(19) **Europäisches Patentamt**
**European Patent Office**
**Office européen des brevets**

(11) **EP 3 502 703 A1**

(12) **EUROPEAN PATENT APPLICATION**

(43) Date of publication:
**26.06.2019 Bulletin 2019/26**

(51) Int Cl.:
***G01N 33/68*** (2006.01)

(21) Application number: **17209996.2**

(22) Date of filing: **22.12.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **metanomics Health GmbH**
**10589 Berlin (DE)**

(72) Inventors:
- **SCHULZE-PELLENGAHR, Silke**
  **10589 Berlin (DE)**
- **WAGNER-GOLBS, Antje**
  **10589 Berlin (DE)**
- **REIN, Dietrich**
  **68623 Lampertheim (DE)**

- **SCHATZ, Philipp**
  **10435 Berlin (DE)**
- **CHRISTIANSEN, Nicole**
  **10589 Berlin (DE)**
- **BERG, Thomas**
  **04103 Leipzig (DE)**
- **HAMPE, Jochen**
  **01307 Dresden (DE)**
- **DATZ, Christian**
  **5110 Oberndorf (AT)**
- **STICKEL, Felix**
  **3013 Bern (CH)**
- **LERCH, Markus**
  **17475 Greifswald (DE)**

(74) Representative: **Herzog, Fiesser & Partner**
**Patentanwälte PartG mbB**
**Dudenstrasse 46**
**68167 Mannheim (DE)**

(54) **METHOD FOR THE ASSESSMENT OF NAFLD**

(57) The present invention relates to a method for assessing non-alcoholic fatty liver disease (NAFLD) comprising determining in a sample from a subject suffering from NAFLD at least the amounts of two metabolite biomarkers, wherein said two metabolite biomarkers are: (i) at least one eicosatrienoic acid metabolite biomarker and (ii) at least one androgen metabolite biomarker and/or at least one bile acid metabolite biomarker. Further, the present invention relates to the vitro use of the two metabolite biomarkers for assessing non-alcoholic fatty liver disease (NAFLD).

**EP 3 502 703 A1**

Printed by Jouve, 75001 PARIS (FR)

**Description**

FIELD OF THE INVENTION

The present invention relates to a method for assessing non-alcoholic fatty liver disease (NAFLD) comprising determining in a sample from a subject suffering from NAFLD at least the amounts of two metabolite biomarkers, wherein said two metabolite biomarkers are: (i) at least one eicosatrienoic acid metabolite biomarker and (ii) at least one androgen metabolite biomarker and/or at least one bile acid metabolite biomarker. Further, the present invention relates to the in vitro use of the two metabolite biomarkers for assessing non-alcoholic fatty liver disease (NAFLD).

BACKGROUND SECTION

Fatty liver disease that develops in the absence of alcohol abuse is recognized increasingly as a major health burden, in particular if an inflammatory component is involved such as in non-alcoholic steatohepatitis (NASH). Estimates based on imaging and biopsy studies suggest that about 20% to 30% of adults in the United States and other Western countries have excess fat accumulation in the liver. About 10% of these individuals, or fully 2% to 3% of adults, are estimated to meet current diagnostic criteria for NASH. Sustained liver injury leads to progressive fibrosis and cirrhosis in about 30% of NASH patients. The diagnostic criteria for NASH continue to evolve and rely on the histologic findings of steatosis, hepatocellular injury (ballooning, Mallory bodies), and the pattern of fibrosis.

Liver biopsy has remained the criterion standard or "gold standard" in the evaluation of the etiology and extent of disease of the liver such as non-alcoholic fatty liver disease (NAFLD) and NASH. Percutaneous liver biopsy is the preferred method to determine NAFLD and to differentiate NASH from NAFLD. Other biopsy methods are typically even more invasive and include transvenous and laparoscopic liver biopsy. The American Gastroenterological Association has published detailed recommendations on how to grade NAFLD comprising NASH into macrovescicular steatosis grades, necroinflammatory activity grades and fibrosis stages (American Gastroenterological Association 2002, Gastroenterology 123: 1705-25; Brunt 1999, Am J Gastroenterol. 94: 2467-74, Brunt 2010, Nat Rev Gastroenterol Hepatol. 7:195-203).

Although liver biopsies are generally regarded as safe, they bare risks that are potentially lethal. Almost two thirds of complications of liver biopsy occur within two hours. Approximately 2% of patients undergoing liver biopsy require hospitalization for the management of an adverse event. Significant bleeding after a liver biopsy occurs in approximately 1% of patients who are biopsied. If bleeding persists, a blood transfusion may be needed. Surgery or angiography, where the bleeding site is identified and treated, may be required if the bleeding is severe or does not stop on its own. Intraperitoneal hemorrhage is the most serious consequence of bleeding. Fatal complications have been reported in up to 0.04% of biopsied patients.

An additional challenge for the liver biopsy is the cost associated with the diagnosis. Moreover, biopsy assessment of the type and severity of hepatic steatosis is associated with a significant time investment for the practitioner and the patient for examination and post-biopsy care.

Metabolite biomarker based methods have been recently reported for the assessment of fatty liver diseases; see WO 2008/021192, WO 2009/059150, WO 2010/018165, WO 2012/000770, WO 2016/081534, S.C. Kalhan et al. (Metabolism Clinical and Experimental 60 (2011) 404-413), Koga et al. (Intern Med 50: 1657-1661, 2011), Tokushige et al. (J Gastroenterol (2013) 48:1392-1400).

A robust minimal invasive test to reliably and efficiently assess NAFLD needed.

Accordingly, the technical problem underlying the present invention could be seen as the provision of means and methods for assessing NAFLD which avoids the aforementioned drawbacks of invasive technologies. The technical problem is solved by the embodiments characterized in the claims and herein below.

SUMMARY OF THE PRESENT INVENTION

The present invention relates to a method for assessing non-alcoholic fatty liver disease (NAFLD) comprising

> (a) determining in a sample from a subject who, preferably, suffers from NAFLD at least the amounts of two metabolite biomarkers,
> (b) comparing

>> (i) the amounts as determined in step (a) to reference amounts for the metabolite biomarkers, or
>> (ii) a score calculated based on the amounts as determined in step (a) to a reference score,

> and

(c) assessing NAFLD based on the results of step (b).

In a preferred embodiment, said at least two metabolite biomarkers are:

- at least one eicosatrienoic acid metabolite biomarker selected from the group consisting of 14,15-Dihydroxyeicosatrienoic acid (C20:cis[5,8,11]3), 11,12-Dihydroxyeicosatrienoic acid (C20:cis[5,8,14]3), and 8,9-Dihydroxyeicosatrienoic acid (C20:cis[5,11,14]3), or the sum of the amounts of 14,15-Dihydroxyeicosatrienoic acid (C20:cis[5,8,11]3), 11,12-Dihydroxyeicosatrienoic acid (C20:cis[5,8,14]3), and 8,9-Dihydroxyeicosatrienoic acid (C20:cis[5,11,14]3), and

- at least one androgen metabolite biomarker and/or at least one bile acid metabolite biomarker:

  wherein the at least one androgen metabolite biomarker is Etiocholanolone sulfate, Androsterone sulfate or Dihydrotestosterone sulfate,
  and/or
  wherein the bile acid metabolite biomarker is selected from Glycocholic acid, Taurochenodeoxycholic acid, Taurodeoxycholic acid, and Taurocholic acid, or the sum of the amounts of Taurochenodeoxycholic acid, Taurodeoxycholic acid, Taurocholic acid, and Glycocholic acid.

In a particularly preferred embodiment, the biomarkers of a panel shown in Table 2a_1, Table 2a_2), Table 2a_3), Table 2b_1), Table 2b_2), Table 2b_3), Table 2c_1), Table 2c_2), Table 2c_3), Table 2d), Table 2e), or Table 2f), and in particular of a panel shown in Table 3 in the Examples section are determined in step a).

In an embodiment of the present invention, the assessment of NAFLD is the differentiation between a mild form and severe form of NAFLD, in particular of NASH.

In an embodiment of the method of the present invention, a mild form of NAFLD is NAFLD with a fibrosis score of lower than 2. E.g., a mild form of NASH is preferably NASH with a fibrosis score of lower than 2.

In an embodiment of the method of the present invention, a severe form of NAFLD is NAFLD with a fibrosis score of equal to or larger than 2. E.g., a severe form of NASH is preferably NASH with a fibrosis score of equal to or larger than 2.

In an embodiment of the present invention, NAFLD is NASH (non-alcoholic steatohepatitis) or NAFL (non-alcoholic fatty liver).

In an embodiment of the method of the present invention, the sample is blood, serum or plasma sample.

In an embodiment of the method of the present invention, the subject is a human subject.

In an embodiment of the method of the present invention, at least the amounts of Etiocholanolone sulfate, Glycocholic acid, and 14,15-Dihydroxyeicosatrienoic acid (C20:cis[5,8,11]3) are determined in step (a).

In another embodiment of the method of the present invention, at least the amounts of Androsterone sulfate, Glycocholic acid, and 14,15-Dihydroxyeicosatrienoic acid (C20:cis[5,8,11]3) are determined in step (a).

In an even further embodiment of the method of the present invention, at least the amounts of Dihydrotestosterone sulfate, Glycocholic acid, and 14,15-Dihydroxyeicosatrienoic acid (C20:cis[5,8,11]3) are determined in step (a).

In an embodiment of the method of the present invention, the amounts of the metabolite biomarkers are determined by mass spectrometry.

In an embodiment of the method of the present invention, the amount of at least one further biomarker selected from the group consisting of Tyrosine, Sphingomyelin (d18:2,C18:0), Lysine, Cystine, Creatinine, Cholesterylester C20:4, Glucose, Sphingomyelin (d18:2,C20:0), Creatine, Phenylalanine, Asparagine, Arginine, Glutamine, Tryptophan, Urea, Histidine, Ethanolamine plasmalogen (C39:5), Choline plasmalogen (C36:5), Phosphatidylcholine (C18:0,C20:4), total cholesterol, Ethanolamine plasmalogen (C39:4), Choline plasmalogen (C36:4), Total Cholesterol, Sphingomyelin (d18:1,C18:0), Cholesterylester C15:0, Choline plasmalogen (C18-vinyl,C20:4), Testosterone, Androstenedione, Uridine, C-reactive protein, alkaline phosphatase, leukocytes, Dehydroepiandrosterone sulfate, and Testosterone-17-sulfate is determined. Also, the at least one further marker may be any additional marker listed in Tables 1 a, 1b and 1 c in the Examples section. E.g., the biomarker may be Urea; Leucocyte; Cholesterylester C18:0; Cholesterylester C20:3; Cholesterylester C20:5; Cholesterylester C22:4; Cholesterylester C22:6; Cholesterylester C18:1; Cholesterylester C18:2; Cholesterylester C20:4; Glucose; Creatinine; Aspartate Aminotransferase (AST); Gamma Glutamyltransferase (GGT); FFA_Elaidic acid (C18:trans[9]1); FFA_Arachidonic acid (C20:cis[5,8,11,14]4); FFA_Docosahexaenoic acid (C22:cis[4,7,10,13,16,19]6); Sphingomyelin (d18:1,C14:0); Sphingomyelin (d18:1,C18:0); Sphingomyelin (d18:1,C18:1); Sphingomyelin (d18:1,C19:0); Sphingomyelin (d18:1,C20:0); Sphingomyelin (d18:1,C20:1); Sphingomyelin (d18:1,C21:0); Sphingomyelin (d18:1,C22:0); Sphingomyelin (d18:1,C23:0); Sphingomyelin (d18:1,C24:0); Sphingomyelin (d18:1,C24:1); Sphingomyelin (d18:2,C14:0); Sphingomyelin (d18:2,C18:0); Sphingomyelin (d18:2,C18:1); Sphingomyelin (d18:2,C20:0); Sphingomyelin (d18:2,C22:0); Sphingomyelin (d18:2,C24:0); Sphingomyelin (d16:1,C18:0);

Sphingomyelin (d17:1,C18:0); Sphingomyelin (d17:1,C20:0); Sphingomyelin (d18:1,C18:2); Sphingomyelin (d18:1,C22:2); Sphingomyelin (d18:2,C17:0); Sphingomyelin (d19:1,C18:0); Testosterone; 18-Hydroxycorticosterone; Ceramide (d18:0,C18:0); Ceramide (d18:1,C18:0); Ceramide (d18:1,C19:0); Ceramide (d18:1,C20:0); Ceramide (d18:1,C21:0); Ceramide (d18:1,C22:1); Ceramide (d18:2,C18:0); Ceramide (d18:2,C20:0); Ceramide (d16:1,C18:0); Ceramide (d17:1,C18:0); Ceramide (d17:1,C20:0); Ceramide (d18:1,C22:0); Creatinine; Creatine; Linoleic acid (C18:cis[9,12]2); gamma-Linolenic acid (C18:cis[6,9,12]3); Arachidonic acid (C20:cis[5,8,11,14]4); Docosapentaenoic acid (C22:cis[4,7,10,13,16]5); conjugated 13-Hydroxylinoleic acid (C18:cis[9]trans[11]2); 14,15-Dihydroxyeicosatrienoic acid (C20:cis[5,8,11]3); 11,12-Dihydroxyeicosatrienoic acid (C20:cis[5,8,14]3); 8,9-Dihydroxyeicosatrienoic acid (C20:cis[5,11,14]3); Glycerol, lipid fraction; Arachidonic acid (C20:cis[5,8,11,14]4); Docosahexaenoic acid (C22:cis[4,7,10,13,16,19]6); Cholesterol, total; Lignoceric acid (C24:0); Eicosanoic acid (C20:0); Tricosanoic acid (C23:0); 6-Aminohexanoic acid; Behenic acid (C22:0); Nervonic acid (C24:cis[15]1); Urea; gamma-Linolenic acid (C18:cis[6,9,12]3); dihomo-gamma-Linolenic acid (C20:cis[8,11,14]3); 3-O-Methylsphingosine (d18:1); threo-Sphingosine (d18:1) ; 5-O-Methylsphingosine (d18:1) ; Sphingolipids; erythro-Sphingosine (d18:1) ; Cholestenol No 02; erythro-Dihydrosphingosine (d16:0); erythro-Sphingosine-1-phosphate (d18:1); 1-Hydroxy-2-amino-(cis,trans)-3,5-octadecadiene (from sphingolipids); 5-O-Methylsphingosine (d16:1); Cysteine ; Lysine; Tryptophan; Erythrol; Aspartate; Phenylalanine; Tyrosine; 1,5-Anhydrosorbitol; scyllo-Inositol; Erythronic acid; PE_Docosatetraenoic acid (C22:cis[7,10,13,16]4); Dehydroepiandrosterone sulfate ; Testosterone-17-sulfate; Hippuric acid; Lysophosphatidylcholine (C18:1); Phosphatidylcholine (C16:0,C22:6) ; Phosphatidylcholine (C18:2,C20:4); Ethanolamine plasmalogen (C39:5) ; Choline plasmalogen (C36:5); Phosphatidylcholine (C16:O,C16:0); Phosphatidylcholine (C18:0,C20:4); DAG (C18:1,C18:2); TAG (C18:2,C18:2) (Triacylglyceride); TAG (C16:0,C18:2) (Triacylglyceride); TAG (C16:O,C18:1,C18:2) (Triacylglyceride); TAG (C18:1,C18:2) (Triacylglyceride); TAG (C18:2,C18:3) (Triacylglyceride); Phosphatidylcholine (C16:0,C20:5); Phosphatidylcholine (C18:0,C18:1); Phosphatidylcholine (C18:0,C20:3) ; Phosphatidylcholine (C20:1,C18:2); Phosphatidylcholine (C20:2,C18:1)); TAG (C16:0,C18:1,C18:3) (Triacylglyceride); TAG (C16:0,C18:2,C18:2) (Triacylglyceride); TAG (C16:1,C18:1,C18:2) (Triacylglyceride); TAG (C18:1,C18:2,C18:3) (Triacylglyceride); TAG (C16:0,C18:1,C20:5) (Triacylglyceride); TAG (C16:0,C18:2,C20:4) (Triacylglyceride); PC_trans-Vaccenic acid (C18:trans[11]1); PC_Oleic acid (C18:cis[9]1); PC_Eicoasenoic acid (C20:cis[11]1); PC_Arachidonic acid (C20:cis[5,8,11,14]4); and LPC_Oleic acid (C18:cis[9]1).

In a particularly preferred embodiment, the biomarkers of a panel shown in Table 2a_1, Table 2a_2), Table 2a_3), Table 2b_1), Table 2b_2), Table 2b_3), Table 2c_1), Table 2c_2), Table 2c_3), Table 2d), Table 2e), or Table 2f), and in particular of a panel shown in Table 3 in the Examples section are determined in step (a) of the method.

The present invention further relates to the in vitro use of the at least two metabolite biomarkers as set forth in connection with the method of the present invention for assessing non-alcoholic fatty liver disease (NAFLD).

DETAILED DESCRIPTION OF THE PRESENT INVENTION - DEFINITIONS

As set forth above, the present invention relates to method for assessing non-alcoholic fatty liver disease (NAFLD) comprising

(a) determining in a sample from a subject suffering from NAFLD at least the amounts of two metabolite biomarkers,
(b) comparing

(i) the amounts as determined in step (a) to reference amounts for the metabolite biomarkers, or
(ii) a score calculated based on the amounts as determined in step (a) to a reference score, and

(c) assessing NAFLD based on the results of step (b).

In a preferred embodiment said two metabolite biomarkers are at least one eicosatrienoic acid metabolite biomarker and at least one further metabolite biomarker, wherein said at least one further metabolite biomarker is at least one androgen metabolite biomarker and/or at least one bile acid metabolite biomarker

Thus, in an embodiment, the amounts of at least one eicosatrienoic acid metabolite biomarker and of at least one androgen metabolite biomarker are determined in step a) of the method of the present invention. In another embodiment of the method of the present invention, the amounts of at least one eicosatrienoic acid metabolite biomarker and of at least one bile acid metabolite biomarker are determined in step a) of the method. In another embodiment of the method of the present invention, the amounts of at least one eicosatrienoic acid metabolite biomarker, of at least one androgen metabolite biomarker, and of at least one bile acid metabolite biomarker are determined in step a) of the method.

In a preferred embodiment of the method of the present invention, the at least one eicosatrienoic acid metabolite biomarker is selected from the group consisting of 14,15-Dihydroxyeicosatrienoic acid (C20:cis[5,8,11]3), 11,12-Dihydroxyeicosatrienoic acid (C20:cis[5,8,14]3), and 8,9-Dihydroxyeicosatrienoic acid (C20:cis[5,11,14]3), or the sum of the amounts of 14,15-Dihydroxyeicosatrienoic acid (C20:cis[5,8,11]3), 11,12-Dihydroxyeicosatrienoic acid (C20:cis[5,8,14]3), and 8,9-Dihydroxyeicosatrienoic acid (C20:cis[5,11,14]3). The aforementioned sum is herein also referred to as "sum.eicosatienoic".

In a preferred embodiment of the method of the present invention, the at least one androgen metabolite biomarker is Etiocholanolone sulfate. In another preferred embodiment of the method of the present invention, the at least one androgen metabolite biomarker is Androsterone sulfate. In another preferred embodiment of the method of the present invention, the at least one androgen metabolite biomarker is Dihydrotestosterone sulfate.

In a preferred embodiment of the method of the present invention, the bile acid metabolite biomarker is selected from Glycocholic acid, Taurochenodeoxycholic acid, Taurodeoxycholic acid, and Taurocholic acid, or the sum (sum.bile) of the amounts of Taurochenodeoxycholic acid, Taurodeoxycholic acid, Taurocholic acid, and Glycocholic acid.

Table 2a_1, Table 2a_2), Table 2a_3), Table 2b_1), Table 2b_2), Table 2b_3), Table 2c_1), Table 2c_2), Table 2c_3), Table 2d), Table 2e), Table 2f), and in particular Table 3 list different panels for the assessment of NAFLD, in particular of NASH. In a particularly preferred embodiment of the method of the present invention, the biomarkers of the individual panels of these tables are determined in step a).

The most preferred marker combinations are summarized in Table 3 in the Examples section. In a particularly preferred embodiment, the amount of the markers of panel AEB_1_2 are determined in step a), see Table 3. Thus, the amounts of Etiocholanolone sulfate; Glycocholic acid; 14,15-Dihydroxyeicosatrienoic acid (C20:cis[5,8,11]3) are determined.

In another embodiment, the amount of the markers of panel AEB_2_2 are determined. In another embodiment, the amount of the markers of panel AEB_3_2; are determined. In another embodiment, the amount of the markers of panel AEB_4_2 are determined. In another embodiment, the amount of the markers of panel AEB_5_2 are determined. In another embodiment, the amount of the markers of panel AEB_6_2 are determined. In another embodiment, the amount of the markers of panel AEB_7_2 are determined. Further, it is envisaged to determine the amounts of the biomarkers of panel AEB_8_2; AEB_9_2; AEB_10_2; AEB_11_2; AEB_ 12_2; AEB_13_2; AEB_14_2; AEA_17_2; AEA_18_2; AEA_19_2; AEA_20_2; AEL_40_2; AEL_41_2; AEL_42_2; AEL_44_2; AEC_59_2; AECL 62_2; or AB_84_2 (again, see Table 3 for all panels).

The method as referred to in accordance with the present invention includes a method which essentially consists of the aforementioned steps or a method which includes further steps. However, it is to be understood that the method, in a preferred embodiment, is a method carried out ex vivo, i.e. not practised on the human or animal body. Thus, it is preferably an in vitro method. The method, preferably, can be assisted by automation.

In accordance with the method of the present invention, non-alcoholic fatty liver disease (NAFLD) shall be assessed. As will be understood by those skilled in the art, the assessment made in connection with present invention is usually not intended to be correct for 100% of the subjects to be tested. In an embodiment, the term requires that a correct assessment (such as the diagnosis or differentiation as referred to herein) can be made for a statistically significant portion of subjects. Whether a portion is statistically significant can be determined without further ado by the person skilled in the art using various well known statistic evaluation tools, e.g., determination of confidence intervals, p-value determination, Student's t-test, Mann-Whitney test etc. Details are found in Dowdy and Wearden, Statistics for Research, John Wiley & Sons, New York 1983. Preferred confidence intervals are at least 90%, at least 95%, at least 97%, at least 98%, or at least 99%. The p-values are, preferably, 0.4, 0.1, 0.05, 0.01, 0.005, or 0.0001.

The term "non-alcoholic fatty liver disease" (abbreviated NAFLD) is well understood by the skilled person. The term preferably refers to fatty liver disease which occurs when fat is deposited in the liver due to causes other than consumption of alcohol. Accordingly, the term refers to an impairment of the liver which is the result of a surplus of triacylglyceride that accumulate in the liver and form large vacuoles. The symptoms accompanying fatty liver disease are well known from standard text books of medicine such as Stedman's or Pschyrembel. Fatty liver disease may result from diabetes mellitus, nutritional defects and wrong diets, toxicity of drugs or genetic predisposition. In accordance with the present invention, the accumulation of fat in the liver shall be not the consequence of alcohol abuse, i.e. the fatty liver disease shall be non-alcoholic fatty liver disease. A subgroup of people with NAFLD may have a more serious condition termed non-alcoholic steatohepatitis (NASH).

The term "NAFLD" preferably includes all conditions reflecting a form of non-alcohol fatty liver disease, but in particular, NASH and NAFL (Non-alcoholic fatty liver).

In NASH, fat accumulation is associated with liver cell inflammation and different degrees of scarring. Cirrhosis occurs when the liver sustains substantial damage, and the liver cells are gradually replaced by scar tissue which results in the inability of the liver to work properly. Patients suffering from NASH frequently have obesity, type 2 diabetes mellitus, dyslipidemia, and/or the metabolic syndrome. Symptoms of NASH are fatigue, malaise, or right upper quadrant abdominal discomfort. Hepatomegaly develops in a larger number of patients. Splenomegaly may develop if advanced hepatic fibrosis is present. Most NASH patients are, however, asymptomatic.

Nonalcoholic fatty liver (NAFL) is a generally benign condition. In fatty liver, the liver functions normally and looks normal under the microscope, except for accumulations of fat within cells. NAFL is often detected when imaging tests of the abdomen are obtained for other reasons (such as an ultrasound being done to look for gallstones).

As set forth above, non-alcoholic fatty liver disease (NAFLD), in particular NASH or NAFL, shall be assessed by carrying out the method of the present invention.

In a preferred embodiment of the present invention, the assessment of NAFLD is the differentiation between a mild form and severe form of NAFLD. Accordingly, it is differentiated between a mild and severe form of NAFLD. In particular, it is differentiated between a mild and severe form of NASH. Preferably, the subject to be tested in connection with the aforementioned differentiation suffers from NAFLD, in particular from NASH.

A mild form of NAFLD is, preferably, NAFLD, in particular NASH, with a fibrosis score of lower than 2. A severe form of NAFLD is, preferably, NAFLD, in particular NASH, with a fibrosis score of equal to or larger than 2. The fibrosis score is the so called NAFLD fibrosis score. It is a non-invasive system that identifies liver fibrosis in patients with NAFLD. In a preferred embodiment, the fibrosis score is determined as disclosed in Treeprasertsuk S et al, NAFLD fibrosis score: a prognostic predictor for mortality and liver complications among NAFLD patients. World J Gastroenterol. 2013 Feb 28;19(8):1219-29. doi: 10.3748/wjg.v19.i8.1219. In another preferred embodiment, the fibrosis score is determined as disclosed in Kleiner et al., 2005, HEPATOLOGY, Vol. 41, No. 6, 2005.

Thus, the present invention also allows a staging of fibrosis. Accordingly, the assessment of NAFLD is the staging of fibrosis, preferably in a subject who suffers from NAFLD, in particular NASH. In order to allow for a staging, one more reference amounts or reference scores might be applied.

In another preferred embodiment of the present invention, the assessment of NAFLD is the diagnosis of NAFLD. Accordingly, it is diagnosed, whether a subject suffers from NAFLD, or not. In particular, it is diagnosed, whether a subject suffers from NASH, or not. Preferably, the subject to be tested in connection with the method for diagnosing of NAFLD is a subject who is suspected to suffer from NAFLD. Preferably, a subject is suspected to suffer from NASH is a subject who shows some or all of the symptoms associated with NASH. Also preferably, a subject is suspected to suffer from NAFLD, in particular NASH, if the subject has at least one risk factor for NAFLD, in particular NASH, in particular obesity, type 2 diabetes mellitus, or dyslipidemia, and/or if the subject has an elevated aminotransferase level. However, it is also contemplated that the subject already has been diagnosed previously to suffer from NAFLD, in particular NASH, and that the previous diagnosis is confirmed by carrying out the method of the present invention.

In another preferred embodiment of the present invention, the assessment of NAFLD is the prediction of the risk of a subject to suffer from NAFLD, in particular from NASH. Accordingly, it is predicted whether a subject is at risk and/or not as risk of suffering from NAFLD. The subject to be tested in connection with the prediction does not suffer from NAFLD. In particular, the subject does not suffer from NASH. However, the subject may have at least one of the above risk factors.

In another preferred embodiment of the method of the present invention, the assessment of NAFLD is the diagnosis of fibrosis. Thus, it is diagnosed whether the subject to be tested suffers from fibrosis, or not. Preferably, the subject is a subject who is suspected to suffer from fibrosis. More preferably, the subject to be tested suffers from NAFLD.

In another preferred embodiment of the method of the present invention, the assessment of NAFLD is the monitoring of a therapy of NAFLD. Thus, it is monitored whether the subject responds to a therapy, or not. Preferably, the subject is a subject who is suspected to suffer from NAFLD. A therapy of NAFLD as used in accordance with the present invention, preferably, comprises surgery, drug treatment or life style recommendations. Drug-based therapies, preferably, include the administration of one or more drugs selected from Statins, Incretin analogues, Metformin, Rimonabant, Thiazolidinediones, Orlistat (see Maryam R. Kashi, Dawn M. Torres, and Stephen A. Harrison: Current and Emerging Therapies in Nonalcoholic Fatty Liver Disease: Therapeutic Modalities; Semin Liver Dis. 2008; 28 (4): 396-406).

It is known in the art that biomarkers could be increased or decreased in various diseases and disorders. This is taken into account by the skilled person. Accordingly, the "assessment of NAFLD" is understood as an aid in the assessment of NAFLD, and thus as an aid in diagnosing NAFLD, an aid in the diagnosis of fibrosis, an aid in monitoring a therapy of NAFLD, an aid in differentiating between a mild or severe form of NAFLD, an aid in the prediction of a risk of a subject to suffer from NAFLD.

Accordingly, the present invention relates to a method for aiding in assessing non-alcoholic fatty liver disease (NAFLD) comprising steps a) and b) as set forth above, and step (c) of aiding in the assessment of NAFLD based on the results of step (b).

The method of the present invention envisages the determination of at least the amounts of at least two biomarkers, i.e. of a combination of at least two biomarkers. Preferred combinations are described elsewhere herein. Preferably, the at least one eicosatrienoic acid biomarker, the at least one androgen metabolite biomarker and the at least one bile acid biomarker are metabolite biomarkers. The term "metabolite biomarker" as used herein refers to a molecular species which serves as an indicator for a disease or effect as referred to in this specification. Said molecular species can be a metabolite itself which is found in a sample of a subject. Moreover, in certain cases the biomarker may also be a molecular species which is derived from said metabolite. In such a case, the actual metabolite will be chemically modified in the sample or during the determination process and, as a result of said modification, a chemically different molecular species, i.e. the analyte, will be the determined molecular species. It is to be understood that in such a case, the analyte represents the actual metabolite and has the same potential as an indicator for the respective medical condition. Preferably, the metabolite biomarker in accordance with the present invention is a small molecule compound.

In the method according to the present invention at least the amounts of at least two metabolite biomarkers are determined. The term "at least two biomarkers" as used herein, means two or more than two. Accordingly, the amounts of two, three, four, five, six, seven, eight, nine, ten or even more biomarkers may be determined (and compared to a reference). Preferably, the amounts of two to ten biomarkers, in particular metabolite biomarkers, are determined (and compared to a reference). In an embodiment, the biomarkers of a panel as shown in the examples section are determined. The panels comprise two or three biomarkers.

Preferably, the at least two biomarkers to be determined are lipid metabolite biomarkers. Thus, the biomarkers are preferably metabolite biomarkers (i.e. lipid biomarkers). The at least two metabolite biomarkers to be determined, preferably, belong to at least two of the three following compound classes

- eicosatrienoic acid metabolite biomarkers
- androgen metabolite biomarkers
- bile acid metabolite biomarker

In an embodiment, the amount of at least one eicosatrienoic acid metabolite biomarker and of at least one androgen metabolite biomarker is determined. In another embodiment, the amount of at least one eicosatrienoic acid metabolite biomarker and of at least one bile acid metabolite biomarker is determined. In another embodiment, the amount of at least one eicosatrienoic acid metabolite biomarker, of at least one androgen metabolite biomarker, and of at least one bile acid metabolite biomarker is determined. Preferred biomarker combinations to be determined in accordance with the present invention are disclosed in column 2 (panel composition long) of Table 2a_1, Table 2a_2), Table 2a_3), Table 2b_1), Table 2b_2), Table 2b_3), Table 2c_1), Table 2c_2), Table 2c_3), Table 2d), Table 2e), and Table 2f) in the examples section. The name of the panels is given in column 1 of these Tables. Further preferred panels are given in Table 3.

As described elsewhere, it is further envisaged to determine the amount of at least one further biomarker selected from the group consisting of Tyrosine, Sphingomyelin (d18:2,C18:0), Lysine, Cystine, Creatinine, Cholesterylester C20:4, Glucose, Sphingomyelin (d18:2,C20:0), Creatine, Phenylalanine, Asparagine, Arginine, Glutamine, Tryptophan, Urea, Histidine, Ethanolamine plasmalogen (C39:5), Choline plasmalogen (C36:5), Phosphatidylcholine (C18:0,C20:4), total cholesterol, Ethanolamine plasmalogen (C39:4), Choline plasmalogen (C36:4), Total Cholesterol, Sphingomyelin (d18:1,C18:0), Cholesterylester C15:0, Choline plasmalogen (C18-vinyl,C20:4), Testosterone, Androstenedione, Uridine, C-reactive protein, alkaline phosphatase, leukocytes, Dehydroepiandrosterone sulfate, and Testosterone-17-sulfate. Also, the at least one further marker may be any additional marker listed in Tables 1 a, 1b and 1c in the Examples section.

In addition, to the at least two biomarkers as referred to herein, and optionally the at least further biomarker as referred to in the previous paragraph, at least one peptide or polypeptide biomarker can be determined. Said peptide or polypeptide biomarker shall be a biomarker which is capable of assessing NAFLD, in particular NASH and/or fibrosis. Such peptide or polypeptide biomarkers are well known in the art and are described elsewhere herein.

Eicosatrienoic acid metabolite biomarker

The eicosatrienoic acid metabolite biomarker is preferably selected from the group consisting of

- 14,15-Dihydroxyeicosatrienoic acid (C20:cis[5,8,11]3),
- 11,12-Dihydroxyeicosatrienoic acid (C20:cis[5,8,14]3),
- 8,9-Dihydroxyeicosatrienoic acid (C20:cis[5,11,14]3),
- the sum of the amounts of 14,15-Dihydroxyeicosatrienoic acid (C20:cis[5,8,11]3), 11,12-Dihydroxyeicosatrienoic acid (C20:cis[5,8,14]3), and 8,9-Dihydroxyeicosatrienoic acid (C20:cis[5,11,14]3).

Thus, in an embodiment, the eicosatrienoic acid metabolite biomarker is 14,15-Dihydroxyeicosatrienoic acid (C20:cis[5,8,11]3). In another embodiment, the eicosatrienoic acid metabolite biomarker is 11,12-Dihydroxyeicosatrienoic acid (C20:cis[5,8,14]3). In another embodiment, the eicosatrienoic acid metabolite biomarker is 8,9-Dihydroxyeicosatrienoic acid (C20:cis[5,11,14]3). Further, it is envisaged to determine two or three of the aforementioned biomarkers. The most preferred eicosatrienoic acid metabolite biomarker is 14,15-Dihydroxyeicosatrienoic acid (C20:cis[5,8,11]3).

In an embodiment of the method of the present invention, the sum of the amounts of the aforementioned three biomarkers is determined in step a) of the present invention, i.e. the sum of the amounts 14,15-Dihydroxyeicosatrienoic acid (C20:cis[5,8,11]3), 11,12-Dihydroxyeicosatrienoic acid (C20:cis[5,8,14]3), and 8,9-Dihydroxyeicosatrienoic acid (C20:cis[5,11,14]3)

(C20:cis[5,11,14]3. Accordingly, a combination of the three biomarkers is determined (referred to as "sum.eicosatienoic" in the Examples).

Bile acid metabolite biomarker

The bile acid metabolite biomarker is preferably selected from the group consisting of

- Glycocholic acid,
- Taurochenodeoxycholic acid,
- Taurodeoxycholic acid, and
- Taurocholic acid, and
- the sum of the amounts of Taurochenodeoxycholic acid, Taurodeoxycholic acid, Taurocholic acid, and Glycocholic acid

Thus, in an embodiment, the bile acid metabolite biomarker is Glycocholic acid. In another embodiment, the bile acid metabolite biomarker is Taurochenodeoxycholic acid. In another embodiment, the bile acid metabolite biomarker is Taurodeoxycholic acid. In another embodiment, the bile acid metabolite biomarker is Taurocholic acid. Further, it is envisaged to determine two, three are all four of the aforementioned biomarkers.

The most preferred bile acid metabolite biomarker is Glycocholic acid.

In an embodiment of the method of the present invention, the sum of the amounts of the aforementioned four biomarkers is determined in step a) of the present invention, i.e. the sum of the amounts of Taurochenodeoxycholic acid, Taurodeoxycholic acid, Taurocholic acid, and Glycocholic acid. Accordingly, a combination of the four biomarkers is determined (referred to as "sum.bile" in the Examples).

Androgen metabolite biomarker

The androgen metabolite biomarker is preferably selected from the group consisting of

- Androsterone sulfate
- Etiocholanolone sulfate
- Dihydrotestosterone sulfate

The term "subject" as used herein relates to animals and, preferably, to mammals. More preferably, the subject is a primate and, most preferably, a human. The subject may be a female or a male subject. The subject to be tested, preferably, is suspected to suffer from non-alcoholic fatty liver disease (NAFLD). More preferably, the subject is known to suffer from non-alcoholic fatty liver disease (NAFLD). Accordingly, the subject shall have been diagnosed to suffer from NAFLD.

Further, it is envisaged that the subject does not have a history of heavy alcohol use.

Moreover, it is envisaged that the test subject does not suffer from liver cancer.

In an embodiment, the subject has a body mass index (BMI) of more than 25 kg/m$^2$, in particular of more than 28.0 kg/m$^2$.

The term "sample" as used herein refers to samples from body fluids, preferably, blood, plasma, serum, saliva or urine, or samples derived, e.g., by biopsy, from cells, tissues or organs, in particular from the liver. More preferably, the sample is a blood, plasma or serum sample, most preferably, a plasma sample. Biological samples can be derived from a subject as specified elsewhere herein. Techniques for obtaining the aforementioned different types of biological samples are well known in the art. For example, blood samples may be obtained by blood taking while tissue or organ samples are to be obtained, e.g., by biopsy.

In a preferred embodiment of the present invention, the sample is a blood (i.e. whole blood), serum or plasma sample. Serum is the liquid fraction of whole blood that is obtained after the blood is allowed to clot. For obtaining the serum, the clot is removed by centrifugation and the supernatant is collected. Plasma is the acellular fluid portion of blood. For obtaining a plasma sample, whole blood is collected in anticoagulant-treated tubes (e.g. citrate-treated or EDTA-treated tubes). Cells are removed from the sample by centrifugation and the supernatant (i.e. the plasma sample) is obtained.

In a preferred embodiment of the present invention, the sample is a fasting sample, in particular a fasting blood, serum or plasma sample. Accordingly, the sample shall have been obtained from a fasting subject. A fasting subject, in particular, is a subject who refrained from food and beverages, except for water, prior to obtaining the sample to be tested. Preferably, a fasting subject refrained from food and beverages, except for water, for at least eight hours prior to obtaining the sample to be tested. More preferably, the sample has been obtained from the subject after an overnight fast.

The aforementioned samples are, preferably, pre-treated before they are used for the method of the present invention. Preferably, the sample is pre-treated as described in the examples section. As described in more detail below, said pre-treatment may include treatments required to release or separate the compounds or to remove excessive material or waste. Suitable techniques comprise centrifugation, extraction, fractioning, ultrafiltration, protein precipitation followed by filtration and purification and/or enrichment of compounds. Moreover, other pre-treatments are carried out in order to provide the compounds in a form or concentration suitable for compound analysis. For example, if gas-chromatography coupled mass spectrometry is used in the method of the present invention, it will be required to derivatize the compounds prior to the said gas chromatography. Suitable and necessary pre-treatments depend on the means used for carrying out the method of the invention and are well known to the person skilled in the art. Pre-treated samples as described before are also comprised by the term "sample" as used in accordance with the present invention.

As set forth herein below in more detail, the determination of the amount of a biomarker as referred to herein, preferably includes a separation step, i.e. a step in which compounds comprised by the sample are separated. Preferably, the separation of the compounds is carried by chromatography, in particular by liquid chromatography (LC) or high performance liquid chromatography (HPLC). Preferably, the pre-treatment of the sample should allow for a subsequent separation of compounds, in particular the metabolite biomarkers as referred to above, comprised by the sample. Molecules of interest, in particular the biomarkers as referred to above may be extracted in an extraction step which comprises mixing of the sample with a suitable extraction solvent. The extraction solvent shall be capable of precipitating the proteins in a sample, thereby facilitating the, preferably, centrifugation-based, removal of protein contaminants which otherwise would interfere with the subsequent analysis of the biomarkers as referred above. Preferably, the pretreatment of the sample comprises an extraction step with a suitable extraction solvent. This extraction step additionally results in the precipitation of proteins comprised by the sample. Subsequently, the proteins comprised by the sample are removed by centrifugation.

The term "determining the amount", in particular of the metabolite biomarkers, as used herein refers to determining at least one characteristic feature of a biomarker to be determined by the method of the present invention in the sample. Characteristic features in accordance with the present invention are features which characterize the physical and/or chemical properties including biochemical properties of a biomarker. Such properties include, e.g., molecular weight, viscosity, density, electrical charge, spin, optical activity, colour, fluorescence, chemiluminescence, elementary composition, chemical structure, capability to react with other compounds, capability to elicit a response in a biological read out system (e.g., induction of a reporter gene) and the like. Values for said properties may serve as characteristic features and can be determined by techniques well known in the art. Moreover, the characteristic feature may be any feature which is derived from the values of the physical and/or chemical properties of a biomarker by standard operations, e.g., mathematical calculations such as multiplication, division or logarithmic calculus. Most preferably, the at least one characteristic feature allows the determination and/or chemical identification of the said at least one biomarker and its amount. Accordingly, the characteristic value, preferably, also comprises information relating to the abundance of the biomarker from which the characteristic value is derived. For example, a characteristic value of a biomarker may be a peak in a mass spectrum. Such a peak contains characteristic information of the biomarker, i.e. the m/z information, as well as an intensity value being related to the abundance of the said biomarker (i.e. its amount) in the sample.

As discussed before, each biomarker comprised by a sample may be, preferably, determined in accordance with the present invention quantitatively or semi-quantitatively. For quantitative determination, either the absolute or precise amount of the biomarker will be determined or the relative amount of the biomarker will be determined based on the value determined for the characteristic feature(s) referred to herein above. The relative amount may be determined in a case were the precise amount of a biomarker can or shall not be determined. In said case, it can be determined whether the amount in which the biomarker is present, is enlarged or diminished with respect to a second sample comprising said biomarker in a second amount. In a preferred embodiment said second sample comprising said biomarker shall be a calculated reference as specified elsewhere herein. Quantitatively analysing a biomarker, thus, also includes what is sometimes referred to as semi-quantitative analysis of a biomarker.

Thus, the determination of the amount of a biomarker as referred to herein is preferably done by a compound separation step and a subsequent mass spectrometry step. Thus, determining as used in the method of the present invention, preferably, includes using a compound separation step prior to the analysis step. Preferably, said compound separation step yields a time resolved separation of the metabolites, in particular of the at least two biomarkers, comprised by the sample. Suitable techniques for separation to be used preferably in accordance with the present invention, therefore, include all chromatographic separation techniques such as liquid chromatography (LC), high performance liquid chromatography (HPLC), gas chromatography (GC), thin layer chromatography, size exclusion or affinity chromatography. These techniques are well known in the art and can be applied by the person skilled in the art without further ado. Most preferably, LC and/or HPLC are chromatographic techniques to be envisaged by the method of the present invention. Suitable devices for such determination of biomarkers are well known in the art. Preferably, mass spectrometry is used in particular gas chromatography mass spectrometry (GC-MS), liquid chromatography mass spectrometry (LC-MS), direct infusion mass spectrometry or Fourier transform ion-cyclotrone-resonance mass spectrometry (FT-ICR-MS), capillary electrophoresis mass spectrometry (CE-MS), high-performance liquid chromatography coupled mass spectrometry (HPLC-MS), quadrupole mass spectrometry, any sequentially coupled mass spectrometry, such as MS-MS or MS-MS-MS, inductively coupled plasma mass spectrometry (ICP-MS), pyrolysis mass spectrometry (Py-MS), ion mobility mass spectrometry or time of flight mass spectrometry (TOF). More preferably, LC-MS, in particular LC-MS/MS, and most preferably HPLC-MS, in particular HPLC-MS/MS, are used as described in detail below. Accordingly, the determination of the amounts to the at least three biomarkers is preferably carried by HPLC-MS, in particular HPLC-MS/MS (high-performance liquid chromatography tandem mass spectrometry). The techniques described above are disclosed in, e.g., Nissen 1995, Journal of Chromatography A, 703: 37-57, US 4,540,884 or US 5,397,894, the disclosure content of which is hereby incorporated by reference.

As an alternative or in addition to mass spectrometry techniques, the following techniques may be used for compound determination: nuclear magnetic resonance (NMR), magnetic resonance imaging (MRI), Fourier transform infrared analysis (FT-IR), ultraviolet (UV) spectroscopy, refraction index (RI), fluorescent detection, radiochemical detection, electrochemical detection, light scattering (LS), dispersive Raman spectroscopy or flame ionisation detection (FID). These techniques are well known to the person skilled in the art and can be applied without further ado.

The method of the present invention shall be, preferably, assisted by automation. For example, sample processing or pre-treatment can be automated by robotics. Data processing and comparison is, preferably, assisted by suitable computer programs and databases. Automation as described herein before allows using the method of the present invention in high-throughput approaches.

As described above, said determining of the at least two biomarkers can, preferably, comprise mass spectrometry (MS). Thus, a mass spectrometry step is carried out after the separation step (e.g. by LC or HPLC). Mass spectrometry as used herein encompasses all techniques which allow for the determination of the molecular weight (i.e. the mass) or a mass variable corresponding to a compound, i.e. a biomarker, to be determined in accordance with the present invention. Preferably, mass spectrometry as used herein relates to GC-MS, LC-MS, direct infusion mass spectrometry, FT-ICR-MS, CE-MS, HPLC-MS, quadrupole mass spectrometry, any sequentially coupled mass spectrometry such as MS-MS or MS-MS-MS, ICP-MS, Py-MS, TOF or any combined approaches using the aforementioned techniques. How to apply these techniques is well known to the person skilled in the art. Moreover, suitable devices are commercially available. More preferably, mass spectrometry as used herein relates to LC-MS and/or HPLC-MS, i.e. to mass spectrometry being operatively linked to a prior liquid chromatography separation step. Preferably, the mass spectrometry is tandem mass spectrometry (also known as MS/MS). Tandem mass spectrometry, also known as MS/MS involves two or more mass spectrometry step, with a fragmentation occurring in between the stages. In tandem mass spectrometry two mass spectrometers in a series connected by a collision cell. The mass spectrometers are coupled to the chromatographic device. The sample that has been separated by a chromatography is sorted and weighed in the first mass spectrometer, then fragmented by an inert gas in the collision cell, and a piece or pieces sorted and weighed in the second mass spectrometer.

The fragments are sorted and weighed in the second mass spectrometer. Identification by MS/MS is more accurate.

In an embodiment, mass spectrometry as used herein encompasses quadrupole MS. Most preferably, said quadrupole MS is carried out as follows: a) selection of a mass/charge quotient (m/z) of an ion created by ionisation in a first analytical quadrupole of the mass spectrometer, b) fragmentation of the ion selected in step a) by applying an acceleration voltage in an additional subsequent quadrupole which is filled with a collision gas and acts as a collision chamber, c) selection of a mass/charge quotient of an ion created by the fragmentation process in step b) in an additional subsequent quadrupole, whereby steps a) to c) of the method are carried out at least once.

More preferably, said mass spectrometry is liquid chromatography (LC) MS such high performance liquid chromatography (HPLC) MS, in particular HPLC-MS/MS. Liquid chromatography as used herein refers to all techniques which allow for separation of compounds (i.e. metabolites) in liquid or supercritical phase. Liquid chromatography is characterized in that compounds in a mobile phase are passed through the stationary phase. When compounds pass through the stationary phase at different rates they become separated in time since each individual compound has its specific retention time (i.e. the time which is required by the compound to pass through the system). Liquid chromatography as used herein also includes HPLC. Devices for liquid chromatography are commercially available, e.g. from Agilent Technologies, USA. For examples, HPLC can be carried out with commercially available reversed phase separation columns with e.g. C8, C18 or C30 stationary phases. The person skilled in the art is capable to select suitable solvents for the HPLC or any other chromatography method as described herein. The eluate that emerges from the chromatography device shall comprise the biomarkers as referred to above.

A suitable solvent for elution for lipid chromatography can be determined by the skilled person. In an embodiment, the solvents for gradient elution in the HPLC separation consist of a polar solvent and a lipid solvent. Preferably, the polar solvent is a mixture of water and a water miscible solvent with an acid modifier. Examples of suitable organic solvents which are completely miscible with water include the C1-C3-alkanols, tetrahydrofurane, dioxane, C3-C4-ketones such as acetone and acetonitril and mixtures thereof, with methanol being particularly preferred. Additionally the lipid solvent is a mixture of the above mentioned solvents together with hydrophobic solvents from the groups consisting of dichloromethane (DCM), chloroform, tertiary butyl methyl ether (tBME or MTBE), ethyl ethanoate, and isooctane. Examples of acidic modifiers are formic acid or acidic acid. Preferred solvents for gradient elution are disclosed in the Examples section.

Gas chromatography which may be also applied in accordance with the present invention, in principle, operates comparable to liquid chromatography. However, rather than having the compounds (i.e. metabolites) in a liquid mobile phase which is passed through the stationary phase, the compounds will be present in a gaseous volume. The compounds pass the column which may contain solid support materials as stationary phase or the walls of which may serve as or are coated with the stationary phase. Again, each compound has a specific time which is required for passing through the column. Moreover, in the case of gas chromatography it is preferably envisaged that the compounds are derivatized prior to gas chromatography. Suitable techniques for derivatization are well known in the art. Preferably, derivatization in accordance with the present invention relates to methoxymation and trimethylsilylation of, preferably, polar compounds and transmethylation, methoxymation and trimethylsilylation of, preferably, non-polar (i.e. lipophilic) compounds.

For mass spectrometry, the analytes in the sample are ionized in order to generate charged molecules or molecule fragments. Afterwards, the mass-to-charge of the ionized analyte, in particular of the ionized biomarkers, or fragments thereof is measured.

Thus, the mass spectrometry step preferably comprises an ionization step in which the biomarkers to be determined are ionized. Of course, other compounds present in the sample/elulate are ionizied as well. Ionization of the biomarkers can be carried out by any method deemed appropriate, in particular by electron impact ionization, fast atom bombardment, electrospray ionization (ESI), atmospheric pressure chemical ionization (APCI), matrix assisted laser desorption ionization (MALDI).

In an embodiment, the ionization step is carried out by atmospheric pressure chemical ionization (AP-CI). In particular, the ionization step (for mass spectrometry) is carried out by electrospray ionization (ESI). Accordingly, the mass spectrometry is preferably ESI-MS (or if tandem MS is carried out: ESI-MS/MS). Electrospray is a soft ionization method which results in the formation of ions without breaking any chemical bonds.

Electrospray ionization (ESI) is a technique used in mass spectrometry to produce ions using an electrospray in which a high voltage is applied to the sample to create an aerosol. It is especially useful in producing ions from macromolecules because it overcomes the propensity of these molecules to fragment when ionized.

Preferably, the electrospray ionization is positive ion mode electrospray ionization. Thus, the ionization is preferably a protonation (or an adduct formation with positive charged ions such as $NH_4^+$, $Na^+$, or $K^+$). According a suitable cation, preferably, a proton ($H^+$) is added to the biomarkers to be determined (and of course to any compound in the sample, i.e. in the eluate from the chromatography column). Therefore, the determination of the amounts of the at metabolite biomarkers might be the determination of the amount of protonated biomarkers.

The person skilled in the art knows that the ionization step is carried out at the beginning of the mass spectrometry step. If tandem MS is carried out, the ionization, in particular the electrospray ionization, is carried out in the first mass spectrometry step.

The ionization of the biomarkers can be preferably carried out by feeding the liquid eluting from the chromatography column (in particular from the LC or HPLC column) directly to an electrospray. Alternatively the fractions can be collected and are later analyzed in a classical nanoelectrospray-mass spectrometry setup.

As set forth above, the mass spectrometry step is carried out after the separation step, in particular the chromatography step. In an embodiment, the eluate that emerges from the chromatography column (e.g. the LC or HPLC column) may be pre-treated prior to subjecting it to the mass spectrometry step.

In a preferred embodiment of the present invention, the metabolite biomarkers, in particular the at least two biomarkers, are determined together in a single measurement. In particular, it is envisaged to determine the amounts together in a single LC-MS (or LC-MS/MS), HPLC-MS (HPLC-MS/MS) measurement (i.e. run).

In an embodiment, the determination of the amounts of the at least two metabolite biomarkers comprises the step as described in the Examples section. For example, the at least two biomarkers can be determined as described in Examples section.

The term "amount" as used herein preferably encompasses the absolute amount of a biomarker as referred to herein, the relative amount or concentration of the said biomarker as well as any value or parameter which correlates thereto or can be derived therefrom. Such values or parameters comprise intensity signal values from specific physical or chemical properties obtained from the said biomarker. In particular, encompassed shall be values or parameters which are obtained by indirect measurements specified elsewhere in this description. It is to be understood that values correlating to the aforementioned amounts or parameters can also be obtained by standard mathematical operations.

In an embodiment, it is contemplated to determine only the amounts of the at least two metabolite biomarkers as referred to herein, i.e. no further biomarkers are determined (and compared to a reference).

In an alternative embodiment, it envisaged to determine in the step (a) of the method of the present invention, the amount of further biomarkers. In particular, it is envisaged the amount of at least one further biomarker selected from the group consisting of Tyrosine, Sphingomyelin (d18:2,C18:0), Lysine, Cystine, Creatinine, Cholesterylester C20:4, Glucose, Sphingomyelin (d18:2,C20:0), Creatine, Phenylalanine, Asparagine, Arginine, Glutamine, Tryptophan, Urea, Histidine, Ethanolamine plasmalogen (C39:5), Choline plasmalogen (C36:5), Phosphatidylcholine (C18:0,C20:4), total cholesterol, Ethanolamine plasmalogen (C39:4), Choline plasmalogen (C36:4), Total Cholesterol, Sphingomyelin (d18:1,C18:0), Cholesterylester C15:0, Choline plasmalogen (C18-vinyl,C20:4), Testosterone, Androstenedione, Uridine, C-reactive protein, alkaline phosphatase, leukocytes, Dehydroepiandrosterone sulfate, and Testosterone-17-sulfate. Also, the at least one further marker may be any additional marker listed in Tables 1a, 1b and 1 c in the Examples section.

In an embodiment, the amount of the least one further biomarker is compared to reference amount in step b) of the method of the present invention. Alternatively, a score is calculated based on the amount of biomarkers as mentioned in step (a), and compared to a reference score.

Of the aforementioned biomarkers, the following are metabolite biomarkers: Tyrosine, Sphingomyelin (d18:2,C18:0), Lysine, Cystine, Cholesterylester C20:4, Glucose, Sphingomyelin (d18:2,C20:0), Creatine, Phenylalanine, Asparagine, Arginine, Glutamine, Tryptophan, Histidine, Ethanolamine plasmalogen (C39:5), Choline plasmalogen (C36:5), Phosphatidylcholine (C18:0,C20:4), Ethanolamine plasmalogen (C39:4), Choline plasmalogen (C36:4), Sphingomyelin (d18:1,C18:0), Cholesterylester C15:0, Choline plasmalogen (C18-vinyl,C20:4), Testosterone, Androstenedione, Uridine, Dehydroepiandrosterone sulfate, and Testosterone-17-sulfate. Preferably, these biomarkers can be determined as the metabolite biomarkers as referred to above.

Creatinine, total cholesterol; Total Cholesterol C-reactive protein, alkaline phosphatase, and leukocytes, Urea are well known biomarkers and can be determined by well known methods. For example, the biomarker C-reactive protein can be determined by using antibody (or fragment thereof) which specifically bind to C-reactive protein. Total cholesterol is the sum of free cholesterol and esterified cholesterol.

As set forth above, it is further contemplated to determine the amount of a least one peptide or polypeptide biomarker in step a) of the method of the present invention. Said peptide or polypeptide biomarker shall be capable of assessing NASH. Said peptide or polypeptide biomarkers are well known in the art:

In a preferred embodiment, said biomarker is procollagen type III aminoterminal peptide (PIIINP). The application of this biomarker is e.g. described in Khan et al., Postgrad Med J. 2006 May; 82(967): 353-354. In a particularly preferred embodiment, the biomarker is Pro-C3 (N-terminal type III collagen propeptide, the precisely cleaved N-terminal propeptide of type III collagen). Pro-C3 is a neo-epitope biomarker reflecting true type III collagen formation. It is released by specific N-proteases during type III collagen formation. It has been shown that Pro-C3 is diagnostic as well able to predict fibrosis progression, see Development and Validation of the Collagen Neo-Epitope Biomarker Pro-C3 "FIB-C3 Score" for Detection and Staging of Advanced Non-Alcoholic Fatty Liver Disease in a Large International Multi-Centre Patient Cohort Boyle, Marie P.; Tiniakos, Dina G.; McPherson, Stuart; et al. HEPATOLOGY Volume: 66 Special Issue:SI Supplement: 1 Pages: 54A-55A Meeting Abstract: 93 Published:OCT 2017.

In another embodiment, said biomarker is Cytokeratin-18 (CK-18). The application of this biomarker is e.g. described in Hasegawa, et al. 2015; Dig Dis. 2015; Vol 33, Usefulness of Cytokeratin-18M65 in Diagnosing Non-Alcoholic Steatohepatitis in Japanese Population, or in Musso, et al. Ann Med 2011; Meta-analysis: natural history of non-alcoholic fatty liver disease (NAFLD) and diagnostic accuracy of non-invasive tests for liver disease severity, or in Feldstein, et al. Hepatology 2009; Cytokeratin-18 fragment levels as noninvasive biomarkers for nonalcoholic steatohepatitis: a multicenter validation study.

In an embodiment, said biomarker is gamma-glutamyl transpeptidase (GGT). The application of this biomarker is e.g. described in Khan et al., Postgrad Med J. 2006 May; 82(967): 353-354.

In an embodiment, said biomarker is procollagen type III aminoterminal peptide (PIIINP). The application of this biomarker is e.g. described in Lemoine M et al., The gamma-glutamyl transpeptidase to platelet ratio (GPR) predicts significant liver fibrosis and cirrhosis in patients with chronic HBV infection in West Africa Gut. 2016 Aug;65(8):1369-76. doi: 10.1136/gutjnl-2015-309260. Epub 2015 Jun 24..

In an embodiment, said biomarker is a liver enzyme. Preferably, said liver enzyme is selected from alanine aminotransferase (ALT), aspartate aminotransferase (AST),alkaline phosphatase (ALP), and gamma glutamyltransferase (GGT), see Chin et. al (2016)Non-invasive Markers of Liver Fibrosis: Adjuncts or Alternatives to Liver Biopsy? Front. Pharmacol.7:159.doi: 10.3389/fphar.2016.00159.

In an embodiment, said biomarker is selected from bilirubin, haptoglobin, albumin, ApolipoproteinA1, a2-macroglobulin, ceruloplasmin, transferrin, a1-antitrypsin, adiponectin and leptin.

The thus determined amount of said peptide or polypeptide biomarkers is then compared to a reference (in step b) for said marker. Thus, the at least two metabolite biomarkers and the peptide or polypeptide biomarker might be determined at the same time (but preferably by varying assays). In another preferred embodiment of the present invention, the amount of the peptide or polypeptide biomarker is determined in a sample in a further step d), and compared to a reference for this marker in a further step e). Based on steps c) and e) NAFLD is assessed. Thus, there may be a time gap between the determination of the amounts of the at least two metabolite biomarkers and the determination of the amount of the peptide or polypeptide biomarker (such as one day or one week). Alternatively, the amount of the peptide or polypeptide biomarker be derived from the medical record of the subject to be tested. Thus, the method of the present invention may comprise the step of providing and/or retrieving information on the amount of the peptide or polypeptide marker (i.e. the value of this marker).

The determination of the amount of the peptide or polypeptide biomarker may differ from the determination of the amount of the at least two metabolite biomarkers as referred to in the context of the method of the present invention (since the amounts of the at least two metabolite biomarkers are preferably determined by the methods involving chromatography and mass spectrometry, see elsewhere herein). Preferably, the amount of the peptide or polypeptide biomarker is determined in a blood, serum or plasma sample. Preferably, the amount is determined by using at least one antibody which specifically binds to peptide or polypeptide biomarker. The at least one antibody forms a complex with the marker to determined (i.e. the peptide or polypeptide biomarker). Afterwards the amount of the formed complex is measured. The complex comprises the marker and the antibody (which might be labelled in order to allow for a detection of the complex).

If the peptide or polypeptide biomarker is an enzyme, the amount of this marker can be also assessed by determining the enzymatic activity of said enzyme in a sample.

In an embodiment, the method of the present invention may further comprise carrying out a correction for confounders. Preferably, the values determined in a sample of a subject according to the present invention are adjusted for age, BMI, gender or other existing diseases, e.g., the presence or absence of diabetes before comparing to a reference. Alternatively, the references can be derived from values or ratios which have likewise been adjusted for age, BMI, gender. Such an adjustment can be made by deriving the references and the underlying values or ratios from a group of subjects the individual subjects of which are essentially identical with respect to these parameters to the subject to be investigated. Alternatively, the adjustment may be done by statistical calculations.

The term "reference" refers to values of characteristic features which can be correlated to a medical condition, i.e. the presence or absence of the disease, diseases status or an effect referred to herein (such as a mild or severe form of NAFLD, or the diagnosis of the presence of absence of NAFLD). Preferably, a reference is a threshold value for a biomarker of the at least two biomarkers as referred to in connection with the present invention whereby values found in a sample to be investigated which are lower than (or depending on the marker larger than) the threshold are indicative for the presence of a severe form of NAFLD while those being lower (or depending on the marker higher than) are indicative for the presence of mild form of NAFLD.

The diagnostic algorithm may depend on the reference.

With respect to the diagnosis, preferably, the following applies: If the reference amount is e.g. derived from a subject or group of subjects known to suffer from NAFLD, the presence of NAFLD is preferably indicated by amounts in the test sample which are essentially identical to the reference(s). If the reference amount is e.g. derived from an apparently healthy subject or group thereof, the presence of NAFLD is preferably indicated by amounts of the at least two biomarkers in the test sample which are different from (e.g. increased ("up") or decreased ("down") as compared to) the reference(s).

With respect to the differentiation, preferably, the following applies: If the reference amount is e.g. derived from a subject or group of subjects known to suffer from a severe form of NAFLD, the presence of a severe form of NAFLD is preferably indicated by amounts in the test sample which are essentially identical to the reference(s). If the reference amount is e.g. derived from a subject or group of subjects known to suffer from a mild form of NAFLD, the presence of a mild form of NAFLD is preferably indicated by amounts in the test sample which are essentially identical to the reference(s).

In accordance with the aforementioned method of the present invention, a reference (or references) is, preferably, a reference (or references) obtained from a sample from a subject or group of subjects known to suffer from a severe form NAFLD. In such a case, a value for each of the at least two biomarkers found in the test sample being essentially identical is indicative for the presence of severe form of NAFLD. Moreover, the reference, also preferably, could be from a subject or group of subjects known not to suffer from NAFLD, preferably, an apparently healthy subject or group of subjects. In such a case, a value for each of the at least two biomarkers found in the test sample being altered with respect to the reference is indicative for the presence of the disease. The same applies mutatis mutandis for a calculated reference, most preferably the average or median, for the relative or absolute value of the biomarkers of a population of individuals comprising the subject to be investigated. The absolute or relative values of the biomarkers of said individuals of the population can be determined as specified elsewhere herein. How to calculate a suitable reference value, preferably, the average or median, is well known in the art. The population of subjects referred to before shall comprise a plurality of subjects, preferably, at least 5, 10, 50, 100, 1,000 or 10,000 subjects. It is to be understood that the subject to be diagnosed by the method of the present invention and the subjects of the said plurality of subjects are of the same species.

The value for a biomarker of the test sample and the reference values are essentially identical, if the values for the characteristic features and, in the case of quantitative determination, the intensity values are essentially identical. Essentially identical means that the difference between two values is, preferably, not significant and shall be characterized in that the values for the intensity are within at least the interval between 1st and 99th percentile, 5th and 95th percentile, 10th and 90th percentile, 20th and 80th percentile, 30th and 70th percentile, 40th and 60th percentile of the reference value, preferably, the 50th, 60th, 70th, 80th, 90th or 95th percentile of the reference value. Statistical test for determining whether two amounts or values are essentially identical are well known in the art and are also described elsewhere herein.

An observed difference for two values, on the other hand, shall be statistically significant. A difference in the relative or absolute value is, preferably, significant outside of the interval between 45th and 55th percentile, 40th and 60th percentile, 30th and 70th percentile, 20th and 80th percentile, 10th and 90th percentile, 5th and 95th percentile, 1st and 99th percentile of the reference value. Preferred changes and ratios of the medians are described in the Examples (see in particular Table 1 a, 1 band 1 c).

In a preferred embodiment the value for the characteristic feature can also be a calculated output such as score of a classification algorithm like "elastic net" as set forth elsewhere herein.

Preferably, the reference, i.e. a value or values for at least one characteristic feature (e.g. the amount) of the biomarkers or ratios thereof, will be stored in a suitable data storage medium such as a database and are, thus, also available for future assessments.

The term "comparing", preferably, refers to determining whether the determined value of the biomarkers, or score (see below) is essentially identical to a reference or differs therefrom. Preferably, a value for a biomarker (or score) is deemed to differ from a reference if the observed difference is statistically significant which can be determined by statistical techniques referred to elsewhere in this description. If the difference is not statistically significant, the biomarker value and the reference are essentially identical. Based on the comparison referred to above, a subject can be assessed to suffer from the NAFLD, or not. Alternatively, a subject can be assessed to suffer from a mild or severe form of NAFLD.

For the specific biomarkers referred to in this specification, preferred values for the changes in the relative amounts or ratios (i.e. the changes expressed as the ratios of the means) or the kind of direction of change (i.e. "up"- or "down" or increase or decrease resulting in a higher or lower relative and/or absolute amount or ratio) are indicated in the Table 1a) to 1c) in the Examples section. The ratio of means indicates the degree of increase or decrease, e.g., a value of 2 means that the amount is twice the amount of the biomarker compared to the reference. Moreover, it is apparent whether there is an "up- regulation" or a "down-regulation". In the case of an "up-regulation" the ratio of the mean shall exceed 1.0 while it will be below 1.0 in case of a "down"-regulation. Accordingly, the direction of regulation can be derived from the Tables as well. It will be understood that instead of the means, medians could be used as well.

For up-regulated markers the following applies: an increased amount of the marker in a sample of a subject as compared to the reference is indicative for a severe form of NAFLD, whereas a decreased amount as compared to the reference is indicative for a mild form of NAFLD.

For down-regulated markers the following applies: a decreased amount of the marker in a sample of a subject as compared to the reference is indicative for a severe form of NAFLD, whereas an increased amount as compared to the reference is indicative for a mild form of NAFLD.

The comparison is, preferably, assisted by automation. For example, a suitable computer program comprising algorithms for the comparison of two different data sets (e.g., data sets comprising the values of the characteristic feature(s)) may be used. Such computer programs and algorithms are well known in the art. Notwithstanding the above, a comparison can also be carried out manually.

In the context of step b) of the present invention, the amounts of a group of biomarkers as referred to in step a) of the methods of the present invention shall be compared to a reference or references. Thereby, the NAFLD is assessed. In an embodiment references for the individual determined biomarkers, i.e. references for each of biomarkers as referred to in step a) are applied. However, it is also envisaged to calculate a score (in particular a single score) based on the amounts of the at least two biomarkers as referred to in step a) of the method of the present invention, i.e. a single score, and to compare this score to a reference score. Preferably, the score is based on the amounts of the at least two biomarkers in the sample from the test subject. For example, if the amounts of the biomarkers Etiocholanolone sulfate, Glycocholic acid, and 14,15-Dihydroxyeicosatrienoic acid (C20:cis[5,8,11]3) are determined, the calculated score is based on the amounts of Etiocholanolone sulfate, Glycocholic acid, and 14,15-Dihydroxyeicosatrienoic acid (C20:cis[5,8,11]3) in the sample from the test subject. If additionally the amount of a peptide or polypeptide biomarker is determined, the score is based on the amount of the amount of the three metabolite biomarkers and the amount of the peptide or polypeptide biomarker of the test subject.

The calculated score combines information on the amounts of the at least two biomarkers. Moreover, in the score, the biomarkers are, preferably, weighted in accordance with their contribution to the establishment of the assessment as referred to herein. Based on the combination of biomarkers applied in the method of the invention, the weight of an individual biomarker may be different.

The score can be regarded as a classifier parameter for the assessment of NAFLD. In particular, it enables the person who provides the diagnosis based on a single score based on the comparison with a reference score. The reference score is preferably a value, in particular a cut-off value which allows for differentiating between the presence of NAFLD and the absence of NAFLD in the subject to be tested, or for differentiating between a mild form of NAFLD and severe form of NAFLD. Preferably, the reference is a single value. Thus, the person does not have to interpret the entire information on the amounts of the individual biomarkers.

Using a scoring system as described herein, advantageously, values of different dimensions or units for the biomarkers may be used since the values will be mathematically transformed into the score.

Thus, in a preferred embodiment of the present invention, the comparison of the amounts of the biomarkers to a reference as set forth in step b) of the method of the present invention encompasses step b1) of calculating a score based on the determined amounts of the biomarkers as referred to in step a), and step b2) of comparing the, thus, calculated score to a reference score. More preferably, a logistic regression method is used for calculating the score and, most preferably, said logistic regression method comprises elastic net regularization.

Alternatively, the amount of each of the at least two biomarkers is compared to a reference, wherein the result of this comparison is used for the calculation of a score (in particular a single score), and wherein said score is compared to a reference score. Thereby, NAFLD is assessed.

Thus, the present invention, in particular, relates to a method for assessing NAFLD in a subject comprising the steps of:

a) determining in a sample of a subject as referred to herein the amounts of at least two metabolite biomarkers as referred to above; and
b1) calculating a score based on the determined amounts of the at least two metabolite biomarkers as referred to in step a), and
b2) comparing the, thus, calculated score to a reference score, whereby NAFLD is to be assessed.

As set forth elsewhere herein, the aforementioned method may further comprise in step a) the determination of the amount a peptide or polypeptide biomarker. The amount a peptide or polypeptide biomarker may contribute to the score calculated in step b). Accordingly, the method comprises the following steps:

a) determining in a sample of a subject as referred to herein the amounts of at least two metabolite biomarkers as referred to above and the amount a peptide or polypeptide biomarker; and
b1) calculating a score based on the determined amounts of the at least two metabolite biomarkers and on the amount a peptide or polypeptide biomarker as referred to in step a), and
b2) comparing the, thus, calculated score to a reference score, whereby NAFLD is to be assessed.

Alternatively, the amount a peptide or polypeptide biomarker may not contribute to the score calculated in step b1). Accordingly, the method comprises the following steps:

a) determining in a sample of a subject as referred to herein the amounts of at least two metabolite biomarkers as referred to above and the amount a peptide or polypeptide biomarker; and
b1) calculating a score based on the determined amounts of the at least two metabolite biomarkers, and
b2) comparing the, thus, calculated score to a reference score, and comparing the amount a peptide or polypeptide biomarker to a reference, whereby NAFLD is to be assessed.

As set forth elsewhere herein, the amount of the peptide or polypeptide biomarker can be also derived from the medical record of the subject to be tested. In this case, it is not required to the determine the amount of this marker in step a).

Preferably, the reference score shall allow for assessing NAFLD as referred to herein. E.g. the assessment is the differentiation between a mild form and a severe form of NAFLD, the reference score shall allow for differentiating between mild form and a severe form of NAFLD (the same applies to the reference amounts as described elsewhere herein). Preferably, the assessment, e.g. the differentiation, is made by assessing whether the score of the test subject is above or below the reference score. Thus, in an embodiment it is not necessary to provide an exact reference score. A relevant reference score can be obtained by correlating the sensitivity and specificity and the sensitivity/specificity for any score, preferably, using ROC-curve-analysis. A reference score resulting in a high sensitivity results in a lower specificity and vice versa. Thus, the reference score may depend on the desired sensitivity and/or specificity. Preferably, the reference score is based on the same markers as the score calculated in step b).

The reference score may be a "Cut-Off" value which allows for the assessment of NAFLD in the subject.

Preferably, the score is calculated based on a suitable scoring algorithm. Said scoring algorithm e.g. shall allow for differentiating whether a subject suffers from a mild form or a severe form of disease as referred to herein (based on the amounts of the biomarkers to be determined)

Preferably, said scoring algorithm has been previously determined by comparing the information regarding the amounts of the individual biomarkers as referred to in step a) in samples from patients suffering from a mild form NAFLD as referred to herein and from patients suffering from a severe form of NAFLD. Accordingly, step b) may also comprise step b0) of determining or implementing a scoring algorithm. Preferably, this step is carried out prior steps b1) and b2).

In an embodiment the reference score is calculated such that an increased value of the score of the test subject as compared to the reference score is indicative for a severe form of NAFLD, and/or a decreased value of the score of the test subject as compared to the reference score is indicative for a mild form of NAFLD. In particular, the score may be a cut-off value.

In a preferred embodiment of the present invention (e.g. of the methods, devices, uses etc.), the reference score is a single cut-off value. Preferably, said value allows for allocating the test subject either into a group of subjects suffering from a severe form of NAFLD or a into a group of subject suffering from a mild NAFLD (or for allocating the test subject either into a group of subjects suffering from NAFLD or a into a group of subjects not suffering from NAFLD). Preferably, a score for a subject lower than the reference score is indicative for a mild form NAFLD in said subject whereas a score for a subject larger than the reference score is indicative for a severe form of NAFLD in said subject. In another preferred embodiment of the present invention (e.g. of the methods, devices, uses etc.), the reference score is a reference score range.

A suitable scoring algorithm can be determined with the at least two biomarkers referred to in step a) (and optionally the at least one further biomarker) by the skilled person without further ado. E.g., the scoring algorithm may be a mathematical function that uses information regarding the amounts of the biomarkers in a cohort of subjects suffering from a severe form NAFLD and a group of subjects suffering from a mild form NAFLD. Methods for determining a scoring algorithm are well known in the art and including Significance Analysis of Microarrays, Tree Harvesting, CART, MARS, Self Organizing Maps, Frequent Item Set, Bayesian networks, Prediction Analysis of Microarray (PAM), SMO, Simple Logistic Regression, Logistic Regression, Multilayer Perceptron, Bayes Net, Naïve Bayes, Naïve Bayes Simple, Naïve Bayes Up, IB1, Ibk, Kstar, LWL, AdaBoost, ClassViaRegression, Decorate, Multiclass Classifier, Random Committee, j48, LMT, NBTree, Part, Random Forest, Ordinal Classifier, Sparse Linear Programming (SPLP), Sparse Logistic Regression (SPLR), Elastic net, Support Vector Machine, Prediction of Residual Error Sum of Squares (PRESS), Penalized Logistic Regression, Mutual Information. Preferably, the scoring algorithm is determined with or without correction for confounders as set forth elsewhere herein.

In a preferred embodiment the score as referred to in step b) in the method of the present invention is calculated by calculating for each amount determined in step a) of the method of the present invention, a scaled amount by first subtracting a predetermined, diagnostic biomarker-specific subtrahend from said amount and then dividing the resulting value by a predetermined, biomarker-specific divisor. The score is then calculated by i) assigning a biomarker-specific weight value to each scaled amount, thereby providing a weighed amount, (ii) summing up said weighed amounts for all diagnostic biomarkers, providing a sum of weighted amounts, (iii) preferably, assigning a bias value to the sum of weighted amounts of step (ii) to provide a bias-corrected sum, and (iv) preferably, scaling the bias-corrected sum of step (iii), preferably to a value between 0 and 1 (or a value between 0 and 100). The score is a measure e.g. for the severity of NAFLD. Preferably, the higher the score is, the more severe the NAFLD. Accordingly, the score is a measure for the prediction probability.

Accordingly, the method of assessing NAFLD may comprise the steps of

(a) determining the amounts of the at least two metabolite biomarkers (and optionally the amount of the further peptide or polypeptide biomarker) according to the present invention in at least one sample of a subject as referred to herein,

(b1) for each amount determined in step (a), calculating a scaled amount by first subtracting a predetermined, diagnostic biomarker-specific subtrahend from said amount and then dividing the resulting value by a predetermined, biomarker-specific divisor,

(b2) calculating a score by

(i) assigning a biomarker-specific weight value to each scaled amount of (b1), thereby providing a weighed amount,

(ii) summing up said weighed amounts for all diagnostic biomarkers, providing a sum of weighted amounts,

(iii) preferably, assigning a bias value to the sum of weighted amounts of step (ii) to provide a bias-corrected sum,

(iv) preferably, scaling the bias-corrected sum of step (iii), preferably to a value between 0 and 1 (or a value between 0 and 100), and

(c) assessing NAFLD based on the score determined in step (b2).

Step (a), preferably, corresponds to step (a) of the method for assessing as specified above. E.g. the amount of the biomarkers or the sum of the amounts of certain biomarkers are determined.

Preferably, the measured values for the amounts are scaled by first subtracting a predetermined, diagnostic biomarker-specific subtrahend from said amount and then dividing the resulting value by a predetermined, diagnostic biomarker-specific divisor. From the values thus obtained, a score is calculated by weighting the individual biomarkers and summing up the weighted values for all biomarkers determined, preferably, followed by assigning a bias value to said sum and, preferably, scaling the bias-corrected sum, preferably to a value between 0 and 1 (or to a value between 0 and 100). Preferably, scaling of measured values comprises log10 transforming the measured values, preferably after subtracting said predetermined, biomarker-specific subtrahend. More preferably, the score is calculated according to the following formula (I) in the Examples section, wherein

$p$ = score;
$e$ = Euler's number;
$\omega_0$ = bias value;
$\omega_i$ = biomarker-specific weight value for diagnostic biomarker i; and
$\hat{x}_i$ = scaled amount for biomarker i..

Preferably, $\hat{x}_i$ is calculated by scaling the $\log_{10}$-transformed input data $x$ by first subtracting an analyte-specific constant $m_i$ and then dividing by a analyte-specific constant $s_i$, resulting in $\log_{10}$-transformed and scaled input data $\hat{x}$, see formula II in the Examples section.

The calculated score can be compared to a reference score.

Preferably, optimized values of the predetermined, biomarker-specific subtrahend, the predetermined, diagnostic biomarker-specific divisor, the diagnostic biomarker-specific weight value, and the bias value are determined by optimizing the assessment of NAFLD, e.g. the differentiation between subjects suffering from a mild form of NAFLD and subjects suffering from a severe form of NAFLD. Thus preferably, the group of diagnostic biomarkers is, preferably, trained on data obtained from two groups of subjects with known disease state. E.g., preferably, one of said groups of subjects is a group of subjects suffering from a mild form of NAFLD and the second group is a group of subjects suffering a severe form of NAFLD; this way, optimized parameters for a differentiation between the two forms.

Preferred scaling factors $m_i$ and $s_i$ and coefficients $w_i$ to the used in the method of the present invention are shown in Table 3 in the Examples section (for the preferred marker combinations).

Advantageously, it has been found in the study underlying the present invention that the amounts of the at least two biomarkers referred to above are indicators for NAFLD. Accordingly, the at least two biomarkers as specified above in a sample can, in principle, be used for assessing whether a subject suffers from a mild or severe form NAFLD. This is particularly helpful for an efficient diagnosis of the disease as well as for improving of the pre-clinical and clinical management of NAFLD.

By carrying out the method of the present invention, a subject can be identified who is in need for a therapy of NAFLD. Accordingly, the present invention relates to a method for identifying whether a subject is in need for a therapy of NAFLD or a change of therapy comprising the steps of the methods of the present invention and the further step of identifying a subject in need if NAFLD is diagnosed.

The phrase "in need for a therapy of NAFLD" as used herein means that the disease in the subject is in a status where therapeutic intervention is necessary or beneficial in order to ameliorate or treat NAFLD or the symptoms associated therewith. Accordingly, the findings of the studies underlying the present invention do not only allow diagnosing NAFLD in a subject but also allow for identifying subjects which should be treated by a NAFLD therapy or whose NAFLD therapy needs adjustment. Once the subject has been identified, the method may further include a step of making recommendations for a therapy of NAFLD.

A therapy of NAFLD as used in accordance with the present invention, preferably, comprises surgery, drug treatment or life style recommendations. Drug-based therapies, preferably, include the administration of one or more drugs selected from Statins, Incretin analogues, Metformin, Rimonabant, Thiazolidinediones, Orlistat (see Maryam R. Kashi, Dawn M. Torres, and Stephen A. Harrison: Current and Emerging Therapies in Nonalcoholic Fatty Liver Disease: Therapeutic Modalities; Semin Liver Dis. 2008; 28 (4): 396-406).

In general, the present invention also contemplates the use, in particular the in vitro use, of the at least two metabolite biomarkers as set forth in the method of the present invention, and optionally of at least one further biomarker as set forth above for assessing non-alcoholic fatty liver disease (NAFLD). Preferably, the biomarkers are used in a sample from a subject as defined herein. The definitions given in connection with the method of the present invention apply accordingly. The same applies to the following methods.

Moreover, the present invention relates to a method for assessing NAFLD in a subject comprising the steps of:

a) determining in a sample of a subject the amount at least one biomarker from tables 1a), 1b) or 1c); and

b) comparing the amounts of the said biomarkers as determined in step a) to a reference, whereby NAFLD is to be assessed.

Moreover, the present invention pertains to the use of at least one biomarker at least one biomarker from tables 1a), 1b) or 1c) in a sample of a subject for the assessment of NAFLD.

All references cited herein are herewith incorporated by reference with respect to their disclosure content in general or with respect to the specific disclosure contents indicated above.

Means and methods for diagnosing fibrosis stage in a subject based on a metabolite panel

## EXAMPLES

The invention will now be illustrated by the following Examples which are not intended to restrict or limit the scope of this invention.

## Example 1: Patients, plasma and serum preparation

A total of 78 patients with Non-alcoholic fatty liver disease (NAFLD and 62 healthy blood donors were enrolled in the clinical study. In this prospective, multicenter study samples of: 36 patients suffering from non-alcoholic steato-hepatitis (NASH) ,42 non-alcoholic fatty liver (NAFL) were included. Among the 78 NAFLD patients, 13 patients were classified into Fibrosis stage > 2 and 61 NAFLD patients were classified into Fibrosis stage < 2 (Kleiner et al., 2005, HEPATOLOGY, Vol. 41, No. 6, 2005). All patients or their legal representatives gave their written informed consent and the local ethics review boards approved the protocol. Patients were consecutively recruited from five centers. After blood drawing and centrifugation, plasma or serum samples were collected in Eppendorf tubes and stored at -80°C for further analysis. Sample processing was performed according to the institutional standard operating procedure.

## Example 2: Metabolite profiling

*MxP® Broad and MxP®Steroids*

Three types of mass spectrometry analyses were applied to all samples. GC-MS (gas chromatography-mass spectrometry; Agilent 6890 GC coupled to an Agilent 5973 MSSystem, Agilent, Waldbronn, Germany) and LC-MS/MS [liquid chromatography-MS/MS; Agilent 1100 HPLC-System (Agilent, Waldbronn, Germany) coupled to an Applied Biosystems API4000 MS/MS-System (Applied Biosystems, Darmstadt, Germany)] were used for broad profiling [van Ravenzwaay, B. et al. The use of metabolomics for the discovery of new biomarkers of effect. Toxicol Lett 172, 21-8 (2007). Solid phase extraction-LC-MS/MS [SPE-LC-MS/MS; Symbiosis Pharma (Spark, Emmen, Netherlands) coupled to an Applied Biosystems API4000 MS/MS-System (Applied Biosystems, Darmstadt, Germany)] was used for the determination steroid levels. Fractionation and derivatisation of samples and detection technologies have been previously described [van Ravenzwaay, B. et al. The use of metabolomics for the discovery of new biomarkers of effect. Toxicol Lett 172, 21-8 (2007, Roessner, U., Wagner, C., Kopka, J., Trethewey, R.N. & Willmitzer, L. Technical advance: simultaneous analysis of metabolites in potato tuber by gas chromatography-mass spectrometry. Plant J23, 131-42 (2000), Mutch, D.M. et al. Metabolite profiling identifies candidate markers reflecting the clinical adaptations associated with Roux-en-Y gastric bypass surgery. PLoS One 4, e7905 (2009)]. Proteins were removed from plasma samples by solvent precipitation by adding the triple amount of acetonitrile. Subsequently polar and non-polar fractions were separated for both GC-MS and LC-MS/MS analyses by adding water and a mixture of ethanol and dichloromethane. For GC-MS analyses, the non-polar fraction was treated with methanol under acidic conditions to yield the fatty acid methyl esters derived from both free fatty acids and hydrolyzed complex lipids. The polar and non-polar fractions were further derivatized with O-methyl-hydroxyamine hydrochloride (20 mg/ml in pyridine, 50 Il) to convert oxo-groups to O-methyloximes and subsequently with a silylating agent (MSTFA, 50 Il) before GC-MS analysis. For LC-MS/MS analyses, both fractions were reconstituted in appropriate solvent mixtures. High performance LC (HPLC) was performed by gradient elution using methanol/water/formic acid on reversed phase separation columns. Mass spectrometric detection technology was applied as described in the patent US 7196323, which allows targeted and high sensitivity "Multiple Reaction Monitoring" profiling in parallel to a full screen analysis. Steroids and their related metabolites were measured by online SPE-LC-MS/MS.

*MxP® Lipids*

Total lipids were extracted from plasma or serum by liquid/liquid extraction using chloroform/methanol. The lipid extracts were subsequently fractionated by normal phase liquid chromatography (NPLC) according to [Christie, W.W. Rapid separation and quantification of lipid classes by high performance liquid chromatography and mass (light-scattering) detection. J Lipid Res 26, 507-12 (1985)] into different lipid groups comprising sphingomyelins, ceramides, sterol esters, free fatty acids, phosphatidylcholines, phosphatidylethanolamines, phosphatidyl-serines, phosphatidylinositols, lysophosphatidylcholines, triacylglycerides, diacylglycerides and monoacylglycerides. The fractions were analyzed by LC-MS/MS using electrospray ionization (ESI) and atmospheric pressure chemical ionization (APCI) with detection of specific multiple reaction monitoring (MRM) transitions for cholesterol esters (CE), free sterols (FS), sphingoymelins (SM), and ceramides (CER) respectively. Sphingosines and sphingosine-1-phosphates (SP) were analyzed by LC-MS/MS using electrospray ionization (ESI) with detection of specific multiple reaction monitoring (MRM) transitions as described by [Schmidt, H., Schmidt, R. & Geisslinger, G. LC-MS/MS-analysis of sphingosine-1-phosphate and related compounds in plasma samples. Prostaglandins Other Lipid Mediat 81, 162-70 (2006)]. The lipid classes Monoacylglycerides (MAG), Triacylglycerides (TAG), Phosphatidylcholines (PC), Phosphatidylserines (PS), Phosphatidylinositoles (PI), Lysophosphatidylcholines (LPC), Diacylglycerols (DAG), Free fatty acids (FFA) were measured by GC-MS. The fractions are analyzed by GC-MS after derivatization with TMSH (Trimethyl sulfonium hydroxide), yielding the fatty acid methyl esters (FAME) corresponding to the acyl moieties of the class-separated lipids. The concentrations of FAME from C14 to C24 are determined in each fraction. In the tables below, any of the abbreviations above is used as prefix for a metabolite to indicate that the respective metabolite has been derived from the respective lipid or lipid fraction.

*MxP® Eicosanoids*

Eicosanoids were measured by online SPE-LC-MS/MS (Solid-phase extraction-LC-MS/MS) (Yamada et al., 2002). First, proteins were removed by precipitation, followed by solid-phase extraction (SPE). During this separation process compounds dissolved in blood were separated based on their physical and chemical properties. Afterwards, the fraction of interest was analyzed by LC-MS/MS (liquid chromatography coupled with tandem mass spectrometry see Absolute quantification (provided in ng/ml) was achieved by means of stable isotope-labeled internal standards and the use of external calibration.

*Clinical Parameter*

The following clinical parameter were analysed by a clinical diagnostic laboratory according methods known to the skilled person in the art and routinely applied in clinical diagnostic laboratories:

Table:01 Clinical parameter

| Metabolite | Unit |
|---|---|
| Urea | mg / dL |
| Leucocyte | 10E9 / L |
| Glucose | mmol / L |
| Total Triacyglyceride | |
| Total Cholesterol | |
| Creatinine | mg / L |
| Aspartate Aminotransferase (AST) | U per L |
| Gamma-Glutamyltransferase (GGT) | U per L |
| C-reactive Protein (CRP) | mg / L |
| Bilirubin | mg /dL |
| Alkaline Posphatase (ALP) | U per L |

**Example 3: Data set analysis and normalization**

Prior to statistical analysis, log10 transformation of ratios was conducted to assure normal distribution of data. The software R 2.8.1 (package nlme) was used for data analyses and visualizations. Statistical analysis was done by a linear mixed model (ANOVA) with "disease", fibrosis, "age", "gender", "BMI", as fixed effects and center as random effect.
For the multivariate statistical analysis, missing values of the log10-transfomed data were imputed by the sample median of the related metabolite, followed by classical scaling ($\hat{x}_i = (\log_{10}(x_i) - m_i)/si$). Non-linear Classification analysis with Random Forest (Liaw and Wiener (2002). Classification and Regression by random Forest. R News 2(3), 18-22.) and linear Elastic Net (Zou and Hastie (2005) Regularization and variable selection via the elastic net, Journal of the Royal Statistical Society, Series B), as well as Linear Discriminant Analysis (Tibshirani, Hasti, Friedman (2008), Elements of Statistical Learning, Chapter 4.3)] were applied on the log10 transformed data. Feature selection was done by a forward search approach where

(a) the metabolite best correlating with the residuals of the last model is added to the next model or
(b) iterative testing, where the metabolite resulting in the best model deviance is selected.

The inbuilt feature selection of Elastic Net was not applied. We used 10-fold cross-validation with the feature selection embedded to repeatedly build a model on nine out of ten training folds to analyse the performance of our selected panels, estimated with the area under the curve (AUC) of a receiver operating characteristic (ROC) analysis on the test fold

Afterwards the final set of metabolites was determined by retraining the classifier on the entire data.

The core panel was selected from results of multivariate analyses and univariate analyses (ANOVA, ROC) for differential diagnosis between Fibrosis stages >2 and Fibrosis stage <2.

The analysis comprised the following comparisons:

a) Diseasase advanced vs disease mild = 74 NAFLD samples (61 NAFLD; Fibrosis < 2 vs 13 NAFLD; Fibrosis > 2)
b) Advanced vs mild = 136 samples (122 samples (NAFLD + Controls); Fibrosis < 2 vs 14 NAFLD samples; Fibrosis > 2)
c) NASH Advancd vs NASH mild = 32 NASH Samples (20 NASH samples; Fibrosis < 2 vs 12 NASH samples; Fibrosis >2)

## Calculation of prediction probability:

The prediction probalitity was calculated as:

$$p = \frac{1}{1+e^{-(w_0+\sum_{i=1}^{n} w_i \hat{x}_i)}}$$ (formula I) with the features $\hat{x}_i$ being $$\hat{x}_i = \frac{x_i - m_i}{s_i}$$ (formula II) where $x_i$ are the log-transformed analytical results taking into account the units listed in Table 3a) - 3f), $m_i$ and $s_i$ are feature-specific scaling factors, and $w_i$ are the coefficients of the model (Table 3a) - 3f); $w_0$, intercept; $w_1$, coefficient for; $w_2 ... w_n$, coefficients for the metabolomic features; $n$, total number of features in the panel plus NT-proBNP).

**The results of the univariate analysis are listed in the following tables:**

**Table 1a)** Ratio, p-Value and AUC of comparison disease advanced vs disease mild
**Table 1b)** Ratio, p-Value and AUC of comparison advanced vs mild
**Table 1c)** Ratio, p-Value and AUC of comparison NASH advanced vs NASH mild

**The results of the multivariate analysis are listed in the following tables:**

**Table 2a_1)** Multimarker panel of comparison "disease advanced vs disease mild" - Androgen Core Panel
**Table 2a_2)** Multimarker panel of comparison "disease advanced vs disease mild" - Androgen Core Panel
**Table 2a_3)** Multimarker panel of comparison "disease advanced vs disease mild" - Androgen Core Panel
**Table 2b_1)** Multimarker panel of comparison "advanced vs mild" - Androgen Core Panel
**Table 2b_2)** Multimarker panel of comparison "advanced vs mild" - Androgen Core Panel
**Table 2b_3)** Multimarker panel of comparison "advanced vs mild" - Androgen Core Panel
**Table 2c_1)** Multimarker panel of comparison "NASH advanced vs NASH mild" - Androgen Core Panel
**Table 2c_2)** Multimarker panel of comparison "NASH advanced vs NASH mild" - Androgen Core Panel
**Table 2c_3)** Multimarker panel of comparison "NASH advanced vs NASH mild" - Androgen Core Panel
**Table 2d)** Multimarker panel of comparison "disease advanced vs disease mild" - Bile acid Core Panel
**Table 2e)** Multimarker panel of comparison "advanced vs mild"- Bile acid Core Panel

**Table 2f)** Multimarker panel of comparison "NASH advanced vs NASH mild" - Bile acid Core Panel

**The scaling factors and coefficients are listed in the following table:**

**Table 3)** Scaling factors $m_i$ and $s_i$ and coefficients $w_i$ used for the calculation of prediction probabilities individual subjects are listed in the table below (Comparison disease advanced vs disease mild)

**Table 1a: ANOVA and ROC result of disease advanced (Fib>2) relative to disease mild (Fib<2)**

| Table 1a: ANOVA and ROC result of disease advanced (Fib>2) relative to disease mild (Fib<2) | | | | |
|---|---|---|---|---|
| Metabolite | Direction | Estimate fold change | p-Value | Area under the curve |
| CE_Cholesterylester C12:0 | up | 1.56240 | 0.05636 | 0.61410 |
| CE_Cholesterylester C14:0 | up | 1.29613 | 0.04199 | 0.63490 |
| CE_Cholesterylester C14:1 | up | 1.32142 | 0.17587 | 0.61280 |
| CE_Cholesterylester C20:3 | down | 0.84763 | 0.12675 | 0.65840 |
| CE_Cholesterylester C20:5 | down | 0.77073 | 0.14770 | 0.69490 |
| CE_Cholesterylester C22:4 | down | 0.84561 | 0.17200 | 0.62580 |
| CE_Cholesterylester C22:6 | down | 0.74068 | 0.02292 | 0.71840 |
| CE_Cholesterylester C18:2 | down | 0.89058 | 0.07004 | 0.64800 |
| CE_Cholesterylester C20:4 | down | 0.72002 | 0.00060 | 0.80700 |
| Creatinine | down | 0.87202 | 0.01122 | 0.75940 |
| Aspartate Aminotransferase (AST) | up | 1.42412 | 0.00530 | 0.77110 |
| Gamma Glutamyltransferase (GGT) | up | 1.66577 | 0.04679 | 0.71310 |
| Bilirubin | up | 1.26598 | 0.18135 | 0.59650 |
| FFA_Myristic acid (C14:0) | up | 1.91995 | 0.15342 | 0.74400 |
| FFA_Elaidic acid (C18:trans[9]1) | up | 1.36768 | 0.14938 | 0.69990 |
| SM_Sphingomyelin (d18:1,C14:0) | up | 1.17674 | 0.07476 | 0.54350 |
| SM_Sphingomyelin (d18:1,C18:0) | down | 0.79187 | 0.00211 | 0.74270 |
| SM_Sphingomyelin (d18:1,C18:1) | down | 0.88553 | 0.08052 | 0.64190 |
| SM_Sphingomyelin (d18:1,C19:0) | down | 0.77197 | 0.01444 | 0.66330 |
| SM_Sphingomyelin (d18:1,C20:0) | down | 0.80176 | 0.00050 | 0.74910 |
| SM_Sphingomyelin (d18:1,C20:1) | down | 0.86139 | 0.11943 | 0.64690 |
| SM_Sphingomyelin (d18:1,C21:0) | down | 0.85154 | 0.03183 | 0.62300 |
| SM_Sphingomyelin (d18:1,C22:0) | down | 0.81235 | 0.01004 | 0.65830 |
| SM_Sphingomyelin (d18:1,C23:0) | down | 0.87440 | 0.09531 | 0.62800 |
| SM_Sphingomyelin (d18:1,C24:0) | down | 0.87064 | 0.09336 | 0.64560 |
| SM_Sphingomyelin (d18:1,C24:1) | down | 0.87831 | 0.11694 | 0.68220 |
| SM_Sphingomyelin (d18:2,C14:0) | up | 1.19937 | 0.08341 | 0.59270 |
| SM_Sphingomyelin (d18:2,C18:0) | down | 0.75473 | 0.00007 | 0.75910 |
| SM_Sphingomyelin (d18:2,C18:1) | down | 0.80345 | 0.01677 | 0.64440 |
| SM_Sphingomyelin (d18:2,C19:0) | down | 0.85421 | 0.14494 | 0.60150 |
| SM_Sphingomyelin (d18:2,C20:0) | down | 0.78789 | 0.00146 | 0.72130 |
| SM_Sphingomyelin (d18:2,C22:0) | down | 0.88855 | 0.05344 | 0.67470 |
| SM_Sphingomyelin (d18:2,C22:1) | down | 0.86925 | 0.14663 | 0.64060 |
| SM_Sphingomyelin (d18:2,C24:0) | down | 0.86235 | 0.04290 | 0.68850 |
| SM_Sphingomyelin (d18:2,C24:1) | down | 0.88981 | 0.18977 | 0.67090 |

(continued)

| Table 1a: ANOVA and ROC result of disease advanced (Fib>2) relative to disease mild (Fib<2) | | | | |
|---|---|---|---|---|
| Metabolite | Direction | Estimate fold change | p-Value | Area under the curve |
| SM_Sphingomyelin (d16:1,C18:0) | down | 0.73211 | 0.00075 | 0.71250 |
| SM_Sphingomyelin (d16:1,C18:1) | down | 0.88276 | 0.16350 | 0.59900 |
| SM_Sphingomyelin (d16:1,C20:0) | down | 0.89128 | 0.13776 | 0.60910 |
| SM_Sphingomyelin (d17:1,C18:0) | down | 0.75942 | 0.00150 | 0.72760 |
| SM_Sphingomyelin (d17:1,C20:0) | down | 0.82943 | 0.02770 | 0.67210 |
| SM_Sphingomyelin (d17:1,C22:0) | down | 0.89231 | 0.16150 | 0.62420 |
| SM_Sphingomyelin (d17:1,C24:1) | down | 0.90648 | 0.18319 | 0.68600 |
| SM_Sphingomyelin (d18:1,C18:2) | down | 0.78374 | 0.00354 | 0.68600 |
| SM_Sphingomyelin (d18:1,C22:2) | down | 0.78145 | 0.03388 | 0.61030 |
| SM_Sphingomyelin (d18:2,C17:0) | down | 0.84956 | 0.11781 | 0.60030 |
| SM_Sphingomyelin (d19:1,C18:0) | down | 0.77235 | 0.04155 | 0.63180 |
| SM_Sphingomyelin (d19:1,C22:0) | down | 0.83663 | 0.14194 | 0.59020 |
| Testosterone | up | 1.70317 | 0.01201 | 0.53460 |
| 18-Hydroxycorticosterone | up | 1.43409 | 0.05111 | 0.69100 |
| CER_Ceramide (d18:0,C16:0) | up | 1.23579 | 0.07558 | 0.74400 |
| CER_Ceramide (d18:1,C14:0) | up | 1.19817 | 0.07382 | 0.60910 |
| CER_Ceramide (d18:1,C18:0) | down | 0.81894 | 0.06584 | 0.55860 |
| CER_Ceramide (d18:1,C20:0) | down | 0.86165 | 0.13039 | 0.56620 |
| CER_Ceramide (d18:2,C14:0) | up | 1.19496 | 0.11336 | 0.55490 |
| CER_Ceramide (d18:2,C18:0) | down | 0.80384 | 0.04967 | 0.59390 |
| CER_Ceramide (d16:1,C18:0) | down | 0.81414 | 0.08673 | 0.58260 |
| CER_Ceramide (d17:1,C18:0) | down | 0.80772 | 0.06489 | 0.57500 |
| Cryptoxanthin | up | 1.42126 | 0.08993 | 0.58130 |
| gamma-Tocopherol | down | 0.75347 | 0.08984 | 0.58510 |
| 2,3-Dimethyl-5-phytylquinol | down | 0.75347 | 0.08984 | 0.58510 |
| beta-Tocopherol | down | 0.75347 | 0.08984 | 0.58510 |
| Glucose-6-phosphate | down | 0.67888 | 0.08369 | 0.50880 |
| Fructose-6-phosphate | down | 0.67888 | 0.08369 | 0.50880 |
| myo-Inositol-1-phosphate | down | 0.67888 | 0.08369 | 0.50880 |
| myo-Inositol-2-phosphate | down | 0.67888 | 0.08369 | 0.50880 |
| myo-Inositol-4-phosphate | down | 0.67888 | 0.08369 | 0.50880 |
| Coenzyme Q9 | up | 1.29299 | 0.18255 | 0.62420 |
| Creatinine | down | 0.85724 | 0.01242 | 0.75660 |
| Creatine | down | 0.65494 | 0.00351 | 0.73010 |
| Taurochenodeoxycholic acid | up | 4.77283 | 0.00005 | 0.86920 |

(continued)

| Table 1a: ANOVA and ROC result of disease advanced (Fib>2) relative to disease mild (Fib<2) | | | | |
|---|---|---|---|---|
| Metabolite | Direction | Estimate fold change | p-Value | Area under the curve |
| Taurodeoxycholic acid | up | 4.77283 | 0.00005 | 0.86920 |
| Tetradecanoylcarnitine | up | 1.23191 | 0.05043 | 0.58510 |
| Hydroxyhexadecenoylcarnitine | up | 1.28183 | 0.02801 | 0.67720 |
| TAG_Myristic acid (C14:0) | up | 1.41686 | 0.14501 | 0.61670 |
| TAG_Myristoleic acid (C14:cis[9]1) | up | 2.14419 | 0.09839 | 0.65900 |
| TAG_Elaidic acid (C18:trans[9]1) | up | 2.44501 | 0.13105 | 0.64360 |
| TAG_Linoleic acid (C18:cis[9,12]2) | down | 0.78059 | 0.15664 | 0.56790 |
| TAG_gamma-Linolenic acid (C18:cis[6,9,12]3) | down | 0.52146 | 0.07819 | 0.57180 |
| TAG_Arachidonic acid (C20:cis[5,8,11,14]4) | down | 0.74224 | 0.09217 | 0.53720 |
| TAG_Docosapentaenoic acid (C22:cis[4,7,10,13,16]5) | down | 0.51727 | 0.10862 | 0.56540 |
| conjugated 13-Hydroxylinoleic acid (C18:cis[9]trans[11]2) | up | 1.28554 | 0.07028 | 0.60420 |
| conjugated 9-Hydroxylinoleic acid (C18:trans[10]cis[12]2) | up | 1.27393 | 0.09597 | 0.58060 |
| 14,15-Dihydroxyeicosatrienoic acid (C20:cis[5,8,11]3) | up | 1.48429 | 0.00057 | 0.78330 |
| 11,12-Dihydroxyeicosatrienoic acid (C20:cis[5,8,14]3) | up | 1.57069 | 0.00150 | 0.76110 |
| 8,9-Dihydroxyeicosatrienoic acid (C20:cis[5,11,14]3) | up | 1.89720 | 0.00093 | 0.70490 |
| Arachidonic acid (C20:cis[5,8,11,14]4) | down | 0.70586 | 0.00288 | 0.75660 |
| Docosahexaenoic acid (C22:cis[4,7,10,13,16,19]6) | down | 0.77073 | 0.09976 | 0.65950 |
| Cholesterol, total | down | 0.83621 | 0.16797 | 0.66150 |
| Lignoceric acid (C24:0) | down | 0.82304 | 0.10069 | 0.70620 |
| Eicosanoic acid (C20:0) | down | 0.84267 | 0.11069 | 0.66080 |
| Tricosanoic acid (C23:0) | down | 0.85294 | 0.19897 | 0.66830 |
| Myristic acid (C14:0) | up | 1.40388 | 0.16699 | 0.58130 |
| 6-Aminohexanoic acid | down | 0.76526 | 0.10655 | 0.50820 |
| Tetradecanol | down | 0.93212 | 0.14177 | 0.66080 |
| myo-Inositol-2-phosphate, lipid fraction (myo-Inositolphospholipids) | up | 1.30652 | 0.15143 | 0.61540 |
| Behenic acid (C22:0) | down | 0.77154 | 0.01043 | 0.72510 |
| Nervonic acid (C24:cis[15]1) | down | 0.81093 | 0.08507 | 0.79570 |
| 3-O-Methylsphingosine (d18:1) (additional: Sphingolipids, erythro-Sphingosine (d18:1), threo-Sphingosine (d18:1)) | down | 0.75541 | 0.03325 | 0.73390 |
| 5-O-Methylsphingosine (d18:1) | down | 0.71278 | 0.00419 | 0.77180 |
| Sphingolipids | down | 0.71278 | 0.00419 | 0.77180 |
| threo-Sphingosine (d18:1) | down | 0.71278 | 0.00419 | 0.77180 |
| erythro-Sphingosine (d18:1) | down | 0.76671 | 0.00606 | 0.76800 |
| Cholestenol No 02 | down | 0.86620 | 0.17515 | 0.68220 |
| erythro-Dihydrosphingosine (d16:0) | down | 0.78383 | 0.03899 | 0.66830 |

(continued)

| Table 1a: ANOVA and ROC result of disease advanced (Fib>2) relative to disease mild (Fib<2) | | | | |
|---|---|---|---|---|
| Metabolite | Direction | Estimate fold change | p-Value | Area under the curve |
| erythro-Sphingosine-1-phosphate (d18:1) | down | 0.73062 | 0.01944 | 0.74270 |
| 1-Hydroxy-2-amino-(cis,trans)-3,5-octadecadiene (from sphingolipids) | down | 0.73750 | 0.01188 | 0.70090 |
| 5-O-Methylsphingosine (d16:1) | down | 0.80109 | 0.07589 | 0.65700 |
| Glycine | down | 0.86672 | 0.08228 | 0.69740 |
| 5-Oxoproline | down | 0.89644 | 0.05779 | 0.53220 |
| Folic acid | down | 0.89644 | 0.05779 | 0.53220 |
| Cysteine | down | 0.89656 | 0.09012 | 0.54980 |
| Citrate | up | 1.10762 | 0.16196 | 0.67280 |
| Lysine | down | 0.81201 | 0.00140 | 0.81210 |
| Glutamine | down | 0.86250 | 0.04824 | 0.65570 |
| Erythrol | down | 0.83482 | 0.15764 | 0.53720 |
| Aspartate | down | 0.78476 | 0.14868 | 0.53250 |
| Phenylalanine | up | 1.09360 | 0.07453 | 0.66330 |
| Asparagine | down | 0.89989 | 0.18385 | 0.63230 |
| Glutamate | down | 0.79365 | 0.13867 | 0.60400 |
| Tyrosine | up | 1.24649 | 0.00502 | 0.79950 |
| 1,5-Anhydrosorbitol | down | 0.82102 | 0.18459 | 0.67720 |
| Histidine | down | 0.89956 | 0.13950 | 0.65200 |
| Indole-3-acetic acid | up | 1.48325 | 0.04152 | 0.59520 |
| Cystine | up | 1.16226 | 0.14494 | 0.73270 |
| PE_Linoleic acid (C18:cis[9,12]2) | up | 1.21633 | 0.16059 | 0.71280 |
| Dehydroepiandrosterone sulfate | down | 0.46200 | 0.00023 | 0.82850 |
| Testosterone-17-sulfate | down | 0.46200 | 0.00023 | 0.82850 |
| Androsterone sulfate | down | 0.41859 | 0.00009 | 0.84740 |
| Etiocholanolone sulfate | down | 0.41859 | 0.00009 | 0.84740 |
| Dihydrotestosterone sulfate | down | 0.41859 | 0.00009 | 0.84740 |
| Lysophosphatidylcholine (C18:1) | up | 1.10359 | 0.12652 | 0.57250 |
| Phosphatidylcholine (C16:0,C22:6) | down | 0.93171 | 0.01291 | 0.73140 |
| Phosphatidylcholine (C18:2,C20:4) | down | 0.93171 | 0.01291 | 0.73140 |
| Ethanolamine plasmalogen (C39:5) | down | 0.91980 | 0.15760 | 0.75910 |
| Choline plasmalogen (C36:5) | down | 0.91980 | 0.15760 | 0.75910 |
| Phosphatidylcholine (C16:0,C16:0) | up | 1.10846 | 0.03140 | 0.76670 |
| Phosphatidylcholine (C18:0,C20:4) | down | 0.93375 | 0.00230 | 0.75350 |
| DAG (C18:1,C18:2) (Diacyglceride) | down | 0.79171 | 0.10564 | 0.58760 |
| TAG (C18:2,C18:2) (Triacylglyceride) | down | 0.65994 | 0.00394 | 0.64690 |

(continued)

| Table 1a: ANOVA and ROC result of disease advanced (Fib>2) relative to disease mild (Fib<2) | | | | |
|---|---|---|---|---|
| Metabolite | Direction | Estimate fold change | p-Value | Area under the curve |
| TAG (C16:0,C18:2) (Triacylglyceride) | down | 0.81833 | 0.03340 | 0.54160 |
| TAG (C16:0,C18:1,C18:2) (Triacylglyceride) | down | 0.82620 | 0.18626 | 0.51200 |
| TAG (C18:1,C18:2) (Triacylglyceride) | down | 0.82895 | 0.11042 | 0.53850 |
| Lysophosphatidylcholine (C20:4) | down | 0.87620 | 0.08491 | 0.74150 |
| TAG (C18:2,C18:3) (Triacylglyceride) | down | 0.63257 | 0.05023 | 0.61540 |
| Phosphatidylcholine (C16:0,C20:5) | down | 0.90126 | 0.06969 | 0.55490 |
| Phosphatidylcholine (C18:0,C18:1) | up | 1.04280 | 0.14074 | 0.59330 |
| Phosphatidylcholine (C18:0,C20:3) | down | 0.94098 | 0.04992 | 0.65890 |
| Phosphatidylcholine (C20:1,C18:2) | down | 0.94098 | 0.04992 | 0.65890 |
| Phosphatidylcholine (C20:2,C18:1) | down | 0.94098 | 0.04992 | 0.65890 |
| TAG (C16:0,C18:1,C18:3) (Triacylglyceride) | down | 0.66143 | 0.00352 | 0.66080 |
| TAG (C16:0,C18:2,C18:2) (Triacylglyceride) | down | 0.66143 | 0.00352 | 0.66080 |
| TAG (C16:1,C18:1,C18:2) (Triacylglyceride) | down | 0.66143 | 0.00352 | 0.66080 |
| TAG (C18:1,C18:2,C18:3) (Triacylglyceride) | down | 0.85351 | 0.09620 | 0.56870 |
| TAG (C16:0,C18:1,C20:5) (Triacylglyceride) | down | 0.85351 | 0.09620 | 0.56870 |
| TAG (C16:0,C18:2,C20:4) (Triacylglyceride) | down | 0.85351 | 0.09620 | 0.56870 |
| Lysophosphatidylethanolamine (C18:2) | up | 1.22336 | 0.06729 | 0.67720 |
| PC_Myristic acid (C14:0) | up | 1.32368 | 0.05171 | 0.66080 |
| PC_Palmitoleic acid (C16:cis[9]1) | up | 1.19815 | 0.17674 | 0.66710 |
| PC_Stearic acid (C18:0) | up | 1.12098 | 0.13091 | 0.62800 |
| PC_trans-Vaccenic acid (C18:trans[11]1) | up | 2.06813 | 0.02690 | 0.68350 |
| PC_Oleic acid (C18:cis[9]1) | up | 1.29941 | 0.00401 | 0.81840 |
| PC_cis-Vaccenic acid (C18:cis[11]1) | up | 1.13489 | 0.17246 | 0.65570 |
| PC_Linoleic acid (C18:cis[9,12]2) | up | 1.15494 | 0.06984 | 0.63930 |
| PC_Linolenic acid (C18:cis[9,12,15]3) | up | 1.53593 | 0.13268 | 0.63930 |
| PC_Eicoasenoic acid (C20:cis[11]1) | up | 1.34957 | 0.05736 | 0.74020 |
| PC_conjugated Linoleic acid (C18:cis[9]trans[11]2) | up | 1.43311 | 0.19745 | 0.69230 |
| PC_Eicosadienoic acid (C20:cis[11,14]2) | up | 1.16681 | 0.17560 | 0.59020 |
| PC_Arachidonic acid (C20:cis[5,8,11,14]4) | down | 0.82749 | 0.03493 | 0.64940 |
| LPC_Oleic acid (C18:cis[9]1) | up | 2.15730 | 0.14620 | 0.58510 |
| Glycocholic acid | up | 4.70265 | 0.00013 | 0.87270 |
| Taurocholic acid | up | 7.23709 | 0.00000 | 0.88150 |
| SUM.BILE | up | 4.77164 | 0.00002 | 0.8772 |
| SUM.EICOSATIENOIC | up | 1.55428 | 0.00023 | 0.7847 |

**Table 1b: ANOVA and ROC result of advanced (Fib >2) relative to mild (Fib<2)**

| Table 1b: ANOVA and ROC result of advanced (Fib >2) relative to mild (Fib<2) | | | | |
|---|---|---|---|---|
| Metabolite | Direction | Estimate fold change | p-Value | Area under the curve |
| CE_Cholesterylester C12:0 | up | 1.62990 | 0.02449 | 0.63040 |
| CE_Cholesterylester C14:0 | up | 1.30339 | 0.02554 | 0.62800 |
| CE_Cholesterylester C14:1 | up | 1.44801 | 0.05377 | 0.61800 |
| CE_Cholesterylester C22:6 | down | 0.81039 | 0.08748 | 0.66580 |
| CE_Cholesterylester C18:2 | down | 0.90424 | 0.09044 | 0.63350 |
| CE_Cholesterylester C20:4 | down | 0.77287 | 0.00396 | 0.76400 |
| CREATININE_MG_DL (Creatinine) | down | 0.84382 | 0.00110 | 0.76910 |
| Aspartate Aminotransferase (AST) | up | 1.53238 | 0.00114 | 0.82980 |
| Gamma Glutamyltransferase (GGT) | up | 2.16762 | 0.00599 | 0.80830 |
| Bilirubin | up | 1.23831 | 0.19238 | 0.62550 |
| SM_Sphingomyelin (d18:1,C14:0) | up | 1.13730 | 0.13241 | 0.53280 |
| SM_Sphingomyelin (d18:1,C18:0) | down | 0.83356 | 0.01024 | 0.70370 |
| SM_Sphingomyelin (d18:1,C18:1) | down | 0.90618 | 0.13207 | 0.65570 |
| SM_Sphingomyelin (d18:1,C19:0) | down | 0.74060 | 0.00252 | 0.71310 |
| SM_Sphingomyelin (d18:1,C20:0) | down | 0.81976 | 0.00075 | 0.75180 |
| SM_Sphingomyelin (d18:1,C20:1) | down | 0.85644 | 0.08131 | 0.69500 |
| SM_Sphingomyelin (d18:1,C21:0) | down | 0.86631 | 0.03943 | 0.63110 |
| SM_Sphingomyelin (d18:1,C22:0) | down | 0.86673 | 0.05877 | 0.61420 |
| SM_Sphingomyelin (d18:1,C23:0) | down | 0.89701 | 0.14844 | 0.63170 |
| SM_Sphingomyelin (d18:1,C24:1) | down | 0.87994 | 0.09529 | 0.69500 |
| SM_Sphingomyelin (d18:2,C14:0) | up | 1.16588 | 0.11652 | 0.53920 |
| SM_Sphingomyelin (d18:2,C18:0) | down | 0.78940 | 0.00033 | 0.73190 |
| SM_Sphingomyelin (d18:2,C18:1) | down | 0.81846 | 0.01935 | 0.66100 |
| SM_Sphingomyelin (d18:2,C19:0) | down | 0.84217 | 0.09081 | 0.64700 |
| SM_Sphingomyelin (d18:2,C20:0) | down | 0.80405 | 0.00196 | 0.73240 |
| SM_Sphingomyelin (d18:2,C21:0) | down | 0.88143 | 0.19221 | 0.60420 |
| SM_Sphingomyelin (d18:2,C22:0) | down | 0.90888 | 0.10740 | 0.68500 |
| SM_Sphingomyelin (d18:2,C22:1) | down | 0.85955 | 0.09169 | 0.67920 |
| SM_Sphingomyelin (d18:2,C24:0) | down | 0.88160 | 0.07917 | 0.70080 |
| SM_Sphingomyelin (d18:2,C24:1) | down | 0.88662 | 0.14592 | 0.69670 |
| SM_Sphingomyelin (d16:1,C18:0) | down | 0.76909 | 0.00236 | 0.67800 |
| SM_Sphingomyelin (d16:1,C20:0) | down | 0.89328 | 0.11753 | 0.63350 |
| SM_Sphingomyelin (d17:1,C18:0) | down | 0.79916 | 0.00567 | 0.68740 |
| SM_Sphingomyelin (d17:1,C20:0) | down | 0.83201 | 0.01989 | 0.68790 |
| SM_Sphingomyelin (d17:1,C24:1) | down | 0.90550 | 0.14658 | 0.71840 |

(continued)

| Table 1b: ANOVA and ROC result of advanced (Fib >2) relative to mild (Fib<2) | | | | |
|---|---|---|---|---|
| Metabolite | Direction | Estimate fold change | p-Value | Area under the curve |
| SM_Sphingomyelin (d18:1,C18:2) | down | 0.80092 | 0.00537 | 0.68620 |
| SM_Sphingomyelin (d18:1,C22:2) | down | 0.78549 | 0.02562 | 0.63700 |
| SM_Sphingomyelin (d18:2,C17:0) | down | 0.85179 | 0.10194 | 0.63230 |
| SM_Sphingomyelin (d19:1,C18:0) | down | 0.83184 | 0.12330 | 0.56150 |
| Testosterone | up | 1.51145 | 0.03738 | 0.55850 |
| 18-Hydroxycorticosterone | up | 1.29979 | 0.12863 | 0.61070 |
| CER_Ceramide (d18:0,C16:0) | up | 1.27069 | 0.03217 | 0.76760 |
| CER_Ceramide (d18:1,C14:0) | up | 1.15537 | 0.12548 | 0.55800 |
| CER_Ceramide (d18:1,C18:0) | down | 0.87354 | 0.19700 | 0.51520 |
| CER_Ceramide (d18:2,C18:0) | down | 0.82886 | 0.07235 | 0.54860 |
| CER_Ceramide (d16:1,C18:0) | down | 0.84488 | 0.13985 | 0.52220 |
| CER_Ceramide (d17:1,C18:0) | down | 0.85164 | 0.14654 | 0.50590 |
| Glucose-6-phosphate | down | 0.74530 | 0.15784 | 0.54870 |
| Fructose-6-phosphate | down | 0.74530 | 0.15784 | 0.54870 |
| myo-Inositol-1-phosphate | down | 0.74530 | 0.15784 | 0.54870 |
| myo-Inositol-2-phosphate | down | 0.74530 | 0.15784 | 0.54870 |
| myo-Inositol-4-phosphate | down | 0.74530 | 0.15784 | 0.54870 |
| Coenzyme Q9 | up | 1.28730 | 0.15806 | 0.62820 |
| Creatinine | down | 0.84265 | 0.00299 | 0.73950 |
| Creatine | down | 0.74894 | 0.03437 | 0.65160 |
| Cholesterol, free | up | 1.08848 | 0.11260 | 0.58430 |
| Taurochenodeoxycholic acid | up | 4.93976 | 0.00001 | 0.87770 |
| Taurodeoxycholic acid | up | 4.93976 | 0.00001 | 0.87770 |
| Tetradecanoylcarnitine | up | 1.15498 | 0.14735 | 0.54920 |
| Hydroxyhexadecenoylcarnitine | up | 1.25148 | 0.03232 | 0.65810 |
| TAG_Myristic acid (C14:0) | up | 1.60203 | 0.03655 | 0.67770 |
| TAG_Myristoleic acid (C14:cis[9]1) | up | 2.48445 | 0.03442 | 0.69950 |
| TAG_Palmitic acid (C16:0) | up | 1.26544 | 0.14723 | 0.67180 |
| TAG_Hexadecenoic acid (C16:trans[9]1) | up | 2.02732 | 0.09152 | 0.68950 |
| TAG_Stearic acid (C18:0) | up | 1.47192 | 0.03633 | 0.72140 |
| TAG_Elaidic acid (C18:trans[9]1) | up | 2.27872 | 0.13389 | 0.65110 |
| TAG_Oleic acid (C18:cis[9]1) | up | 1.24489 | 0.13297 | 0.67410 |
| TAG_Docosapentaenoic acid (C22:cis[4,7,10,13,16]5) | down | 0.55942 | 0.12871 | 0.54930 |
| conjugated 13-Hydroxylinoleic acid (C18:cis[9]trans[11]2) | up | 1.26860 | 0.07056 | 0.56050 |

(continued)

| Table 1b: ANOVA and ROC result of advanced (Fib >2) relative to mild (Fib<2) | | | | |
|---|---|---|---|---|
| Metabolite | Direction | Estimate fold change | p-Value | Area under the curve |
| conjugated 9-Hydroxylinoleic acid (C18:trans[10]cis [12]2) | up | 1.25792 | 0.10405 | 0.53310 |
| 5-Hydroxyeicosatetraenoic acid (C20:trans[6]cis [8,11,14]4) (5-HETE) | down | 0.74597 | 0.15435 | 0.51740 |
| 14,15-Dihydroxyeicosatrienoic acid (C20:cis[5,8,11]3) | up | 1.52683 | 0.00010 | 0.80610 |
| 11,12-Dihydroxyeicosatrienoic acid (C20:cis[5,8,14]3) | up | 1.49263 | 0.00270 | 0.74980 |
| 8,9-Dihydroxyeicosatrienoic acid (C20:cis[5,11,14]3) | up | 1.99841 | 0.00016 | 0.74750 |
| Arachidonic acid (C20:cis[5,8,11,14]4) | down | 0.76816 | 0.01595 | 0.70840 |
| alpha-Tocopherol | up | 1.22253 | 0.15907 | 0.54560 |
| Myristic acid (C14:0) | up | 1.55776 | 0.05488 | 0.61590 |
| 6-Aminohexanoic acid | down | 0.59133 | 0.00217 | 0.52050 |
| myo-Inositol-2-phosphate, lipid fraction (myo-Inositolphospholipids) | up | 1.44137 | 0.03787 | 0.64290 |
| Behenic acid (C22:0) | down | 0.83445 | 0.05941 | 0.69790 |
| Nervonic acid (C24:cis[15]1) | down | 0.82664 | 0.09108 | 0.80180 |
| 3-O-Methylsphingosine (d18:1) | down | 0.77452 | 0.03744 | 0.74590 |
| 5-O-Methylsphingosine (d18:1) | down | 0.73764 | 0.00573 | 0.77630 |
| Sphingolipids | down | 0.73764 | 0.00573 | 0.77630 |
| threo-Sphingosine (d18:1) | down | 0.73764 | 0.00573 | 0.77630 |
| erythro-Sphingosine (d18:1) (additional: Sphingolipids) | down | 0.78400 | 0.00699 | 0.77050 |
| conjugated Linoleic acid (C18:trans[9,11]2) | up | 1.32369 | 0.09312 | 0.62060 |
| conjugated Linoleic acid (C18:cis[9]trans[11]2) | up | 1.32369 | 0.09312 | 0.62060 |
| myo-Inositol, lipid fraction | up | 1.16717 | 0.14061 | 0.63110 |
| erythro-Dihydrosphingosine (d16:0) | down | 0.80590 | 0.04957 | 0.68790 |
| erythro-Sphingosine-1-phosphate (d18:1) | down | 0.75865 | 0.02698 | 0.75230 |
| 1-Hydroxy-2-amino-(cis,trans)-3,5-octadecadiene (from sphingolipids) | down | 0.75989 | 0.01418 | 0.70650 |
| 5-O-Methylsphingosine (d16:1) | down | 0.83573 | 0.12401 | 0.65980 |
| Alanine | down | 0.90813 | 0.18003 | 0.53220 |
| Glycine | down | 0.86816 | 0.06554 | 0.68270 |
| Methionine | up | 1.09404 | 0.15954 | 0.64230 |
| 5-Oxoproline | down | 0.92650 | 0.15506 | 0.54570 |
| Folic acid | down | 0.92650 | 0.15506 | 0.54570 |
| Cysteine | down | 0.91762 | 0.15041 | 0.62650 |
| Citrate | up | 1.11415 | 0.11196 | 0.67030 |
| Lysine | down | 0.89644 | 0.08619 | 0.65810 |

(continued)

| Table 1b: ANOVA and ROC result of advanced (Fib >2) relative to mild (Fib<2) | | | | |
|---|---|---|---|---|
| **Metabolite** | **Direction** | **Estimate fold change** | **p-Value** | **Area under the curve** |
| Tryptophan | up | 1.09268 | 0.10478 | 0.62530 |
| alpha-Ketoglutarate | up | 1.17143 | 0.18124 | 0.74220 |
| Ornithine | up | 1.11022 | 0.19790 | 0.52220 |
| Arginine | up | 1.11022 | 0.19790 | 0.52220 |
| Citrulline | up | 1.11022 | 0.19790 | 0.52220 |
| Erythrol | down | 0.85505 | 0.18682 | 0.53160 |
| Phenylalanine | up | 1.11667 | 0.01876 | 0.73190 |
| Tyrosine | up | 1.29474 | 0.00053 | 0.84780 |
| Indole-3-acetic acid | up | 1.55631 | 0.01576 | 0.62760 |
| Cystine | up | 1.15726 | 0.12765 | 0.76460 |
| Isocitrate | up | 1.10751 | 0.19554 | 0.75150 |
| PE_Oleic acid (C18:cis[9]1) | up | 2.27760 | 0.05122 | 0.71900 |
| PE_Linoleic acid (C18:cis[9,12]2) | up | 1.28914 | 0.05175 | 0.72610 |
| PE_Docosatetraenoic acid (C22:cis[7,10,13,16]4) | up | 1.42827 | 0.18317 | 0.69100 |
| PE_Docosapentaenoic acid (C22:cis[7,10,13,16,19]5) | up | 1.67775 | 0.16583 | 0.67150 |
| Dehydroepiandrosterone sulfate | down | 0.48897 | 0.00024 | 0.79390 |
| Testosterone-17-sulfate | down | 0.48897 | 0.00024 | 0.79390 |
| Androsterone sulfate | down | 0.50633 | 0.00116 | 0.78860 |
| Etiocholanolone sulfate | down | 0.50633 | 0.00116 | 0.78860 |
| Dihydrotestosterone sulfate | down | 0.50633 | 0.00116 | 0.78860 |
| Lysophosphatidylcholine (C18:1) | up | 1.08260 | 0.18283 | 0.53280 |
| Phosphatidylcholine (C16:0,C22:6) | down | 0.94033 | 0.01986 | 0.71870 |
| Phosphatidylcholine (C18:2,C20:4) | down | 0.94033 | 0.01986 | 0.71870 |
| Ethanolamine plasmalogen (C39:5) | down | 0.91966 | 0.12868 | 0.74180 |
| Choline plasmalogen (C36:5) | down | 0.91966 | 0.12868 | 0.74180 |
| Phosphatidylcholine (C16:0,C16:0) | up | 1.08261 | 0.07565 | 0.69610 |
| Phosphatidylcholine (C18:0,C20:4) | down | 0.94050 | 0.00341 | 0.72800 |
| TAG (C18:2,C18:2) (Triacylglyceride) | down | 0.75235 | 0.03712 | 0.55800 |
| Lysophosphatidylcholine (C20:4) | down | 0.87671 | 0.06674 | 0.70140 |
| Phosphatidylcholine (C16:0,C20:5) | down | 0.91612 | 0.09989 | 0.56150 |
| Phosphatidylcholine (C18:0,C18:1) | up | 1.04315 | 0.10924 | 0.57440 |
| TAG (C16:0,C18:1,C18:3) (Triacylglyceride) | down | 0.73556 | 0.02118 | 0.57670 |
| TAG (C16:0,C18:2,C18:2) (Triacylglyceride) | down | 0.73556 | 0.02118 | 0.57670 |
| TAG (C16:1,C18:1,C18:2) (Triacylglyceride) | down | 0.73556 | 0.02118 | 0.57670 |
| Lysophosphatidylethanolamine (C18:2) | up | 1.22371 | 0.04840 | 0.65370 |

(continued)

| Table 1b: ANOVA and ROC result of advanced (Fib >2) relative to mild (Fib<2) | | | | |
|---|---|---|---|---|
| Metabolite | Direction | Estimate fold change | p-Value | Area under the curve |
| PC_Myristic acid (C14:0) | up | 1.37705 | 0.01718 | 0.63520 |
| PC_Palmitoleic acid (C16:cis[9]1) | up | 1.22389 | 0.10601 | 0.66630 |
| PC_Stearic acid (C18:0) | up | 1.18158 | 0.02021 | 0.69320 |
| PC_trans-Vaccenic acid (C18:trans[11]1) | up | 2.00310 | 0.02302 | 0.64870 |
| PC_Oleic acid (C18:cis[9]1) | up | 1.31962 | 0.00107 | 0.81560 |
| PC_Linoleic acid (C18:cis[9,12]2) | up | 1.14867 | 0.06001 | 0.60300 |
| PC_Linolenic acid (C18:cis[9,12,15]3) | up | 1.54180 | 0.10467 | 0.60690 |
| PC_Eicoasenoic acid (C20:cis[11]1) | up | 1.31029 | 0.06485 | 0.71900 |
| PC_conjugated Linoleic acid (C18:cis[9]trans[11]2) | up | 1.42629 | 0.17141 | 0.67510 |
| PC_Eicosadienoic acid (C20:cis[11,14]2) | up | 1.17150 | 0.13616 | 0.55210 |
| PC_Arachidonic acid (C20:cis[5,8,11,14]4) | down | 0.86719 | 0.08969 | 0.60950 |
| Glycocholic acid | up | 4.92410 | 0.00003 | 0.88980 |
| Taurocholic acid | up | 6.68852 | 0.00000 | 0.88580 |
| SUM.BILE | up | 4.92330 | 0.000004 | 0.8938 |
| SUM.EICOSATIENOIC | up | 1.57565 | 0.000059 | 0.8028 |

Table 1c: ANOVA and ROC result of NASH advanced (Fib >2) relative to NASH mild (Fib<2)

| Table 1c: ANOVA and ROC result of NASH advanced (Fib >2) relative to NASH mild (Fib<2) | | | | |
|---|---|---|---|---|
| Metabolite | Direction | Estimate fold change | p-Value | Area under the curve |
| Urea | down | 0.75221 | 0.03068 | 0.75490 |
| Leucocyte | down | 0.83663 | 0.08803 | 0.59210 |
| CE_Cholesterylester C18:0 | down | 0.84229 | 0.17770 | 0.66670 |
| CE_Cholesterylester C20:3 | down | 0.78636 | 0.06167 | 0.72500 |
| CE_Cholesterylester C20:5 | down | 0.75995 | 0.19175 | 0.67080 |
| CE_Cholesterylester C22:4 | down | 0.77475 | 0.07897 | 0.64580 |
| CE_Cholesterylester C22:6 | down | 0.71211 | 0.02729 | 0.74580 |
| CE_Cholesterylester C18:1 | down | 0.88199 | 0.16598 | 0.63330 |
| CE_Cholesterylester C18:2 | down | 0.86530 | 0.05721 | 0.67920 |
| CE_Cholesterylester C20:4 | down | 0.69362 | 0.00111 | 0.77080 |
| Glucose | down | 0.92365 | 0.16976 | 0.56620 |
| Creatinine | down | 0.83522 | 0.00514 | 0.77370 |
| Aspartate Aminotransferase (AST) | up | 1.44088 | 0.01361 | 0.80420 |
| Gamma Glutamyltransferase (GGT) | up | 1.78360 | 0.05081 | 0.74790 |
| FFA_Elaidic acid (C18:trans[9]1) | up | 1.57519 | 0.07313 | 0.72920 |

(continued)

| Table 1c: ANOVA and ROC result of NASH advanced (Fib >2) relative to NASH mild (Fib<2) | | | | |
|---|---|---|---|---|
| Metabolite | Direction | Estimate fold change | p-Value | Area under the curve |
| FFA_Arachidonic acid (C20:cis[5,8,11,14]4) | down | 0.72703 | 0.10459 | 0.59170 |
| FFA_Docosahexaenoic acid (C22:cis [4,7,10,13,16,19]6) | down | 0.40053 | 0.18624 | 0.59170 |
| SM_Sphingomyelin (d18:1,C14:0) | up | 1.15012 | 0.19625 | 0.53330 |
| SM_Sphingomyelin (d18:1,C18:0) | down | 0.74292 | 0.00069 | 0.80420 |
| SM_Sphingomyelin (d18:1,C18:1) | down | 0.87004 | 0.08629 | 0.70000 |
| SM_Sphingomyelin (d18:1,C19:0) | down | 0.69881 | 0.00401 | 0.75830 |
| SM_Sphingomyelin (d18:1,C20:0) | down | 0.74781 | 0.00007 | 0.84170 |
| SM_Sphingomyelin (d18:1,C20:1) | down | 0.84310 | 0.12795 | 0.71250 |
| SM_Sphingomyelin (d18:1,C21:0) | down | 0.80391 | 0.01379 | 0.68750 |
| SM_Sphingomyelin (d18:1,C22:0) | down | 0.80196 | 0.01901 | 0.68750 |
| SM_Sphingomyelin (d18:1,C23:0) | down | 0.85186 | 0.09339 | 0.68330 |
| SM_Sphingomyelin (d18:1,C24:0) | down | 0.87041 | 0.15525 | 0.64170 |
| SM_Sphingomyelin (d18:1,C24:1) | down | 0.86191 | 0.11015 | 0.72920 |
| SM_Sphingomyelin (d18:2,C14:0) | up | 1.17321 | 0.19939 | 0.51250 |
| SM_Sphingomyelin (d18:2,C18:0) | down | 0.73535 | 0.00019 | 0.81670 |
| SM_Sphingomyelin (d18:2,C18:1) | down | 0.79608 | 0.03091 | 0.72920 |
| SM_Sphingomyelin (d18:2,C20:0) | down | 0.76925 | 0.00278 | 0.77080 |
| SM_Sphingomyelin (d18:2,C22:0) | down | 0.88989 | 0.10794 | 0.70830 |
| SM_Sphingomyelin (d18:2,C24:0) | down | 0.89564 | 0.19868 | 0.67500 |
| SM_Sphingomyelin (d16:1,C18:0) | down | 0.69986 | 0.00113 | 0.75830 |
| SM_Sphingomyelin (d17:1,C18:0) | down | 0.73083 | 0.00223 | 0.77080 |
| SM_Sphingomyelin (d17:1,C20:0) | down | 0.79035 | 0.01950 | 0.75000 |
| SM_Sphingomyelin (d18:1,C18:2) | down | 0.78043 | 0.01220 | 0.72500 |
| SM_Sphingomyelin (d18:1,C22:2) | down | 0.80861 | 0.12167 | 0.60830 |
| SM_Sphingomyelin (d18:2,C17:0) | down | 0.79260 | 0.05841 | 0.67920 |
| SM_Sphingomyelin (d19:1,C18:0) | down | 0.75742 | 0.06484 | 0.66670 |
| Testosterone | up | 1.53130 | 0.08646 | 0.60090 |
| 18-Hydroxycorticosterone | up | 1.41224 | 0.11272 | 0.70420 |
| CER_Ceramide (d18:0,C18:0) | down | 0.77183 | 0.17131 | 0.55420 |
| CER_Ceramide (d18:1,C18:0) | down | 0.71537 | 0.00783 | 0.69170 |
| CER_Ceramide (d18:1,C19:0) | down | 0.82352 | 0.11764 | 0.65420 |
| CER_Ceramide (d18:1,C20:0) | down | 0.78562 | 0.03412 | 0.52500 |
| CER_Ceramide (d18:1,C21:0) | down | 0.80945 | 0.10120 | 0.61250 |
| CER_Ceramide (d18:1,C22:1) | down | 0.80283 | 0.15019 | 0.52920 |
| CER_Ceramide (d18:2,C18:0) | down | 0.72573 | 0.01479 | 0.68750 |

(continued)

| Table 1c: ANOVA and ROC result of NASH advanced (Fib >2) relative to NASH mild (Fib<2) | | | | |
|---|---|---|---|---|
| Metabolite | Direction | Estimate fold change | p-Value | Area under the curve |
| CER_Ceramide (d18:2,C20:0) | down | 0.84140 | 0.19425 | 0.53750 |
| CER_Ceramide (d16:1,C18:0) | down | 0.74353 | 0.03463 | 0.66670 |
| CER_Ceramide (d17:1,C18:0) | down | 0.71890 | 0.01330 | 0.71250 |
| CER_Ceramide (d17:1,C20:0) | down | 0.83675 | 0.18102 | 0.52920 |
| CER_Ceramide (d18:1,C22:0) | down | 0.86220 | 0.19981 | 0.55000 |
| Creatinine | down | 0.84271 | 0.01926 | 0.73750 |
| Creatine | down | 0.69965 | 0.03320 | 0.72500 |
| Taurochenodeoxycholic acid | up | 3.77552 | 0.00223 | 0.85960 |
| Taurodeoxycholic acid | up | 3.77552 | 0.00223 | 0.85960 |
| TAG_Linoleic acid (C18:cis[9,12]2) | down | 0.70395 | 0.08886 | 0.61400 |
| TAG_gamma-Linolenic acid (C18:cis[6,9,12]3) | down | 0.44137 | 0.05610 | 0.58770 |
| TAG_Arachidonic acid (C20:cis[5,8,11,14]4) | down | 0.67383 | 0.05645 | 0.57020 |
| TAG_Docosapentaenoic acid (C22:cis[4,7,10,13,16]5) | down | 0.50191 | 0.15464 | 0.56800 |
| conjugated 13-Hydroxylinoleic acid (C18:cis[9]trans[11]2) | up | 1.27295 | 0.12986 | 0.63640 |
| 14,15-Dihydroxyeicosatrienoic acid (C20:cis[5,8,11]3) | up | 1.48760 | 0.00203 | 0.85000 |
| 11,12-Dihydroxyeicosatrienoic acid (C20:cis[5,8,14]3) | up | 1.53158 | 0.00964 | 0.75230 |
| 8,9-Dihydroxyeicosatrienoic acid (C20:cis[5,11,14]3) | up | 1.79929 | 0.00781 | 0.71820 |
| Glycerol, lipid fraction | down | 0.68174 | 0.14304 | 0.61250 |
| Arachidonic acid (C20:cis[5,8,11,14]4) | down | 0.65340 | 0.00167 | 0.78330 |
| Docosahexaenoic acid (C22:cis[4,7,10,13,16,19]6) | down | 0.68886 | 0.04286 | 0.71670 |
| Cholesterol, total | down | 0.78928 | 0.11962 | 0.67110 |
| Lignoceric acid (C24:0) | down | 0.82417 | 0.16172 | 0.68750 |
| Eicosanoic acid (C20:0) | down | 0.72600 | 0.01019 | 0.75000 |
| Tricosanoic acid (C23:0) | down | 0.80598 | 0.13291 | 0.70830 |
| 6-Aminohexanoic acid | down | 0.69562 | 0.05907 | 0.55420 |
| Behenic acid (C22:0) | down | 0.75041 | 0.01417 | 0.74170 |
| Nervonic acid (C24:cis[15]1) | down | 0.80834 | 0.13363 | 0.76670 |
| Urea | down | 0.77724 | 0.03193 | 0.67080 |
| gamma-Linolenic acid (C18:cis[6,9,12]3) | down | 0.63994 | 0.07731 | 0.65420 |
| dihomo-gamma-Linolenic acid (C20:cis[8,11,14]3) | down | 0.75798 | 0.05151 | 0.69580 |
| 3-O-Methylsphingosine (d18:1) | down | 0.71880 | 0.03171 | 0.74580 |
| threo-Sphingosine (d18:1) | down | 0.86049 | 0.19110 | 0.67920 |
| 5-O-Methylsphingosine (d18:1) | down | 0.66635 | 0.00328 | 0.79170 |

(continued)

| Table 1c: ANOVA and ROC result of NASH advanced (Fib >2) relative to NASH mild (Fib<2) | | | | |
|---|---|---|---|---|
| Metabolite | Direction | Estimate fold change | p-Value | Area under the curve |
| Sphingolipids | down | 0.66635 | 0.00328 | 0.79170 |
| erythro-Sphingosine (d18:1) | down | 0.73407 | 0.00622 | 0.78750 |
| Cholestenol No 02 | down | 0.79418 | 0.06189 | 0.72500 |
| erythro-Dihydrosphingosine (d16:0) | down | 0.76858 | 0.05456 | 0.70830 |
| erythro-Sphingosine-1-phosphate (d18:1) | down | 0.70496 | 0.02611 | 0.74580 |
| 1-Hydroxy-2-amino-(cis,trans)-3,5-octadecadiene (from sphingolipids) | down | 0.68835 | 0.00821 | 0.73680 |
| 5-O-Methylsphingosine (d16:1) | down | 0.78024 | 0.08954 | 0.70000 |
| Cysteine | down | 0.87665 | 0.07946 | 0.58750 |
| Lysine | down | 0.80944 | 0.00509 | 0.84580 |
| Tryptophan | up | 1.09967 | 0.16320 | 0.66250 |
| Erythrol | down | 0.66914 | 0.00598 | 0.57080 |
| Aspartate | down | 0.77561 | 0.19201 | 0.55910 |
| Phenylalanine | up | 1.10174 | 0.09828 | 0.74580 |
| Tyrosine | up | 1.29667 | 0.00500 | 0.84580 |
| 1,5-Anhydrosorbitol | down | 0.68247 | 0.02733 | 0.75000 |
| scyllo-Inositol | down | 0.76935 | 0.15842 | 0.50880 |
| Erythronic acid | down | 0.85669 | 0.07351 | 0.53120 |
| PE_Docosatetraenoic acid (C22:cis[7,10,13,16]4) | up | 1.54412 | 0.19369 | 0.66670 |
| Dehydroepiandrosterone sulfate | down | 0.42151 | 0.00049 | 0.81250 |
| Testosterone-17-sulfate | down | 0.42151 | 0.00049 | 0.81250 |
| Hippuric acid | up | 1.76553 | 0.14634 | 0.62500 |
| Androsterone sulfate | down | 0.45927 | 0.00269 | 0.80000 |
| Etiocholanolone sulfate | down | 0.45927 | 0.00269 | 0.80000 |
| Dihydrotestosterone sulfate | down | 0.45927 | 0.00269 | 0.80000 |
| Lysophosphatidylcholine (C18:1) | up | 1.11064 | 0.16815 | 0.60830 |
| Phosphatidylcholine (C16:0,C22:6) | down | 0.93524 | 0.04387 | 0.69580 |
| Phosphatidylcholine (C18:2,C20:4) | down | 0.93524 | 0.04387 | 0.69580 |
| Ethanolamine plasmalogen (C39:5) | down | 0.91180 | 0.18122 | 0.73750 |
| Choline plasmalogen (C36:5) | down | 0.91180 | 0.18122 | 0.73750 |
| Phosphatidylcholine (C16:0,C16:0) | up | 1.08655 | 0.13653 | 0.71670 |
| Phosphatidylcholine (C18:0,C20:4) | down | 0.91727 | 0.00120 | 0.81670 |
| DAG (C18:1,C18:2) | down | 0.70524 | 0.03894 | 0.60830 |
| TAG (C18:2,C18:2) (Triacylglyceride) | down | 0.60685 | 0.00291 | 0.69580 |
| TAG (C16:0,C18:2) (Triacylglyceride) | down | 0.78975 | 0.03249 | 0.53540 |
| TAG (C16:0,C18:1,C18:2) (Triacylglyceride) | down | 0.73817 | 0.07157 | 0.56670 |

(continued)

| Table 1c: ANOVA and ROC result of NASH advanced (Fib >2) relative to NASH mild (Fib<2) | | | | |
|---|---|---|---|---|
| Metabolite | Direction | Estimate fold change | p-Value | Area under the curve |
| TAG (C18:1,C18:2) (Triacylglyceride) | down | 0.78376 | 0.07750 | 0.57080 |
| TAG (C18:2,C18:3) (Triacylglyceride) | down | 0.53100 | 0.01992 | 0.65420 |
| Phosphatidylcholine (C16:0,C20:5) | down | 0.88844 | 0.07701 | 0.53750 |
| Phosphatidylcholine (C18:0,C18:1) | up | 1.04963 | 0.14113 | 0.60630 |
| Phosphatidylcholine (C18:0,C20:3) | down | 0.90396 | 0.00573 | 0.77080 |
| Phosphatidylcholine (C20:1,C18:2) | down | 0.90396 | 0.00573 | 0.77080 |
| Phosphatidylcholine (C20:2,C18:1) | down | 0.90396 | 0.00573 | 0.77080 |
| TAG (C16:0,C18:1,C18:3) (Triacylglyceride) | down | 0.61427 | 0.00335 | 0.68750 |
| TAG (C16:0,C18:2,C18:2) (Triacylglyceride) | down | 0.61427 | 0.00335 | 0.68750 |
| TAG (C16:1,C18:1,C18:2) (Triacylglyceride) | down | 0.61427 | 0.00335 | 0.68750 |
| TAG (C18:1,C18:2,C18:3) (Triacylglyceride) | down | 0.81612 | 0.06775 | 0.60000 |
| TAG (C16:0,C18:1,C20:5) (Triacylglyceride) | down | 0.81612 | 0.06775 | 0.60000 |
| TAG (C16:0,C18:2,C20:4) (Triacylglyceride) | down | 0.81612 | 0.06775 | 0.60000 |
| PC_trans-Vaccenic acid (C18:trans[11]1) | up | 2.14354 | 0.04429 | 0.65000 |
| PC_Oleic acid (C18:cis[9]1) | up | 1.20777 | 0.07106 | 0.73330 |
| PC_Eicoasenoic acid (C20:cis[11]1) | up | 1.26941 | 0.19616 | 0.70830 |
| PC_Arachidonic acid (C20:cis[5,8,11,14]4) | down | 0.78666 | 0.02393 | 0.66250 |
| LPC_Oleic acid (C18:cis[9]1) | up | 2.54520 | 0.13669 | 0.62500 |
| Glycocholic acid | up | 4.48348 | 0.00154 | 0.83330 |
| Taurocholic acid | up | 6.14009 | 0.00016 | 0.85420 |
| SUM.BILE | up | 3.98811 | 0.00109 | 0.8553 |
| SUM.EICOSATIENOIC | up | 1.51089 | 0.00212 | 0.8136 |

**Table 2a_1: Multimarker panel composition and their respective Area under the curve (AUC) values of receiver operating characteristics (ROC) analysis**

| Panel Number | Panel Composition long | No of Metabolites | Positive Group | Negative Group | AUC |
|---|---|---|---|---|---|
| AEB_1_1 | Androsterone sulfate; Glycocholic acid; 14,15-Dihydroxyeicosatrienoic acid (C20:cis[5,8,11]3) | 3 | Dis_adv (Fib >2) | Dis_mild (Fib <2) | 0.95082 |
| AEB_2_1 | Androsterone sulfate; SUM.BILE; 14,15-Dihydroxyeicosatrienoic acid (C20:cis[5,8,11]3) | 3 | Dis_adv (Fib >2) | Dis_mild (Fib <2) | 0.94451 |
| AEB_3_1 | Androsterone sulfate; SUM.BILE; SUM.EICOSATIENOIC | 3 | Dis_adv (Fib >2) | Dis_mild (Fib <2) | 0.92055 |
| AEB_4_1 | Androsterone sulfate; Taurochenodeoxycholic acid; 8,9-Dihydroxyeicosatrienoic acid (C20:cis [5,11,14]3) | 3 | Dis_adv (Fib >2) | Dis_mild (Fib <2) | 0.91929 |
| AEB_4a_1 | Androsterone sulfate; Taurodeoxycholic acid; 8,9-Dihydroxyeicosatrienoic acid (C20:cis[5,11,14]3) | 3 | Dis_adv (Fib >2) | Dis_mild (Fib <2) | 0.91929 |

(continued)

| Panel Number | Panel Composition long | No of Metabolites | Positive Group | Negative Group | AUC |
|---|---|---|---|---|---|
| AEB_ 5_1 | Androsterone sulfate; SUM.BILE; 11,12-Dihydroxyeicosatrienoic acid (C20:cis[5,8,14]3) | 3 | Dis_adv (Fib >2) | Dis_mild (Fib <2) | 0.9192 9 |
| AEB_ 6_1 | Androsterone sulfate; SUM.BILE; 8,9-Dihydroxyeicosatrienoic acid (C20:cis[5,11,14]3) | 3 | Dis_adv (Fib >2) | Dis_mild (Fib <2) | 0.9180 3 |
| AEB_ 7_1 | Androsterone sulfate; Taurochenodeoxycholic acid ; SUM.EICOSATIENOIC | 3 | Dis_adv (Fib >2) | Dis_mild (Fib <2) | 0.9180 3 |
| AEB_ 7a_1 | Androsterone sulfate; Taurodeoxycholic acid; SUM.EICOSATIENOIC | 3 | Dis_adv (Fib >2) | Dis_mild (Fib <2) | 0.9180 3 |
| AEB_ 8_1 | Androsterone sulfate; Taurochenodeoxycholic acid; 14,15-Dihydroxyeicosatrienoic acid (C20:cis [5,8,11]3) | 3 | Dis_adv (Fib >2) | Dis_mild (Fib <2) | 0.9167 7 |
| AEB_ 8a_1 | Androsterone sulfate; Taurodeoxycholic acid; 14,15-Dihydroxyeicosatrienoic acid (C20:cis [5,8,11]3) | 3 | Dis_adv (Fib >2) | Dis_mild (Fib <2) | 0.9167 7 |
| AEB_ 9_1 | Androsterone sulfate; Taurocholic acid; 14,15-Dihydroxyeicosatrienoic acid (C20:cis[5,8,11]3) | 3 | Dis_adv (Fib >2) | Dis_mild (Fib <2) | 0.9129 9 |
| AEB_ 10_1 | Androsterone sulfate; Taurochenodeoxy-cholic acid; 11,12-Dihydroxyeicosatrienoic acid (C20:cis [5,8,14]3) | 3 | Dis_adv (Fib >2) | Dis_mild (Fib <2) | 0.9104 7 |
| AEB_ 10a_1 | Androsterone sulfate; Taurodeoxycholic acid; 11,12-Dihydroxyeicosatrienoic acid (C20:cis [5,8,14]3) | 3 | Dis_adv (Fib >2) | Dis_mild (Fib <2) | 0.9104 7 |
| AEB_ 11_1 | Androsterone sulfate; Taurocholic acid; SUM.EICOSATIENOIC | 3 | Dis_adv (Fib >2) | Dis_mild (Fib <2) | 0.9029 0 |
| AEB_ 12_1 | Androsterone sulfate; Glycocholic acid; 11,12-Dihydroxyeicosatrienoic acid (C20:cis[5,8,14]3) | 3 | Dis_adv (Fib >2) | Dis_mild (Fib <2) | 0.8991 2 |
| AEB_ 13_1 | Androsterone sulfate; Taurocholic acid; 8,9-Dihydroxyeicosatrienoic acid (C20:cis[5,11,14]3) | 3 | Dis_adv (Fib >2) | Dis_mild (Fib <2) | 0.8966 0 |
| AEB_ 14_1 | Androsterone sulfate; Taurocholic acid; 11,12-Dihydroxyeicosatrienoic acid (C20:cis[5,8,14]3) | 3 | Dis_adv (Fib >2) | Dis_mild (Fib <2) | 0.8940 7 |
| AEB_ 15_1 | Androsterone sulfate; Glycocholic acid; SUM.EICOSATIENOIC | 3 | Dis_adv (Fib >2) | Dis_mild (Fib <2) | 0.8890 3 |
| AEB_ 16_1 | Androsterone sulfate; Glycocholic acid; 8,9-Dihydroxyeicosatrienoic acid (C20:cis[5,11,14]3) | 3 | Dis_adv (Fib >2) | Dis_mild (Fib <2) | 0.8713 7 |
| AEA_ 17_1 | Androsterone sulfate; 14,15-Dihydroxyeicosatrienoic acid (C20:cis[5,8,11]3); Creatine | 3 | Dis_adv (Fib >2) | Dis_mild (Fib <2) | 0.9407 3 |
| AEA_ 18_1 | Androsterone sulfate; 14,15-Dihydroxyeicosatrienoic acid (C20:cis[5,8,11]3); Cystine | 3 | Dis_adv (Fib >2) | Dis_mild (Fib <2) | 0.9243 4 |
| AEA_ 19_1 | Androsterone sulfate; 14,15-Dihydroxyeicosatrienoic acid (C20:cis[5,8,11]3); Tyrosine | 3 | Dis_adv (Fib >2) | Dis_mild (Fib <2) | 0.8940 7 |
| AEA_ 20_1 | Androsterone sulfate; Lysine; 14,15-Dihydroxyeicosatrienoic acid (C20:cis[5,8,11]3) | 3 | Dis_adv (Fib >2) | Dis_mild (Fib <2) | 0.8940 7 |

(continued)

| Panel Number | Panel Composition long | No of Metabolites | Positive Group | Negative Group | AUC |
|---|---|---|---|---|---|
| AEA_21_1 | Androsterone sulfate; SUM.EICOSATIENOIC; Creatine | 3 | Dis_adv (Fib >2) | Dis_mild (Fib <2) | 0.88903 |
| AEA_22_1 | Androsterone sulfate; 11,12-Dihydroxyeicosatrienoic acid (C20:cis[5,8,14]3); Creatine | 3 | Dis_adv (Fib >2) | Dis_mild (Fib <2) | 0.88272 |
| AEA_23_1 | Androsterone sulfate; SUM.EICOSATIENOIC; Cystine | 3 | Dis_adv (Fib >2) | Dis_mild (Fib <2) | 0.87516 |
| AEA_24_1 | Androsterone sulfate; 14,15-Dihydroxyeicosatrienoic acid (C20:cis[5,8,11]3); Phenylalanine | 3 | Dis_adv (Fib >2) | Dis_mild (Fib <2) | 0.86885 |
| AEA_25_1 | Androsterone sulfate; 14,15-Dihydroxyeicosatrienoic acid (C20:cis[5,8,11]3); Asparagine | 3 | Dis_adv (Fib >2) | Dis_mild (Fib <2) | 0.86759 |
| AEA_26_1 | Androsterone sulfate; 14,15-Dihydroxyeicosatrienoic acid (C20:cis[5,8,11]3); Creatinine | 3 | Dis_adv (Fib >2) | Dis_mild (Fib <2) | 0.86381 |
| AEA_27_1 | Androsterone sulfate; 14,15-Dihydroxyeicosatrienoic acid (C20:cis[5,8,11]3); Arginine | 3 | Dis_adv (Fib >2) | Dis_mild (Fib <2) | 0.86003 |
| AEA_28_1 | Androsterone sulfate; Lysine; 11,12-Dihydroxyeicosatrienoic acid (C20:cis[5,8,14]3) | 3 | Dis_adv (Fib >2) | Dis_mild (Fib <2) | 0.86003 |
| AEA_29_1 | Androsterone sulfate; 14,15-Dihydroxyeicosatrienoic acid (C20:cis[5,8,11]3); Glutamine | 3 | Dis_adv (Fib >2) | Dis_mild (Fib <2) | 0.85624 |
| AEA_30_1 | Androsterone sulfate; Tyrosine; SUM.EICOSATIENOIC | 3 | Dis_adv (Fib >2) | Dis_mild (Fib <2) | 0.84868 |
| AEA_31_1 | Androsterone sulfate; 14,15-Dihydroxyeicosatrienoic acid (C20:cis[5,8,11]3); Tryptophan | 3 | Dis_adv (Fib >2) | Dis_mild (Fib <2) | 0.84615 |
| AEE_32_1 | Androsterone sulfate; 14,15-Dihydroxyeicosatrienoic acid (C20:cis[5,8,11]3); SUM.EICOSATIENOIC | 3 | Dis_adv (Fib >2) | Dis_mild (Fib <2) | 0.82850 |
| AEA_33_1 | Androsterone sulfate; Cystine; 11,12-Dihydroxyeicosatrienoic acid (C20:cis[5,8,14]3) | 3 | Dis_adv (Fib >2) | Dis_mild (Fib <2) | 0.84615 |
| AEE_34_1 | Androsterone sulfate; 14,15-Dihydroxyeicosatrienoic acid (C20:cis[5,8,11]3); SUM.EICOSATIENOIC | 3 | Dis_adv (Fib >2) | Dis_mild (Fib <2) | 0.82850 |
| AEA_35_1 | Androsterone sulfate; 14,15-Dihydroxyeicosatrienoic acid (C20:cis[5,8,11]3); Urea | 3 | Dis_adv (Fib >2) | Dis_mild (Fib <2) | 0.83607 |
| AEA_36_1 | Androsterone sulfate; Creatinine; 11,12-Dihydroxyeicosatrienoic acid (C20:cis[5,8,14]3) | 3 | Dis_adv (Fib >2) | Dis_mild (Fib <2) | 0.82724 |
| AEA_37_1 | Androsterone sulfate; 14,15-Dihydroxyeicosatrienoic acid (C20:cis[5,8,11]3); Histidine | 3 | Dis_adv (Fib >2) | Dis_mild (Fib <2) | 0.82598 |

(continued)

| Panel Number | Panel Composition long | No of Metabolites | Positive Group | Negative Group | AUC |
|---|---|---|---|---|---|
| AEA_ 38_1 | Androsterone sulfate; Lysine; SUM.EICOSATIENOIC | 3 | Dis_adv (Fib >2) | Dis_mild (Fib <2) | 0.8234 6 |
| AEA_ 39_1 | Androsterone sulfate; Tyrosine; 11,12-Dihydroxyeicosatrienoic acid (C20:cis[5,8,14]3) | 3 | Dis_adv (Fib >2) | Dis_mild (Fib <2) | 0.8171 5 |
| AEL_ 40_1 | Androsterone sulfate; CE_Cholesterylester C20:4; 14,15-Dihydroxyeicosatrienoic acid (C20:cis [5,8,11]3) | 3 | Dis_adv (Fib >2) | Dis_mild (Fib <2) | 0.9180 3 |
| AEL_ 41_1 | Androsterone sulfate; 14,15-Dihydroxyeicosatrienoic acid (C20:cis[5,8,11]3); SM_Sphingomyelin (d18:2,C20:0) | 3 | Dis_adv (Fib >2) | Dis_mild (Fib <2) | 0.9054 2 |
| AEL_ 42_1 | Androsterone sulfate; 14,15-Dihydroxyeicosatrienoic acid (C20:cis[5,8,11]3); SM_Sphingomyelin (d18:2,C18:0) | 3 | Dis_adv (Fib >2) | Dis_mild (Fib <2) | 0.9016 4 |
| AEL_ 43_1 | Androsterone sulfate; CE_Cholesterylester C20:4; SUM.EICOSATIENOIC | 3 | Dis_adv (Fib >2) | Dis_mild (Fib <2) | 0.8839 8 |
| AEL_ 44_1 | Androsterone sulfate; 14,15-Dihydroxyeicosatrienoic acid (C20:cis[5,8,11]3); Ethanolamine plasmalogen (C39:5) | 3 | Dis_adv (Fib >2) | Dis_mild (Fib <2) | 0.8827 2 |
| AEL_ 44a_1 | Androsterone sulfate; 14,15-Dihydroxyeicosatrienoic acid (C20:cis[5,8,11]3); Choline plasmalogen (C36:5) | 3 | Dis_adv (Fib >2) | Dis_mild (Fib <2) | 0.8827 2 |
| AEL_ 45_1 | Androsterone sulfate; 14,15-Dihydroxyeicosatrienoic acid (C20:cis[5,8,11]3); Phosphatidylcholine (C18:0,C20:4) | 3 | Dis_adv (Fib >2) | Dis_mild (Fib <2) | 0.8726 4 |
| AEL_ 46_1 | Androsterone sulfate; CE_Cholesterylester C20:4; 11,12-Dihydroxyeicosatrienoic acid (C20:cis [5,8,14]3) | 3 | Dis_adv (Fib >2) | Dis_mild (Fib <2) | 0.8675 9 |
| AEL_ 47_1 | Androsterone sulfate; SUM.EICOSATIENOIC; SM_Sphingomyelin (d18:2,C20:0) | 3 | Dis_adv (Fib >2) | Dis_mild (Fib <2) | 0.8600 3 |
| AEL_ 48_1 | Androsterone sulfate; SM_Sphingomyelin (d18: 2,C18:0); SUM.EICOSATIENOIC | 3 | Dis_adv (Fib >2) | Dis_mild (Fib <2) | 0.8537 2 |
| AEL_ 49_1 | Androsterone sulfate; 14,15-Dihydroxyeicosatrienoic acid (C20:cis[5,8,11]3); total triacylglyceride | 3 | Dis_adv (Fib >2) | Dis_mild (Fib <2) | 0.8448 9 |
| AEL_ 50_1 | Androsterone sulfate; 14,15-Dihydroxyeicosatrienoic acid (C20:cis[5,8,11]3); Ethanolamine plasmalogen (C39:4) | 3 | Dis_adv (Fib >2) | Dis_mild (Fib <2) | 0.8411 1 |
| AEL_ 50a_1 | Androsterone sulfate; 14,15-Dihydroxyeicosatrienoic acid (C20:cis[5,8,11]3); Choline plasmalogen (C36:4) | 3 | Dis_adv (Fib >2) | Dis_mild (Fib <2) | 0.8411 1 |
| AEL_ 51_1 | Androsterone sulfate; 14,15-Dihydroxyeicosatrienoic acid (C20:cis[5,8,11]3); total cholesterol | 3 | Dis_adv (Fib >2) | Dis_mild (Fib <2) | 0.8398 5 |
| AEL_ 52_1 | Androsterone sulfate; SM_Sphingomyelin (d18: 2,C18:0); 11,12-Dihydroxyeicosatrienoic acid (C20:cis[5,8,14]3) | 3 | Dis_adv (Fib >2) | Dis_mild (Fib <2) | 0.8398 5 |

(continued)

| Panel Number | Panel Composition long | No of Metabolites | Positive Group | Negative Group | AUC |
|---|---|---|---|---|---|
| AEL_53_1 | Androsterone sulfate; 14,15-Dihydroxyeicosatrienoic acid (C20:cis[5,8,11]3); SM_Sphingomyelin (d18:1,C18:0) | 3 | Dis_adv (Fib >2) | Dis_mild (Fib <2) | 0.83985 |
| AEL_54_1 | Androsterone sulfate; 11,12-Dihydroxyeicosatrienoic acid (C20:cis[5,8,14]3); SM_Sphingomyelin (d18:2,C20:0) | 3 | Dis_adv (Fib >2) | Dis_mild (Fib <2) | 0.83733 |
| AEL_55_1 | Androsterone sulfate; 14,15-Dihydroxyeicosatrienoic acid (C20:cis[5,8,11]3); CE_Cholesterylester C15:0 | 3 | Dis_adv (Fib >2) | Dis_mild (Fib <2) | 0.83733 |
| AEL_56_1 | Androsterone sulfate; 14,15-Dihydroxyeicosatrienoic acid (C20:cis[5,8,11]3); Choline plasmalogen (C18-vinyl,C20:4) | 3 | Dis_adv (Fib >2) | Dis_mild (Fib <2) | 0.83354 |
| AEL_57_1 | Androsterone sulfate; CE_Cholesterylester C20:4; 8,9-Dihydroxyeicosatrienoic acid (C20:cis [5,11,14]3) | 3 | Dis_adv (Fib >2) | Dis_mild (Fib <2) | 0.80832 |
| AEL_58_1 | Androsterone sulfate; SM_Sphingomyelin (d18:2,C18:0); 8,9-Dihydroxyeicosatrienoic acid (C20:cis[5,11,14]3) | 3 | Dis_adv (Fib >2) | Dis_mild (Fib <2) | 0.80580 |
| AEC_59_1 | Androsterone sulfate; 14,15-Dihydroxyeicosatrienoic acid (C20:cis[5,8,11]3); Glucose | 3 | Dis_adv (Fib >2) | Dis_mild (Fib <2) | 0.90794 |
| AEC_60_1 | Androsterone sulfate; Glucose; SUM.EICOSATIENOIC | 3 | Dis_adv (Fib >2) | Dis_mild (Fib <2) | 0.86507 |
| AEC_61_1 | Androsterone sulfate; Glucose; 11,12-Dihydroxyeicosatrienoic acid (C20:cis[5,8,14]3) | 3 | Dis_adv (Fib >2) | Dis_mild (Fib <2) | 0.86507 |
| AECL 62_1 | Androsterone sulfate; 14,15-Dihydroxyeicosatrienoic acid (C20:cis[5,8,11]3); C-reactive Protein (CRP) | 3 | Dis_adv (Fib >2) | Dis_mild (Fib <2) | 0.90164 |
| AECL 63_1 | Androsterone sulfate; 14,15-Dihydroxyeicosatrienoic acid (C20:cis[5,8,11]3); leucocyte | 3 | Dis_adv (Fib >2) | Dis_mild (Fib <2) | 0.87011 |
| AECL 64_1 | Androsterone sulfate; 11,12-Dihydroxyeicosatrienoic acid (C20:cis[5,8,14]3); C-reactive Protein (CRP) | 3 | Dis_adv (Fib >2) | Dis_mild (Fib <2) | 0.85876 |
| AECL 65_1 | Androsterone sulfate; 14,15-Dihydroxyeicosatrienoic acid (C20:cis[5,8,11]3); alkaline phosphatase (ALP) | 3 | Dis_adv (Fib >2) | Dis_mild (Fib <2) | 0.85120 |
| AECL 66_1 | Androsterone sulfate; SUM.EICOSATIENOIC; C-reactive Protein (CRP) | 3 | Dis_adv (Fib >2) | Dis_mild (Fib <2) | 0.83228 |
| AEN_67_1 | Androsterone sulfate; 14,15-Dihydroxyeicosatrienoic acid (C20:cis[5,8,11]3); Uridine | 3 | Dis_adv (Fib >2) | Dis_mild (Fib <2) | 0.84994 |
| AEE_68_1 | Androsterone sulfate; 14,15-Dihydroxyeicosatrienoic acid (C20:cis[5,8,11]3); 11,12-Dihydroxyeicosatrienoic acid (C20:cis [5,8,14]3) | 3 | Dis_adv (Fib >2) | Dis_mild (Fib <2) | 0.84615 |

(continued)

| Panel Number | Panel Composition long | No of Metabolites | Positive Group | Negative Group | AUC |
|---|---|---|---|---|---|
| AEE_ 69_1 | Androsterone sulfate; 14,15-Dihydroxyeicosatrienoic acid (C20:cis[5,8,11]3); 11,12-Dihydroxyeicosatrienoic acid (C20:cis [5,8,14]3) | 3 | Dis_adv (Fib >2) | Dis_mild (Fib <2) | 0.8461 5 |
| AEE_ 70_1 | Androsterone sulfate; 14,15-Dihydroxyeicosatrienoic acid (C20:cis[5,8,11]3); 8,9-Dihydroxyeicosatrienoic acid (C20:cis [5,11,14]3) | 3 | Dis_adv (Fib >2) | Dis_mild (Fib <2) | 0.8423 7 |
| AEE_ 71_1 | Androsterone sulfate; 14,15-Dihydroxyeicosatrienoic acid (C20:cis[5,8,11]3); 8,9-Dihydroxyeicosatrienoic acid (C20:cis [5,11,14]3) | 3 | Dis_adv (Fib >2) | Dis_mild (Fib <2) | 0.8423 7 |
| AEH_ 72_1 | Androsterone sulfate; 14,15-Dihydroxyeicosatrienoic acid (C20:cis[5,8,11]3); Testosterone | 3 | Dis_adv (Fib >2) | Dis_mild (Fib <2) | 0.8499 4 |
| AEH_ 73_1 | Androsterone sulfate; 14,15-Dihydroxyeicosatrienoic acid (C20:cis[5,8,11]3); Androstenedione | 3 | Dis_adv (Fib >2) | Dis_mild (Fib <2) | 0.8348 0 |
| AE_ 74_1 | Androsterone sulfate; 14,15-Dihydroxyeicosatrienoic acid (C20:cis[5,8,11]3); | 2 | Dis_adv (Fib >2) | Dis_mild (Fib <2) | 0.8575 0 |
| AE_ 75_1 | Androsterone sulfate; 14,15-Dihydroxyeicosatrienoic acid (C20:cis[5,8,11]3); | 2 | Dis_adv (Fib >2) | Dis_mild (Fib <2) | 0.8575 0 |
| AE_ 76_1 | Androsterone sulfate; 11,12-Dihydroxyeicosatrienoic acid (C20:cis[5,8,14]3); | 2 | Dis_adv (Fib >2) | Dis_mild (Fib <2) | 0.8146 3 |
| AE_ 77_1 | Androsterone sulfate; 11,12-Dihydroxyeicosatrienoic acid (C20:cis[5,8,14]3); | 2 | Dis_adv (Fib >2) | Dis_mild (Fib <2) | 0.8146 3 |
| AA_ 78_1 | Androsterone sulfate; Lysine; | 2 | Dis_adv (Fib >2) | Dis_mild (Fib <2) | 0.8423 7 |
| AA_ 79_1 | Androsterone sulfate; Cystine; | 2 | Dis_adv (Fib >2) | Dis_mild (Fib <2) | 0.8297 6 |
| AA_ 80_1 | Androsterone sulfate; Creatine; | 2 | Dis_adv (Fib >2) | Dis_mild (Fib <2) | 0.8184 1 |
| AL_81_1 | Androsterone sulfate; SM_Sphingomyelin (d18: 2,C20:0); | 2 | Dis_adv (Fib >2) | Dis_mild (Fib <2) | 0.8196 7 |
| AL_82_1 | Androsterone sulfate; SM_Sphingomyelin (d18: 2,C18:0); | 2 | Dis_adv (Fib >2) | Dis_mild (Fib <2) | 0.8184 1 |
| AL_83_1 | Androsterone sulfate; CE_Cholesterylester C20:4; | 2 | Dis_adv (Fib >2) | Dis_mild (Fib <2) | 0.8158 9 |
| AB_ 84_1 | Androsterone sulfate; SUM.BILE; | 2 | Dis_adv (Fib >2) | Dis_mild (Fib <2) | 0.9331 7 |

**Table 2a_2: Multimarker panel composition and their respective Area under the curve (AUC) values of receiver operating characteristics (ROC) analysis**

| Panel Number | Panel Composition long | No of Metabolites | Positive Group | Negative Group | AUC |
|---|---|---|---|---|---|
| AEB_ 1_2 | Etiocholanolone sulfate; Glycocholic acid; 14,15-Dihydroxyeicosatrienoic acid (C20:cis[5,8,11]3) | 3 | Dis_adv (Fib >2) | Dis_mild (Fib <2) | 0.95082 |
| AEB_ 2_2 | Etiocholanolone sulfate; SUM.BILE; 14,15-Dihydroxyeicosatrienoic acid (C20:cis[5,8,11]3) | 3 | Dis_adv (Fib >2) | Dis_mild (Fib <2) | 0.94451 |
| AEB_ 3_2 | Etiocholanolone sulfate; SUM.BILE; SUM.EICOSATIENOIC | 3 | Dis_adv (Fib >2) | Dis_mild (Fib <2) | 0.92055 |
| AEB_ 4_2 | Etiocholanolone sulfate; Taurochenodeoxycholic acid ; 8,9-Dihydroxyeicosatrienoic acid (C20:cis[5,11,14]3) | 3 | Dis_adv (Fib >2) | Dis_mild (Fib <2) | 0.91929 |
| AEB_ 4a_2 | Etiocholanolone sulfate; Taurodeoxycholic acid; 8,9-Dihydroxyeicosatrienoic acid (C20:cis[5,11,14]3) | 3 | Dis_adv (Fib >2) | Dis_mild (Fib <2) | 0.91929 |
| AEB_ 5_2 | Etiocholanolone sulfate; SUM.BILE; 11,12-Dihydroxyeicosatrienoic acid (C20:cis[5,8,14]3) | 3 | Dis_adv (Fib >2) | Dis_mild (Fib <2) | 0.91929 |
| AEB_ 6_2 | Etiocholanolone sulfate; SUM.BILE; 8,9-Dihydroxyeicosatrienoic acid (C20:cis[5,11,14]3) | 3 | Dis_adv (Fib >2) | Dis_mild (Fib <2) | 0.91803 |
| AEB_ 7_2 | Etiocholanolone sulfate; Taurochenodeoxycholic acid ; SUM.EICOSATIENOIC | 3 | Dis_adv (Fib >2) | Dis_mild (Fib <2) | 0.91803 |
| AEB_ 7a_2 | Etiocholanolone sulfate; Taurodeoxycholic acid; SUM.EICOSATIENOIC | 3 | Dis_adv (Fib >2) | Dis_mild (Fib <2) | 0.91803 |
| AEB_ 8_2 | Etiocholanolone sulfate; Taurochenodeoxycholic acid; 14,15-Dihydroxyeicosatrienoic acid (C20:cis[5,8,11]3) | 3 | Dis_adv (Fib >2) | Dis_mild (Fib <2) | 0.91677 |
| AEB_ 8a_2 | Etiocholanolone sulfate; Taurodeoxycholic acid; 14,15-Dihydroxyeicosatrienoic acid (C20:cis[5,8,11]3) | 3 | Dis_adv (Fib >2) | Dis_mild (Fib <2) | 0.91677 |
| AEB_ 9_2 | Etiocholanolone sulfate; Taurocholic acid; 14,15-Dihydroxyeicosatrienoic acid (C20:cis[5,8,11]3) | 3 | Dis_adv (Fib >2) | Dis_mild (Fib <2) | 0.91299 |
| AEB_ 10_2 | Etiocholanolone sulfate; Taurochenodeoxycholic acid; 11,12-Dihydroxyeicosatrienoic acid (C20:cis[5,8,14]3) | 3 | Dis_adv (Fib >2) | Dis_mild (Fib <2) | 0.91047 |
| AEB_ 10a_2 | Etiocholanolone sulfate; Taurodeoxycholic acid; 11,12-Dihydroxyeicosatrienoic acid (C20:cis[5,8,14]3) | 3 | Dis_adv (Fib >2) | Dis_mild (Fib <2) | 0.91047 |
| AEB_ 11_2 | Etiocholanolone sulfate; Taurocholic acid; SUM.EICOSATIENOIC | 3 | Dis_adv (Fib >2) | Dis_mild (Fib <2) | 0.90290 |
| AEB_ 12 | Etiocholanolone sulfate; Glycocholic acid; 11,12-Dihydroxyeicosatrienoic acid (C20:cis[5,8,14]3) | 3 | Dis_adv (Fib >2) | Dis_mild (Fib <2) | 0.89912 |
| AEB_ 13_2 | Etiocholanolone sulfate; Taurocholic acid; 8,9-Dihydroxyeicosatrienoic acid (C20:cis[5,11,14]3) | 3 | Dis_adv (Fib >2) | Dis_mild (Fib <2) | 0.89660 |
| AEB_ 14_2 | Etiocholanolone sulfate; Taurocholic acid; 11,12-Dihydroxyeicosatrienoic acid (C20:cis[5,8,14]3) | 3 | Dis_adv (Fib >2) | Dis_mild (Fib <2) | 0.89407 |
| AEB_ 15_2 | Etiocholanolone sulfate; Glycocholic acid; SUM.EICOSATIENOIC | 3 | Dis_adv (Fib >2) | Dis_mild (Fib <2) | 0.88903 |

(continued)

| Panel Number | Panel Composition long | No of Metabolites | Positive Group | Negative Group | AUC |
|---|---|---|---|---|---|
| AEB_16_2 | Etiocholanolone sulfate; Glycocholic acid; 8,9-Dihydroxyeicosatrienoic acid (C20:cis[5,11,14]3) | 3 | Dis_adv (Fib >2) | Dis_mild (Fib <2) | 0.87137 |
| AEA_17_2 | Etiocholanolone sulfate; 14,15-Dihydroxyeicosatrienoic acid (C20:cis[5,8,11]3); Creatine | 3 | Dis_adv (Fib >2) | Dis_mild (Fib <2) | 0.94073 |
| AEA_18_2 | Etiocholanolone sulfate; 14,15-Dihydroxyeicosatrienoic acid (C20:cis[5,8,11]3); Cystine | 3 | Dis_adv (Fib >2) | Dis_mild (Fib <2) | 0.92434 |
| AEA_19_2 | Etiocholanolone sulfate; 14,15-Dihydroxyeicosatrienoic acid (C20:cis[5,8,11]3); Tyrosine | 3 | Dis_adv (Fib >2) | Dis_mild (Fib <2) | 0.89407 |
| AEA_20_2 | Etiocholanolone sulfate; Lysine; 14,15-Dihydroxyeicosatrienoic acid (C20:cis[5,8,11]3) | 3 | Dis_adv (Fib >2) | Dis_mild (Fib <2) | 0.89407 |
| AEA_21_2 | Etiocholanolone sulfate; SUM.EICOSATIENOIC; Creatine | 3 | Dis_adv (Fib >2) | Dis_mild (Fib <2) | 0.88903 |
| AEA_22_2 | Etiocholanolone sulfate; 11,12-Dihydroxyeicosatrienoic acid (C20:cis[5,8,14]3); Creatine | 3 | Dis_adv (Fib >2) | Dis_mild (Fib <2) | 0.88272 |
| AEA_23_2 | Etiocholanolone sulfate; SUM.EICOSATIENOIC; Cystine | 3 | Dis_adv (Fib >2) | Dis_mild (Fib <2) | 0.87516 |
| AEA_24_2 | Etiocholanolone sulfate; 14,15-Dihydroxyeicosatrienoic acid (C20:cis[5,8,11]3); Phenylalanine | 3 | Dis_adv (Fib >2) | Dis_mild (Fib <2) | 0.86885 |
| AEA_25_2 | Etiocholanolone sulfate; 14,15-Dihydroxyeicosatrienoic acid (C20:cis[5,8,11]3); Asparagine | 3 | Dis_adv (Fib >2) | Dis_mild (Fib <2) | 0.86759 |
| AEA_26_2 | Etiocholanolone sulfate; 14,15-Dihydroxyeicosatrienoic acid (C20:cis[5,8,11]3); Creatinine | 3 | Dis_adv (Fib >2) | Dis_mild (Fib <2) | 0.86381 |
| AEA_27_2 | Etiocholanolone sulfate; 14,15-Dihydroxyeicosatrienoic acid (C20:cis[5,8,11]3); Arginine | 3 | Dis_adv (Fib >2) | Dis_mild (Fib <2) | 0.86003 |
| AEA_28_2 | Etiocholanolone sulfate; Lysine; 11,12-Dihydroxyeicosatrienoic acid (C20:cis[5,8,14]3) | 3 | Dis_adv (Fib >2) | Dis_mild (Fib <2) | 0.86003 |
| AEA_29_2 | Etiocholanolone sulfate; 14,15-Dihydroxyeicosatrienoic acid (C20:cis[5,8,11]3); Glutamine | 3 | Dis_adv (Fib >2) | Dis_mild (Fib <2) | 0.85624 |
| AEA_30_2 | Etiocholanolone sulfate; Tyrosine; SUM.EICOSATIENOIC | 3 | Dis_adv (Fib >2) | Dis_mild (Fib <2) | 0.84868 |
| AEA_31_2 | Etiocholanolone sulfate; 14,15-Dihydroxyeicosatrienoic acid (C20:cis[5,8,11]3); Tryptophan | 3 | Dis_adv (Fib >2) | Dis_mild (Fib <2) | 0.84615 |
| AEE_32_2 | Etiocholanolone sulfate; 14,15-Dihydroxyeicosatrienoic acid (C20:cis[5,8,11]3); SUM.EICOSATIENOIC | 3 | Dis_adv (Fib >2) | Dis_mild (Fib <2) | 0.82850 |

(continued)

| Panel Number | Panel Composition long | No of Metabolites | Positive Group | Negative Group | AUC |
|---|---|---|---|---|---|
| AEA_33_2 | Etiocholanolone sulfate; Cystine; 11,12-Dihydroxyeicosatrienoic acid (C20:cis[5,8,14]3) | 3 | Dis_adv (Fib >2) | Dis_mild (Fib <2) | 0.84615 |
| AEE_34_2 | Etiocholanolone sulfate; 14,15-Dihydroxyeicosatrienoic acid (C20:cis[5,8,11]3); SUM.EICOSATIENOIC | 3 | Dis_adv (Fib >2) | Dis_mild (Fib <2) | 0.82850 |
| AEA_35_2 | Etiocholanolone sulfate; 14,15-Dihydroxyeicosatrienoic acid (C20:cis[5,8,11]3); Urea | 3 | Dis_adv (Fib >2) | Dis_mild (Fib <2) | 0.83607 |
| AEA_36_2 | Etiocholanolone sulfate; Creatinine; 11,12-Dihydroxyeicosatrienoic acid (C20:cis[5,8,14]3) | 3 | Dis_adv (Fib >2) | Dis_mild (Fib <2) | 0.82724 |
| AEA_37_2 | Etiocholanolone sulfate; 14,15-Dihydroxyeicosatrienoic acid (C20:cis[5,8,11]3); Histidine | 3 | Dis_adv (Fib >2) | Dis_mild (Fib <2) | 0.82598 |
| AEA_38_2 | Etiocholanolone sulfate; Lysine; SUM.EICOSATIENOIC | 3 | Dis_adv (Fib >2) | Dis_mild (Fib <2) | 0.82346 |
| AEA_39_2 | Etiocholanolone sulfate; Tyrosine; 11,12-Dihydroxyeicosatrienoic acid (C20:cis[5,8,14]3) | 3 | Dis_adv (Fib >2) | Dis_mild (Fib <2) | 0.81715 |
| AEL_40_2 | Etiocholanolone sulfate; CE_Cholesterylester C20:4; 14,15-Dihydroxyeicosatrienoic acid (C20:cis[5,8,11]3) | 3 | Dis_adv (Fib >2) | Dis_mild (Fib <2) | 0.91803 |
| AEL_41_2 | Etiocholanolone sulfate; 14,15-Dihydroxyeicosatrienoic acid (C20:cis[5,8,11]3); SM_Sphingomyelin (d18:2,C20:0) | 3 | Dis_adv (Fib >2) | Dis_mild (Fib <2) | 0.90542 |
| AEL_42_2 | Etiocholanolone sulfate; 14,15-Dihydroxyeicosatrienoic acid (C20:cis[5,8,11]3); SM_Sphingomyelin (d18:2,C18:0) | 3 | Dis_adv (Fib >2) | Dis_mild (Fib <2) | 0.90164 |
| AEL_43_2 | Etiocholanolone sulfate; CE_Cholesterylester C20:4; SUM.EICOSATIENOIC | 3 | Dis_adv (Fib >2) | Dis_mild (Fib <2) | 0.88398 |
| AEL_44_2 | Etiocholanolone sulfate; 14,15-Dihydroxyeicosatrienoic acid (C20:cis[5,8,11]3); Ethanolamine plasmalogen (C39:5) | 3 | Dis_adv (Fib >2) | Dis_mild (Fib <2) | 0.88272 |
| AEL_44a_2 | Etiocholanolone sulfate; 14,15-Dihydroxyeicosatrienoic acid (C20:cis[5,8,11]3); Choline plasmalogen (C36:5) | 3 | Dis_adv (Fib >2) | Dis_mild (Fib <2) | 0.88272 |
| AEL_45_2 | Etiocholanolone sulfate; 14,15-Dihydroxyeicosatrienoic acid (C20:cis[5,8,11]3); Phosphatidylcholine (C18:0,C20:4) | 3 | Dis_adv (Fib >2) | Dis_mild (Fib <2) | 0.87264 |
| AEL_46_2 | Etiocholanolone sulfate; CE_Cholesterylester C20:4; 11,12-Dihydroxyeicosatrienoic acid (C20:cis[5,8,14]3) | 3 | Dis_adv (Fib >2) | Dis_mild (Fib <2) | 0.86759 |
| AEL_47_2 | Etiocholanolone sulfate; SUM.EICOSATIENOIC; SM_Sphingomyelin (d18:2,C20:0) | 3 | Dis_adv (Fib >2) | Dis_mild (Fib <2) | 0.86003 |
| AEL_48_2 | Etiocholanolone sulfate; SM_Sphingomyelin (d18:2,C18:0); SUM.EICOSATIENOIC | 3 | Dis_adv (Fib >2) | Dis_mild (Fib <2) | 0.85372 |

(continued)

| Panel Number | Panel Composition long | No of Metabolites | Positive Group | Negative Group | AUC |
|---|---|---|---|---|---|
| AEL_49_2 | Etiocholanolone sulfate; 14,15-Dihydroxyeicosatrienoic acid (C20:cis[5,8,11]3); total triacylglyceride | 3 | Dis_adv (Fib >2) | Dis_mild (Fib <2) | 0.84489 |
| AEL_50_2 | Etiocholanolone sulfate; 14,15-Dihydroxyeicosatrienoic acid (C20:cis[5,8,11]3); Ethanolamine plasmalogen (C39:4) | 3 | Dis_adv (Fib >2) | Dis_mild (Fib <2) | 0.84111 |
| AEL_50a_2 | Etiocholanolone sulfate; 14,15-Dihydroxyeicosatrienoic acid (C20:cis[5,8,11]3); Choline plasmalogen (C36:4) | 3 | Dis_adv (Fib >2) | Dis_mild (Fib <2) | 0.84111 |
| AEL_51_2 | Etiocholanolone sulfate; 14,15-Dihydroxyeicosatrienoic acid (C20:cis[5,8,11]3); total cholesterol | 3 | Dis_adv (Fib >2) | Dis_mild (Fib <2) | 0.83985 |
| AEL_52_2 | Etiocholanolone sulfate; SM_Sphingomyelin (d18:2,C18:0); 11,12-Dihydroxyeicosatrienoic acid (C20:cis[5,8,14]3) | 3 | Dis_adv (Fib >2) | Dis_mild (Fib <2) | 0.83985 |
| AEL_53_2 | Etiocholanolone sulfate; 14,15-Dihydroxyeicosatrienoic acid (C20:cis[5,8,11]3); SM_Sphingomyelin (d18:1,C18:0) | 3 | Dis_adv (Fib >2) | Dis_mild (Fib <2) | 0.83985 |
| AEL_54_2 | Etiocholanolone sulfate; 11,12-Dihydroxyeicosatrienoic acid (C20:cis[5,8,14]3); SM_Sphingomyelin (d18:2,C20:0) | 3 | Dis_adv (Fib >2) | Dis_mild (Fib <2) | 0.83733 |
| AEL_55_2 | Etiocholanolone sulfate; 14,15-Dihydroxyeicosatrienoic acid (C20:cis[5,8,11]3); CE_Cholesterylester C15:0 | 3 | Dis_adv (Fib >2) | Dis_mild (Fib <2) | 0.83733 |
| AEL_56_2 | Etiocholanolone sulfate; 14,15-Dihydroxyeicosatrienoic acid (C20:cis[5,8,11]3); Choline plasmalogen (C18-vinyl,C20:4) | 3 | Dis_adv (Fib >2) | Dis_mild (Fib <2) | 0.83354 |
| AEL_57_2 | Etiocholanolone sulfate; CE_Cholesterylester C20:4; 8,9-Dihydroxyeicosatrienoic acid (C20:cis [5,11,14]3) | 3 | Dis_adv (Fib >2) | Dis_mild (Fib <2) | 0.80832 |
| AEL_58_2 | Etiocholanolone sulfate; SM_Sphingomyelin (d18:2,C18:0); 8,9-Dihydroxyeicosatrienoic acid (C20:cis[5,11,14]3) | 3 | Dis_adv (Fib >2) | Dis_mild (Fib <2) | 0.80580 |
| AEC_59_2 | Etiocholanolone sulfate; 14,15-Dihydroxyeicosatrienoic acid (C20:cis[5,8,11]3); Glucose | 3 | Dis_adv (Fib >2) | Dis_mild (Fib <2) | 0.90794 |
| AEC_60_2 | Etiocholanolone sulfate; Glucose; SUM.EICOSATIENOIC | 3 | Dis_adv (Fib >2) | Dis_mild (Fib <2) | 0.86507 |
| AEC_61_2 | Etiocholanolone sulfate; Glucose; 11,12-Dihydroxyeicosatrienoic acid (C20:cis[5,8,14]3) | 3 | Dis_adv (Fib >2) | Dis_mild (Fib <2) | 0.86507 |
| AECL62_2 | Etiocholanolone sulfate; 14,15-Dihydroxyeicosatrienoic acid (C20:cis[5,8,11]3); C-reactive Protein (CRP) | 3 | Dis_adv (Fib >2) | Dis_mild (Fib <2) | 0.90164 |
| AECL63_2 | Etiocholanolone sulfate; 14,15-Dihydroxyeicosatrienoic acid (C20:cis[5,8,11]3); leucocyte | 3 | Dis_adv (Fib >2) | Dis_mild (Fib <2) | 0.87011 |

(continued)

| Panel Number | Panel Composition long | No of Metabolites | Positive Group | Negative Group | AUC |
|---|---|---|---|---|---|
| AECL 64_2 | Etiocholanolone sulfate; 11,12-Dihydroxyeicosatrienoic acid (C20:cis[5,8,14]3); C-reactive Protein (CRP) | 3 | Dis_adv (Fib >2) | Dis_mild (Fib <2) | 0.85876 |
| AECL 65_2 | Etiocholanolone sulfate; 14,15-Dihydroxyeicosatrienoic acid (C20:cis[5,8,11]3); alkaline phosphatase (ALP) | 3 | Dis_adv (Fib >2) | Dis_mild (Fib <2) | 0.85120 |
| AECL 66_2 | Etiocholanolone sulfate; SUM.EICOSATIENOIC; C-reactive Protein (CRP) | 3 | Dis_adv (Fib >2) | Dis_mild (Fib <2) | 0.83228 |
| AEN_ 67_2 | Etiocholanolone sulfate; 14,15-Dihydroxyeicosatrienoic acid (C20:cis[5,8,11]3); Uridine | 3 | Dis_adv (Fib >2) | Dis_mild (Fib <2) | 0.84994 |
| AEE_ 68_2 | Etiocholanolone sulfate; 14,15-Dihydroxyeicosatrienoic acid (C20:cis[5,8,11]3); 11,12-Dihydroxyeicosatrienoic acid (C20:cis [5,8,14]3) | 3 | Dis_adv (Fib >2) | Dis_mild (Fib <2) | 0.84615 |
| AEE_ 69_2 | Etiocholanolone sulfate; 14,15-Dihydroxyeicosatrienoic acid (C20:cis[5,8,11]3); 11,12-Dihydroxyeicosatrienoic acid (C20:cis [5,8,14]3) | 3 | Dis_adv (Fib >2) | Dis_mild (Fib <2) | 0.84615 |
| AEE_ 70_2 | Etiocholanolone sulfate; 14,15-Dihydroxyeicosatrienoic acid (C20:cis[5,8,11]3); 8,9-Dihydroxyeicosatrienoic acid (C20:cis [5,11,14]3) | 3 | Dis_adv (Fib >2) | Dis_mild (Fib <2) | 0.84237 |
| AEE_ 71_2 | Etiocholanolone sulfate; 14,15-Dihydroxyeicosatrienoic acid (C20:cis[5,8,11]3); 8,9-Dihydroxyeicosatrienoic acid (C20:cis [5,11,14]3) | 3 | Dis_adv (Fib >2) | Dis_mild (Fib <2) | 0.84237 |
| AEH_ 72_2 | Etiocholanolone sulfate; 14,15-Dihydroxyeicosatrienoic acid (C20:cis[5,8,11]3); Testosterone | 3 | Dis_adv (Fib >2) | Dis_mild (Fib <2) | 0.84994 |
| AEH_ 73_2 | Etiocholanolone sulfate; 14,15-Dihydroxyeicosatrienoic acid (C20:cis[5,8,11]3); Androstenedione | 3 | Dis_adv (Fib >2) | Dis_mild (Fib <2) | 0.83480 |
| AE_ 74_2 | Etiocholanolone sulfate; 14,15-Dihydroxyeicosatrienoic acid (C20:cis[5,8,11]3); | 2 | Dis_adv (Fib >2) | Dis_mild (Fib <2) | 0.85750 |
| AE_ 75_2 | Etiocholanolone sulfate; 14,15-Dihydroxyeicosatrienoic acid (C20:cis[5,8,11]3); | 2 | Dis_adv (Fib >2) | Dis_mild (Fib <2) | 0.85750 |
| AE_ 76_2 | Etiocholanolone sulfate; 11,12-Dihydroxyeicosatrienoic acid (C20:cis[5,8,14]3); | 2 | Dis_adv (Fib >2) | Dis_mild (Fib <2) | 0.81463 |
| AE_ 77_2 | Etiocholanolone sulfate; 11,12-Dihydroxyeicosatrienoic acid (C20:cis[5,8,14]3); | 2 | Dis_adv (Fib >2) | Dis_mild (Fib <2) | 0.81463 |
| AA_ 78_2 | Etiocholanolone sulfate; Lysine; | 2 | Dis_adv (Fib >2) | Dis_mild (Fib <2) | 0.84237 |
| AA_ 79_2 | Etiocholanolone sulfate; Cystine; | 2 | Dis_adv (Fib >2) | Dis_mild (Fib <2) | 0.82976 |

(continued)

| Panel Number | Panel Composition long | No of Metabolites | Positive Group | Negative Group | AUC |
|---|---|---|---|---|---|
| AA_80_2 | Etiocholanolone sulfate; Creatine; | 2 | Dis_adv (Fib >2) | Dis_mild (Fib <2) | 0.81841 |
| AL_81_2 | Etiocholanolone sulfate; SM_Sphingomyelin (d18:2,C20:0); | 2 | Dis_adv (Fib >2) | Dis_mild (Fib <2) | 0.81967 |
| AL_82_2 | Etiocholanolone sulfate; SM_Sphingomyelin (d18:2,C18:0); | 2 | Dis_adv (Fib >2) | Dis_mild (Fib <2) | 0.81841 |
| AL_83_2 | Etiocholanolone sulfate; CE_Cholesterylester C20:4; | 2 | Dis_adv (Fib >2) | Dis_mild (Fib <2) | 0.81589 |
| AB_84_2 | Etiocholanolone sulfate; SUM.BILE; | 2 | Dis_adv (Fib >2) | Dis_mild (Fib <2) | 0.93317 |

**Table 2a_3: Multimarker panel composition and their respective Area under the curve (AUC) values of receiver operating characteristics (ROC) analysis**

| Panel Number | Panel Composition long | No of Metabolites | Positive Group | Negative Group | AUC |
|---|---|---|---|---|---|
| AEB_1_3 | Dihydrotestosterone sulfate; Glycocholic acid; 14,15-Dihydroxyeicosatrienoic acid (C20:cis[5,8,11]3) | 3 | Dis_adv (Fib >2) | Dis_mild (Fib <2) | 0.95082 |
| AEB_2_3 | Dihydrotestosterone sulfate; SUM.BILE; 14,15-Dihydroxyeicosatrienoic acid (C20:cis[5,8,11]3) | 3 | Dis_adv (Fib >2) | Dis_mild (Fib <2) | 0.94451 |
| AEB_3 | Dihydrotestosterone sulfate; SUM.BILE; SUM.EICOSATIENOIC | 3 | Dis_adv (Fib >2) | Dis_mild (Fib <2) | 0.92055 |
| AEB_4_3 | Dihydrotestosterone sulfate; Taurochenodeoxycholic acid; 8,9-Dihydroxyeicosatrienoic acid (C20:cis[5,11,14]3) | 3 | Dis_adv (Fib >2) | Dis_mild (Fib <2) | 0.91929 |
| AEB_4a_3 | Dihydrotestosterone sulfate; Taurodeoxycholic acid; 8,9-Dihydroxyeicosatrienoic acid (C20:cis[5,11,14]3) | 3 | Dis_adv (Fib >2) | Dis_mild (Fib <2) | 0.91929 |
| AEB_5_3 | Dihydrotestosterone sulfate; SUM.BILE; 11,12-Dihydroxyeicosatrienoic acid (C20:cis[5,8,14]3) | 3 | Dis_adv (Fib >2) | Dis_mild (Fib <2) | 0.91929 |
| AEB_6_3 | Dihydrotestosterone sulfate; SUM.BILE; 8,9-Dihydroxyeicosatrienoic acid (C20:cis[5,11,14]3) | 3 | Dis_adv (Fib >2) | Dis_mild (Fib <2) | 0.91803 |
| AEB_7_3 | Dihydrotestosterone sulfate; Taurochenodeoxycholic acid ; SUM.EICOSATIENOIC | 3 | Dis_adv (Fib >2) | Dis_mild (Fib <2) | 0.91803 |
| AEB_7a_3 | Dihydrotestosterone sulfate; Taurodeoxycholic acid; SUM.EICOSATIENOIC | 3 | Dis_adv (Fib >2) | Dis_mild (Fib <2) | 0.91803 |
| AEB_8_3 | Dihydrotestosterone sulfate; Taurochenodeoxycholic acid ; 14,15-Dihydroxyeicosatrienoic acid (C20:cis[5,8,11]3) | 3 | Dis_adv (Fib >2) | Dis_mild (Fib <2) | 0.91677 |
| AEB_8a_3 | Dihydrotestosterone sulfate; Taurodeoxycholic acid; 14,15-Dihydroxyeicosatrienoic acid (C20:cis[5,8,11]3) | 3 | Dis_adv (Fib >2) | Dis_mild (Fib <2) | 0.91677 |

(continued)

| Panel Number | Panel Composition long | No of Metabolites | Positive Group | Negative Group | AUC |
|---|---|---|---|---|---|
| AEB_9_3 | Dihydrotestosterone sulfate; Taurocholic acid; 14,15-Dihydroxyeicosatrienoic acid (C20:cis [5,8,11]3) | 3 | Dis_adv (Fib >2) | Dis_mild (Fib <2) | 0.91299 |
| AEB_10_3 | Dihydrotestosterone sulfate; Taurochenodeoxycholic acid; 11,12-Dihydroxyeicosatrienoic acid (C20:cis[5,8,14]3) | 3 | Dis_adv (Fib >2) | Dis_mild (Fib <2) | 0.91047 |
| AEB_10a_3 | Dihydrotestosterone sulfate; Taurodeoxycholic acid; 11,12-Dihydroxyeicosatrienoic acid (C20:cis [5,8,14]3) | 3 | Dis_adv (Fib >2) | Dis_mild (Fib <2) | 0.91047 |
| AEB_11_3 | Dihydrotestosterone sulfate; Taurocholic acid; SUM.EICOSATIENOIC | 3 | Dis_adv (Fib >2) | Dis_mild (Fib <2) | 0.90290 |
| AEB_12_3 | Dihydrotestosterone sulfate; Glycocholic acid; 11,12-Dihydroxyeicosatrienoic acid (C20:cis [5,8,14]3) | 3 | Dis_adv (Fib >2) | Dis_mild (Fib <2) | 0.89912 |
| AEB_13_ | Dihydrotestosterone sulfate; Taurocholic acid; 8,9-Dihydroxyeicosatrienoic acid (C20:cis [5,11,14]3) | 3 | Dis_adv (Fib >2) | Dis_mild (Fib <2) | 0.89660 |
| AEB_14_3 | Dihydrotestosterone sulfate; Taurocholic acid; 11,12-Dihydroxyeicosatrienoic acid (C20:cis [5,8,14]3) | 3 | Dis_adv (Fib >2) | Dis_mild (Fib <2) | 0.89407 |
| AEB_15_3 | Dihydrotestosterone sulfate; Glycocholic acid; SUM.EICOSATIENOIC | 3 | Dis_adv (Fib >2) | Dis_mild (Fib <2) | 0.88903 |
| AEB_16_3 | Dihydrotestosterone sulfate; Glycocholic acid; 8,9-Dihydroxyeicosatrienoic acid (C20:cis [5,11,14]3) | 3 | Dis_adv (Fib >2) | Dis_mild (Fib <2) | 0.87137 |
| AEA_17_3 | Dihydrotestosterone sulfate; 14,15-Dihydroxyeicosatrienoic acid (C20:cis[5,8,11]3); Creatine | 3 | Dis_adv (Fib >2) | Dis_mild (Fib <2) | 0.94073 |
| AEA_18_3 | Dihydrotestosterone sulfate; 14,15-Dihydroxyeicosatrienoic acid (C20:cis[5,8,11]3); Cystine | 3 | Dis_adv (Fib >2) | Dis_mild (Fib <2) | 0.92434 |
| AEA_19_3 | Dihydrotestosterone sulfate; 14,15-Dihydroxyeicosatrienoic acid (C20:cis[5,8,11]3); Tyrosine | 3 | Dis_adv (Fib >2) | Dis_mild (Fib <2) | 0.89407 |
| AEA_20_3 | Dihydrotestosterone sulfate; Lysine; 14,15-Dihydroxyeicosatrienoic acid (C20:cis[5,8,11]3) | 3 | Dis_adv (Fib >2) | Dis_mild (Fib <2) | 0.89407 |
| AEA_21_3 | Dihydrotestosterone sulfate; SUM.EICOSATIENOIC; Creatine | 3 | Dis_adv (Fib >2) | Dis_mild (Fib <2) | 0.88903 |
| AEA_22_3 | Dihydrotestosterone sulfate; 11,12-Dihydroxyeicosatrienoic acid (C20:cis[5,8,14]3); Creatine | 3 | Dis_adv (Fib >2) | Dis_mild (Fib <2) | 0.88272 |
| AEA_23_ | Dihydrotestosterone sulfate; SUM.EICOSATIENOIC; Cystine | 3 | Dis_adv (Fib >2) | Dis_mild (Fib <2) | 0.87516 |
| AEA_24_3 | Dihydrotestosterone sulfate; 14,15-Dihydroxyeicosatrienoic acid (C20:cis[5,8,11]3); Phenylalanine | 3 | Dis_adv (Fib >2) | Dis_mild (Fib <2) | 0.86885 |

(continued)

| Panel Number | Panel Composition long | No of Metabolites | Positive Group | Negative Group | AUC |
|---|---|---|---|---|---|
| AEA_25_3 | Dihydrotestosterone sulfate; 14,15-Dihydroxyeicosatrienoic acid (C20:cis[5,8,11]3); Asparagine | 3 | Dis_adv (Fib >2) | Dis_mild (Fib <2) | 0.86759 |
| AEA_26_3 | Dihydrotestosterone sulfate; 14,15-Dihydroxyeicosatrienoic acid (C20:cis[5,8,11]3); Creatinine | 3 | Dis_adv (Fib >2) | Dis_mild (Fib <2) | 0.86381 |
| AEA_27_3 | Dihydrotestosterone sulfate; 14,15-Dihydroxyeicosatrienoic acid (C20:cis[5,8,11]3); Arginine | 3 | Dis_adv (Fib >2) | Dis_mild (Fib <2) | 0.86003 |
| AEA_28_3 | Dihydrotestosterone sulfate; Lysine; 11,12-Dihydroxyeicosatrienoic acid (C20:cis[5,8,14]3) | 3 | Dis_adv (Fib >2) | Dis_mild (Fib <2) | 0.86003 |
| AEA_29_3 | Dihydrotestosterone sulfate; 14,15-Dihydroxyeicosatrienoic acid (C20:cis[5,8,11]3); Glutamine | 3 | Dis_adv (Fib >2) | Dis_mild (Fib <2) | 0.85624 |
| AEA_30_3 | Dihydrotestosterone sulfate; Tyrosine; SUM.EICOSATIENOIC | 3 | Dis_adv (Fib >2) | Dis_mild (Fib <2) | 0.84868 |
| AEA_31_3 | Dihydrotestosterone sulfate; 14,15-Dihydroxyeicosatrienoic acid (C20:cis[5,8,11]3); Tryptophan | 3 | Dis_adv (Fib >2) | Dis_mild (Fib <2) | 0.84615 |
| AEE_32_3 | Dihydrotestosterone sulfate; 14,15-Dihydroxyeicosatrienoic acid (C20:cis[5,8,11]3); SUM.EICOSATIENOIC | 3 | Dis_adv (Fib >2) | Dis_mild (Fib <2) | 0.82850 |
| AEA_33_3 | Dihydrotestosterone sulfate; Cystine; 11,12-Dihydroxyeicosatrienoic acid (C20:cis[5,8,14]3) | 3 | Dis_adv (Fib >2) | Dis_mild (Fib <2) | 0.84615 |
| AEE_34_3 | Dihydrotestosterone sulfate; 14,15-Dihydroxyeicosatrienoic acid (C20:cis[5,8,11]3); SUM.EICOSATIENOIC | 3 | Dis_adv (Fib >2) | Dis_mild (Fib <2) | 0.82850 |
| AEA_35_3 | Dihydrotestosterone sulfate; 14,15-Dihydroxyeicosatrienoic acid (C20:cis[5,8,11]3); Urea | 3 | Dis_adv (Fib >2) | Dis_mild (Fib <2) | 0.83607 |
| AEA_36_3 | Dihydrotestosterone sulfate; Creatinine; 11,12-Dihydroxyeicosatrienoic acid (C20:cis[5,8,14]3) | 3 | Dis_adv (Fib >2) | Dis_mild (Fib <2) | 0.82724 |
| AEA_37_3 | Dihydrotestosterone sulfate; 14,15-Dihydroxyeicosatrienoic acid (C20:cis[5,8,11]3); Histidine | 3 | Dis_adv (Fib >2) | Dis_mild (Fib <2) | 0.82598 |
| AEA_38_3 | Dihydrotestosterone sulfate; Lysine; SUM.EICOSATIENOIC | 3 | Dis_adv (Fib >2) | Dis_mild (Fib <2) | 0.82346 |
| AEA_39_3 | Dihydrotestosterone sulfate; Tyrosine; 11,12-Dihydroxyeicosatrienoic acid (C20:cis[5,8,14]3) | 3 | Dis_adv (Fib >2) | Dis_mild (Fib <2) | 0.81715 |
| AEL_40_3 | Dihydrotestosterone sulfate; CE_Cholesterylester C20:4; 14,15-Dihydroxyeicosatrienoic acid (C20:cis[5,8,11]3) | 3 | Dis_adv (Fib >2) | Dis_mild (Fib <2) | 0.91803 |
| AEL_41_3 | Dihydrotestosterone sulfate; 14,15-Dihydroxyeicosatrienoic acid (C20:cis[5,8,11]3); SM_Sphingomyelin (d18:2,C20:0) | 3 | Dis_adv (Fib >2) | Dis_mild (Fib <2) | 0.90542 |

(continued)

| Panel Number | Panel Composition long | No of Metabolites | Positive Group | Negative Group | AUC |
|---|---|---|---|---|---|
| AEL_42_3 | Dihydrotestosterone sulfate; 14,15-Dihydroxyeicosatrienoic acid (C20:cis[5,8,11]3); SM_Sphingomyelin (d18:2,C18:0) | 3 | Dis_adv (Fib >2) | Dis_mild (Fib <2) | 0.90164 |
| AEL_43 | Dihydrotestosterone sulfate; CE_Cholesterylester C20:4; SUM.EICOSATIENOIC | 3 | Dis_adv (Fib >2) | Dis_mild (Fib <2) | 0.88398 |
| AEL_44_3 | Dihydrotestosterone sulfate; 14,15-Dihydroxyeicosatrienoic acid (C20:cis[5,8,11]3); Ethanolamine plasmalogen (C39:5) | 3 | Dis_adv (Fib >2) | Dis_mild (Fib <2) | 0.88272 |
| AEL_44a_3 | Dihydrotestosterone sulfate; 14,15-Dihydroxyeicosatrienoic acid (C20:cis[5,8,11]3); Choline plasmalogen (C36:5) | 3 | Dis_adv (Fib >2) | Dis_mild (Fib <2) | 0.88272 |
| AEL_45_3 | Dihydrotestosterone sulfate; 14,15-Dihydroxyeicosatrienoic acid (C20:cis[5,8,11]3); Phosphatidylcholine (C18:0,C20:4) | 3 | Dis_adv (Fib >2) | Dis_mild (Fib <2) | 0.87264 |
| AEL_46_3 | Dihydrotestosterone sulfate; CE_Cholesterylester C20:4; 11,12-Dihydroxyeicosatrienoic acid (C20:cis[5,8,14]3) | 3 | Dis_adv (Fib >2) | Dis_mild (Fib <2) | 0.86759 |
| AEL_47_3 | Dihydrotestosterone sulfate; SUM.EICOSATIENOIC; SM_Sphingomyelin (d18:2,C20:0) | 3 | Dis_adv (Fib >2) | Dis_mild (Fib <2) | 0.86003 |
| AEL_48_3 | Dihydrotestosterone sulfate; SM_Sphingomyelin (d18:2,C18:0); SUM.EICOSATIENOIC | 3 | Dis_adv (Fib >2) | Dis_mild (Fib <2) | 0.85372 |
| AEL_49_3 | Dihydrotestosterone sulfate; 14,15-Dihydroxyeicosatrienoic acid (C20:cis[5,8,11]3); total triacylglyceride | 3 | Dis_adv (Fib >2) | Dis_mild (Fib <2) | 0.84489 |
| AEL_50_3 | Dihydrotestosterone sulfate; 14,15-Dihydroxyeicosatrienoic acid (C20:cis[5,8,11]3); Ethanolamine plasmalogen (C39:4) | 3 | Dis_adv (Fib >2) | Dis_mild (Fib <2) | 0.84111 |
| AEL_50a_3 | Dihydrotestosterone sulfate; 14,15-Dihydroxyeicosatrienoic acid (C20:cis[5,8,11]3); Choline plasmalogen (C36:4) | 3 | Dis_adv (Fib >2) | Dis_mild (Fib <2) | 0.84111 |
| AEL_51_3 | Dihydrotestosterone sulfate; 14,15-Dihydroxyeicosatrienoic acid (C20:cis[5,8,11]3); total cholesterol | 3 | Dis_adv (Fib >2) | Dis_mild (Fib <2) | 0.83985 |
| AEL_52_3 | Dihydrotestosterone sulfate; SM_Sphingomyelin (d18:2,C18:0); 11,12-Dihydroxyeicosatrienoic acid (C20:cis[5,8,14]3) | 3 | Dis_adv (Fib >2) | Dis_mild (Fib <2) | 0.83985 |
| AEL_53_3 | Dihydrotestosterone sulfate; 14,15-Dihydroxyeicosatrienoic acid (C20:cis[5,8,11]3); SM_Sphingomyelin (d18:1,C18:0) | 3 | Dis_adv (Fib >2) | Dis_mild (Fib <2) | 0.83985 |
| AEL_54_3 | Dihydrotestosterone sulfate; 11,12-Dihydroxyeicosatrienoic acid (C20:cis[5,8,14]3); SM_Sphingomyelin (d18:2,C20:0) | 3 | Dis_adv (Fib >2) | Dis_mild (Fib <2) | 0.83733 |
| AEL_55_3 | Dihydrotestosterone sulfate; 14,15-Dihydroxyeicosatrienoic acid (C20:cis[5,8,11]3); CE_Cholesterylester C15:0 | 3 | Dis_adv (Fib >2) | Dis_mild (Fib <2) | 0.83733 |

(continued)

| Panel Number | Panel Composition long | No of Metabolites | Positive Group | Negative Group | AUC |
|---|---|---|---|---|---|
| AEL_56_3 | Dihydrotestosterone sulfate; 14,15-Dihydroxyeicosatrienoic acid (C20:cis[5,8,11]3); Choline plasmalogen (C18-vinyl,C20:4) | 3 | Dis_adv (Fib >2) | Dis_mild (Fib <2) | 0.83354 |
| AEL_57_3 | Dihydrotestosterone sulfate; CE_Cholesterylester C20:4; 8,9-Dihydroxyeicosatrienoic acid (C20:cis[5,11,14]3) | 3 | Dis_adv (Fib >2) | Dis_mild (Fib <2) | 0.80832 |
| AEL_58_3 | Dihydrotestosterone sulfate; SM_Sphingomyelin (d18:2,C18:0); 8,9-Dihydroxyeicosatrienoic acid (C20:cis[5,11,14]3) | 3 | Dis_adv (Fib >2) | Dis_mild (Fib <2) | 0.80580 |
| AEC_59_3 | Dihydrotestosterone sulfate; 14,15-Dihydroxyeicosatrienoic acid (C20:cis[5,8,11]3); Glucose | 3 | Dis_adv (Fib >2) | Dis_mild (Fib <2) | 0.90794 |
| AEC_60_3 | Dihydrotestosterone sulfate; Glucose; SUM.EICOSATIENOIC | 3 | Dis_adv (Fib >2) | Dis_mild (Fib <2) | 0.86507 |
| AEC_61_3 | Dihydrotestosterone sulfate; Glucose; 11,12-Dihydroxyeicosatrienoic acid (C20:cis[5,8,14]3) | 3 | Dis_adv (Fib >2) | Dis_mild (Fib <2) | 0.86507 |
| AECL 62_3 | Dihydrotestosterone sulfate; 14,15-Dihydroxyeicosatrienoic acid (C20:cis[5,8,11]3); C-reactive Protein (CRP) | 3 | Dis_adv (Fib >2) | Dis_mild (Fib <2) | 0.90164 |
| AECL 63_3 | Dihydrotestosterone sulfate; 14,15-Dihydroxyeicosatrienoic acid (C20:cis[5,8,11]3); leucocyte | 3 | Dis_adv (Fib >2) | Dis_mild (Fib <2) | 0.87011 |
| AECL 64_3 | Dihydrotestosterone sulfate; 11,12-Dihydroxyeicosatrienoic acid (C20:cis[5,8,14]3); C-reactive Protein (CRP) | 3 | Dis_adv (Fib >2) | Dis_mild (Fib <2) | 0.85876 |
| AECL 65_3 | Dihydrotestosterone sulfate; 14,15-Dihydroxyeicosatrienoic acid (C20:cis[5,8,11]3); alkaline phosphatase (ALP) | 3 | Dis_adv (Fib >2) | Dis_mild (Fib <2) | 0.85120 |
| AECL 66_3 | Dihydrotestosterone sulfate; SUM.EICOSATIENOIC; C-reactive Protein (CRP) | 3 | Dis_adv (Fib >2) | Dis_mild (Fib <2) | 0.83228 |
| AEN_67_3 | Dihydrotestosterone sulfate; 14,15-Dihydroxyeicosatrienoic acid (C20:cis[5,8,11]3); Uridine | 3 | Dis_adv (Fib >2) | Dis_mild (Fib <2) | 0.84994 |
| AEE_68_3 | Dihydrotestosterone sulfate; 14,15-Dihydroxyeicosatrienoic acid (C20:cis[5,8,11]3); 11,12-Dihydroxyeicosatrienoic acid (C20:cis[5,8,14]3) | 3 | Dis_adv (Fib >2) | Dis_mild (Fib <2) | 0.84615 |
| AEE_69_3 | Dihydrotestosterone sulfate; 14,15-Dihydroxyeicosatrienoic acid (C20:cis[5,8,11]3); 11,12-Dihydroxyeicosatrienoic acid (C20:cis[5,8,14]3) | 3 | Dis_adv (Fib >2) | Dis_mild (Fib <2) | 0.84615 |
| AEE_70_3 | Dihydrotestosterone sulfate; 14,15-Dihydroxyeicosatrienoic acid (C20:cis[5,8,11]3); 8,9-Dihydroxyeicosatrienoic acid (C20:cis[5,11,14]3) | 3 | Dis_adv (Fib >2) | Dis_mild (Fib <2) | 0.84237 |

(continued)

| Panel Number | Panel Composition long | No of Metabolites | Positive Group | Negative Group | AUC |
|---|---|---|---|---|---|
| AEE_ 71_3 | Dihydrotestosterone sulfate; 14,15-Dihydroxyeicosatrienoic acid (C20:cis[5,8,11]3); 8,9-Dihydroxyeicosatrienoic acid (C20:cis [5,11,14]3) | 3 | Dis_adv (Fib >2) | Dis_mild (Fib <2) | 0.84237 |
| AEH_ 72_3 | Dihydrotestosterone sulfate; 14,15-Dihydroxyeicosatrienoic acid (C20:cis[5,8,11]3); Testosterone | 3 | Dis_adv (Fib >2) | Dis_mild (Fib <2) | 0.84994 |
| AEH_ 73_3 | Dihydrotestosterone sulfate; 14,15-Dihydroxyeicosatrienoic acid (C20:cis[5,8,11]3); Androstenedione | 3 | Dis_adv (Fib >2) | Dis_mild (Fib <2) | 0.83480 |
| AE_ 74_3 | Dihydrotestosterone sulfate; 14,15-Dihydroxyeicosatrienoic acid (C20:cis[5,8,11]3); | 2 | Dis_adv (Fib >2) | Dis_mild (Fib <2) | 0.85750 |
| AE_ 75_3 | Dihydrotestosterone sulfate; 14,15-Dihydroxyeicosatrienoic acid (C20:cis[5,8,11]3); | 2 | Dis_adv (Fib >2) | Dis_mild (Fib <2) | 0.85750 |
| AE_ 76_3 | Dihydrotestosterone sulfate; 11,12-Dihydroxyeicosatrienoic acid (C20:cis[5,8,14]3); | 2 | Dis_adv (Fib >2) | Dis_mild (Fib <2) | 0.81463 |
| AE_ 77_3 | Dihydrotestosterone sulfate; 11,12-Dihydroxyeicosatrienoic acid (C20:cis[5,8,14]3); | 2 | Dis_adv (Fib >2) | Dis_mild (Fib <2) | 0.81463 |
| AA_ 78_3 | Dihydrotestosterone sulfate; Lysine; | 2 | Dis_adv (Fib >2) | Dis_mild (Fib <2) | 0.84237 |
| AA_ 79_3 | Dihydrotestosterone sulfate; Cystine; | 2 | Dis_adv (Fib >2) | Dis_mild (Fib <2) | 0.82976 |
| AA_ 80_3 | Dihydrotestosterone sulfate; Creatine; | 2 | Dis_adv (Fib >2) | Dis_mild (Fib >2) | 0.81841 |
| AL_81_3 | Dihydrotestosterone sulfate; SM_Sphingomyelin (d18:2,C20:0); | 2 | Dis_adv (Fib >2) | Dis_mild (Fib <2) | 0.81967 |
| AL_82_3 | Dihydrotestosterone sulfate; SM_Sphingomyelin (d18:2,C18:0); | 2 | Dis_adv (Fib >2) | Dis_mild (Fib <2) | 0.81841 |
| AL_83_3 | Dihydrotestosterone sulfate; CE_ Cholesterylester C20:4; | 2 | Dis_adv (Fib >2) | Dis_mild (Fib <2) | 0.81589 |
| AB_ 84_3 | Dihydrotestosterone sulfate; SUM.BILE; | 2 | Dis_adv (Fib >2) | Dis_mild (Fib <2) | 0.93317 |

**Table 2b_1: Multimarker panel composition and their respective Area under the curve (AUC) values of receiver operating characteristics (ROC) analysis**

| Panel Number | Panel Composition long | No of Metabolites | Positive Group | Negative Group | AUC |
|---|---|---|---|---|---|
| AEB_ 83_1 | Glycocholic acid; Androsterone sulfate; 14,15-Dihydroxyeicosatrienoic acid (C20:cis[5,8,11] 3) | 3 | adv (Fib >2) | mild (Fib < 2) | 0.90632 |
| AEB_ 84_1 | SUM.BILE; Androsterone sulfate; 14,15-Dihydroxyeicosatrienoic acid (C20:cis[5,8,11] 3) | 3 | adv (Fib >2) | mild (Fib < 2) | 0.90574 |

(continued)

| Panel Number | Panel Composition long | No of Metabolites | Positive Group | Negative Group | AUC |
|---|---|---|---|---|---|
| AEB_85_1 | Taurocholic acid; Androsterone sulfate; 14,15-Dihydroxyeicosatrienoic acid (C20:cis[5,8,11]3) | 3 | adv (Fib >2) | mild (Fib < 2) | 0.90105 |
| AEB_86_1 | SUM.BILE; Androsterone sulfate; SUM.EICOSATIENOIC | 3 | adv (Fib >2) | mild (Fib < 2) | 0.88115 |
| AEB_87_1 | Taurocholic acid; Androsterone sulfate; SUM.EICOSATIENOIC | 3 | adv (Fib >2) | mild (Fib < 2) | 0.87705 |
| AEB_88_1 | Taurochenodeoxycholic acid; Androsterone sulfate; 14,15-Dihydroxyeicosatrienoic acid (C20:cis[5,8,11]3) | 3 | adv (Fib >2) | mild (Fib < 2) | 0.87354 |
| AEB_88a_1 | Taurodeoxycholic acid; Androsterone sulfate; 14,15-Dihydroxyeicosatrienoic acid (C20:cis [5,8,11]3) | 3 | adv (Fib >2) | mild (Fib < 2) | 0.87354 |
| AEB_89_1 | Glycocholic acid; Androsterone sulfate; SUM.EICOSATIENOIC | 3 | adv (Fib >2) | mild (Fib < 2) | 0.87295 |
| AEB_90_1 | SUM.BILE; Androsterone sulfate; 8,9-Dihydroxyeicosatrienoic acid (C20:cis [5,11,14]3) | 3 | adv (Fib >2) | mild (Fib < 2) | 0.86300 |
| AEB_91_1 | SUM.BILE; Androsterone sulfate; 11,12-Dihydroxyeicosatrienoic acid (C20:cis[5,8,14] 3) | 3 | adv (Fib >2) | mild (Fib < 2) | 0.85597 |
| AEB_92_1 | Taurochenodeoxycholic acid; Androsterone sulfate; SUM.EICOSATIENOIC | 3 | adv (Fib >2) | mild (Fib < 2) | 0.84895 |
| AEB_92a_1 | Taurodeoxycholic acid; Androsterone sulfate; SUM.EICOSATIENOIC | 3 | adv (Fib >2) | mild (Fib < 2) | 0.84895 |
| AEB_93_1 | Taurochenodeoxycholic acid; Androsterone sulfate; 11,12-Dihydroxyeicosatrienoic acid (C20:cis[5,8,14]3) | 3 | adv (Fib >2) | mild (Fib < 2) | 0.83548 |
| AEB_93a_1 | Taurodeoxycholic acid; Androsterone sulfate; 11,12-Dihydroxyeicosatrienoic acid (C20:cis [5,8,14]3) | 3 | adv (Fib >2) | mild (Fib < 2) | 0.83548 |
| AEB_94_1 | Glycocholic acid; Androsterone sulfate; 11,12-Dihydroxyeicosatrienoic acid (C20:cis[5,8,14] 3) | 3 | adv (Fib >2) | mild (Fib < 2) | 0.81938 |
| AEB_95_1 | Taurochenodeoxycholic acid; Androsterone sulfate; 8,9-Dihydroxyeicosatrienoic acid (C20:cis[5,11,14]3) | 3 | adv (Fib >2) | mild (Fib < 2) | 0.81879 |
| AEB_95a_1 | Taurodeoxycholic acid; Androsterone sulfate; 8,9-Dihydroxyeicosatrienoic acid (C20:cis [5,11,14]3) | 3 | adv (Fib >2) | mild (Fib < 2) | 0.81879 |
| AEB_96_1 | Glycocholic acid; Androsterone sulfate; 8,9-Dihydroxyeicosatrienoic acid (C20:cis [5,11,14]3) | 3 | adv (Fib >2) | mild (Fib < 2) | 0.81821 |
| AEB_97_1 | Taurocholic acid; Androsterone sulfate; 11,12-Dihydroxyeicosatrienoic acid (C20:cis[5,8,14] 3) | 3 | adv (Fib >2) | mild (Fib < 2) | 0.81060 |

(continued)

| Panel Number | Panel Composition long | No of Metabolites | Positive Group | Negative Group | AUC |
|---|---|---|---|---|---|
| AEB_98_1 | Taurocholic acid; Androsterone sulfate; 8,9-Dihydroxyeicosatrienoic acid (C20:cis [5,11,14]3) | 3 | adv (Fib >2) | mild (Fib < 2) | 0.81001 |
| AEL_99_1 | Androsterone sulfate; CE_Cholesterylester C20:4; SUM.EICOSATIENOIC | 3 | adv (Fib >2) | mild (Fib < 2) | 0.83138 |
| AEL_100_1 | Androsterone sulfate; SM_Sphingomyelin (d18:2,C20:0); 8,9-Dihydroxyeicosatrienoic acid (C20:cis[5,11,14]3) | 3 | adv (Fib >2) | mild (Fib < 2) | 0.80035 |
| AEL_101_1 | Androsterone sulfate; 14,15-Dihydroxyeicosatrienoic acid (C20:cis[5,8,11]3); SM_Sphingomyelin (d18:2,C20:0) | 3 | adv (Fib >2) | mild (Fib < 2) | 0.84895 |
| AEL_102_1 | Androsterone sulfate; 14,15-Dihydroxyeicosatrienoic acid (C20:cis[5,8,11]3); CE_Cholesterylester C20:4 | 3 | adv (Fib >2) | mild (Fib < 2) | 0.84368 |
| AEL_103_1 | Androsterone sulfate; SUM.EICOSATIENOIC; SM_Sphingomyelin (d18:2,C20:0) | 3 | adv (Fib >2) | mild (Fib < 2) | 0.83489 |
| AEL_104_1 | Androsterone sulfate; 14,15-Dihydroxyeicosatrienoic acid (C20:cis[5,8,11]3); SM_Sphingomyelin (d18:2,C18:0) | 3 | adv (Fib >2) | mild (Fib < 2) | 0.81909 |
| AEA_105_1 | Androsterone sulfate; SUM.EICOSATIENOIC; Cystine | 3 | adv (Fib >2) | mild (Fib < 2) | 0.82875 |
| AEA_106_1 | Androsterone sulfate; 14,15-Dihydroxyeicosatrienoic acid (C20:cis[5,8,11]3); Cystine | 3 | adv (Fib >2) | mild (Fib < 2) | 0.82875 |
| AEA_106a_1 | Androsterone sulfate; Cystine; 8,9-Dihydroxyeicosatrienoic acid (C20:cis [5,11,14]3) | 3 | adv (Fib >2) | mild (Fib < 2) | 0.80006 |
| AB_107_1 | SUM.BILE; Androsterone sulfate; | 2 | adv (Fib >2) | mild (Fib < 2) | 0.90047 |

**Table 2b_2: Multimarker panel composition and their respective Area under the curve (AUC) values of receiver operating characteristics (ROC) analysis**

| Panel Number | Panel Composition long | No of Metabolites | Positive Group | Negative Group | AUC |
|---|---|---|---|---|---|
| AEB_83_2 | Glycocholic acid; Etiocholanolone sulfate; 14,15-Dihydroxyeicosatrienoic acid (C20:cis [5,8,11]3) | 3 | adv (Fib >2) | mild (Fib < 2) | 0.90632 |
| AEB_84_2 | SUM.BILE; Etiocholanolone sulfate; 14,15-Dihydroxyeicosatrienoic acid (C20:cis[5,8,11]3) | 3 | adv (Fib >2) | mild (Fib < 2) | 0.90574 |
| AEB_85_2 | Taurocholic acid; Etiocholanolone sulfate; 14,15-Dihydroxyeicosatrienoic acid (C20:cis [5,8,11]3) | 3 | adv (Fib >2) | mild (Fib < 2) | 0.90105 |

(continued)

| Panel Number | Panel Composition long | No of Metabolites | Positive Group | Negative Group | AUC |
|---|---|---|---|---|---|
| AEB_86_2 | SUM.BILE; Etiocholanolone sulfate; SUM.EICOSATIENOIC | 3 | adv (Fib >2) | mild (Fib < 2) | 0.88115 |
| AEB_87_2 | Taurocholic acid; Etiocholanolone sulfate; SUM.EICOSATIENOIC | 3 | adv (Fib >2) | mild (Fib < 2) | 0.87705 |
| AEB_88_2 | Taurochenodeoxycholic acid; Etiocholanolone sulfate; 14,15-Dihydroxyeicosatrienoic acid (C20:cis[5,8,11]3) | 3 | adv (Fib >2) | mild (Fib < 2) | 0.87354 |
| AEB_88a_2 | Taurodeoxycholic acid; Etiocholanolone sulfate; 14,15-Dihydroxyeicosatrienoic acid (C20:cis[5,8,11]3) | 3 | adv (Fib >2) | mild (Fib < 2) | 0.87354 |
| AEB_89_2 | Glycocholic acid; Etiocholanolone sulfate; SUM.EICOSATIENOIC | 3 | adv (Fib >2) | mild (Fib < 2) | 0.87295 |
| AEB_90_2 | SUM.BILE; Etiocholanolone sulfate; 8,9-Dihydroxyeicosatrienoic acid (C20:cis [5,11,14]3) | 3 | adv (Fib >2) | mild (Fib < 2) | 0.86300 |
| AEB_91_2 | SUM.BILE; Etiocholanolone sulfate; 11,12-Dihydroxyeicosatrienoic acid (C20:cis[5,8,14]3) | 3 | adv (Fib >2) | mild (Fib < 2) | 0.85597 |
| AEB_92_2 | Taurochenodeoxycholic acid; Etiocholanolone sulfate; SUM.EICOSATIENOIC | 3 | adv (Fib >2) | mild (Fib < 2) | 0.84895 |
| AEB_92a_2 | Taurodeoxycholic acid; Etiocholanolone sulfate; SUM.EICOSATIENOIC | 3 | adv (Fib >2) | mild (Fib < 2) | 0.84895 |
| AEB_93_2 | Taurochenodeoxycholic acid; Etiocholanolone sulfate; 11,12-Dihydroxyeicosatrienoic acid (C20:cis[5,8,14]3) | 3 | adv (Fib >2) | mild (Fib < 2) | 0.83548 |
| AEB_93a_2 | Taurodeoxycholic acid; Etiocholanolone sulfate; 11,12-Dihydroxyeicosatrienoic acid (C20:cis[5,8,14]3) | 3 | adv (Fib >2) | mild (Fib < 2) | 0.83548 |
| AEB_94_2 | Glycocholic acid; Etiocholanolone sulfate; 11,12-Dihydroxyeicosatrienoic acid (C20:cis [5,8,14]3) | 3 | adv (Fib >2) | mild (Fib < 2) | 0.81938 |
| AEB_95_2 | Taurochenodeoxycholic acid; Etiocholanolone sulfate; 8,9-Dihydroxyeicosatrienoic acid (C20:cis[5,11,14]3) | 3 | adv (Fib >2) | mild (Fib < 2) | 0.81879 |
| AEB_95a_2 | Taurodeoxycholic acid; Etiocholanolone sulfate; 8,9-Dihydroxyeicosatrienoic acid (C20:cis[5,11,14]3) | 3 | adv (Fib >2) | mild (Fib < 2) | 0.81879 |
| AEB_96_2 | Glycocholic acid; Etiocholanolone sulfate; 8,9-Dihydroxyeicosatrienoic acid (C20:cis [5,11,14]3) | 3 | adv (Fib >2) | mild (Fib < 2) | 0.81821 |
| AEB_97_2 | Taurocholic acid; Etiocholanolone sulfate; 11,12-Dihydroxyeicosatrienoic acid (C20:cis [5,8,14]3) | 3 | adv (Fib >2) | mild (Fib < 2) | 0.81060 |
| AEB_98_2 | Taurocholic acid; Etiocholanolone sulfate; 8,9-Dihydroxyeicosatrienoic acid (C20:cis [5,11,14]3) | 3 | adv (Fib >2) | mild (Fib < 2) | 0.81001 |

(continued)

| Panel Number | Panel Composition long | No of Metabolites | Positive Group | Negative Group | AUC |
|---|---|---|---|---|---|
| AEL_99_2 | Etiocholanolone sulfate; CE_Cholesterylester C20:4; SUM.EICOSATIENOIC | 3 | adv (Fib >2) | mild (Fib < 2) | 0.83138 |
| AEL_100_2 | Etiocholanolone sulfate; SM_Sphingomyelin (d18:2,C20:0); 8,9-Dihydroxyeicosatrienoic acid (C20:cis[5,11,14]3) | 3 | adv (Fib >2) | mild (Fib < 2) | 0.80035 |
| AEL_101_2 | Etiocholanolone sulfate; 14,15-Dihydroxyeicosatrienoic acid (C20:cis[5,8,11]3); SM_Sphingomyelin (d18:2,C20:0) | 3 | adv (Fib >2) | mild (Fib < 2) | 0.84895 |
| AEL_102_2 | Etiocholanolone sulfate; 14,15-Dihydroxyeicosatrienoic acid (C20:cis[5,8,11]3); CE_Cholesterylester C20:4 | 3 | adv (Fib >2) | mild (Fib < 2) | 0.84368 |
| AEL_103_2 | Etiocholanolone sulfate; SUM.EICOSATIENOIC; SM_Sphingomyelin (d18:2,C20:0) | 3 | adv (Fib >2) | mild (Fib < 2) | 0.83489 |
| AEL_104_2 | Etiocholanolone sulfate; 14,15-Dihydroxyeicosatrienoic acid (C20:cis[5,8,11]3); SM_Sphingomyelin (d18:2,C18:0) | 3 | adv (Fib >2) | mild (Fib < 2) | 0.81909 |
| AEA_105_2 | Etiocholanolone sulfate; SUM.EICOSATIENOIC; Cystine | 3 | adv (Fib >2) | mild (Fib < 2) | 0.82875 |
| AEA_106_2 | Etiocholanolone sulfate; 14,15-Dihydroxyeicosatrienoic acid (C20:cis[5,8,11]3); Cystine | 3 | adv (Fib >2) | mild (Fib < 2) | 0.82875 |
| AEA_106a_2 | Etiocholanolone sulfate; Cystine; 8,9-Dihydroxyeicosatrienoic acid (C20:cis[5,11,14]3) | 3 | adv (Fib >2) | mild (Fib < 2) | 0.80006 |
| AB_107_2 | SUM.BILE; Etiocholanolone sulfate; | 2 | adv (Fib >2) | mild (Fib < 2) | 0.90047 |

**Table 2b_3: Multimarker panel composition and their respective Area under the curve (AUC) values of receiver operating characteristics (ROC) analysis**

| Panel Number | Panel Composition long | No of Metabolites | Positive Group | Negative Group | AUC |
|---|---|---|---|---|---|
| AEB_83_3 | Glycocholic acid; Dihydrotestosterone sulfate; 14,15-Dihydroxyeicosatrienoic acid (C20:cis[5,8,11]3) | 3 | adv (Fib >2) | mild (Fib < 2) | 0.90632 |
| AEB_84_3 | SUM.BILE; Dihydrotestosterone sulfate; 14,15-Dihydroxyeicosatrienoic acid (C20:cis[5,8,11]3) | 3 | adv (Fib >2) | mild (Fib < 2) | 0.90574 |
| AEB_85_3 | Taurocholic acid; Dihydrotestosterone sulfate; 14,15-Dihydroxyeicosatrienoic acid (C20:cis[5,8,11]3) | 3 | adv (Fib >2) | mild (Fib < 2) | 0.90105 |
| AEB_86_3 | SUM.BILE; Dihydrotestosterone sulfate; SUM.EICOSATIENOIC | 3 | adv (Fib >2) | mild (Fib < 2) | 0.88115 |
| AEB_87_3 | Taurocholic acid; Dihydrotestosterone sulfate; SUM.EICOSATIENOIC | 3 | adv (Fib >2) | mild (Fib < 2) | 0.87705 |

(continued)

| Panel Number | Panel Composition long | No of Metabolites | Positive Group | Negative Group | AUC |
|---|---|---|---|---|---|
| AEB_88_3 | Taurochenodeoxycholic acid; Dihydrotestosterone sulfate; 14,15-Dihydroxyeicosatrienoic acid (C20:cis[5,8,11]3) | 3 | adv (Fib >2) | mild (Fib < 2) | 0.87354 |
| AEB_88a_3 | Taurodeoxycholic acid; Dihydrotestos-terone sulfate; 14,15-Dihydroxyeicosatrienoic acid (C20: cis[5,8,11]3) | 3 | adv (Fib >2) | mild (Fib < 2) | 0.87354 |
| AEB_89_3 | Glycocholic acid; Dihydrotestosterone sulfate; SUM.EICOSATIENOIC | 3 | adv (Fib >2) | mild (Fib < 2) | 0.87295 |
| AEB_90_3 | SUM.BILE; Dihydrotestosterone sulfate; 8,9-Dihydroxyeicosatrienoic acid (C20:cis[5,11,14]3) | 3 | adv (Fib >2) | mild (Fib < 2) | 0.86300 |
| AEB_91_3 | SUM.BILE; Dihydrotestosterone sulfate; 11,12-Dihydroxyeicosatrienoic acid (C20:cis[5,8,14]3) | 3 | adv (Fib >2) | mild (Fib < 2) | 0.85597 |
| AEB_92_3 | Taurochenodeoxycholic acid; Dihydrotestosterone sulfate; SUM.EICOSATIENOIC | 3 | adv (Fib >2) | mild (Fib < 2) | 0.84895 |
| AEB_92a_3 | Taurodeoxycholic acid; Dihydrotestosterone sulfate; SUM.EICOSATIENOIC | 3 | adv (Fib >2) | mild (Fib < 2) | 0.84895 |
| AEB_93_3 | Taurochenodeoxycholic acid; Dihydrotestosterone sulfate; 11,12-Dihydroxyeicosatrienoic acid (C20:cis[5,8,14]3) | 3 | adv (Fib >2) | mild (Fib < 2) | 0.83548 |
| AEB_93a_3 | Taurodeoxycholic acid; Dihydrotestosterone sulfate; 11,12-Dihydroxyeicosatrienoic acid (C20: cis[5,8,14]3) | 3 | adv (Fib >2) | mild (Fib < 2) | 0.83548 |
| AEB_94_3 | Glycocholic acid; Dihydrotestosterone sulfate; 11,12-Dihydroxyeicosatrienoic acid (C20:cis [5,8,14]3) | 3 | adv (Fib >2) | mild (Fib < 2) | 0.81938 |
| AEB_95_3 | Taurochenodeoxycholic acid; Dihydrotestosterone sulfate; 8,9-Dihydroxyeicosatrienoic acid (C20:cis[5,11,14]3) | 3 | adv (Fib >2) | mild (Fib < 2) | 0.81879 |
| AEB_95a_3 | Taurodeoxycholic acid; Dihydrotestosterone sulfate; 8,9-Dihydroxyeicosatrienoic acid (C20:cis [5,11,14]3) | 3 | adv (Fib >2) | mild (Fib < 2) | 0.81879 |
| AEB_96_3 | Glycocholic acid; Dihydrotestosterone sulfate; 8,9-Dihydroxyeicosatrienoic acid (C20:cis [5,11,14]3) | 3 | adv (Fib >2) | mild (Fib < 2) | 0.81821 |
| AEB_97_3 | Taurocholic acid; Dihydrotestosterone sulfate; 11,12-Dihydroxyeicosatrienoic acid (C20:cis [5,8,14]3) | 3 | adv (Fib >2) | mild (Fib < 2) | 0.81060 |
| AEB_98_3 | Taurocholic acid; Dihydrotestosterone sulfate; 8,9-Dihydroxyeicosatrienoic acid (C20:cis [5,11,14]3) | 3 | adv (Fib >2) | mild (Fib < 2) | 0.81001 |
| AEL_99_3 | Dihydrotestosterone sulfate; CE_Cholesterylester C20:4; SUM.EICOSATIENOIC | 3 | adv (Fib >2) | mild (Fib < 2) | 0.83138 |
| AEL_100_3 | Dihydrotestosterone sulfate; SM_Sphingomyelin (d18:2,C20:0); 8,9-Dihydroxyeicosatrienoic acid (C20:cis[5,11,14]3) | 3 | adv (Fib >2) | mild (Fib < 2) | 0.80035 |

(continued)

| Panel Number | Panel Composition long | No of Metabolites | Positive Group | Negative Group | AUC |
|---|---|---|---|---|---|
| AEL_101_3 | Dihydrotestosterone sulfate; 14,15-Dihydroxyeicosatrienoic acid (C20:cis[5,8,11]3); SM_Sphingomyelin (d18:2,C20:0) | 3 | adv (Fib >2) | mild (Fib < 2) | 0.84895 |
| AEL_102_3 | Dihydrotestosterone sulfate; 14,15-Dihydroxyeicosatrienoic acid (C20:cis[5,8,11]3); CE_Cholesterylester C20:4 | 3 | adv (Fib >2) | mild (Fib < 2) | 0.84368 |
| AEL_103_3 | Dihydrotestosterone sulfate; SUM.EICOSATIENOIC; SM_Sphingomyelin (d18:2,C20:0) | 3 | adv (Fib >2) | mild (Fib < 2) | 0.83489 |
| AEL_104_3 | Dihydrotestosterone sulfate; 14,15-Dihydroxyeicosatrienoic acid (C20:cis[5,8,11]3); SM_Sphingomyelin (d18:2,C18:0) | 3 | adv (Fib >2) | mild (Fib < 2) | 0.81909 |
| AEA_105_3 | Dihydrotestosterone sulfate; SUM.EICOSATIENOIC; Cystine | 3 | adv (Fib >2) | mild (Fib < 2) | 0.82875 |
| AEA_106_3 | Dihydrotestosterone sulfate; 14,15-Dihydroxyeicosatrienoic acid (C20:cis[5,8,11]3); Cystine | 3 | adv (Fib >2) | mild (Fib < 2) | 0.82875 |
| AEA_106a_3 | Dihydrotestosterone sulfate; Cystine; 8,9-Dihydroxyeicosatrienoic acid (C20:cis[5,11,14]3) | 3 | adv (Fib >2) | mild (Fib < 2) | 0.80006 |
| AB_107_3 | SUM.BILE; Dihydrotestosterone sulfate; | 2 | adv (Fib >2) | mild (Fib < 2) | 0.90047 |

**Table 2c_1: Multimarker panel composition and their respective Area under the curve (AUC) values of receiver operating characteristics (ROC) analysis**

| Panel Number | Panel Composition long | No of Metabolites | Positive Group | Negative Group | AUC |
|---|---|---|---|---|---|
| AEA_108_1 | 14,15-Dihydroxyeicosatrienoic acid (C20:cis[5,8,11]3); Androsterone sulfate; Creatine | 3 | NASH adv (Fib >2) | NASH mild (Fib < 2) | 0.92500 |
| AEA_109_1 | 14,15-Dihydroxyeicosatrienoic acid (C20:cis[5,8,11]3); Androsterone sulfate; Cystine | 3 | NASH adv (Fib >2) | NASH mild (Fib < 2) | 0.86667 |
| AEA_110_1 | 14,15-Dihydroxyeicosatrienoic acid (C20:cis[5,8,11]3); Androsterone sulfate; Asparagine | 3 | NASH adv (Fib >2) | NASH mild (Fib < 2) | 0.82917 |
| AEA_111_1 | 14,15-Dihydroxyeicosatrienoic acid (C20:cis[5,8,11]3); Androsterone sulfate; Arginine | 3 | NASH adv (Fib >2) | NASH mild (Fib < 2) | 0.81250 |
| AEA_112_1 | 14,15-Dihydroxyeicosatrienoic acid (C20:cis[5,8,11]3); Androsterone sulfate; Phenylalanine | 3 | NASH adv (Fib >2) | NASH mild (Fib < 2) | 0.80833 |
| AEA_113_1 | Tyrosine; 14,15-Dihydroxyeicosatrienoic acid (C20:cis[5,8,11]3); Androsterone sulfate | 3 | NASH adv (Fib >2) | NASH mild (Fib < 2) | 0.88750 |

(continued)

| Panel Number | Panel Composition long | No of Metabolites | Positive Group | Negative Group | AUC |
|---|---|---|---|---|---|
| AEL_114_1 | 14,15-Dihydroxyeicosatrienoic acid (C20:cis [5,8,11]3); Androsterone sulfate; Phosphatidylcholine (C18:0,C20:4) | 3 | NASH adv (Fib >2) | NASH mild (Fib < 2) | 0.89583 |
| AEL_115_1 | 14,15-Dihydroxyeicosatrienoic acid (C20:cis [5,8,11]3); Androsterone sulfate; CE_Cholesterylester C20:4 | 3 | NASH adv (Fib >2) | NASH mild (Fib < 2) | 0.89583 |
| AEL_116_1 | 14,15-Dihydroxyeicosatrienoic acid (C20:cis [5,8,11]3); Androsterone sulfate; total triacylglyceride | 3 | NASH adv (Fib >2) | NASH mild (Fib < 2) | 0.82500 |
| AEL_117_1 | 14,15-Dihydroxyeicosatrienoic acid (C20:cis [5,8,11]3); Androsterone sulfate; total cholesterol | 3 | NASH adv (Fib >2) | NASH mild (Fib < 2) | 0.81250 |
| AEL_118_1 | 14,15-Dihydroxyeicosatrienoic acid (C20:cis [5,8,11]3); Androsterone sulfate; SM_Sphingomyelin (d18:2,C20:0) | 3 | NASH adv (Fib >2) | NASH mild (Fib < 2) | 0.81250 |
| AEL_119_1 | 14,15-Dihydroxyeicosatrienoic acid (C20:cis [5,8,11]3); Androsterone sulfate; SM_Sphingomyelin (d18:1,C18:0) | 3 | NASH adv (Fib >2) | NASH mild (Fib < 2) | 0.80833 |
| AEL_120_1 | 14,15-Dihydroxyeicosatrienoic acid (C20:cis [5,8,11]3); Androsterone sulfate; Ethanolamine plasmalogen (C39:5) | 3 | NASH adv (Fib >2) | NASH mild (Fib < 2) | 0.80833 |
| AEL_120a_1 | 14,15-Dihydroxyeicosatrienoic acid (C20:cis [5,8,11]3); Androsterone sulfate; Choline plasmalogen (C36:5) | 3 | NASH adv (Fib >2) | NASH mild (Fib < 2) | 0.80833 |
| AEL_121_1 | SM_Sphingomyelin (d18:2,C18:0); Androsterone sulfate; 14,15-Dihydroxyeicosatrienoic acid (C20:cis[5,8,11]3) | 3 | NASH adv (Fib >2) | NASH mild (Fib < 2) | 0.83750 |
| AEB_122_1 | SUM.BILE; Androsterone sulfate; 14,15-Dihydroxyeicosatrienoic acid (C20:cis[5,8,11]3) | 3 | NASH adv (Fib >2) | NASH mild (Fib < 2) | 0.87500 |
| AEB_123_1 | Taurochenodeoxycholic acid; Androsterone sulfate; 14,15-Dihydroxyeicosatrienoic acid (C20:cis[5,8,11]3) | 3 | NASH adv (Fib >2) | NASH mild (Fib < 2) | 0.87083 |
| AEB_123a_1 | Taurodeoxycholic acid; Androsterone sulfate; 14,15-Dihydroxyeicosatrienoic acid (C20:cis [5,8,11]3) | 3 | NASH adv (Fib >2) | NASH mild (Fib < 2) | 0.87083 |
| AEB_124_1 | Taurocholic acid; Androsterone sulfate; 14,15-Dihydroxyeicosatrienoic acid (C20:cis[5,8,11]3) | 3 | NASH adv (Fib >2) | NASH mild (Fib < 2) | 0.87083 |
| AEB_125_1 | Glycocholic acid; 14,15-Dihydroxyeicosatrienoic acid (C20:cis[5,8,11]3); Androsterone sulfate | 3 | NASH adv (Fib >2) | NASH mild (Fib < 2) | 0.88750 |
| AEE_126_1 | 14,15-Dihydroxyeicosatrienoic acid (C20:cis [5,8,11]3); Androsterone sulfate; 8,9-Dihydroxyeicosatrienoic acid (C20:cis[5,11,14]3) | 3 | NASH adv (Fib >2) | NASH mild (Fib < 2) | 0.80000 |

(continued)

| Panel Number | Panel Composition long | No of Metabolites | Positive Group | Negative Group | AUC |
|---|---|---|---|---|---|
| AEE_ 127_1 | 14,15-Dihydroxyeicosatrienoic acid (C20:cis [5,8,11]3); Androsterone sulfate; 11,12-Dihydroxyeicosatrienoic acid (C20:cis[5,8,14]3) | 3 | NASH adv (Fib >2) | NASH mild (Fib < 2) | 0.80000 |
| AEE_ 128_1 | 14,15-Dihydroxyeicosatrienoic acid (C20:cis [5,8,11]3); Androsterone sulfate; 8,9-Dihydroxyeicosatrienoic acid (C20:cis[5,11,14]3) | 3 | NASH adv (Fib >2) | NASH mild (Fib < 2) | 0.80000 |
| AEE_ 129_1 | 14,15-Dihydroxyeicosatrienoic acid (C20:cis [5,8,11]3); Androsterone sulfate; 11,12-Dihydroxyeicosatrienoic acid (C20:cis[5,8,14]3) | 3 | NASH adv (Fib >2) | NASH mild (Fib < 2) | 0.80000 |
| AEH_ 130_1 | 14,15-Dihydroxyeicosatrienoic acid (C20:cis [5,8,11]3); Androsterone sulfate; Androstenedione | 3 | NASH adv (Fib >2) | NASH mild (Fib < 2) | 0.82917 |
| AEH_ 131_1 | 14,15-Dihydroxyeicosatrienoic acid (C20:cis [5,8,11]3); Androsterone sulfate; Testosterone | 3 | NASH adv (Fib >2) | NASH mild (Fib < 2) | 0.82083 |
| AEN_ 132_1 | 14,15-Dihydroxyeicosatrienoic acid (C20:cis [5,8,11]3); Androsterone sulfate; Uridine | 3 | NASH adv (Fib >2) | NASH mild (Fib < 2) | 0.81667 |
| AECP_ 133_1 | 14,15-Dihydroxyeicosatrienoic acid (C20:cis [5,8,11]3); Androsterone sulfate; Leucocyte | 3 | NASH adv (Fib >2) | NASH mild (Fib < 2) | 0.83333 |
| AE_ 134_1 | 14,15-Dihydroxyeicosatrienoic acid (C20:cis [5,8,11]3); Androsterone sulfate; | 2 | NASH adv (Fib >2) | NASH mild (Fib < 2) | 0.83750 |

**Table 2c_2: Multimarker panel composition and their respective Area under the curve (AUC) values of receiver operating characteristics (ROC) analysis**

| Panel Number | Panel Composition long | No of Metabolites | Positive Group | Negative Group | AUC |
|---|---|---|---|---|---|
| AEA_ 108_2 | 14,15-Dihydroxyeicosatrienoic acid (C20:cis [5,8,11]3); Etiocholanolone sulfate; Creatine | 3 | NASH adv (Fib >2) | NASH mild (Fib < 2) | 0.92500 |
| AEA_ 109_2 | 14,15-Dihydroxyeicosatrienoic acid (C20:cis [5,8,11]3); Etiocholanolone sulfate; Cystine | 3 | NASH adv (Fib >2) | NASH mild (Fib < 2) | 0.86667 |
| AEA_ 110_2 | 14,15-Dihydroxyeicosatrienoic acid (C20:cis [5,8,11]3); Etiocholanolone sulfate; Asparagine | 3 | NASH adv (Fib >2) | NASH mild (Fib < 2) | 0.82917 |
| AEA_ 111_2 | 14,15-Dihydroxyeicosatrienoic acid (C20:cis [5,8,11]3); Etiocholanolone sulfate; Arginine | 3 | NASH adv (Fib >2) | NASH mild (Fib < 2) | 0.81250 |
| AEA_ 112_2 | 14,15-Dihydroxyeicosatrienoic acid (C20:cis [5,8,11]3); Etiocholanolone sulfate; Phenylalanine | 3 | NASH adv (Fib >2) | NASH mild (Fib < 2) | 0.80833 |

(continued)

| Panel Number | Panel Composition long | No of Metabolites | Positive Group | Negative Group | AUC |
|---|---|---|---|---|---|
| AEA_ 113_2 | Tyrosine; 14,15-Dihydroxyeicosatrienoic acid (C20:cis[5,8,11]3); Etiocholanolone sulfate | 3 | NASH adv (Fib >2) | NASH mild (Fib < 2) | 0.88750 |
| AEL_ 114_2 | 14,15-Dihydroxyeicosatrienoic acid (C20:cis [5,8,11]3); Etiocholanolone sulfate; Phosphatidylcholine (C18:0,C20:4) | 3 | NASH adv (Fib >2) | NASH mild (Fib < 2) | 0.89583 |
| AEL_ 115_2 | 14,15-Dihydroxyeicosatrienoic acid (C20:cis [5,8,11]3); Etiocholanolone sulfate; CE_ Cholesterylester C20:4 | 3 | NASH adv (Fib >2) | NASH mild (Fib < 2) | 0.89583 |
| AEL_ 116_2 | 14,15-Dihydroxyeicosatrienoic acid (C20:cis [5,8,11]3); Etiocholanolone sulfate; total triacylglyceride | 3 | NASH adv (Fib >2) | NASH mild (Fib < 2) | 0.82500 |
| AEL_ 117_2 | 14,15-Dihydroxyeicosatrienoic acid (C20:cis [5,8,11]3); Etiocholanolone sulfate; total cholesterol | 3 | NASH adv (Fib >2) | NASH mild (Fib < 2) | 0.81250 |
| AEL_ 118_2 | 14,15-Dihydroxyeicosatrienoic acid (C20:cis [5,8,11]3); Etiocholanolone sulfate; SM_ Sphingomyelin (d18:2,C20:0) | 3 | NASH adv (Fib >2) | NASH mild (Fib < 2) | 0.81250 |
| AEL_ 119_2 | 14,15-Dihydroxyeicosatrienoic acid (C20:cis [5,8,11]3); Etiocholanolone sulfate; SM_ Sphingomyelin (d18:1,C18:0) | 3 | NASH adv (Fib >2) | NASH mild (Fib < 2) | 0.80833 |
| AEL_ 120_2 | 14,15-Dihydroxyeicosatrienoic acid (C20:cis [5,8,11]3); Etiocholanolone sulfate; Ethanolamine plasmalogen (C39:5) | 3 | NASH adv (Fib >2) | NASH mild (Fib < 2) | 0.80833 |
| AEL_ 120a_2 | 14,15-Dihydroxyeicosatrienoic acid (C20:cis [5,8,11]3); Etiocholanolone sulfate; Choline plasmalogen (C36:5) | 3 | NASH adv (Fib >2) | NASH mild (Fib < 2) | 0.80833 |
| AEL_ 121_2 | SM_Sphingomyelin (d18:2,C18:0); Etiocholanolone sulfate; 14,15-Dihydroxyeicosatrienoic acid (C20:cis[5,8,11]3) | 3 | NASH adv (Fib >2) | NASH mild (Fib < 2) | 0.83750 |
| AEB_ 122_2 | SUM.BILE; Etiocholanolone sulfate; 14,15-Dihydroxyeicosatrienoic acid (C20:cis[5,8,11]3) | 3 | NASH adv (Fib >2) | NASH mild (Fib < 2) | 0.87500 |
| AEB_ 123_2 | Taurochenodeoxycholic acid; Etiocholanolone sulfate; 14,15-Dihydroxyeicosatrienoic acid (C20: cis[5,8,11]3) | 3 | NASH adv (Fib >2) | NASH mild (Fib < 2) | 0.87083 |
| AEB_ 123a_2 | Taurodeoxycholic acid; Etiocholanolone sulfate; 14,15-Dihydroxyeicosatrienoic acid (C20:cis [5,8,11]3) | 3 | NASH adv (Fib >2) | NASH mild (Fib < 2) | 0.87083 |
| AEB_ 124_2 | Taurocholic acid; Etiocholanolone sulfate; 14,15-Dihydroxyeicosatrienoic acid (C20:cis[5,8,11]3) | 3 | NASH adv (Fib >2) | NASH mild (Fib < 2) | 0.87083 |
| AEB_ 125_2 | Glycocholic acid; 14,15-Dihydroxyeicosatrienoic acid (C20:cis[5,8,11]3); Etiocholanolone sulfate | 3 | NASH adv (Fib >2) | NASH mild (Fib < 2) | 0.88750 |

(continued)

| Panel Number | Panel Composition long | No of Metabolites | Positive Group | Negative Group | AUC |
|---|---|---|---|---|---|
| AEE_ 126_2 | 14,15-Dihydroxyeicosatrienoic acid (C20:cis [5,8,11]3); Etiocholanolone sulfate; 8,9-Dihydroxyeicosatrienoic acid (C20:cis[5,11,14]3) | 3 | NASH adv (Fib >2) | NASH mild (Fib < 2) | 0.80000 |
| AEE_ 127_2 | 14,15-Dihydroxyeicosatrienoic acid (C20:cis [5,8,11]3); Etiocholanolone sulfate; 11,12-Dihydroxyeicosatrienoic acid (C20:cis[5,8,14]3) | 3 | NASH adv (Fib >2) | NASH mild (Fib < 2) | 0.80000 |
| AEE_ 128_2 | 14,15-Dihydroxyeicosatrienoic acid (C20:cis [5,8,11]3); Etiocholanolone sulfate; 8,9-Dihydroxyeicosatrienoic acid (C20:cis[5,11,14]3) | 3 | NASH adv (Fib >2) | NASH mild (Fib >2) | 0.80000 |
| AEE_ 129_2 | 14,15-Dihydroxyeicosatrienoic acid (C20:cis [5,8,11]3); Etiocholanolone sulfate; 11,12-Dihydroxyeicosatrienoic acid (C20:cis[5,8,14]3) | 3 | NASH adv (Fib >2) | NASH mild (Fib < 2) | 0.80000 |
| AEH_ 130_2 | 14,15-Dihydroxyeicosatrienoic acid (C20:cis [5,8,11]3); Etiocholanolone sulfate; Androstenedione | 3 | NASH adv (Fib >2) | NASH mild (Fib < 2) | 0.82917 |
| AEH_ 131_2 | 14,15-Dihydroxyeicosatrienoic acid (C20:cis [5,8,11]3); Etiocholanolone sulfate; Testosterone | 3 | NASH adv (Fib >2) | NASH mild (Fib < 2) | 0.82083 |
| AEN_ 132_2 | 14,15-Dihydroxyeicosatrienoic acid (C20:cis [5,8,11]3); Etiocholanolone sulfate; Uridine | 3 | NASH adv (Fib >2) | NASH mild (Fib < 2) | 0.81667 |
| AECP_ 133_2 | 14,15-Dihydroxyeicosatrienoic acid (C20:cis [5,8,11]3); Etiocholanolone sulfate; Leucocyte | 3 | NASH adv (Fib >2) | NASH mild (Fib < 2) | 0.83333 |
| AE_ 134_2 | 14,15-Dihydroxyeicosatrienoic acid (C20:cis [5,8,11]3); Etiocholanolone sulfate; | 2 | NASH adv (Fib >2) | NASH mild (Fib < 2) | 0.83750 |

**Table 2c_3: Multimarker panel composition and their respective Area under the curve (AUC) values of receiver operating characteristics (ROC) analysis**

| Panel Number | Panel Composition long | No of Metabolites | Positive Group | Negative Group | AUC |
|---|---|---|---|---|---|
| AEA_ 108_3 | 14,15-Dihydroxyeicosatrienoic acid (C20:cis [5,8,11]3); Dihydrotestosterone sulfate; Creatine | 3 | NASH adv (Fib >2) | NASH mild (Fib < 2) | 0.92500 |
| AEA_ 109_3 | 14,15-Dihydroxyeicosatrienoic acid (C20:cis [5,8,11]3); Dihydrotestosterone sulfate; Cystine | 3 | NASH adv (Fib >2) | NASH mild (Fib < 2) | 0.86667 |
| AEA_ 110_3 | 14,15-Dihydroxyeicosatrienoic acid (C20:cis [5,8,11]3); Dihydrotestosterone sulfate; Asparagine | 3 | NASH adv (Fib >2) | NASH mild (Fib < 2) | 0.82917 |
| AEA_ 111_3 | 14,15-Dihydroxyeicosatrienoic acid (C20:cis [5,8,11]3); Dihydrotestosterone sulfate; Arginine | 3 | NASH adv (Fib >2) | NASH mild (Fib < 2) | 0.81250 |

(continued)

| Panel Number | Panel Composition long | No of Metabolites | Positive Group | Negative Group | AUC |
|---|---|---|---|---|---|
| AEA_ 112_3 | 14,15-Dihydroxyeicosatrienoic acid (C20:cis [5,8,11]3); Dihydrotestosterone sulfate; Phenylalanine | 3 | NASH adv (Fib >2) | NASH mild (Fib < 2) | 0.80833 |
| AEA_ 113_3 | Tyrosine; 14,15-Dihydroxyeicosatrienoic acid (C20:cis[5,8,11]3); Dihydrotestosterone sulfate | 3 | NASH adv (Fib >2) | NASH mild (Fib < 2) | 0.88750 |
| AEL_ 114_3 | 14,15-Dihydroxyeicosatrienoic acid (C20:cis [5,8,11]3); Dihydrotestosterone sulfate; Phosphatidylcholine (C18:0,C20:4) | 3 | NASH adv (Fib >2) | NASH mild (Fib < 2) | 0.89583 |
| AEL_ 115_3 | 14,15-Dihydroxyeicosatrienoic acid (C20:cis [5,8,11]3); Dihydrotestosterone sulfate; CE_ Cholesterylester C20:4 | 3 | NASH adv (Fib >2) | NASH mild (Fib >2) | 0.89583 |
| AEL_ 116_3 | 14,15-Dihydroxyeicosatrienoic acid (C20:cis [5,8,11]3); Dihydrotestosterone sulfate; total triacylglyceride | 3 | NASH adv (Fib >2) | NASH mild (Fib < 2) | 0.82500 |
| AEL_ 117_3 | 14,15-Dihydroxyeicosatrienoic acid (C20:cis [5,8,11]3); Dihydrotestosterone sulfate; total cholesterol | 3 | NASH adv (Fib >2) | NASH mild (Fib < 2) | 0.81250 |
| AEL_ 118_3 | 14,15-Dihydroxyeicosatrienoic acid (C20:cis [5,8,11]3); Dihydrotestosterone sulfate; SM_ Sphingomyelin (d18:2,C20:0) | 3 | NASH adv (Fib >2) | NASH mild (Fib < 2) | 0.81250 |
| AEL_ 119_3 | 14,15-Dihydroxyeicosatrienoic acid (C20:cis [5,8,11]3); Dihydrotestosterone sulfate; SM_ Sphingomyelin (d18:1,C18:0) | 3 | NASH adv (Fib >2) | NASH mild (Fib < 2) | 0.80833 |
| AEL_ 120_3 | 14,15-Dihydroxyeicosatrienoic acid (C20:cis [5,8,11]3); Dihydrotestosterone sulfate; Ethanolamine plasmalogen (C39:5) | 3 | NASH adv (Fib >2) | NASH mild (Fib < 2) | 0.80833 |
| AEL_ 120a_3 | 14,15-Dihydroxyeicosatrienoic acid (C20:cis [5,8,11]3); Dihydrotestosterone sulfate; Choline plasmalogen (C36:5) | 3 | NASH adv (Fib >2) | NASH mild (Fib < 2) | 0.80833 |
| AEL_ 121_3 | SM_Sphingomyelin (d18:2,C18:0); Dihydrotestosterone sulfate; 14,15-Dihydroxyeicosatrienoic acid (C20:cis[5,8,11]3) | 3 | NASH adv (Fib >2) | NASH mild (Fib < 2) | 0.83750 |
| AEB_ 122_3 | SUM.BILE; Dihydrotestosterone sulfate; 14,15-Dihydroxyeicosatrienoic acid (C20:cis[5,8,11]3) | 3 | NASH adv (Fib >2) | NASH mild (Fib < 2) | 0.87500 |
| AEB_ 123_3 | Taurochenodeoxycholic acid; Dihydrotestosterone sulfate; 14,15-Dihydroxyeicosatrienoic acid (C20:cis[5,8,11]3) | 3 | NASH adv (Fib >2) | NASH mild (Fib < 2) | 0.87083 |
| AEB_ 123a_3 | Taurodeoxycholic acid; Dihydrotestosterone sulfate; 14,15-Dihydroxyeicosatrienoic acid (C20: cis[5,8,11]3) | 3 | NASH adv (Fib >2) | NASH mild (Fib < 2) | 0.87083 |
| AEB_ 124_3 | Taurocholic acid; Dihydrotestosterone sulfate; 14,15-Dihydroxyeicosatrienoic acid (C20:cis [5,8,11]3) | 3 | NASH adv (Fib >2) | NASH mild (Fib < 2) | 0.87083 |

(continued)

| Panel Number | Panel Composition long | No of Metabolites | Positive Group | Negative Group | AUC |
|---|---|---|---|---|---|
| AEB_125_3 | Glycocholic acid; 14,15-Dihydroxyeicosatrienoic acid (C20:cis[5,8,11]3); Dihydrotestosterone sulfate | 3 | NASH adv (Fib >2) | NASH mild (Fib < 2) | 0.88750 |
| AEE_126_3 | 14,15-Dihydroxyeicosatrienoic acid (C20:cis[5,8,11]3); Dihydrotestosterone sulfate; 8,9-Dihydroxyeicosatrienoic acid (C20:cis[5,11,14]3) | 3 | NASH adv (Fib >2) | NASH mild (Fib < 2) | 0.80000 |
| AEE_127_3 | 14,15-Dihydroxyeicosatrienoic acid (C20:cis[5,8,11]3); Dihydrotestosterone sulfate; 11,12-Dihydroxyeicosatrienoic acid (C20:cis[5,8,14]3) | 3 | NASH adv (Fib >2) | NASH mild (Fib < 2) | 0.80000 |
| AEE_128_3 | 14,15-Dihydroxyeicosatrienoic acid (C20:cis[5,8,11]3); Dihydrotestosterone sulfate; 8,9-Dihydroxyeicosatrienoic acid (C20:cis[5,11,14]3) | 3 | NASH adv (Fib >2) | NASH mild (Fib < 2) | 0.80000 |
| AEE_129_3 | 14,15-Dihydroxyeicosatrienoic acid (C20:cis[5,8,11]3); Dihydrotestosterone sulfate; 11,12-Dihydroxyeicosatrienoic acid (C20:cis[5,8,14]3) | 3 | NASH adv (Fib >2) | NASH mild (Fib < 2) | 0.80000 |
| AEH_130_3 | 14,15-Dihydroxyeicosatrienoic acid (C20:cis[5,8,11]3); Dihydrotestosterone sulfate; Androstenedione | 3 | NASH adv (Fib >2) | NASH mild (Fib < 2) | 0.82917 |
| AEH_131_3 | 14,15-Dihydroxyeicosatrienoic acid (C20:cis[5,8,11]3); Dihydrotestosterone sulfate; Testosterone | 3 | NASH adv (Fib >2) | NASH mild (Fib < 2) | 0.82083 |
| AEN_132_3 | 14,15-Dihydroxyeicosatrienoic acid (C20:cis[5,8,11]3); Dihydrotestosterone sulfate; Uridine | 3 | NASH adv (Fib >2) | NASH mild (Fib < 2) | 0.81667 |
| AECP_133_3 | 14,15-Dihydroxyeicosatrienoic acid (C20:cis[5,8,11]3); Dihydrotestosterone sulfate; Leucocyte | 3 | NASH adv (Fib >2) | NASH mild (Fib < 2) | 0.83333 |
| AE_134_3 | 14,15-Dihydroxyeicosatrienoic acid (C20:cis[5,8,11]3); Dihydrotestosterone sulfate; | 2 | NASH adv (Fib >2) | NASH mild (Fib < 2) | 0.83750 |

**Table 2d: Multimarker panel composition and their respective Area under the curve (AUC) values of receiver operating characteristics (ROC)**

| Panel Number | Panel Composition long | No of Metabolites | Positive Group | Negative Group | AUC |
|---|---|---|---|---|---|
| BEH_1_1 | Androsterone sulfate; Taurochenodeoxycholic acid; 14,15-Dihydroxyeicosatrienoic acid (C20:cis[5,8,11]3) | 3 | Dis_adv (Fib >2) | Dis_mild (Fib <2) | 0.91677 |
| BEH_1a_1 | Androsterone sulfate; Taurodeoxycholic acid; 14,15-Dihydroxyeicosatrienoic acid (C20:cis[5,8,11]3) | 3 | Dis_adv (Fib >2) | Dis_mild (Fib <2) | 0.91677 |
| BEH_1_2 | Etiocholanolone sulfate; Taurochenodeoxycholic acid; 14,15-Dihydroxyeicosatrienoic acid (C20:cis[5,8,11]3) | 3 | Dis_adv (Fib >2) | Dis_mild (Fib <2) | 0.91677 |

(continued)

| Panel Number | Panel Composition long | No of Metabolites | Positive Group | Negative Group | AUC |
|---|---|---|---|---|---|
| BEH_1a_2 | Etiocholanolone sulfate; Taurodeoxycholic acid; 14,15-Dihydroxyeicosatrienoic acid (C20:cis[5,8,11]3) | 3 | Dis_adv (Fib >2) | Dis_mild (Fib <2) | 0.91677 |
| BEH_1_3 | Dihydrotestosterone sulfate; Taurochenodeoxycholic acid; 14,15-Dihydroxyeicosatrienoic acid (C20:cis[5,8,11]3) | 3 | Dis_adv (Fib >2) | Dis_mild (Fib <2) | 0.91677 |
| BEH_1a_3 | Dihydrotestosterone sulfate; Taurodeox-ycholic acid; 14,15-Dihydroxyeicosatrienoic acid (C20:cis[5,8,11]3) | 3 | Dis_adv (Fib >2) | Dis_mild (Fib >2) | 0.91677 |
| BEH_2_1 | Androsterone sulfate; Taurochenodeoxycholic acid; 11,12-Dihydroxyeicosatrienoic acid (C20:cis[5,8,14]3) | 3 | Dis_adv (Fib >2) | Dis_mild (Fib <2) | 0.91047 |
| BEH_2a_1 | Androsterone sulfate; Taurodeoxycholic acid; 11,12-Dihydroxyeicosatrienoic acid (C20:cis[5,8,14]3) | 3 | Dis_adv (Fib >2) | Dis_mild (Fib <2) | 0.91047 |
| BEH_2_2 | Etiocholanolone sulfate; Taurochenodeoxycholic acid; 11,12-Dihydroxyeicosatrienoic acid (C20:cis[5,8,14]3) | 3 | Dis_adv (Fib >2) | Dis_mild (Fib <2) | 0.91047 |
| BEH_2a_2 | Etiocholanolone sulfate; Taurodeoxycholic acid; 11,12-Dihydroxyeicosatrienoic acid (C20:cis[5,8,14]3) | 3 | Dis_adv (Fib >2) | Dis_mild (Fib <2) | 0.91047 |
| BEH_2_3 | Dihydrotestosterone sulfate; Taurochenodeoxycholic acid; 11,12-Dihydroxyeicosatrienoic acid (C20:cis[5,8,14]3) | 3 | Dis_adv (Fib >2) | Dis_mild (Fib <2) | 0.91047 |
| BEH_2a_3 | Dihydrotestosterone sulfate; Taurodeoxycholic acid; 11,12-Dihydroxyeicosatrienoic acid (C20:cis[5,8,14]3) | 3 | Dis_adv (Fib >2) | Dis_mild (Fib <2) | 0.91047 |
| BEH_3 | Dehydroepiandrosterone sulfate; Taurochenodeoxycholic acid; 14,15-Dihydroxyeicosatrienoic acid (C20:cis[5,8,11]3) | 3 | Dis_adv (Fib >2) | Dis_mild (Fib <2) | 0.88651 |
| BEH_3a | Dehydroepiandrosterone sulfate; Taurodeoxycholic acid; 14,15-Dihydroxyeicosatrienoic acid (C20:cis[5,8,11]3) | 3 | Dis_adv (Fib >2) | Dis_mild (Fib <2) | 0.88651 |
| BEH_3b | Testosterone-17-sulfate; Taurochenodeoxycholic acid; 14,15-Dihydroxyeicosatrienoic acid (C20:cis[5,8,11]3) | 3 | Dis_adv (Fib >2) | Dis_mild (Fib <2) | 0.88651 |
| BEH_3c | Testosterone-17-sulfate; Taurodeoxycholic acid; 14,15-Dihydroxyeicosatrienoic acid (C20:cis[5,8,11]3) | 3 | Dis_adv (Fib >2) | Dis_mild (Fib <2) | 0.88651 |
| BEH_4 | Dehydroepiandrosterone sulfate; Taurochenodeoxycholic acid; 11,12-Dihydroxyeicosatrienoic acid (C20:cis[5,8,14]3) | 3 | Dis_adv (Fib >2) | Dis_mild (Fib <2) | 0.86885 |
| BEH_4a | Dehydroepiandrosterone sulfate; Taurodeoxycholic acid; 11,12-Dihydroxyeicosatrienoic acid (C20:cis[5,8,14]3) | 3 | Dis_adv (Fib >2) | Dis_mild (Fib <2) | 0.86885 |

(continued)

| Panel Number | Panel Composition long | No of Metabolites | Positive Group | Negative Group | AUC |
|---|---|---|---|---|---|
| BEH_4b | Testosterone-17-sulfate; Tau-rochenodeoxycholic acid; 11,12-Dihydroxyeicosatrienoic acid (C20:cis[5,8,14]3) | 3 | Dis_adv (Fib >2) | Dis_mild (Fib >2) | 0.86885 |
| BEH_4c | Testosterone-17-sulfate; Taurodeoxycholic acid; 11,12-Dihydroxyeicosatrienoic acid (C20:cis [5,8,14]3) | 3 | Dis_adv (Fib >2) | Dis_mild (Fib <2) | 0.86885 |
| BEH_5 | Taurochenodeoxycholic acid; 14,15-Dihydroxyeicosatrienoic acid (C20:cis[5,8,11]3); Testosterone | 3 | Dis_adv (Fib >2) | Dis_mild (Fib <2) | 0.84237 |
| BEH_5a | Taurodeoxycholic acid; 14,15-Dihydroxyeicosatrienoic acid (C20:cis[5,8,11]3); Testosterone | 3 | Dis_adv (Fib >2) | Dis_mild (Fib <2) | 0.84237 |
| BEH_6 | Taurochenodeoxycholic acid; 11,12-Dihydroxyeicosatrienoic acid (C20:cis[5,8,14]3); Testosterone | 3 | Dis_adv (Fib >2) | Dis_mild (Fib <2) | 0.80706 |
| BEH_6a | Taurodeoxycholic acid; 11,12-Dihydroxyeicosatrienoic acid (C20:cis[5,8,14]3); Testosterone | 3 | Dis_adv (Fib >2) | Dis_mild (Fib <2) | 0.80706 |
| BEH_7 | Taurochenodeoxycholic acid; 14,15-Dihydroxyeicosatrienoic acid (C20:cis[5,8,11]3); Androstenedione | 3 | Dis_adv (Fib >2) | Dis_mild (Fib <2) | 0.82976 |
| BEH_7a | Taurodeoxycholic acid; 14,15-Dihydroxyeicosatrienoic acid (C20:cis[5,8,11]3); Androstenedione | 3 | Dis_adv (Fib >2) | Dis_mild (Fib <2) | 0.82976 |
| BEH_8 | Taurochenodeoxycholic acid; 11,12-Dihydroxyeicosatrienoic acid (C20:cis[5,8,14]3); Androstenedione | 3 | Dis_adv (Fib >2) | Dis_mild (Fib <2) | 0.80454 |
| BEH_8a | Taurodeoxycholic acid; 11,12-Dihydroxyeicosatrienoic acid (C20:cis[5,8,14]3); Androstenedione | 3 | Dis_adv (Fib >2) | Dis_mild (Fib <2) | 0.80454 |
| BEL_9 | Taurochenodeoxycholic acid; 14,15-Dihydroxyeicosatrienoic acid (C20:cis[5,8,11]3); Ethanolamine plasmalogen (C39:5) | 3 | Dis_adv (Fib >2) | Dis_mild (Fib <2) | 0.89912 |
| BEL_9a | Taurochenodeoxycholic acid; 14,15-Dihydroxyeicosatrienoic acid (C20:cis[5,8,11]3); Choline plasmalogen (C36:5) | 3 | Dis_adv (Fib >2) | Dis_mild (Fib <2) | 0.89912 |
| BEL_9b | Taurodeoxycholic acid; 14,15-Dihydroxyeicosatrienoic acid (C20:cis[5,8,11]3); Ethanolamine plasmalogen (C39:5) | 3 | Dis_adv (Fib >2) | Dis_mild (Fib <2) | 0.89912 |
| BEL_9c | Taurodeoxycholic acid; 14,15-Dihydroxyeicosatrienoic acid (C20:cis[5,8,11]3); Choline plasmalogen (C36:5) | 3 | Dis_adv (Fib >2) | Dis_mild (Fib <2) | 0.89912 |
| BEL_10 | Taurochenodeoxycholic acid; CE_Cholesterylester C20:4; 14,15-Dihydroxyeicosatrienoic acid (C20:cis[5,8,11]3) | 3 | Dis_adv (Fib >2) | Dis_mild (Fib <2) | 0.87264 |

(continued)

| Panel Number | Panel Composition long | No of Metabolites | Positive Group | Negative Group | AUC |
|---|---|---|---|---|---|
| BEL_ 10a | Taurodeoxycholic acid; CE_Cholesterylester C20:4; 14,15-Dihydroxyeicosatrienoic acid (C20:cis[5,8,11]3) | 3 | Dis_adv (Fib >2) | Dis_mild (Fib <2) | 0.87264 |
| BEL_11 | Taurochenodeoxycholic acid; Ethanolamine plasmalogen (C39:5); 11,12-Dihydroxyeicosatrienoic acid (C20:cis[5,8,14]3) | 3 | Dis_adv (Fib >2) | Dis_mild (Fib <2) | 0.86885 |
| BEL_ 11a | Taurochenodeoxycholic acid; Choline plasmalogen (C36:5); 11,12-Dihydroxyeicosatrienoic acid (C20:cis[5,8,14]3) | 3 | Dis_adv (Fib >2) | Dis_mild (Fib <2) | 0.86885 |
| BEL_ 11b | Taurodeoxycholic acid; Ethanolamine plasmalogen (C39:5); 11,12-Dihydroxyeicosatrienoic acid (C20:cis[5,8,14]3) | 3 | Dis_adv (Fib >2) | Dis_mild (Fib <2) | 0.86885 |
| BEL_11c | Taurodeoxycholic acid; Choline plasmalogen (C36:5); 11,12-Dihydroxyeicosatrienoic acid (C20:cis[5,8,14]3) | 3 | Dis_adv (Fib >2) | Dis_mild (Fib <2) | 0.86885 |
| BEL_12 | Taurochenodeoxycholic acid; CE_Cholesterylester C20:4; 11,12-Dihydroxyeicosatrienoic acid (C20:cis[5,8,14]3) | 3 | Dis_adv (Fib >2) | Dis_mild (Fib <2) | 0.85750 |
| BEL_ 12a | Taurodeoxycholic acid; CE_Cholesterylester C20:4; 11,12-Dihydroxyeicosatrienoic acid (C20:cis[5,8,14]3) | 3 | Dis_adv (Fib >2) | Dis_mild (Fib <2) | 0.85750 |
| BEL_13 | Taurochenodeoxycholic acid; 14,15-Dihydroxyeicosatrienoic acid (C20:cis[5,8,11]3); Ethanolamine plasmalogen (C39:4) | 3 | Dis_adv (Fib >2) | Dis_mild (Fib <2) | 0.85372 |
| BEL_ 13a | Taurochenodeoxycholic acid; 14,15-Dihydroxyeicosatrienoic acid (C20:cis[5,8,11]3); Choline plasmalogen (C36:4) | 3 | Dis_adv (Fib >2) | Dis_mild (Fib <2) | 0.85372 |
| BEL_ 13b | Taurodeoxycholic acid; 14,15-Dihydroxyeicosatrienoic acid (C20:cis[5,8,11]3); Ethanolamine plasmalogen (C39:4) | 3 | Dis_adv (Fib >2) | Dis_mild (Fib <2) | 0.85372 |
| BEL_13c | Taurodeoxycholic acid; 14,15-Dihydroxyeicosatrienoic acid (C20:cis[5,8,11]3); Choline plasmalogen (C36:4) | 3 | Dis_adv (Fib >2) | Dis_mild (Fib <2) | 0.85372 |
| BEL_14 | Taurochenodeoxycholic acid; SM_Sphingomyelin (d18:2,C18:0); 14,15-Dihydroxyeicosatrienoic acid (C20:cis[5,8,11]3) | 3 | Dis_adv (Fib >2) | Dis_mild (Fib <2) | 0.84994 |
| BEL_ 14a | Taurodeoxycholic acid; SM_Sphingomyelin (d18:2,C18:0); 14,15-Dihydroxyeicosatrienoic acid (C20:cis[5,8,11]3) | 3 | Dis_adv (Fib >2) | Dis_mild (Fib <2) | 0.84994 |
| BEL_15 | Taurochenodeoxycholic acid; 14,15-Dihydroxyeicosatrienoic acid (C20:cis[5,8,11]3); Phosphatidylcholine (C18:0,C20:4) | 3 | Dis_adv (Fib >2) | Dis_mild (Fib <2) | 0.84615 |
| BEL_ 15a | Taurodeoxycholic acid; 14,15-Dihydroxyeicosatrienoic acid (C20:cis[5,8,11]3); Phosphatidylcholine (C18:0,C20:4) | 3 | Dis_adv (Fib >2) | Dis_mild (Fib <2) | 0.84615 |

(continued)

| Panel Number | Panel Composition long | No of Metabolites | Positive Group | Negative Group | AUC |
|---|---|---|---|---|---|
| BEL_16 | Taurochenodeoxycholic acid; 14,15-Dihydroxyeicosatrienoic acid (C20:cis[5,8,11]3); Choline plasmalogen (C18-vinyl,C20:4) | 3 | Dis_adv (Fib >2) | Dis_mild (Fib <2) | 0.84237 |
| BEL_16a | Taurodeoxycholic acid; 14,15-Dihydroxyeicosatrienoic acid (C20:cis[5,8,11]3); Choline plasmalogen (C18-vinyl,C20:4) | 3 | Dis_adv (Fib >2) | Dis_mild (Fib <2) | 0.84237 |
| BEL_17 | Taurochenodeoxycholic acid; 11,12-Dihydroxyeicosatrienoic acid (C20:cis[5,8,14]3); Ethanolamine plasmalogen (C39:4) | 3 | Dis_adv (Fib >2) | Dis_mild (Fib <2) | 0.83859 |
| BEL_17a | Taurochenodeoxycholic acid; 11,12-Dihydroxyeicosatrienoic acid (C20:cis[5,8,14]3); Choline plasmalogen (C36:4) | 3 | Dis_adv (Fib >2) | Dis_mild (Fib <2) | 0.83859 |
| BEL_17b | Taurodeoxycholic acid; 11,12-Dihydroxyeicosatrienoic acid (C20:cis[5,8,14]3); Ethanolamine plasmalogen (C39:4) | 3 | Dis_adv (Fib >2) | Dis_mild (Fib <2) | 0.83859 |
| BEL_17c | Taurodeoxycholic acid; 11,12-Dihydroxyeicosatrienoic acid (C20:cis[5,8,14]3); Choline plasmalogen (C36:4) | 3 | Dis_adv (Fib >2) | Dis_mild (Fib <2) | 0.83859 |
| BEL_18 | Taurochenodeoxycholic acid; 14,15-Dihydroxyeicosatrienoic acid (C20:cis[5,8,11]3); total cholesterol | 3 | Dis_adv (Fib >2) | Dis_mild (Fib <2) | 0.83733 |
| BEL_18a | Taurodeoxycholic acid; 14,15-Dihydroxyeicosatrienoic acid (C20:cis[5,8,11]3); total cholesterol | 3 | Dis_adv (Fib >2) | Dis_mild (Fib <2) | 0.83733 |
| BEL_19 | Taurochenodeoxycholic acid; SM_Sphingomyelin (d18:2,C18:0); 11,12-Dihydroxyeicosatrienoic acid (C20:cis[5,8,14]3) | 3 | Dis_adv (Fib >2) | Dis_mild (Fib <2) | 0.83607 |
| BEL_19a | Taurodeoxycholic acid; SM_Sphingomyelin (d18:2,C18:0); 11,12-Dihydroxyeicosatrienoic acid (C20:cis[5,8,14]3) | 3 | Dis_adv (Fib >2) | Dis_mild (Fib <2) | 0.83607 |
| BEL_20 | Taurochenodeoxycholic acid; 14,15-Dihydroxyeicosatrienoic acid (C20:cis[5,8,11]3); total triacylglyceride | 3 | Dis_adv (Fib >2) | Dis_mild (Fib <2) | 0.82976 |
| BEL_20a | Taurodeoxycholic acid; 14,15-Dihydroxyeicosatrienoic acid (C20:cis[5,8,11]3); total triacylglyceride | 3 | Dis_adv (Fib >2) | Dis_mild (Fib <2) | 0.82976 |
| BEL_21 | Taurochenodeoxycholic acid; 14,15-Dihydroxyeicosatrienoic acid (C20:cis[5,8,11]3); CE_Cholesterylester C15:0 | 3 | Dis_adv (Fib >2) | Dis_mild (Fib <2) | 0.82219 |
| BEL_21a | Taurodeoxycholic acid; 14,15-Dihydroxyeicosatrienoic acid (C20:cis[5,8,11]3); CE_Cholesterylester C15:0 | 3 | Dis_adv (Fib >2) | Dis_mild (Fib <2) | 0.82219 |
| BEL_22 | Taurochenodeoxycholic acid; Phosphatidylcholine (C18:0,C20:4); 11,12-Dihydroxyeicosatrienoic acid (C20:cis[5,8,14]3) | 3 | Dis_adv (Fib >2) | Dis_mild (Fib <2) | 0.81463 |

(continued)

| Panel Number | Panel Composition long | No of Metabolites | Positive Group | Negative Group | AUC |
|---|---|---|---|---|---|
| BEL_22a | Taurodeoxycholic acid; Phosphatidylcholine (C18:0,C20:4); 11,12-Dihydroxyeicosatrienoic acid (C20:cis[5,8,14]3) | 3 | Dis_adv (Fib >2) | Dis_mild (Fib <2) | 0.81463 |
| BEL_23 | Taurochenodeoxycholic acid; 14,15-Dihydroxyeicosatrienoic acid (C20:cis[5,8,11]3); SM_Sphingomyelin (d18:2,C20:0) | 3 | Dis_adv (Fib >2) | Dis_mild (Fib <2) | 0.81084 |
| BEL_23a | Taurodeoxycholic acid; 14,15-Dihydroxyeicosatrienoic acid (C20:cis[5,8,11]3); SM_Sphingomyelin (d18:2,C20:0) | 3 | Dis_adv (Fib >2) | Dis_mild (Fib <2) | 0.81084 |
| BEL_24 | Taurochenodeoxycholic acid; 11,12-Dihydroxyeicosatrienoic acid (C20:cis[5,8,14]3); Choline plasmalogen (C18-vinyl,C20:4) | 3 | Dis_adv (Fib >2) | Dis_mild (Fib <2) | 0.81084 |
| BEL_24a | Taurodeoxycholic acid; 11,12-Dihydroxyeicosatrienoic acid (C20:cis[5,8,14]3); Choline plasmalogen (C18-vinyl,C20:4) | 3 | Dis_adv (Fib >2) | Dis_mild (Fib <2) | 0.81084 |
| BEL_25 | Taurochenodeoxycholic acid; 11,12-Dihydroxyeicosatrienoic acid (C20:cis[5,8,14]3); total cholesterol | 3 | Dis_adv (Fib >2) | Dis_mild (Fib <2) | 0.80202 |
| BEL_25a | Taurodeoxycholic acid; 11,12-Dihydroxyeicosatrienoic acid (C20:cis[5,8,14]3); total cholesterol | 3 | Dis_adv (Fib >2) | Dis_mild (Fib <2) | 0.80202 |
| BEL_26 | Taurochenodeoxycholic acid; 11,12-Dihydroxyeicosatrienoic acid (C20:cis[5,8,14]3); total triacylglyceride | 3 | Dis_adv (Fib >2) | Dis_mild (Fib <2) | 0.80202 |
| BEL_26a | Taurodeoxycholic acid; 11,12-Dihydroxyeicosatrienoic acid (C20:cis[5,8,14]3); total triacylglyceride | 3 | Dis_adv (Fib >2) | Dis_mild (Fib <2) | 0.80202 |
| BEC_27 | Taurochenodeoxycholic acid; Glucose; 14,15-Dihydroxyeicosatrienoic acid (C20:cis[5,8,11]3) | 3 | Dis_adv (Fib >2) | Dis_mild (Fib <2) | 0.87137 |
| BEC_27a | Taurodeoxycholic acid; Glucose; 14,15-Dihydroxyeicosatrienoic acid (C20:cis[5,8,11]3) | 3 | Dis_adv (Fib >2) | Dis_mild (Fib <2) | 0.87137 |
| BEC_28 | Taurochenodeoxycholic acid; Glucose; 11,12-Dihydroxyeicosatrienoic acid (C20:cis[5,8,14]3) | 3 | Dis_adv (Fib >2) | Dis_mild (Fib <2) | 0.87137 |
| BEC_28a | Taurodeoxycholic acid; Glucose; 11,12-Dihydroxyeicosatrienoic acid (C20:cis[5,8,14]3) | 3 | Dis_adv (Fib >2) | Dis_mild (Fib <2) | 0.87137 |
| BEA_29 | Taurochenodeoxycholic acid; 14,15-Dihydroxyeicosatrienoic acid (C20:cis[5,8,11]3); Cystine | 3 | Dis_adv (Fib >2) | Dis_mild (Fib <2) | 0.86129 |
| BEA_29a | Taurodeoxycholic acid; 14,15-Dihydroxyeicosatrienoic acid (C20:cis[5,8,11]3); Cystine | 3 | Dis_adv (Fib >2) | Dis_mild (Fib <2) | 0.86129 |
| BEA_30 | Taurochenodeoxycholic acid; Creatinine; 14,15-Dihydroxyeicosatrienoic acid (C20:cis[5,8,11]3) | 3 | Dis_adv (Fib >2) | Dis_mild (Fib <2) | 0.84741 |
| BEA_30a | Taurodeoxycholic acid; Creatinine; 14,15-Dihydroxyeicosatrienoic acid (C20:cis[5,8,11]3) | 3 | Dis_adv (Fib >2) | Dis_mild (Fib <2) | 0.84741 |

(continued)

| Panel Number | Panel Composition long | No of Metabolites | Positive Group | Negative Group | AUC |
|---|---|---|---|---|---|
| BEA_31 | Taurochenodeoxycholic acid; 14,15-Dihydroxyeicosatrienoic acid (C20:cis[5,8,11]3); Histidine | 3 | Dis_adv (Fib >2) | Dis_mild (Fib <2) | 0.83985 |
| BEA_31a | Taurodeoxycholic acid; 14,15-Dihydroxyeicosatrienoic acid (C20:cis[5,8,11]3); Histidine | 3 | Dis_adv (Fib >2) | Dis_mild (Fib <2) | 0.83985 |
| BEA_32 | Taurochenodeoxycholic acid; 14,15-Dihydroxyeicosatrienoic acid (C20:cis[5,8,11]3); Tryptophan | 3 | Dis_adv (Fib >2) | Dis_mild (Fib <2) | 0.83859 |
| BEA_32a | Taurodeoxycholic acid; 14,15-Dihydroxyeicosatrienoic acid (C20:cis[5,8,11]3); Tryptophan | 3 | Dis_adv (Fib >2) | Dis_mild (Fib <2) | 0.83859 |
| BEA_33 | Taurochenodeoxycholic acid; 14,15-Dihydroxyeicosatrienoic acid (C20:cis[5,8,11]3); Arginine | 3 | Dis_adv (Fib >2) | Dis_mild (Fib <2) | 0.83859 |
| BEA_33a | Taurodeoxycholic acid; 14,15-Dihydroxyeicosatrienoic acid (C20:cis[5,8,11]3); Arginine | 3 | Dis_adv (Fib >2) | Dis_mild (Fib <2) | 0.83859 |
| BEA_34 | Taurochenodeoxycholic acid; 14,15-Dihydroxyeicosatrienoic acid (C20:cis[5,8,11]3); Glutamine | 3 | Dis_adv (Fib >2) | Dis_mild (Fib <2) | 0.83859 |
| BEA_34a | Taurodeoxycholic acid; 14,15-Dihydroxyeicosatrienoic acid (C20:cis[5,8,11]3); Glutamine | 3 | Dis_adv (Fib >2) | Dis_mild (Fib <2) | 0.83859 |
| BEA_35 | Taurochenodeoxycholic acid; 14,15-Dihydroxyeicosatrienoic acid (C20:cis[5,8,11]3); Asparagine | 3 | Dis_adv (Fib >2) | Dis_mild (Fib <2) | 0.83859 |
| BEA_35a | Taurodeoxycholic acid; 14,15-Dihydroxyeicosatrienoic acid (C20:cis[5,8,11]3); Asparagine | 3 | Dis_adv (Fib >2) | Dis_mild (Fib <2) | 0.83859 |
| BEA_36 | Taurochenodeoxycholic acid; Creatinine; 11,12-Dihydroxyeicosatrienoic acid (C20:cis[5,8,14]3) | 3 | Dis_adv (Fib >2) | Dis_mild (Fib <2) | 0.83859 |
| BEA_36a | Taurodeoxycholic acid; Creatinine; 11,12-Dihydroxyeicosatrienoic acid (C20:cis[5,8,14]3) | 3 | Dis_adv (Fib >2) | Dis_mild (Fib <2) | 0.83859 |
| BEA_37 | Taurochenodeoxycholic acid; Cystine; 11,12-Dihydroxyeicosatrienoic acid (C20:cis[5,8,14]3) | 3 | Dis_adv (Fib >2) | Dis_mild (Fib <2) | 0.83733 |
| BEA_37a | Taurodeoxycholic acid; Cystine; 11,12-Dihydroxyeicosatrienoic acid (C20:cis[5,8,14]3) | 3 | Dis_adv (Fib >2) | Dis_mild (Fib <2) | 0.83733 |
| BEA_38 | Taurochenodeoxycholic acid; 14,15-Dihydroxyeicosatrienoic acid (C20:cis[5,8,11]3); Phenylalanine | 3 | Dis_adv (Fib >2) | Dis_mild (Fib <2) | 0.83480 |
| BEA_38a | Taurodeoxycholic acid; 14,15-Dihydroxyeicosatrienoic acid (C20:cis[5,8,11]3); Phenylalanine | 3 | Dis_adv (Fib >2) | Dis_mild (Fib <2) | 0.83480 |
| BEA_39 | Taurochenodeoxycholic acid; Tyrosine; 14,15-Dihydroxyeicosatrienoic acid (C20:cis[5,8,11]3) | 3 | Dis_adv (Fib >2) | Dis_mild (Fib <2) | 0.83228 |

(continued)

| Panel Number | Panel Composition long | No of Metabolites | Positive Group | Negative Group | AUC |
|---|---|---|---|---|---|
| BEA_39a | Taurodeoxycholic acid; Tyrosine; 14,15-Dihydroxyeicosatrienoic acid (C20:cis[5,8,11]3) | 3 | Dis_adv (Fib >2) | Dis_mild (Fib <2) | 0.83228 |
| BEA_40 | Taurochenodeoxycholic acid; 11,12-Dihydroxyeicosatrienoic acid (C20:cis[5,8,14]3); Histidine | 3 | Dis_adv (Fib >2) | Dis_mild (Fib <2) | 0.82724 |
| BEA_40a | Taurodeoxycholic acid; 11,12-Dihydroxyeicosatrienoic acid (C20:cis[5,8,14]3); Histidine | 3 | Dis_adv (Fib >2) | Dis_mild (Fib <2) | 0.82724 |
| BEA_41 | Taurochenodeoxycholic acid; Lysine; 14,15-Dihydroxyeicosatrienoic acid (C20:cis[5,8,11]3) | 3 | Dis_adv (Fib >2) | Dis_mild (Fib <2) | 0.82472 |
| BEA_41a | Taurodeoxycholic acid; Lysine; 14,15-Dihydroxyeicosatrienoic acid (C20:cis[5,8,11]3) | 3 | Dis_adv (Fib >2) | Dis_mild (Fib <2) | 0.82472 |
| BEA_42 | Taurochenodeoxycholic acid; Lysine; 11,12-Dihydroxyeicosatrienoic acid (C20:cis[5,8,14]3) | 3 | Dis_adv (Fib >2) | Dis_mild (Fib <2) | 0.81589 |
| BEA_42a | Taurodeoxycholic acid; Lysine; 11,12-Dihydroxyeicosatrienoic acid (C20:cis[5,8,14]3) | 3 | Dis_adv (Fib >2) | Dis_mild (Fib <2) | 0.81589 |
| BEA_43 | Taurochenodeoxycholic acid; 11,12-Dihydroxyeicosatrienoic acid (C20:cis[5,8,14]3); Urea | 3 | Dis_adv (Fib >2) | Dis_mild (Fib <2) | 0.81211 |
| BEA_43a | Taurodeoxycholic acid; 11,12-Dihydroxyeicosatrienoic acid (C20:cis[5,8,14]3); Urea | 3 | Dis_adv (Fib >2) | Dis_mild (Fib <2) | 0.81211 |
| BEA_44 | Taurochenodeoxycholic acid; 11,12-Dihydroxyeicosatrienoic acid (C20:cis[5,8,14]3); Tryptophan | 3 | Dis_adv (Fib >2) | Dis_mild (Fib >2) | 0.80454 |
| BEA_44a | Taurodeoxycholic acid; 11,12-Dihydroxyeicosatrienoic acid (C20:cis[5,8,14]3); Tryptophan | 3 | Dis_adv (Fib >2) | Dis_mild (Fib <2) | 0.80454 |
| BEA_45 | Taurochenodeoxycholic acid; 11,12-Dihydroxyeicosatrienoic acid (C20:cis[5,8,14]3); Glutamine | 3 | Dis_adv (Fib >2) | Dis_mild (Fib <2) | 0.80328 |
| BEA_45a | Taurodeoxycholic acid; 11,12-Dihydroxyeicosatrienoic acid (C20:cis[5,8,14]3); Glutamine | 3 | Dis_adv (Fib >2) | Dis_mild (Fib <2) | 0.80328 |
| BEA_46 | Taurochenodeoxycholic acid; 11,12-Dihydroxyeicosatrienoic acid (C20:cis[5,8,14]3); Asparagine | 3 | Dis_adv (Fib >2) | Dis_mild (Fib <2) | 0.80328 |
| BEA_46a | Taurodeoxycholic acid; 11,12-Dihydroxyeicosatrienoic acid (C20:cis[5,8,14]3); Asparagine | 3 | Dis_adv (Fib >2) | Dis_mild (Fib <2) | 0.80328 |
| BECP_47 | Taurochenodeoxycholic acid; 14,15-Dihydroxyeicosatrienoic acid (C20:cis[5,8,11]3); C-reactive Protein (CRP) | 3 | Dis_adv (Fib >2) | Dis_mild (Fib <2) | 0.85246 |
| BECP_47a | Taurodeoxycholic acid; 14,15-Dihydroxyeicosatrienoic acid (C20:cis[5,8,11]3); C-reactive Protein (CRP) | 3 | Dis_adv (Fib >2) | Dis_mild (Fib <2) | 0.85246 |

(continued)

| Panel Number | Panel Composition long | No of Metabolites | Positive Group | Negative Group | AUC |
|---|---|---|---|---|---|
| BECP_ 48 | Taurochenodeoxycholic acid; 14,15-Dihydroxyeicosatrienoic acid (C20:cis[5,8,11]3); leucocytes | 3 | Dis_adv (Fib >2) | Dis_mild (Fib <2) | 0.83985 |
| BECP_ 48a | Taurodeoxycholic acid; 14,15-Dihydroxyeicosatrienoic acid (C20:cis[5,8,11]3); leucocytes | 3 | Dis_adv (Fib >2) | Dis_mild (Fib <2) | 0.83985 |
| BECP_ 49 | Taurochenodeoxycholic acid; 11,12-Dihydroxyeicosatrienoic acid (C20:cis[5,8,14]3); C-reactive Protein (CRP) | 3 | Dis_adv (Fib >2) | Dis_mild (Fib <2) | 0.82724 |
| BECP_ 49a | Taurodeoxycholic acid; 11,12-Dihydroxyeicosatrienoic acid (C20:cis[5,8,14]3); C-reactive Protein (CRP) | 3 | Dis_adv (Fib >2) | Dis_mild (Fib <2) | 0.82724 |
| BECP_ 50 | Taurochenodeoxycholic acid; 14,15-Dihydroxyeicosatrienoic acid (C20:cis[5,8,11]3); alkaline phosphatase (ALP) | 3 | Dis_adv (Fib >2) | Dis_mild (Fib <2) | 0.81967 |
| BECP_ 50a | Taurodeoxycholic acid; 14,15-Dihydroxyeicosatrienoic acid (C20:cis[5,8,11]3); alkaline phosphatase (ALP) | 3 | Dis_adv (Fib >2) | Dis_mild (Fib <2) | 0.81967 |
| BEN_51 | Taurochenodeoxycholic acid; 14,15-Dihydroxyeicosatrienoic acid (C20:cis[5,8,11]3); Uridine | 3 | Dis_adv (Fib >2) | Dis_mild (Fib <2) | 0.84237 |
| BEN_ 51a | Taurodeoxycholic acid; 14,15-Dihydroxyeicosatrienoic acid (C20:cis[5,8,11]3); Uridine | 3 | Dis_adv (Fib >2) | Dis_mild (Fib <2) | 0.84237 |
| BEE_52 | Taurochenodeoxycholic acid; 14,15-Dihydroxyeicosatrienoic acid (C20:cis[5,8,11]3); 8,9-Dihydroxyeicosatrienoic acid (C20:cis [5,11,14]3) | 3 | Dis_adv (Fib >2) | Dis_mild (Fib <2) | 0.83985 |
| BEE_ 52a | Taurodeoxycholic acid; 14,15-Dihydroxyeicosatrienoic acid (C20:cis[5,8,11]3); 8,9-Dihydroxyeicosatrienoic acid (C20:cis [5,11,14]3) | 3 | Dis_adv (Fib >2) | Dis_mild (Fib <2) | 0.83985 |
| BEE_53 | Taurochenodeoxycholic acid; 14,15-Dihydroxyeicosatrienoic acid (C20:cis[5,8,11]3); 11,12-Dihydroxyeicosatrienoic acid (C20:cis [5,8,14]3) | 3 | Dis_adv (Fib >2) | Dis_mild (Fib <2) | 0.82346 |
| BEE_ 53a | Taurodeoxycholic acid; 14,15-Dihydroxyeicosatrienoic acid (C20:cis[5,8,11]3); 11,12-Dihydroxyeicosatrienoic acid (C20:cis [5,8,14]3) | 3 | Dis_adv (Fib >2) | Dis_mild (Fib <2) | 0.82346 |
| BEB_54 | Taurocholic acid; Taurochenodeoxycholic acid; 11,12-Dihydroxyeicosatrienoic acid (C20:cis [5,8,14]3) | 3 | Dis_adv (Fib >2) | Dis_mild (Fib <2) | 0.81715 |
| BEB_ 54a | Taurocholic acid; Taurodeoxycholic acid; 11,12-Dihydroxyeicosatrienoic acid (C20:cis[5,8,14]3) | 3 | Dis_adv (Fib >2) | Dis_mild (Fib <2) | 0.81715 |

(continued)

| Panel Number | Panel Composition long | No of Metabolites | Positive Group | Negative Group | AUC |
|---|---|---|---|---|---|
| BEB_55 | Taurochenodeoxycholic acid; Glycocholic acid; 11,12-Dihydroxyeicosatrienoic acid (C20:cis [5,8,14]3) | 3 | Dis_adv (Fib >2) | Dis_mild (Fib <2) | 0.81084 |
| BEB_55a | Taurodeoxycholic acid; Glycocholic acid; 11,12-Dihydroxyeicosatrienoic acid (C20:cis[5,8,14]3) | 3 | Dis_adv (Fib >2) | Dis_mild (Fib <2) | 0.81084 |
| BE_56 | Taurocholic acid; 8,9-Dihydroxyeicosatrienoic acid (C20:cis[5,11,14]3); | 2 | Dis_adv (Fib >2) | Dis_mild (Fib <2) | 0.83733 |
| BE_57 | Glycocholic acid; 14,15-Dihydroxyeicosatrienoic acid (C20:cis[5,8,11]3); | 2 | Dis_adv (Fib >2) | Dis_mild (Fib <2) | 0.83607 |
| BE_58 | Taurocholic acid; 11,12-Dihydroxyeicosatrienoic acid (C20:cis[5,8,14]3); | 2 | Dis_adv (Fib >2) | Dis_mild (Fib <2) | 0.83480 |
| BE_59 | SUM.BILE; 14,15-Dihydroxyeicosatrienoic acid (C20:cis[5,8,11]3); | 2 | Dis_adv (Fib >2) | Dis_mild (Fib <2) | 0.82850 |
| BE_60 | Taurocholic acid; 14,15-Dihydroxyeicosatrienoic acid (C20:cis[5,8,11]3); | 2 | Dis_adv (Fib >2) | Dis_mild (Fib <2) | 0.82472 |
| BE_61 | SUM.BILE; 8,9-Dihydroxyeicosatrienoic acid (C20:cis[5,11,14]3); | 2 | Dis_adv (Fib >2) | Dis_mild (Fib >2) | 0.82472 |
| BE_62 | SUM.BILE; 11,12-Dihydroxyeicosatrienoic acid (C20:cis[5,8,14]3); | 2 | Dis_adv (Fib >2) | Dis_mild (Fib <2) | 0.82346 |
| BE_63 | Taurochenodeoxycholic acid; 14,15-Dihydroxyeicosatrienoic acid (C20:cis[5,8,11]3); | 2 | Dis_adv (Fib >2) | Dis_mild (Fib <2) | 0.82346 |
| BE_63a | Taurodeoxycholic acid; 14,15-Dihydroxyeicosatrienoic acid (C20:cis[5,8,11]3); | 2 | Dis_adv (Fib >2) | Dis_mild (Fib <2) | 0.82346 |
| BE_64 | Taurochenodeoxycholic acid; 14,15-Dihydroxyeicosatrienoic acid (C20:cis[5,8,11]3); | 2 | Dis_adv (Fib >2) | Dis_mild (Fib <2) | 0.82346 |
| BE_64a | Taurodeoxycholic acid; 14,15-Dihydroxyeicosatrienoic acid (C20:cis[5,8,11]3); | 2 | Dis_adv (Fib >2) | Dis_mild (Fib <2) | 0.82346 |
| BE_65 | Glycocholic acid; 11,12-Dihydroxyeicosatrienoic acid (C20:cis[5,8,14]3); | 2 | Dis_adv (Fib >2) | Dis_mild (Fib <2) | 0.81841 |
| BE_66 | Taurochenodeoxycholic acid; 11,12-Dihydroxyeicosatrienoic acid (C20:cis[5,8,14]3); | 2 | Dis_adv (Fib >2) | Dis_mild (Fib <2) | 0.80958 |
| BE_66a | Taurodeoxycholic acid; 11,12-Dihydroxyeicosatrienoic acid (C20:cis[5,8,14]3); | 2 | Dis_adv (Fib >2) | Dis_mild (Fib <2) | 0.80958 |
| BE_67 | Taurochenodeoxycholic acid; 8,9-Dihydroxyeicosatrienoic acid (C20:cis[5,11,14]3); | 2 | Dis_adv (Fib >2) | Dis_mild (Fib <2) | 0.80958 |
| BE_67a | Taurodeoxycholic acid; 8,9-Dihydroxyeicosatrienoic acid (C20:cis[5,11,14]3); | 2 | Dis_adv (Fib >2) | Dis_mild (Fib <2) | 0.80958 |
| BE_68 | Taurochenodeoxycholic acid; 11,12-Dihydroxyeicosatrienoic acid (C20:cis[5,8,14]3); | 2 | Dis_adv (Fib >2) | Dis_mild (Fib <2) | 0.80958 |
| BE_68a | Taurodeoxycholic acid; 11,12-Dihydroxyeicosatrienoic acid (C20:cis[5,8,14]3); | 2 | Dis_adv (Fib >2) | Dis_mild (Fib <2) | 0.80958 |

**Table 2e: Multimarker panel composition and their respective Area under the curve (AUC) values of receiver operating characteristics (ROC)**

| Panel Number | Panel Composition long | No of Metabolites | Positive Group | Negative Group | AUC |
|---|---|---|---|---|---|
| BEH_69 | Taurochenodeoxycholic acid; Dehydroepiandrosterone sulfate; 14,15-Dihydroxyeicosatrienoic acid (C20:cis[5,8,11]3) | 3 | adv (Fib >2) | mild (Fib < 2) | 0.89988 |
| BEH_69a | Taurochenodeoxycholic acid; Testosterone-17-sulfate; 14,15-Dihydroxyeicosatrienoic acid (C20:cis[5,8,11]3) | 3 | adv (Fib >2) | mild (Fib < 2) | 0.89988 |
| BEH_69b | Taurodeoxycholic acid; Dehydroepiandros-terone sulfate; 14,15-Dihydroxyeicosatrienoic acid (C20:cis[5,8,11]3) | 3 | adv (Fib >2) | mild (Fib <2) | 0.89988 |
| BEH_69c | Taurodeoxycholic acid; Testosterone-17-sulfate; 14,15-Dihydroxyeicosatrienoic acid (C20:cis [5,8,11]3) | 3 | adv (Fib >2) | mild (Fib < 2) | 0.89988 |
| BEH_70 | Taurochenodeoxycholic acid; Dehydroepiandrosterone sulfate; 11,12-Dihydroxyeicosatrienoic acid (C20:cis[5,8,14]3) | 3 | adv (Fib >2) | mild (Fib < 2) | 0.88173 |
| BEH_70a | Taurochenodeoxycholic acid; Testosterone-17-sulfate; 11,12-Dihydroxyeicosatrienoic acid (C20:cis[5,8,14]3) | 3 | adv (Fib >2) | mild (Fib < 2) | 0.88173 |
| BEH_70b | Taurodeoxycholic acid; Dehydroepiandrosterone sulfate; 11,12-Dihydroxyeicosatrienoic acid (C20:cis[5,8,14]3) | 3 | adv (Fib >2) | mild (Fib < 2) | 0.88173 |
| BEH_70c | Taurodeoxycholic acid; Testosterone-17-sulfate; 11,12-Dihydroxyeicosatrienoic acid (C20:cis [5,8,14]3) | 3 | adv (Fib >2) | mild (Fib < 2) | 0.88173 |
| BEH_71_1 | Taurochenodeoxycholic acid ; Androsterone sulfate; 14,15-Dihydroxyeicosatrienoic acid (C20:cis[5,8,11]3) | 3 | adv (Fib >2) | mild (Fib < 2) | 0.87354 |
| BEH_71a_1 | Taurodeoxycholic acid; Androsterone sulfate; 14,15-Dihydroxyeicosatrienoic acid (C20:cis [5,8,11]3) | 3 | adv (Fib >2) | mild (Fib < 2) | 0.87354 |
| BEH_71_2 | Taurochenodeoxycholic acid; Etiocholanolone sulfate; 14,15-Dihydroxyeicosatrienoic acid (C20:cis[5,8,11]3) | 3 | adv (Fib >2) | mild (Fib < 2) | 0.87354 |
| BEH_71a_2 | Taurodeoxycholic acid; Etiocholanolone sulfate; 14,15-Dihydroxyeicosatrienoic acid (C20:cis [5,8,11]3) | 3 | adv (Fib >2) | mild (Fib < 2) | 0.87354 |
| BEH_71_3 | Taurochenodeoxycholic acid; Dihydrotestosterone sulfate; 14,15-Dihydroxyeicosatrienoic acid (C20:cis[5,8,11]3) | 3 | adv (Fib >2) | mild (Fib < 2) | 0.87354 |
| BEH_71a_3 | Taurodeoxycholic acid; Dihydrotestosterone sulfate; 14,15-Dihydroxyeicosatrienoic acid (C20:cis[5,8,11]3) | 3 | adv (Fib >2) | mild (Fib < 2) | 0.87354 |
| BEH_72 | Taurochenodeoxycholic acid ; 14,15-Dihydroxyeicosatrienoic acid (C20:cis[5,8,11]3); Testosterone | 3 | adv (Fib >2) | mild (Fib < 2) | 0.86710 |

(continued)

| Panel Number | Panel Composition long | No of Metabolites | Positive Group | Negative Group | AUC |
|---|---|---|---|---|---|
| BEH_72a | Taurodeoxycholic acid; 14,15-Dihydroxyeicosatrienoic acid (C20:cis[5,8,11]3); Testosterone | 3 | adv (Fib >2) | mild (Fib < 2) | 0.86710 |
| BEH_73 | Taurochenodeoxycholic acid ; 14,15-Dihydroxyeicosatrienoic acid (C20:cis[5,8,11]3); Androstenedione | 3 | adv (Fib >2) | mild (Fib < 2) | 0.84778 |
| BEH_73a | Taurodeoxycholic acid; 14,15-Dihydroxyeicosatrienoic acid (C20:cis[5,8,11]3); Androstenedione | 3 | adv (Fib >2) | mild (Fib < 2) | 0.84778 |
| BEH_74_1 | Taurochenodeoxycholic acid ; Androsterone sulfate; 11,12-Dihydroxyeicosatrienoic acid (C20: cis[5,8,14]3) | 3 | adv (Fib >2) | mild (Fib < 2) | 0.83548 |
| BEH_74a_1 | Taurodeoxycholic acid; Androsterone sulfate; 11,12-Dihydroxyeicosatrienoic acid (C20:cis [5,8,14]3) | 3 | adv (Fib >2) | mild (Fib < 2) | 0.83548 |
| BEH_74_2 | Taurochenodeoxycholic acid; Etiocholanolone sulfate; 11,12-Dihydroxyeicosatrienoic acid (C20: cis[5,8,14]3) | 3 | adv (Fib >2) | mild (Fib < 2) | 0.83548 |
| BEH_74a_2 | Taurodeoxycholic acid; Etiocholanolone sulfate; 11,12-Dihydroxyeicosatrienoic acid (C20:cis [5,8,14]3) | 3 | adv (Fib >2) | mild (Fib < 2) | 0.83548 |
| BEH_74_3 | Taurochenodeoxycholic acid; Dihydrotestosterone sulfate; 11,12-Dihydroxyeicosatrienoic acid (C20:cis[5,8,14]3) | 3 | adv (Fib >2) | mild (Fib < 2) | 0.83548 |
| BEH_74a_3 | Taurodeoxycholic acid; Dihydrotestosterone sulfate; 11,12-Dihydroxyeicosatrienoic acid (C20: cis[5,8,14]3) | 3 | adv (Fib >2) | mild (Fib < 2) | 0.83548 |
| BEH_75 | Taurochenodeoxycholic acid; 11,12-Dihydroxyeicosatrienoic acid (C20:cis[5,8,14]3); Testosterone | 3 | adv (Fib >2) | mild (Fib < 2) | 0.80855 |
| BEH_75a | Taurodeoxycholic acid; 11,12-Dihydroxyeicosatrienoic acid (C20:cis[5,8,14]3); Testosterone | 3 | adv (Fib >2) | mild (Fib < 2) | 0.80855 |
| BEL_76 | Taurochenodeoxycholic acid; 14,15-Dihydroxyeicosatrienoic acid (C20:cis[5,8,11]3); Ethanolamine plasmalogen (C39:5) | 3 | adv (Fib >2) | mild (Fib < 2) | 0.90515 |
| BEL_76a | Taurochenodeoxycholic acid; 14,15-Dihydroxyeicosatrienoic acid (C20:cis[5,8,11]3); Choline plasmalogen (C36:5) | 3 | adv (Fib >2) | mild (Fib < 2) | 0.90515 |
| BEL_76b | Taurodeoxycholic acid; 14,15-Dihydroxyeicosatrienoic acid (C20:cis[5,8,11]3); Ethanolamine plasmalogen (C39:5) | 3 | adv (Fib >2) | mild (Fib < 2) | 0.90515 |
| BEL_76c | Taurodeoxycholic acid; 14,15-Dihydroxyeicosatrienoic acid (C20:cis[5,8,11]3); Choline plasmalogen (C36:5) | 3 | adv (Fib >2) | mild (Fib < 2) | 0.90515 |

(continued)

| Panel Number | Panel Composition long | No of Metabolites | Positive Group | Negative Group | AUC |
|---|---|---|---|---|---|
| BEL_77 | Taurochenodeoxycholic acid ; CE_ Cholesterylester C20:4; 14,15-Dihydroxyeicosatrienoic acid (C20:cis[5,8,11]3) | 3 | adv (Fib >2) | mild (Fib < 2) | 0.88525 |
| BEL_ 77a | Taurodeoxycholic acid; CE_Cholesterylester C20:4; 14,15-Dihydroxyeicosatrienoic acid (C20: cis[5,8,11]3) | 3 | adv (Fib >2) | mild (Fib < 2) | 0.88525 |
| BEL_78 | Taurochenodeoxycholic acid; Ethanolamine plasmalogen (C39:5); 11,12-Dihydroxyeicosatrienoic acid (C20:cis[5,8,14]3) | 3 | adv (Fib >2) | mild (Fib < 2) | 0.87588 |
| BEL_ 78a | Taurochenodeoxycholic acid ; Choline plasmalogen (C36:5); 11,12-Dihydroxyeicosatrienoic acid (C20:cis[5,8,14]3) | 3 | adv (Fib >2) | mild (Fib < 2) | 0.87588 |
| BEL_ 78b | Taurodeoxycholic acid; Ethanolamine plasmalogen (C39:5); 11,12-Dihydroxyeicosatrienoic acid (C20:cis[5,8,14]3) | 3 | adv (Fib >2) | mild (Fib < 2) | 0.87588 |
| BEL_78c | Taurodeoxycholic acid; Choline plasmalogen (C36:5); 11,12-Dihydroxyeicosatrienoic acid (C20:cis[5,8,14]3) | 3 | adv (Fib >2) | mild (Fib < 2) | 0.87588 |
| BEL_79 | Taurochenodeoxycholic acid; 14,15-Dihydroxyeicosatrienoic acid (C20:cis[5,8,11]3); SM_Sphingomyelin (d18:2,C18:0) | 3 | adv (Fib >2) | mild (Fib < 2) | 0.87354 |
| BEL_ 79a | Taurodeoxycholic acid; 14,15-Dihydroxyeicosatrienoic acid (C20:cis[5,8,11]3); SM_Sphingomyelin (d18:2,C18:0) | 3 | adv (Fib >2) | mild (Fib < 2) | 0.87354 |
| BEL_80 | Taurochenodeoxycholic acid; 14,15-Dihydroxyeicosatrienoic acid (C20:cis[5,8,11]3); Phosphatidylcholine (C18:0,C20:4) | 3 | adv (Fib >2) | mild (Fib < 2) | 0.86827 |
| BEL_ 80a | Taurodeoxycholic acid; 14,15-Dihydroxyeicosatrienoic acid (C20:cis[5,8,11]3); Phosphatidylcholine (C18:0,C20:4) | 3 | adv (Fib >2) | mild (Fib < 2) | 0.86827 |
| BEL_81 | Taurochenodeoxycholic acid; 14,15-Dihydroxyeicosatrienoic acid (C20:cis[5,8,11]3); Ethanolamine plasmalogen (C39:4) | 3 | adv (Fib >2) | mild (Fib < 2) | 0.86710 |
| BEL_ 81a | Taurochenodeoxycholic acid; 14,15-Dihydroxyeicosatrienoic acid (C20:cis[5,8,11]3); Choline plasmalogen (C36:4) | 3 | adv (Fib >2) | mild (Fib < 2) | 0.86710 |
| BEL_ 81b | Taurodeoxycholic acid; 14,15-Dihydroxyeicosatrienoic acid (C20:cis[5,8,11]3); Ethanolamine plasmalogen (C39:4) | 3 | adv (Fib >2) | mild (Fib < 2) | 0.86710 |
| BEL_81c | Taurodeoxycholic acid; 14,15-Dihydroxyeicosatrienoic acid (C20:cis[5,8,11]3); Choline plasmalogen (C36:4) | 3 | adv (Fib >2) | mild (Fib < 2) | 0.86710 |
| BEL_82 | Taurochenodeoxycholic acid; 14,15-Dihydroxyeicosatrienoic acid (C20:cis[5,8,11]3); Choline plasmalogen (C18-vinyl,C20:4) | 3 | adv (Fib >2) | mild (Fib < 2) | 0.86593 |

(continued)

| Panel Number | Panel Composition long | No of Metabolites | Positive Group | Negative Group | AUC |
|---|---|---|---|---|---|
| BEL_82a | Taurodeoxycholic acid; 14,15-Dihydroxyeicosatrienoic acid (C20:cis[5,8,11]3); Choline plasmalogen (C18-vinyl,C20:4) | 3 | adv (Fib >2) | mild (Fib < 2) | 0.86593 |
| BEL_83 | Taurochenodeoxycholic acid; CE_Cholesterylester C20:4; 11,12-Dihydroxyeicosatrienoic acid (C20:cis[5,8,14]3) | 3 | adv (Fib >2) | mild (Fib < 2) | 0.86241 |
| BEL_83a | Taurodeoxycholic acid; CE_Cholesterylester C20:4; 11,12-Dihydroxyeicosatrienoic acid (C20: cis[5,8,14]3) | 3 | adv (Fib >2) | mild (Fib < 2) | 0.86241 |
| BEL_84 | Taurochenodeoxycholic acid; 14,15-Dihydroxyeicosatrienoic acid (C20:cis[5,8,11]3); total cholesterol | 3 | adv (Fib >2) | mild (Fib < 2) | 0.86124 |
| BEL_84a | Taurodeoxycholic acid; 14,15-Dihydroxyeicosatrienoic acid (C20:cis[5,8,11]3); total cholesterol | 3 | adv (Fib >2) | mild (Fib < 2) | 0.86124 |
| BEL_85 | Taurochenodeoxycholic acid; 14,15-Dihydroxyeicosatrienoic acid (C20:cis[5,8,11]3); CE_Cholesterylester C15:0 | 3 | adv (Fib >2) | mild (Fib < 2) | 0.86007 |
| BEL_85a | Taurodeoxycholic acid; 14,15-Dihydroxyeicosatrienoic acid (C20:cis[5,8,11]3); CE_Cholesterylester C15:0 | 3 | adv (Fib >2) | mild (Fib < 2) | 0.86007 |
| BEL_86 | Taurochenodeoxycholic acid; SM_Sphingomyelin (d18:2,C18:0); 11,12-Dihydroxyeicosatrienoic acid (C20:cis[5,8,14]3) | 3 | adv (Fib >2) | mild (Fib < 2) | 0.85539 |
| BEL_86a | Taurodeoxycholic acid; SM_Sphingomyelin (d18:2,C18:0); 11,12-Dihydroxyeicosatrienoic acid (C20:cis[5,8,14]3) | 3 | adv (Fib >2) | mild (Fib < 2) | 0.85539 |
| BEL_87 | Taurochenodeoxycholic acid; 14,15-Dihydroxyeicosatrienoic acid (C20:cis[5,8,11]3); SM_Sphingomyelin (d18:1,C18:0) | 3 | adv (Fib >2) | mild (Fib < 2) | 0.85129 |
| BEL_87a | Taurodeoxycholic acid; 14,15-Dihydroxyeicosatrienoic acid (C20:cis[5,8,11]3); SM_Sphingomyelin (d18:1,C18:0) | 3 | adv (Fib >2) | mild (Fib < 2) | 0.85129 |
| BEL_88 | Taurochenodeoxycholic acid; 14,15-Dihydroxyeicosatrienoic acid (C20:cis[5,8,11]3); SM_Sphingomyelin (d18:2,C20:0) | 3 | adv (Fib >2) | mild (Fib < 2) | 0.84836 |
| BEL_88a | Taurodeoxycholic acid; 14,15-Dihydroxyeicosatrienoic acid (C20:cis[5,8,11]3); SM_Sphingomyelin (d18:2,C20:0) | 3 | adv (Fib >2) | mild (Fib < 2) | 0.84836 |
| BEL_89 | Taurochenodeoxycholic acid; 14,15-Dihydroxyeicosatrienoic acid (C20:cis[5,8,11]3); total triacylglyceride | 3 | adv (Fib >2) | mild (Fib < 2) | 0.84602 |
| BEL_89a | Taurodeoxycholic acid; 14,15-Dihydroxyeicosatrienoic acid (C20:cis[5,8,11]3); total triacylglyceride | 3 | adv (Fib >2) | mild (Fib < 2) | 0.84602 |

(continued)

| Panel Number | Panel Composition long | No of Metabolites | Positive Group | Negative Group | AUC |
|---|---|---|---|---|---|
| BEL_90 | Taurochenodeoxycholic acid; SM_ Sphingomyelin (d18:1,C18:0); 11,12-Dihydroxyeicosatrienoic acid (C20:cis[5,8,14]3) | 3 | adv (Fib >2) | mild (Fib < 2) | 0.82611 |
| BEL_90a | Taurodeoxycholic acid; SM_Sphingomyelin (d18:1,C18:0); 11,12-Dihydroxyeicosatrienoic acid (C20:cis[5,8,14]3) | 3 | adv (Fib >2) | mild (Fib < 2) | 0.82611 |
| BEL_91 | Taurochenodeoxycholic acid; 11,12-Dihydroxyeicosatrienoic acid (C20:cis[5,8,14]3); Choline plasmalogen (C18-vinyl,C20:4) | 3 | adv (Fib >2) | mild (Fib < 2) | 0.82319 |
| BEL_91a | Taurodeoxycholic acid; 11,12-Dihydroxyeicosatrienoic acid (C20:cis[5,8,14]3); Choline plasmalogen (C18-vinyl,C20:4) | 3 | adv (Fib >2) | mild (Fib < 2) | 0.82319 |
| BEL_92 | Taurochenodeoxycholic acid; SM_ Sphingomyelin (d18:2,C20:0); 11,12-Dihydroxyeicosatrienoic acid (C20:cis[5,8,14]3) | 3 | adv (Fib >2) | mild (Fib < 2) | 0.82260 |
| BEL_92a | Taurodeoxycholic acid; SM_Sphingomyelin (d18:2,C20:0); 11,12-Dihydroxyeicosatrienoic acid (C20:cis[5,8,14]3) | 3 | adv (Fib >2) | mild (Fib < 2) | 0.82260 |
| BEL_93 | Taurochenodeoxycholic acid; 11,12-Dihydroxyeicosatrienoic acid (C20:cis[5,8,14]3); CE_Cholesterylester C15:0 | 3 | adv (Fib >2) | mild (Fib < 2) | 0.81557 |
| BEL_93a | Taurodeoxycholic acid; 11,12-Dihydroxyeicosatrienoic acid (C20:cis[5,8,14]3); CE_Cholesterylester C15:0 | 3 | adv (Fib >2) | mild (Fib < 2) | 0.81557 |
| BEL_94 | Taurochenodeoxycholic acid; 11,12-Dihydroxyeicosatrienoic acid (C20:cis[5,8,14]3); total cholesterol | 3 | adv (Fib >2) | mild (Fib < 2) | 0.81557 |
| BEL_94a | Taurodeoxycholic acid; 11,12-Dihydroxyeicosatrienoic acid (C20:cis[5,8,14]3); total cholesterol | 3 | adv (Fib >2) | mild (Fib < 2) | 0.81557 |
| BEL_95 | Taurochenodeoxycholic acid; 11,12-Dihydroxyeicosatrienoic acid (C20:cis[5,8,14]3); Ethanolamine plasmalogen (C39:4) | 3 | adv (Fib >2) | mild (Fib < 2) | 0.81440 |
| BEL_95a | Taurochenodeoxycholic acid; 11,12-Dihydroxyeicosatrienoic acid (C20:cis[5,8,14]3); Choline plasmalogen (C36:4) | 3 | adv (Fib >2) | mild (Fib < 2) | 0.81440 |
| BEL_95b | Taurodeoxycholic acid; 11,12-Dihydroxyeicosatrienoic acid (C20:cis[5,8,14]3); Ethanolamine plasmalogen (C39:4) | 3 | adv (Fib >2) | mild (Fib < 2) | 0.81440 |
| BEL_95c | Taurodeoxycholic acid; 11,12-Dihydroxyeicosatrienoic acid (C20:cis[5,8,14]3); Choline plasmalogen (C36:4) | 3 | adv (Fib >2) | mild (Fib < 2) | 0.81440 |
| BEA_96 | Taurochenodeoxycholic acid; Cystine; 14,15-Dihydroxyeicosatrienoic acid (C20:cis[5,8,11]3) | 3 | adv (Fib >2) | mild (Fib < 2) | 0.89286 |
| BEA_96a | Taurodeoxycholic acid; Cystine; 14,15-Dihydroxyeicosatrienoic acid (C20:cis[5,8,11]3) | 3 | adv (Fib >2) | mild (Fib < 2) | 0.89286 |

(continued)

| Panel Number | Panel Composition long | No of Metabolites | Positive Group | Negative Group | AUC |
|---|---|---|---|---|---|
| BEL_96 | Taurochenodeoxycholic acid; Phosphatidylcholine (C18:0,C20:4); 11,12-Dihydroxyeicosatrienoic acid (C20:cis[5,8,14]3) | 3 | adv (Fib >2) | mild (Fib < 2) | 0.81440 |
| BEL_96a | Taurodeoxycholic acid; Phosphatidylcholine (C18:0,C20:4); 11,12-Dihydroxyeicosatrienoic acid (C20:cis[5,8,14]3) | 3 | adv (Fib >2) | mild (Fib < 2) | 0.81440 |
| BEA_97 | Taurochenodeoxycholic acid; Cystine; 11,12-Dihydroxyeicosatrienoic acid (C20:cis[5,8,14]3) | 3 | adv (Fib >2) | mild (Fib < 2) | 0.89227 |
| BEA_97a | Taurodeoxycholic acid; Cystine; 11,12-Dihydroxyeicosatrienoic acid (C20:cis[5,8,14]3) | 3 | adv (Fib >2) | mild (Fib < 2) | 0.89227 |
| BEA_98 | Taurochenodeoxycholic acid; Tyrosine; 14,15-Dihydroxyeicosatrienoic acid (C20:cis[5,8,11]3) | 3 | adv (Fib >2) | mild (Fib < 2) | 0.88876 |
| BEA_98a | Taurodeoxycholic acid; Tyrosine; 14,15-Dihydroxyeicosatrienoic acid (C20:cis[5,8,11]3) | 3 | adv (Fib >2) | mild (Fib < 2) | 0.88876 |
| BEA_99 | Taurochenodeoxycholic acid; 14,15-Dihydroxyeicosatrienoic acid (C20:cis[5,8,11]3); Creatinine | 3 | adv (Fib >2) | mild (Fib < 2) | 0.87354 |
| BEA_99a | Taurodeoxycholic acid; 14,15-Dihydroxyeicosatrienoic acid (C20:cis[5,8,11]3); Creatinine | 3 | adv (Fib >2) | mild (Fib < 2) | 0.87354 |
| BEA_100 | Taurochenodeoxycholic acid; 14,15-Dihydroxyeicosatrienoic acid (C20:cis[5,8,11]3); Histidine | 3 | adv (Fib >2) | mild (Fib < 2) | 0.87178 |
| BEA_100a | Taurodeoxycholic acid; 14,15-Dihydroxyeicosatrienoic acid (C20:cis[5,8,11]3); Histidine | 3 | adv (Fib >2) | mild (Fib < 2) | 0.87178 |
| BEA_101 | Taurochenodeoxycholic acid; Tyrosine; 11,12-Dihydroxyeicosatrienoic acid (C20:cis[5,8,14]3) | 3 | adv (Fib >2) | mild (Fib < 2) | 0.86944 |
| BEA_101a | Taurodeoxycholic acid; Tyrosine; 11,12-Dihydroxyeicosatrienoic acid (C20:cis[5,8,14]3) | 3 | adv (Fib >2) | mild (Fib < 2) | 0.86944 |
| BEA_102 | Taurochenodeoxycholic acid; 14,15-Dihydroxyeicosatrienoic acid (C20:cis[5,8,11]3); Phenylalanine | 3 | adv (Fib >2) | mild (Fib < 2) | 0.86651 |
| BEA_102a | Taurodeoxycholic acid; 14,15-Dihydroxyeicosatrienoic acid (C20:cis[5,8,11]3); Phenylalanine | 3 | adv (Fib >2) | mild (Fib < 2) | 0.86651 |
| BEA_103 | Taurochenodeoxycholic acid; 14,15-Dihydroxyeicosatrienoic acid (C20:cis[5,8,11]3); Glutamine | 3 | adv (Fib >2) | mild (Fib < 2) | 0.86651 |
| BEA_103a | Taurodeoxycholic acid; 14,15-Dihydroxyeicosatrienoic acid (C20:cis[5,8,11]3); Glutamine | 3 | adv (Fib >2) | mild (Fib < 2) | 0.86651 |
| BEA_104 | Taurochenodeoxycholic acid; 14,15-Dihydroxyeicosatrienoic acid (C20:cis[5,8,11]3); Asparagine | 3 | adv (Fib >2) | mild (Fib < 2) | 0.86651 |

(continued)

| Panel Number | Panel Composition long | No of Metabolites | Positive Group | Negative Group | AUC |
|---|---|---|---|---|---|
| BEA_ 104a | Taurodeoxycholic acid; 14,15-Dihydroxyeicosatrienoic acid (C20:cis[5,8,11]3); Asparagine | 3 | adv (Fib >2) | mild (Fib < 2) | 0.86651 |
| BEA_ 105 | Taurochenodeoxycholic acid; 14,15-Dihydroxyeicosatrienoic acid (C20:cis[5,8,11]3); Arginine | 3 | adv (Fib >2) | mild (Fib < 2) | 0.86534 |
| BEA_ 105a | Taurodeoxycholic acid; 14,15-Dihydroxyeicosatrienoic acid (C20:cis[5,8,11]3); Arginine | 3 | adv (Fib >2) | mild (Fib < 2) | 0.86534 |
| BEA_ 106 | Taurochenodeoxycholic acid; Creatinine; 11,12-Dihydroxyeicosatrienoic acid (C20:cis[5,8,14]3) | 3 | adv (Fib >2) | mild (Fib < 2) | 0.86241 |
| BEA_ 106a | Taurodeoxycholic acid; Creatinine; 11,12-Dihydroxyeicosatrienoic acid (C20:cis[5,8,14]3) | 3 | adv (Fib >2) | mild (Fib < 2) | 0.86241 |
| BEA_ 107 | Taurochenodeoxycholic acid; 14,15-Dihydroxyeicosatrienoic acid (C20:cis[5,8,11]3); Creatine | 3 | adv (Fib >2) | mild (Fib < 2) | 0.86183 |
| BEA_ 107a | Taurodeoxycholic acid; 14,15-Dihydroxyeicosatrienoic acid (C20:cis[5,8,11]3); Creatine | 3 | adv (Fib >2) | mild (Fib < 2) | 0.86183 |
| BEA_ 108 | Taurochenodeoxycholic acid; 14,15-Dihydroxyeicosatrienoic acid (C20:cis[5,8,11]3); Tryptophan | 3 | adv (Fib >2) | mild (Fib < 2) | 0.86183 |
| BEA_ 108a | Taurodeoxycholic acid; 14,15-Dihydroxyeicosatrienoic acid (C20:cis[5,8,11]3); Tryptophan | 3 | adv (Fib >2) | mild (Fib < 2) | 0.86183 |
| BEA_ 109 | Taurochenodeoxycholic acid; 14,15-Dihydroxyeicosatrienoic acid (C20:cis[5,8,11]3); Urea | 3 | adv (Fib >2) | mild (Fib < 2) | 0.85539 |
| BEA_ 109a | Taurodeoxycholic acid; 14,15-Dihydroxyeicosatrienoic acid (C20:cis[5,8,11]3); Urea | 3 | adv (Fib >2) | mild (Fib < 2) | 0.85539 |
| BEA_ 110 | Taurochenodeoxycholic acid; 14,15-Dihydroxyeicosatrienoic acid (C20:cis[5,8,11]3); Lysine | 3 | adv (Fib >2) | mild (Fib < 2) | 0.84953 |
| BEA_ 110a | Taurodeoxycholic acid; 14,15-Dihydroxyeicosatrienoic acid (C20:cis[5,8,11]3); Lysine | 3 | adv (Fib >2) | mild (Fib < 2) | 0.84953 |
| BEA_ 111 | Taurochenodeoxycholic acid; 11,12-Dihydroxyeicosatrienoic acid (C20:cis[5,8,14]3); Histidine | 3 | adv (Fib >2) | mild (Fib < 2) | 0.83021 |
| BEA_ 111a | Taurodeoxycholic acid; 11,12-Dihydroxyeicosatrienoic acid (C20:cis[5,8,14]3); Histidine | 3 | adv (Fib >2) | mild (Fib < 2) | 0.83021 |
| BEA_ 112 | Taurochenodeoxycholic acid; 11,12-Dihydroxyeicosatrienoic acid (C20:cis[5,8,14]3); Urea | 3 | adv (Fib >2) | mild (Fib < 2) | 0.82728 |

(continued)

| Panel Number | Panel Composition long | No of Metabolites | Positive Group | Negative Group | AUC |
|---|---|---|---|---|---|
| BEA_112a | Taurodeoxycholic acid; 11,12-Dihydroxyeicosatrienoic acid (C20:cis[5,8,14]3); Urea | 3 | adv (Fib >2) | mild (Fib < 2) | 0.82728 |
| BEA_113 | Taurochenodeoxycholic acid; 11,12-Dihydroxyeicosatrienoic acid (C20:cis[5,8,14]3); Arginine | 3 | adv (Fib >2) | mild (Fib < 2) | 0.82143 |
| BEA_113a | Taurodeoxycholic acid; 11,12-Dihydroxyeicosatrienoic acid (C20:cis[5,8,14]3); Arginine | 3 | adv (Fib >2) | mild (Fib < 2) | 0.82143 |
| BEA_114 | Taurochenodeoxycholic acid; 11,12-Dihydroxyeicosatrienoic acid (C20:cis[5,8,14]3); Phenylalanine | 3 | adv (Fib >2) | mild (Fib < 2) | 0.82084 |
| BEA_114a | Taurodeoxycholic acid; 11,12-Dihydroxyeicosatrienoic acid (C20:cis[5,8,14]3); Phenylalanine | 3 | adv (Fib >2) | mild (Fib < 2) | 0.82084 |
| BEA_115 | Taurochenodeoxycholic acid; 11,12-Dihydroxyeicosatrienoic acid (C20:cis[5,8,14]3); Asparagine | 3 | adv (Fib >2) | mild (Fib < 2) | 0.81616 |
| BEA_115a | Taurodeoxycholic acid; 11,12-Dihydroxyeicosatrienoic acid (C20:cis[5,8,14]3); Asparagine | 3 | adv (Fib >2) | mild (Fib < 2) | 0.81616 |
| BEA_116 | Taurochenodeoxycholic acid; 11,12-Dihydroxyeicosatrienoic acid (C20:cis[5,8,14]3); Tryptophan | 3 | adv (Fib >2) | mild (Fib < 2) | 0.81382 |
| BEA_116a | Taurodeoxycholic acid; 11,12-Dihydroxyeicosatrienoic acid (C20:cis[5,8,14]3); Tryptophan | 3 | adv (Fib >2) | mild (Fib < 2) | 0.81382 |
| BEA_117 | Taurochenodeoxycholic acid; 11,12-Dihydroxyeicosatrienoic acid (C20:cis[5,8,14]3); Glutamine | 3 | adv (Fib >2) | mild (Fib < 2) | 0.80386 |
| BEA_117a | Taurodeoxycholic acid; 11,12-Dihydroxyeicosatrienoic acid (C20:cis[5,8,14]3); Glutamine | 3 | adv (Fib >2) | mild (Fib < 2) | 0.80386 |
| BEA_118 | Taurochenodeoxycholic acid; 11,12-Dihydroxyeicosatrienoic acid (C20:cis[5,8,14]3); Creatine | 3 | adv (Fib >2) | mild (Fib < 2) | 0.80211 |
| BEA_118a | Taurodeoxycholic acid; 11,12-Dihydroxyeicosatrienoic acid (C20:cis[5,8,14]3); Creatine | 3 | adv (Fib >2) | mild (Fib < 2) | 0.80211 |
| BEC_119 | Taurochenodeoxycholic acid; 14,15-Dihydroxyeicosatrienoic acid (C20:cis[5,8,11]3); Glucose | 3 | adv (Fib >2) | mild (Fib < 2) | 0.87705 |
| BEC_119a | Taurodeoxycholic acid; 14,15-Dihydroxyeicosatrienoic acid (C20:cis[5,8,11]3); Glucose | 3 | adv (Fib >2) | mild (Fib < 2) | 0.87705 |

(continued)

| Panel Number | Panel Composition long | No of Metabolites | Positive Group | Negative Group | AUC |
|---|---|---|---|---|---|
| BEC_ 120 | Taurochenodeoxycholic acid; Glucose; 11,12-Dihydroxyeicosatrienoic acid (C20:cis[5,8,14]3) | 3 | adv (Fib >2) | mild (Fib < 2) | 0.86124 |
| BEC_ 120a | Taurodeoxycholic acid; Glucose; 11,12-Dihydroxyeicosatrienoic acid (C20:cis[5,8,14]3) | 3 | adv (Fib >2) | mild (Fib < 2) | 0.86124 |
| BEN_ 121 | Taurochenodeoxycholic acid; 14,15-Dihydroxyeicosatrienoic acid (C20:cis[5,8,11]3); Uridine | 3 | adv (Fib >2) | mild (Fib < 2) | 0.86651 |
| BEN_ 121a | Taurodeoxycholic acid; 14,15-Dihydroxyeicosatrienoic acid (C20:cis[5,8,11]3); Uridine | 3 | adv (Fib >2) | mild (Fib < 2) | 0.86651 |
| BEN_ 122 | Taurochenodeoxycholic acid; 11,12-Dihydroxyeicosatrienoic acid (C20:cis[5,8,14]3); Uridine | 3 | adv (Fib >2) | mild (Fib < 2) | 0.81557 |
| BEN_ 122a | Taurodeoxycholic acid; 11,12-Dihydroxyeicosatrienoic acid (C20:cis[5,8,14]3); Uridine | 3 | adv (Fib >2) | mild (Fib < 2) | 0.81557 |
| BEE_ 123 | Taurochenodeoxycholic acid; 14,15-Dihydroxyeicosatrienoic acid (C20:cis[5,8,11]3); 8,9-Dihydroxyeicosatrienoic acid (C20:cis[5,11,14]3) | 3 | adv (Fib >2) | mild (Fib < 2) | 0.86124 |
| BEE_ 123a | Taurodeoxycholic acid; 14,15-Dihydroxyeicosatrienoic acid (C20:cis[5,8,11]3); 8,9-Dihydroxyeicosatrienoic acid (C20:cis[5,11,14]3) | 3 | adv (Fib >2) | mild (Fib < 2) | 0.86124 |
| BEE_ 124 | Taurochenodeoxycholic acid; 14,15-Dihydroxyeicosatrienoic acid (C20:cis[5,8,11]3); 11,12-Dihydroxyeicosatrienoic acid (C20:cis[5,8,14]3) | 3 | adv (Fib >2) | mild (Fib < 2) | 0.84309 |
| BEE_ 124a | Taurodeoxycholic acid; 14,15-Dihydroxyeicosatrienoic acid (C20:cis[5,8,11]3); 11,12-Dihydroxyeicosatrienoic acid (C20:cis[5,8,14]3) | 3 | adv (Fib >2) | mild (Fib < 2) | 0.84309 |
| BEB_ 125 | Taurochenodeoxycholic acid; Glycocholic acid; 11,12-Dihydroxyeicosatrienoic acid (C20:cis[5,8,14]3) | 3 | adv (Fib >2) | mild (Fib < 2) | 0.85597 |
| BEB_ 125a | Taurodeoxycholic acid; Glycocholic acid; 11,12-Dihydroxyeicosatrienoic acid (C20:cis[5,8,14]3) | 3 | adv (Fib >2) | mild (Fib < 2) | 0.85597 |
| BEB_ 126 | Taurocholic acid; Taurochenodeoxycholic acid; 11,12-Dihydroxyeicosatrienoic acid (C20:cis[5,8,14]3) | 3 | adv (Fib >2) | mild (Fib < 2) | 0.85070 |
| BEB_ 126 | Taurocholic acid; Taurodeoxycholic acid; 11,12-Dihydroxyeicosatrienoic acid (C20:cis[5,8,14]3) | 3 | adv (Fib >2) | mild (Fib < 2) | 0.85070 |
| BECP_ 127 | Taurochenodeoxycholic acid ; 14,15-Dihydroxyeicosatrienoic acid (C20:cis[5,8,11]3); C-reactive Proteine (CRP) | 3 | adv (Fib >2) | mild (Fib < 2) | 0.87763 |

(continued)

| Panel Number | Panel Composition long | No of Metabolites | Positive Group | Negative Group | AUC |
|---|---|---|---|---|---|
| BECP_ 127a | Taurodeoxycholic acid; 14,15-Dihydroxyeicosatrienoic acid (C20:cis[5,8,11]3); C-reactive Proteine (CRP) | 3 | adv (Fib >2) | mild (Fib < 2) | 0.87763 |
| BECP_ 128 | Taurochenodeoxycholic acid; 14,15-Dihydroxyeicosatrienoic acid (C20:cis[5,8,11]3); leucocyte | 3 | adv (Fib >2) | mild (Fib < 2) | 0.86651 |
| BECP_ 128a | Taurodeoxycholic acid; 14,15-Dihydroxyeicosatrienoic acid (C20:cis[5,8,11]3); leucocyte | 3 | adv (Fib >2) | mild (Fib < 2) | 0.86651 |
| BECP_ 129 | Taurochenodeoxycholic acid; 14,15-Dihydroxyeicosatrienoic acid (C20:cis[5,8,11]3); alkaline phosphatase (ALP) | 3 | adv (Fib >2) | mild (Fib < 2) | 0.85597 |
| BECP_ 129a | Taurodeoxycholic acid; 14,15-Dihydroxyeicosatrienoic acid (C20:cis[5,8,11]3); alkaline phosphatase (ALP) | 3 | adv (Fib >2) | mild (Fib < 2) | 0.85597 |
| BECP_ 130 | Taurochenodeoxycholic acid ; 11,12-Dihydroxyeicosatrienoic acid (C20:cis[5,8,14]3); C-reactive Proteine (CRP) | 3 | adv (Fib >2) | mild (Fib < 2) | 0.85012 |
| BECP_ 130a | Taurodeoxycholic acid; 11,12-Dihydroxyeicosatrienoic acid (C20:cis[5,8,14]3); C-reactive Proteine (CRP) | 3 | adv (Fib >2) | mild (Fib < 2) | 0.85012 |
| BECP_ 131 | Taurochenodeoxycholic acid ; 11,12-Dihydroxyeicosatrienoic acid (C20:cis[5,8,14]3); leucocyte | 3 | adv (Fib >2) | mild (Fib < 2) | 0.81733 |
| BECP_ 131a | Taurodeoxycholic acid; 11,12-Dihydroxyeicosatrienoic acid (C20:cis[5,8,14]3); leucocyte | 3 | adv (Fib >2) | mild (Fib < 2) | 0.81733 |
| BE_132 | Glycocholic acid; 14,15-Dihydroxyeicosatrienoic acid (C20:cis[5,8,11]3); | 2 | adv (Fib >2) | mild (Fib < 2) | 0.89578 |
| BE_132 | Glycocholic acid; 14,15-Dihydroxyeicosatrienoic acid (C20:cis[5,8,11]3); | 2 | adv (Fib >2) | mild (Fib < 2) | 0.89578 |
| BECP_ 132 | Taurochenodeoxycholic acid ; 11,12-Dihydroxyeicosatrienoic acid (C20:cis[5,8,14]3); alkaline phosphatase (ALP) | 3 | adv (Fib >2) | mild (Fib < 2) | 0.80269 |
| BECP_ 132a | Taurodeoxycholic acid; 11,12-Dihydroxyeicosatrienoic acid (C20:cis[5,8,14]3); alkaline phosphatase (ALP) | 3 | adv (Fib >2) | mild (Fib < 2) | 0.80269 |
| BE_133 | SUM.BILE; 14,15-Dihydroxyeicosatrienoic acid (C20:cis[5,8,11]3); | 2 | adv (Fib >2) | mild (Fib < 2) | 0.88934 |
| BE_134 | Taurocholic acid; 14,15-Dihydroxyeicosatrienoic acid (C20:cis[5,8,11]3); | 2 | adv (Fib >2) | mild (Fib < 2) | 0.87939 |
| BE_135 | SUM.BILE; 8,9-Dihydroxyeicosatrienoic acid (C20:cis[5,11,14]3); | 2 | adv (Fib >2) | mild (Fib < 2) | 0.87529 |
| BE_136 | Glycocholic acid; 11,12-Dihydroxyeicosatrienoic acid (C20:cis[5,8,14]3); | 2 | adv (Fib >2) | mild (Fib < 2) | 0.87354 |

(continued)

| Panel Number | Panel Composition long | No of Metabolites | Positive Group | Negative Group | AUC |
|---|---|---|---|---|---|
| BE_137 | Taurochenodeoxycholic acid; 14,15-Dihydroxyeicosatrienoic acid (C20:cis[5,8,11]3); | 2 | adv (Fib >2) | mild (Fib < 2) | 0.86944 |
| BE_137a | Taurodeoxycholic acid; 14,15-Dihydroxyeicosatrienoic acid (C20:cis[5,8,11]3); | 2 | adv (Fib >2) | mild (Fib < 2) | 0.86944 |
| BE_138 | Taurochenodeoxycholic acid; 14,15-Dihydroxyeicosatrienoic acid (C20:cis[5,8,11]3); | 2 | adv (Fib >2) | mild (Fib < 2) | 0.86944 |
| BE_138a | Taurodeoxycholic acid; 14,15-Dihydroxyeicosatrienoic acid (C20:cis[5,8,11]3); | 2 | adv (Fib >2) | mild (Fib < 2) | 0.86944 |
| BE_139 | SUM.BILE; 11,12-Dihydroxyeicosatrienoic acid (C20:cis[5,8,14]3); | 2 | adv (Fib >2) | mild (Fib < 2) | 0.86475 |
| BE_140 | Taurocholic acid; 8,9-Dihydroxyeicosatrienoic acid (C20:cis[5,11,14]3); | 2 | adv (Fib >2) | mild (Fib < 2) | 0.86007 |
| BE_141 | Glycocholic acid; 8,9-Dihydroxyeicosatrienoic acid (C20:cis[5,11,14]3); | 2 | adv (Fib >2) | mild (Fib < 2) | 0.85363 |
| BE_142 | Taurocholic acid; 11,12-Dihydroxyeicosatrienoic acid (C20:cis[5,8,14]3); | 2 | adv (Fib >2) | mild (Fib < 2) | 0.85304 |
| BE_143 | Taurochenodeoxycholic acid; 8,9-Dihydroxyeicosatrienoic acid (C20:cis[5,11,14]3); | 2 | adv (Fib >2) | mild (Fib < 2) | 0.85187 |
| BE_143a | Taurodeoxycholic acid; 8,9-Dihydroxyeicosatrienoic acid (C20:cis[5,11,14]3); | 2 | adv (Fib >2) | mild (Fib < 2) | 0.85187 |
| BE_144 | Taurochenodeoxycholic acid; 11,12-Dihydroxyeicosatrienoic acid (C20:cis[5,8,14]3); | 2 | adv (Fib >2) | mild (Fib < 2) | 0.84133 |
| BE_144a | Taurodeoxycholic acid; 11,12-Dihydroxyeicosatrienoic acid (C20:cis[5,8,14]3); | 2 | adv (Fib >2) | mild (Fib < 2) | 0.84133 |
| BE_145 | Taurochenodeoxycholic acid; 11,12-Dihydroxyeicosatrienoic acid (C20:cis[5,8,14]3); | 2 | adv (Fib >2) | mild (Fib < 2) | 0.84133 |
| BE_145a | Taurodeoxycholic acid; 11,12-Dihydroxyeicosatrienoic acid (C20:cis[5,8,14]3); | 2 | adv (Fib >2) | mild (Fib < 2) | 0.84133 |

**Table 2f: Multimarker panel composition and their respective Area under the curve (AUC) values of receiver operating characteristics (ROC)**

| Panel Number | Panel Composition long | No of Metabolites | Positive Group | Negative Group | AUC |
|---|---|---|---|---|---|
| BEH_146 | Dehydroepiandrosterone sulfate; 14,15-Dihydroxyeicosatrienoic acid (C20:cis[5,8,11]3); Taurochenodeoxycholic acid | 3 | NASH adv (Fib >2) | NASH mild (Fib < 2) | 0.91250 |
| BEH_146a | Testosterone-17-sulfate; 14,15-Dihydroxyeicosatrienoic acid (C20:cis[5,8,11]3); Taurochenodeoxycholic acid | 3 | NASH adv (Fib >2) | NASH mild (Fib < 2) | 0.91250 |
| BEH_146b | Dehydroepiandrosterone sulfate; 14,15-Dihydroxyeicosatrienoic acid (C20:cis[5,8,11]3); Taurodeoxycholic acid | 3 | NASH adv (Fib >2) | NASH mild (Fib < 2) | 0.91250 |

(continued)

| Panel Number | Panel Composition long | No of Metabolites | Positive Group | Negative Group | AUC |
|---|---|---|---|---|---|
| BEH_ 146c | Testosterone-17-sulfate; 14,15-Dihydroxyeicosatrienoic acid (C20:cis[5,8,11]3); Taurodeoxycholic acid | 3 | NASH adv (Fib >2) | NASH mild (Fib < 2) | 0.91250 |
| BEH_ 147_1 | Taurochenodeoxycholic acid; Androsterone sulfate; 14,15-Dihydroxyeicosatrienoic acid (C20:cis[5,8,11]3) | 3 | NASH adv (Fib >2) | NASH mild (Fib < 2) | 0.87083 |
| BEH_ 147a_1 | Taurodeoxycholic acid; Androsterone sulfate; 14,15-Dihydroxyeicosatrienoic acid (C20:cis [5,8,11]3) | 3 | NASH adv (Fib >2) | NASH mild (Fib < 2) | 0.87083 |
| BEH_ 147_2 | Taurochenodeoxycholic acid; Etiocholanolone sulfate; 14,15-Dihydroxyeicosatrienoic acid (C20:cis[5,8,11]3) | 3 | NASH adv (Fib >2) | NASH mild (Fib < 2) | 0.87083 |
| BEH_ 147a_2 | Taurodeoxycholic acid; Etiocholanolone sulfate; 14,15-Dihydroxyeicosatrienoic acid (C20:cis [5,8,11]3) | 3 | NASH adv (Fib >2) | NASH mild (Fib < 2) | 0.87083 |
| BEH_ 147_3 | Taurochenodeoxycholic acid; Dihydrotestosterone sulfate; 14,15-Dihydroxyeicosatrienoic acid (C20:cis[5,8,11]3) | 3 | NASH adv (Fib >2) | NASH mild (Fib < 2) | 0.87083 |
| BEH_ 147a_3 | Taurodeoxycholic acid; Dihydrotestosterone sulfate; 14,15-Dihydroxyeicosatrienoic acid (C20:cis[5,8,11]3) | 3 | NASH adv (Fib >2) | NASH mild (Fib < 2) | 0.87083 |
| BEH_ 148 | Taurochenodeoxycholic acid; 14,15-Dihydroxyeicosatrienoic acid (C20:cis[5,8,11]3); Androstenedione | 3 | NASH adv (Fib >2) | NASH mild (Fib < 2) | 0.81667 |
| BEH_ 148a | Taurodeoxycholic acid; 14,15-Dihydroxyeicosatrienoic acid (C20:cis[5,8,11]3); Androstenedione | 3 | NASH adv (Fib >2) | NASH mild (Fib < 2) | 0.81667 |
| BEL_ 149 | Taurochenodeoxycholic acid; 14,15-Dihydroxyeicosatrienoic acid (C20:cis[5,8,11]3); Ethanolamine plasmalogen (C39:5) | 3 | NASH adv (Fib >2) | NASH mild (Fib < 2) | 0.90417 |
| BEL_ 149a | Taurochenodeoxycholic acid; 14,15-Dihydroxyeicosatrienoic acid (C20:cis[5,8,11]3); Choline plasmalogen (C36:5) | 3 | NASH adv (Fib >2) | NASH mild (Fib < 2) | 0.90417 |
| BEL_ 149b | Taurodeoxycholic acid; 14,15-Dihydroxyeicosatrienoic acid (C20:cis[5,8,11]3); Ethanolamine plasmalogen (C39:5) | 3 | NASH adv (Fib >2) | NASH mild (Fib < 2) | 0.90417 |
| BEL_ 149c | Taurodeoxycholic acid; 14,15-Dihydroxyeicosatrienoic acid (C20:cis[5,8,11]3); Choline plasmalogen (C36:5) | 3 | NASH adv (Fib >2) | NASH mild (Fib < 2) | 0.90417 |
| BEL_ 150 | Taurochenodeoxycholic acid; Phosphatidylcholine (C18:0,C20:4); 14,15-Dihydroxyeicosatrienoic acid (C20:cis[5,8,11]3) | 3 | NASH adv (Fib >2) | NASH mild (Fib < 2) | 0.90417 |
| BEL_ 150a | Taurodeoxycholic acid; Phosphatidylcholine (C18:0,C20:4); 14,15-Dihydroxyeicosatrienoic acid (C20:cis[5,8,11]3) | 3 | NASH adv (Fib >2) | NASH mild (Fib < 2) | 0.90417 |

(continued)

| Panel Number | Panel Composition long | No of Metabolites | Positive Group | Negative Group | AUC |
|---|---|---|---|---|---|
| BEL_151 | Taurochenodeoxycholic acid; CE_ Cholesterylester C20:4; 14,15-Dihydroxyeicosatrienoic acid (C20:cis[5,8,11]3) | 3 | NASH adv (Fib >2) | NASH mild (Fib < 2) | 0.89167 |
| BEL_151a | Taurodeoxycholic acid; CE_Cholesterylester C20:4; 14,15-Dihydroxyeicosatrienoic acid (C20: cis[5,8,11]3) | 3 | NASH adv (Fib >2) | NASH mild (Fib < 2) | 0.89167 |
| BEL_152 | Taurochenodeoxycholic acid; Ethanolamine plasmalogen (C39:5); 11,12-Dihydroxyeicosatrienoic acid (C20:cis[5,8,14]3) | 3 | NASH adv (Fib >2) | NASH mild (Fib < 2) | 0.87083 |
| BEL_152a | Taurochenodeoxycholic acid; Choline plasmalogen (C36:5); 11,12-Dihydroxyeicosatrienoic acid (C20:cis[5,8,14]3) | 3 | NASH adv (Fib >2) | NASH mild (Fib < 2) | 0.87083 |
| BEL_152b | Taurodeoxycholic acid; Ethanolamine plasmalogen (C39:5); 11,12-Dihydroxyeicosatrienoic acid (C20:cis[5,8,14]3) | 3 | NASH adv (Fib >2) | NASH mild (Fib < 2) | 0.87083 |
| BEL_152c | Taurodeoxycholic acid; Choline plasmalogen (C36:5); 11,12-Dihydroxyeicosatrienoic acid (C20:cis[5,8,14]3) | 3 | NASH adv (Fib >2) | NASH mild (Fib < 2) | 0.87083 |
| BEL_153 | Taurochenodeoxycholic acid; 14,15-Dihydroxyeicosatrienoic acid (C20:cis[5,8,11]3); Ethanolamine plasmalogen (C39:4) | 3 | NASH adv (Fib >2) | NASH mild (Fib < 2) | 0.84583 |
| BEL_153a | Taurochenodeoxycholic acid; 14,15-Dihydroxyeicosatrienoic acid (C20:cis[5,8,11]3); Choline plasmalogen (C36:4) | 3 | NASH adv (Fib >2) | NASH mild (Fib < 2) | 0.84583 |
| BEL_153b | Taurodeoxycholic acid; 14,15-Dihydroxyeicosatrienoic acid (C20:cis[5,8,11]3); Ethanolamine plasmalogen (C39:4) | 3 | NASH adv (Fib >2) | NASH mild (Fib < 2) | 0.84583 |
| BEL_153c | Taurodeoxycholic acid; 14,15-Dihydroxyeicosatrienoic acid (C20:cis[5,8,11]3); Choline plasmalogen (C36:4) | 3 | NASH adv (Fib >2) | NASH mild (Fib < 2) | 0.84583 |
| BEL_154 | Taurochenodeoxycholic acid; 14,15-Dihydroxyeicosatrienoic acid (C20:cis[5,8,11]3); total cholesterol | 3 | NASH adv (Fib >2) | NASH mild (Fib < 2) | 0.82083 |
| BEL_154a | Taurodeoxycholic acid; 14,15-Dihydroxyeicosatrienoic acid (C20:cis[5,8,11]3); total cholesterol | 3 | NASH adv (Fib >2) | NASH mild (Fib < 2) | 0.82083 |
| BEL_155 | Taurochenodeoxycholic acid; SM_ Sphingomyelin (d18:2,C18:0); 14,15-Dihydroxyeicosatrienoic acid (C20:cis[5,8,11]3) | 3 | NASH adv (Fib >2) | NASH mild (Fib < 2) | 0.81250 |
| BEL_155a | Taurodeoxycholic acid; SM_Sphingomyelin (d18:2,C18:0); 14,15-Dihydroxyeicosatrienoic acid (C20:cis[5,8,11]3) | 3 | NASH adv (Fib >2) | NASH mild (Fib < 2) | 0.81250 |
| BEL_156 | Taurochenodeoxycholic acid; 14,15-Dihydroxyeicosatrienoic acid (C20:cis[5,8,11]3); CE_Cholesterylester C15:0 | 3 | NASH adv (Fib >2) | NASH mild (Fib < 2) | 0.80833 |

(continued)

| Panel Number | Panel Composition long | No of Metabolites | Positive Group | Negative Group | AUC |
|---|---|---|---|---|---|
| BEL_ 156a | Taurodeoxycholic acid; 14,15-Dihydroxyeicosatrienoic acid (C20:cis[5,8,11]3); CE_Cholesterylester C15:0 | 3 | NASH adv (Fib >2) | NASH mild (Fib < 2) | 0.80833 |
| BEL_ 157 | Taurochenodeoxycholic acid; Phosphatidylcholine (C18:0,C20:4); 11,12-Dihydroxyeicosatrienoic acid (C20:cis[5,8,14]3) | 3 | NASH adv (Fib >2) | NASH mild (Fib < 2) | 0.80833 |
| BEL_ 157a | Taurodeoxycholic acid; Phosphatidylcholine (C18:0,C20:4); 11,12-Dihydroxyeicosatrienoic acid (C20:cis[5,8,14]3) | 3 | NASH adv (Fib >2) | NASH mild (Fib < 2) | 0.80833 |
| BEL_ 158 | Taurochenodeoxycholic acid; SM_ Sphingomyelin (d18:1,C18:0); 14,15-Dihydroxyeicosatrienoic acid (C20:cis[5,8,11]3) | 3 | NASH adv (Fib >2) | NASH mild (Fib < 2) | 0.80417 |
| BEL_ 158a | Taurodeoxycholic acid; SM_Sphingomyelin (d18: 1,C18:0); 14,15-Dihydroxyeicosatrienoic acid (C20:cis[5,8,11]3) | 3 | NASH adv (Fib >2) | NASH mild (Fib < 2) | 0.80417 |
| BEA_ 159 | Taurochenodeoxycholic acid; 14,15-Dihydroxyeicosatrienoic acid (C20:cis[5,8,11]3); Cystine | 3 | NASH adv (Fib >2) | NASH mild (Fib < 2) | 0.89167 |
| BEA_ 159a | Taurodeoxycholic acid; 14,15-Dihydroxyeicosatrienoic acid (C20:cis[5,8,11]3); Cystine | 3 | NASH adv (Fib >2) | NASH mild (Fib < 2) | 0.89167 |
| BEA_ 160 | Taurochenodeoxycholic acid; 14,15-Dihydroxyeicosatrienoic acid (C20:cis[5,8,11]3); Urea | 3 | NASH adv (Fib >2) | NASH mild (Fib < 2) | 0.85000 |
| BEA_ 160a | Taurodeoxycholic acid; 14,15-Dihydroxyeicosatrienoic acid (C20:cis[5,8,11]3); Urea | 3 | NASH adv (Fib >2) | NASH mild (Fib < 2) | 0.85000 |
| BEA_ 161 | Taurochenodeoxycholic acid; 14,15-Dihydroxyeicosatrienoic acid (C20:cis[5,8,11]3); Arginine | 3 | NASH adv (Fib >2) | NASH mild (Fib < 2) | 0.84167 |
| BEA_ 161a | Taurodeoxycholic acid; 14,15-Dihydroxyeicosatrienoic acid (C20:cis[5,8,11]3); Arginine | 3 | NASH adv (Fib >2) | NASH mild (Fib < 2) | 0.84167 |
| BEA_ 162 | Taurochenodeoxycholic acid; Cystine; 11,12-Dihydroxyeicosatrienoic acid (C20:cis[5,8,14]3) | 3 | NASH adv (Fib >2) | NASH mild (Fib < 2) | 0.84167 |
| BEA_ 162a | Taurodeoxycholic acid; Cystine; 11,12-Dihydroxyeicosatrienoic acid (C20:cis[5,8,14]3) | 3 | NASH adv (Fib >2) | NASH mild (Fib < 2) | 0.84167 |
| BEA_ 163 | Tyrosine; 14,15-Dihydroxyeicosatrienoic acid (C20:cis[5,8,11]3); Taurochenodeoxycholic acid | 3 | NASH adv (Fib >2) | NASH mild (Fib < 2) | 0.83750 |
| BEA_ 163a | Tyrosine; 14,15-Dihydroxyeicosatrienoic acid (C20:cis[5,8,11]3); Taurodeoxycholic acid | 3 | NASH adv (Fib >2) | NASH mild (Fib < 2) | 0.83750 |

(continued)

| Panel Number | Panel Composition long | No of Metabolites | Positive Group | Negative Group | AUC |
|---|---|---|---|---|---|
| BEA_164 | Taurochenodeoxycholic acid; 14,15-Dihydroxyeicosatrienoic acid (C20:cis[5,8,11]3); Asparagine | 3 | NASH adv (Fib >2) | NASH mild (Fib < 2) | 0.83750 |
| BEA_164a | Taurodeoxycholic acid; 14,15-Dihydroxyeicosatrienoic acid (C20:cis[5,8,11]3); Asparagine | 3 | NASH adv (Fib >2) | NASH mild (Fib < 2) | 0.83750 |
| BEA_165 | Taurochenodeoxycholic acid; 14,15-Dihydroxyeicosatrienoic acid (C20:cis[5,8,11]3); Histidine | 3 | NASH adv (Fib >2) | NASH mild (Fib < 2) | 0.82083 |
| BEA_165a | Taurodeoxycholic acid; 14,15-Dihydroxyeicosatrienoic acid (C20:cis[5,8,11]3); Histidine | 3 | NASH adv (Fib >2) | NASH mild (Fib < 2) | 0.82083 |
| BEA_166 | Taurochenodeoxycholic acid; 14,15-Dihydroxyeicosatrienoic acid (C20:cis[5,8,11]3); Creatine | 3 | NASH adv (Fib >2) | NASH mild (Fib < 2) | 0.81667 |
| BEA_166a | Taurodeoxycholic acid; 14,15-Dihydroxyeicosatrienoic acid (C20:cis[5,8,11]3); Creatine | 3 | NASH adv (Fib >2) | NASH mild (Fib < 2) | 0.81667 |
| BEA_167 | Taurochenodeoxycholic acid; 14,15-Dihydroxyeicosatrienoic acid (C20:cis[5,8,11]3); Glutamine | 3 | NASH adv (Fib >2) | NASH mild (Fib < 2) | 0.80417 |
| BEA_167a | Taurodeoxycholic acid; 14,15-Dihydroxyeicosatrienoic acid (C20:cis[5,8,11]3); Glutamine | 3 | NASH adv (Fib >2) | NASH mild (Fib < 2) | 0.80417 |
| BEA_168 | Taurochenodeoxycholic acid; 14,15-Dihydroxyeicosatrienoic acid (C20:cis[5,8,11]3); Phenylalanine | 3 | NASH adv (Fib >2) | NASH mild (Fib < 2) | 0.80000 |
| BEA_168a | Taurodeoxycholic acid; 14,15-Dihydroxyeicosatrienoic acid (C20:cis[5,8,11]3); Phenylalanine | 3 | NASH adv (Fib >2) | NASH mild (Fib < 2) | 0.80000 |
| BEE_169 | Taurochenodeoxycholic acid; 14,15-Dihydroxyeicosatrienoic acid (C20:cis[5,8,11]3); 8,9-Dihydroxyeicosatrienoic acid (C20:cis [5,11,14]3) | 3 | NASH adv (Fib >2) | NASH mild (Fib < 2) | 0.83750 |
| BEE_169a | Taurodeoxycholic acid; 14,15-Dihydroxyeicosatrienoic acid (C20:cis[5,8,11]3); 8,9-Dihydroxyeicosatrienoic acid (C20:cis [5,11,14]3) | 3 | NASH adv (Fib >2) | NASH mild (Fib < 2) | 0.83750 |
| BEE_170 | Taurochenodeoxycholic acid; 14,15-Dihydroxyeicosatrienoic acid (C20:cis[5,8,11]3); 11,12-Dihydroxyeicosatrienoic acid (C20:cis [5,8,14]3) | 3 | NASH adv (Fib >2) | NASH mild (Fib < 2) | 0.82500 |
| BEE_170a | Taurodeoxycholic acid; 14,15-Dihydroxyeicosatrienoic acid (C20:cis[5,8,11]3); 11,12-Dihydroxyeicosatrienoic acid (C20:cis [5,8,14]3) | 3 | NASH adv (Fib >2) | NASH mild (Fib < 2) | 0.82500 |

(continued)

| Panel Number | Panel Composition long | No of Metabolites | Positive Group | Negative Group | AUC |
|---|---|---|---|---|---|
| BEC_171 | Taurochenodeoxycholic acid; 14,15-Dihydroxyeicosatrienoic acid (C20:cis[5,8,11]3); Glucose | 3 | NASH adv (Fib >2) | NASH mild (Fib < 2) | 0.83333 |
| BEC_171a | Taurodeoxycholic acid; 14,15-Dihydroxyeicosatrienoic acid (C20:cis[5,8,11]3); Glucose | 3 | NASH adv (Fib >2) | NASH mild (Fib < 2) | 0.83333 |
| BEN_172 | Taurochenodeoxycholic acid; 14,15-Dihydroxyeicosatrienoic acid (C20:cis[5,8,11]3); Uridine | 3 | NASH adv (Fib >2) | NASH mild (Fib < 2) | 0.83333 |
| BEN_172a | Taurodeoxycholic acid; 14,15-Dihydroxyeicosatrienoic acid (C20:cis[5,8,11]3); Uridine | 3 | NASH adv (Fib >2) | NASH mild (Fib < 2) | 0.83333 |
| BECP_173 | Taurochenodeoxycholic acid; 14,15-Dihydroxyeicosatrienoic acid (C20:cis[5,8,11]3); C-reractive Proteine (CRP) | 3 | NASH adv (Fib >2) | NASH mild (Fib < 2) | 0.82917 |
| BECP_173a | Taurodeoxycholic acid; 14,15-Dihydroxyeicosatrienoic acid (C20:cis[5,8,11]3); C-reractive Proteine (CRP) | 3 | NASH adv (Fib >2) | NASH mild (Fib < 2) | 0.82917 |
| BE_174 | Taurocholic acid; 14,15-Dihydroxyeicosatrienoic acid (C20:cis[5,8,11]3); | 2 | NASH adv (Fib >2) | NASH mild (Fib < 2) | 0.82500 |
| BE_175 | Taurochenodeoxycholic acid; 14,15-Dihydroxyeicosatrienoic acid (C20:cis[5,8,11]3); | 2 | NASH adv (Fib >2) | NASH mild (Fib < 2) | 0.80833 |
| BE_175a | Taurodeoxycholic acid; 14,15-Dihydroxyeicosatrienoic acid (C20:cis[5,8,11]3); | 2 | NASH adv (Fib >2) | NASH mild (Fib < 2) | 0.80833 |
| BE_176 | Glycocholic acid; 14,15-Dihydroxyeicosatrienoic acid (C20:cis[5,8,11]3); | 2 | NASH adv (Fib >2) | NASH mild (Fib < 2) | 0.80833 |
| BE_177 | SUM.BILE; 14,15-Dihydroxyeicosatrienoic acid (C20:cis[5,8,11]3); | 2 | NASH adv (Fib >2) | NASH mild (Fib < 2) | 0.80833 |
| BE_178 | Taurochenodeoxycholic acid; 14,15-Dihydroxyeicosatrienoic acid (C20:cis[5,8,11]3); | 2 | NASH adv (Fib >2) | NASH mild (Fib < 2) | 0.80833 |
| BE_178a | Taurodeoxycholic acid; 14,15-Dihydroxyeicosatrienoic acid (C20:cis[5,8,11]3); | 2 | NASH adv (Fib >2) | NASH mild (Fib < 2) | 0.80833 |

Table 3 : Scaling factors $m_i$ and $s_i$ and coefficients $w_i$ (input data for the metabolomic features in the form of absolute concentrations based on external calibration standards); NA = Not Applicable; P.A.R. = Peak Area Ratio

| Panel Number | Positive Group | Negative Group | Metabolite / Feature | Bias | Coefficients | Scaling Factors | | Unit |
|---|---|---|---|---|---|---|---|---|
| | | | | $w_0$ | $w_i$ | $m_i$ | $s_i$ | |
| AEB_ 1_2 | Dis_ad v (Fib >2) | Dis_mil d (Fib <2) | NA | -1.84000 | -1.84000 | NA | NA | NA |
| AEB_ 1_2 | Dis_ad v (Fib >2) | Dis_mil d (Fib <2) | Glycocholic acid | | 0.50449 | -1.08153 | 0.5312 5 | μmol /L |
| AEB_ 1_2 | Dis_ad v (Fib >2) | Dis_mil d (Fib <2) | 14,15-Dihydroxyeicosatrienoic acid (C20: cis[5,8,11]3) | | 0.36368 | -0.51864 | 0.1247 4 | μmol / L |
| AEB_ 1_2 | Dis_ad v (Fib >2) | Dis_mil d (Fib <2) | Etiocholanolone sulfate | | -0.64499 | -0.19521 | 0.3424 4 | P.A. R. |
| AEB_ 2_2 | Dis_ad v (Fib >2) | Dis_mil d (Fib <2) | NA | -1.90018 | -1.90018 | NA | NA | NA |
| AEB_ 2_2 | Dis_ad v (Fib >2) | Dis_mil d (Fib <2) | SUM.BILE | | 0.62867 | -0.60521 | 0.4855 8 | μmol /L |
| AEB_ 2_2 | Dis_ad v (Fib >2) | Dis_mil d (Fib <2) | 14,15-Dihydroxyeicosatrienoic acid (C20: cis[5,8,11]3) | | 0.32206 | -0.51864 | 0.1247 4 | μmol / L |
| AEB_ 2_2 | Dis_ad v (Fib >2) | Dis_mil d (Fib <2) | Etiocholanolone sulfate | | -0.68617 | -0.19521 | 0.3424 4 | P.A. R. |
| AEB_ 3_2 | Dis_ad v (Fib >2) | Dis_mil d (Fib <2) | NA | -1.75567 | -1.75567 | NA | NA | NA |
| AEB_ 3_2 | Dis_ad v (Fib >2) | Dis_mil d (Fib <2) | SUM.BILE | | 0.50372 | -0.60521 | 0.4855 8 | μmol /L |
| AEB_ 3_2 | Dis_ad v (Fib >2) | Dis_mil d (Fib <2) | SUM.EICOSATIENOIC | | 0.11928 | -0.25469 | 0.1394 6 | μmol /L |
| AEB_ 3_2 | Dis_ad v (Fib >2) | Dis_mil d (Fib <2) | Etiocholanolone sulfate | | -0.52481 | -0.19521 | 0.3424 4 | P.A. R. |
| AEB_ 4_2 | Dis_ad v (Fib >2) | Dis_mil d (Fib <2) | NA | -1.84038 | -1.84038 | NA | NA | NA |
| AEB_ 4_2 | Dis_ad v (Fib >2) | Dis_mil d (Fib <2) | Taurochenodeoxycholic acid | | 0.68703 | -0.96771 | 0.5138 2 | μmol /L |

(continued)

| Panel Number | Positive Group | Negative Group | Metabolite / Feature | Bias | Coefficients | Scaling Factors | | Unit |
|---|---|---|---|---|---|---|---|---|
| | | | | $w_0$ | $w_i$ | $m_i$ | $s_i$ | |
| AEB_4_2 | Dis_ad v (Fib >2) | Dis_mil d (Fib <2) | 8,9-Dihydroxyeicosatrienoic acid (C20:cis[5,11,14]3) | | 0 | -1.10011 | 0.2496 4 | μmol/L |
| AEB_4_2 | Dis_ad v (Fib >2) | Dis_mil d (Fib <2) | Etiocholanolone sulfate | | -0.68824 | -0.19521 | 0.3424 4 | P.A. R. |
| AEB_5_2 | Dis_ad v (Fib >2) | Dis_mil d (Fib <2) | NA | -1.85030 | -1.85030 | NA | NA | NA |
| AEB_5_2 | Dis_ad v (Fib >2) | Dis_mil d (Fib <2) | SUM.BILE | | 0.62822 | -0.60521 | 0.4855 8 | μmol/L |
| AEB_5_2 | Dis_ad v (Fib >2) | Dis_mil d (Fib <2) | 11,12-Dihydroxyeicosatrienoic acid (C20: cis[5,8,14]3) | | 0.14931 | -0.78593 | 0.1726 0 | μmol/L |
| AEB_5_2 | Dis_ad v (Fib >2) | Dis_mil d (Fib <2) | Etiocholanolone sulfate | | -0.63911 | -0.19521 | 0.3424 4 | P.A. R. |
| AEB_6_2 | Dis_ad v (Fib >2) | Dis_mil d (Fib <2) | NA | -1.77908 | -1.77908 | NA | NA | NA |
| AEB_6_2 | Dis_ad v (Fib >2) | Dis_mil d (Fib <2) | SUM.BILE | | 0.58778 | -0.60521 | 0.4855 8 | μmol/L |
| AEB_6_2 | Dis_ad v (Fib >2) | Dis_mil d (Fib <2) | 8,9-Dihydroxyeicosatrienoic acid (C20:cis[5,11,14]3) | | 0 | -1.10011 | 0.2496 4 | μmol/L |
| AEB_6_2 | Dis_ad v (Fib >2) | Dis_mil d (Fib <2) | Etiocholanolone sulfate | | -0.57529 | -0.19521 | 0.3424 4 | P.A. R. |
| AEB_7_2 | Dis_ad v (Fib >2) | Dis_mil d (Fib <2) | NA | -1.81604 | -1.81604 | NA | NA | NA |
| AEB_7_2 | Dis_ad v (Fib >2) | Dis_mil d (Fib <2) | SUM.EICOSATIENOIC | | 0.12392 | -0.25469 | 0.1394 6 | μmol/L |
| AEB_7_2 | Dis_ad v (Fib >2) | Dis_mil d (Fib <2) | Taurochenodeoxycholic acid | | 0.59268 | -0.96771 | 0.5138 2 | μmol/L |
| AEB_7_2 | Dis_ad v (Fib >2) | Dis_mil d (Fib <2) | Etiocholanolone sulfate | | -0.63340 | -0.19521 | 0.3424 4 | P.A. R. |

| Panel Number | Positive Group | Negative Group | Metabolite / Feature | Bias | Coefficients | Scaling Factors | | Unit |
|---|---|---|---|---|---|---|---|---|
| | | | | $w_0$ | $w_i$ | $m_i$ | $s_i$ | |
| AEB_ 8_2 | Dis_ad v (Fib >2) | Dis_mil d (Fib <2) | NA | -1.93549 | -1.93549 | NA | NA | NA |
| AEB_ 8_2 | Dis_ad v (Fib >2) | Dis_mil d (Fib <2) | Taurochenodeoxycholic acid | | 0.67259 | -0.96771 | 0.5138 2 | μmol /L |
| AEB_ 8_2 | Dis_ad v (Fib >2) | Dis_mil d (Fib <2) | 14,15-Dihydroxyeicosatrienoic acid (C20: cis[5,8,11]3) | | 0.29760 | -0.51864 | 0.1247 4 | μmol /L |
| AEB_ 8_2 | Dis_ad v (Fib >2) | Dis_mil d (Fib <2) | Etiocholanolone sulfate | | -0.76540 | -0.19521 | 0.3424 4 | P.A. R. |
| AEB_ 9_2 | Dis_ad v (Fib >2) | Dis_mil d (Fib <2) | NA | -1.91082 | -1.91082 | NA | NA | NA |
| AEB_ 9_2 | Dis_ad v (Fib >2) | Dis_mil d (Fib <2) | Taurocholic acid | | 0.61111 | -1.57569 | 0.5807 0 | μmol /L |
| AEB_ 9_2 | Dis_ad v (Fib >2) | Dis_mil d (Fib >2) | 14,15-Dihydroxyeicosatri-enoic acid (C20: cis[5,8,11]3) | | 0.32550 | -0.51864 | 0.1247 4 | μmol / L |
| AEB_ 9_2 | Dis_ad v (Fib >2) | Dis_mil d (Fib <2) | Etiocholanolone sulfate | | -0.65547 | -0.19521 | 0.3424 4 | P.A. R. |
| AEB_ 10_2 | Dis_ad v (Fib >2) | Dis_mil d (Fib <2) | NA | -1.88230 | -1.88230 | NA | NA | NA |
| AEB_ 10_2 | Dis_ad v (Fib >2) | Dis_mil d (Fib <2) | Taurochenodeoxycholic acid | | 0.68197 | -0.96771 | 0.5138 2 | μmol /L |
| AEB_ 10_2 | Dis_ad v (Fib >2) | Dis_mil d (Fib <2) | 11,12-Dihydroxyeicosatrienoic acid (C20: cis[5,8,14]3) | | 0.13884 | -0.78593 | 0.1726 0 | μmol /L |
| AEB_ 10_2 | Dis_ad v (Fib >2) | Dis_mil d (Fib <2) | Etiocholanolone sulfate | | -0.71695 | -0.19521 | 0.3424 4 | P.A. R. |
| AEB_ 11_2 | Dis_ad v (Fib >2) | Dis_mil d (Fib <2) | NA | -1.76155 | -1.76155 | NA | NA | NA |
| AEB_ 11_2 | Dis_ad v (Fib >2) | Dis_mil d (Fib <2) | SUM.EICOSATIENOIC | | 0.12819 | -0.25469 | 0.1394 6 | μmol /L |

| Panel Number | Positive Group | Negative Group | Metabolite / Feature | Bias | Coefficients | Scaling Factors | | Unit |
|---|---|---|---|---|---|---|---|---|
| | | | | $w_0$ | $w_i$ | $m_i$ | $s_i$ | |
| AEB_ 11_2 | Dis_ad v (Fib >2) | Dis_mil d (Fib <2) | Taurocholic acid | | 0.49472 | -1.57569 | 0.5807 0 | μmol /L |
| AEB_ 11_2 | Dis_ad v (Fib >2) | Dis_mil d (Fib <2) | Etiocholanolone sulfate | | -0.49906 | -0.19521 | 0.3424 4 | P.A. R. |
| AEB_ 12_2 | Dis_ad v (Fib >2) | Dis_mil d (Fib <2) | NA | -1.76507 | -1.76507 | NA | NA | NA |
| AEB_ 12_2 | Dis_ad v (Fib >2) | Dis_mil d (Fib <2) | Glycocholic acid | | 0.44971 | -1.08153 | 0.5312 5 | μmol /L |
| AEB_ 12_2 | Dis_ad v (Fib >2) | Dis_mil d (Fib <2) | 11,12-Dihydroxyeicosatrienoic acid (C20: cis[5,8,14]3) | | 0.17697 | -0.78593 | 0.1726 0 | μmol /L |
| AEB_ 12_2 | Dis_ad v (Fib >2) | Dis_mil d (Fib <2) | Etiocholanolone sulfate | | -0.56362 | -0.19521 | 0.3424 4 | P.A. R. |
| AEB_ 13_2 | Dis_ad v (Fib >2) | Dis_mil d (Fib <2) | NA | -1.75352 | -1.75352 | NA | NA | NA |
| AEB_ 13_2 | Dis_ad v (Fib >2) | Dis_mil d (Fib <2) | Taurocholic acid | | 0.53317 | -1.57569 | 0.5807 0 | μmol /L |
| AEB_13_2 | Dis_ad v (Fib >2) | Dis_mil d (Fib <2) | 8,9-Dihydroxyeicosatrienoic acid (C20:cis [5,11,14]3) | | 0 | -1.10011 | 0.2496 4 | μmol /L |
| AEB_ 13_2 | Dis_ad v (Fib >2) | Dis_mil d (Fib <2) | Etiocholanolone sulfate | | -0.50996 | -0.19521 | 0.3424 4 | P.A. R. |
| AEB_ 14_2 | Dis_ad v (Fib >2) | Dis_mil d (Fib <2) | NA | -1.82249 | -1.82249 | NA | NA | NA |
| AEB_ 14_2 | Dis_ad v (Fib >2) | Dis_mil d (Fib <2) | Taurocholic acid | | 0.57416 | -1.57569 | 0.5807 0 | μmol /L |
| AEB_ 14_2 | Dis_ad v (Fib >2) | Dis_mil d (Fib <2) | 11,12-Dihydroxyeicosatrienoic acid (C20: cis[5,8,14]3) | | 0.13817 | -0.78593 | 0.1726 0 | μmol /L |
| AEB_ 14_2 | Dis_ad v (Fib >2) | Dis_mil d (Fib <2) | Etiocholanolone sulfate | | -0.57161 | -0.19521 | 0.3424 4 | P.A. R. |

EP 3 502 703 A1

| Panel Number | Positive Group | Negative Group | Metabolite / Feature | Bias | Coefficients | Scaling Factors | | Unit |
|---|---|---|---|---|---|---|---|---|
| | | | | $w_0$ | $w_i$ | $m_i$ | $s_i$ | |
| AEA_ 17_2 | Dis_ad v (Fib >2) | Dis_mil d (Fib <2) | NA | -2.11532 | -2.11532 | NA | NA | NA |
| AEA_ 17_2 | Dis_ad v (Fib >2) | Dis_mil d (Fib <2) | Creatine | | -0.67984 | 0.51567 | 0.1765 8 | μmol /L |
| AEA_ 17_2 | Dis_ad v (Fib >2) | Dis_mil d (Fib <2) | 14,15-Dihydroxyeicosatrienoic acid (C20: cis[5,8,11]3) | | 0.70666 | -0.51864 | 0.1247 4 | μmol /L |
| AEA_ 17_2 | Dis_ad v (Fib >2) | Dis_mil d (Fib <2) | Etiocholanolone sulfate | | -1.07756 | -0.19521 | 0.3424 4 | P.A. R. |
| AEA_ 18_2 | Dis_ad v (Fib >2) | Dis_mil d (Fib <2) | NA | -1.97905 | -1.97905 | NA | NA | NA |
| AEA_ 18_2 | Dis_ad v (Fib >2) | Dis_mil d (Fib <2) | 14,15-Dihydroxyeicosatrienoic acid (C20: cis[5,8,11]3) | | 0.56123 | -0.51864 | 0.1247 4 | μmol /L |
| AEA_ 18_2 | Dis_ad v (Fib >2) | Dis_mil d (Fib <2) | Cystine | | 0.45623 | 0.95105 | 0.1551 3 | μmol /L |
| AEA_ 18_2 | Dis_ad v (Fib >2) | Dis_mil d (Fib <2) | Etiocholanolone sulfate | | -0.90654 | -0.19521 | 0.3424 4 | P.A. R. |
| AEA_ 19_2 | Dis_ad v (Fib >2) | Dis_mil d (Fib <2) | NA | -2.00175 | -2.00175 | NA | NA | NA |
| AEA_ 19_2 | Dis_ad v (Fib >2) | Dis_mil d (Fib >2) | 14,15-Dihydroxyeicosatri-enoic acid (C20: cis[5,8,11]3) | | 0.64434 | -0.51864 | 0.1247 4 | μmol / L |
| AEA_ 19_2 | Dis_ad v (Fib >2) | Dis_mil d (Fib <2) | Tyrosine | | 0.53317 | 1.10156 | 0.1097 7 | μmol /L |
| AEA_ 19_2 | Dis_ad v (Fib >2) | Dis_mil d (Fib <2) | Etiocholanolone sulfate | | -0.86906 | -0.19521 | 0.3424 4 | P.A. R. |
| AEA_ 20_2 | Dis_ad v (Fib >2) | Dis_mil d (Fib <2) | NA | -1.78451 | -1.78451 | NA | NA | NA |
| AEA_ 20_2 | Dis_ad v (Fib >2) | Dis_mil d (Fib <2) | 14,15-Dihydroxyeicosatrienoic acid (C20: cis[5,8,11]3) | | 0.34315 | -0.51864 | 0.1247 4 | μmol /L |

EP 3 502 703 A1

(continued)

| Panel Number | Positive Group | Negative Group | Metabolite / Feature | Bias w0 | Coefficients wi | Scaling Factors mi | Scaling Factors si | Unit |
|---|---|---|---|---|---|---|---|---|
| AEA_ 20_2 | Dis_ad v (Fib >2) | Dis_mil d (Fib <2) | Lysine | | -0.30475 | 1.42355 | 0.0822 1 | µmol /L |
| AEA_ 20_2 | Dis_ad v (Fib >2) | Dis_mil d (Fib <2) | Etiocholanolone sulfate | | -0.64948 | -0.19521 | 0.3424 4 | P.A. R. |
| AEL_ 40_2 | Dis_ad v (Fib >2) | Dis_mil d (Fib <2) | NA | -2.07062 | -2.07062 | NA | NA | NA |
| AEL_ 40_2 | Dis_ad v (Fib >2) | Dis_mil d (Fib <2) | CE_Cholesterylester C20:4 | | -0.62699 | -0.01985 | 0.1279 8 | µmol /L |
| AEL_ 40_2 | Dis_ad v (Fib >2) | Dis_mil d (Fib <2) | 14,15-Dihydroxyeicosatrienoic acid (C20: cis[5,8,11]3) | | 0.68349 | -0.51864 | 0.1247 4 | µmol /L |
| AEL_ 40_2 | Dis_ad v (Fib >2) | Dis_mil d (Fib <2) | Etiocholanolone sulfate | | -0.86761 | -0.19521 | 0.3424 4 | P.A. R. |
| AEL_ 41_2 | Dis_ad v (Fib >2) | Dis_mil d (Fib <2) | NA | -1.99095 | -1.99095 | NA | NA | NA |
| AEL_ 41_2 | Dis_ad v (Fib >2) | Dis_mil d (Fib <2) | SM_Sphingomyelin (d18:2,C20:0) | | -0.46703 | -0.02006 | 0.1228 8 | µmol /L |
| AEL_ 41_2 | Dis_ad v (Fib >2) | Dis_mil d (Fib <2) | 14,15-Dihydroxyeicosatrienoic acid (C20: cis[5,8,11]3) | | 0.53788 | -0.51864 | 0.1247 4 | µmol /L |
| AEL_ 41_2 | Dis_ad v (Fib >2) | Dis_mil d (Fib <2) | Etiocholanolone sulfate | | -0.98525 | -0.19521 | 0.3424 4 | P.A. R. |
| AEL_ 42_2 | Dis_ad v (Fib >2) | Dis_mil d (Fib <2) | NA | -1.92411 | -1.92411 | NA | NA | NA |
| AEL_ 42_2 | Dis_ad v (Fib >2) | Dis_mil d (Fib <2) | SM_Sphingomyelin (d18:2,C18:0) | | -0.43137 | 0.03698 | 0.1170 9 | µmol /L |
| AEL_ 42_2 | Dis_ad v (Fib >2) | Dis_mil d (Fib <2) | 14,15-Dihydroxyeicosatrienoic acid (C20: cis[5,8,11]3) | | 0.48267 | -0.51864 | 0.1247 4 | µmol /L |
| AEL_ 42_2 | Dis_ad v (Fib >2) | Dis_mil d (Fib <2) | Etiocholanolone sulfate | | -0.85334 | -0.19521 | 0.3424 4 | P.A. R. |

(continued)

| Panel Number | Positive Group | Negative Group | Metabolite / Feature | Bias | Coefficients | Scaling Factors | | Unit |
|---|---|---|---|---|---|---|---|---|
| | | | | $w_0$ | $w_i$ | $m_i$ | $s_i$ | |
| AEL_ 44_2 | Dis_ad v (Fib >2) | Dis_mil d (Fib <2) | NA | -1.76829 | -1.76829 | NA | NA | NA |
| AEL_ 44_2 | Dis_ad v (Fib >2) | Dis_mil d (Fib <2) | 14,15-Dihydroxyeicosatrienoic acid (C20: cis[5,8,11]3) | | 0.40606 | -0.51864 | 0.1247 4 | μmol /L |
| AEL_ 44_2 | Dis_ad v (Fib >2) | Dis_mil d (Fib <2) | Etiocholanolone sulfate | | -0.64196 | -0.19521 | 0.3424 4 | P.A. R. |
| AEL_ 44_2 | Dis_ad v (Fib >2) | Dis_mil d (Fib <2) | Ethanolamine plasmalogen (C39:5) | | -0.20356 | 0.01107 | 0.0799 1 | μmol /L |
| AEC_ 59_2 | Dis_ad v (Fib >2) | Dis_mil d (Fib <2) | NA | -1.84047 | -1.84047 | NA | NA | NA |
| AEC_ 59_2 | Dis_ad v (Fib >2) | Dis_mil d (Fib <2) | 14,15-Dihydroxyeicosatrienoic acid (C20: cis[5,8,11]3) | | 0.45795 | -0.51864 | 0.1247 4 | μmol /L |
| AEC_ 59_2 | Dis_ad v (Fib >2) | Dis_mil d (Fib <2) | Glucose | | 0.29102 | 3.02372 | 0.0896 0 | μmol /L |
| AEC_ 59_2 | Dis_ad v (Fib >2) | Dis_mil d (Fib <2) | Etiocholanolone sulfate | | -0.77038 | -0.19521 | 0.3424 4 | P.A. R. |
| AECL 62_2 | Dis_ad v (Fib >2) | Dis_mil d (Fib <2) | NA | -1.82762 | -1.82762 | NA | NA | NA |
| AECL 62_2 | Dis_ad v (Fib >2) | Dis_mil d (Fib <2) | C-reactive Protein (CRP) | | 0.29012 | 0.51234 | 0.2658 7 | mg/ L |
| AECL 62_2 | Dis_ad v (Fib >2) | Dis_mil d (Fib <2) | 14,15-Dihydroxyeicosatrienoic acid (C20: cis[5,8,11]3) | | 0.47187 | -0.51864 | 0.1247 4 | μmol /L |
| AECL 62_2 | Dis_ad v (Fib >2) | Dis_mil d (Fib <2) | Etiocholanolone sulfate | | -0.76856 | -0.19521 | 0.3424 4 | P.A. R. |
| AB_84_2 | Dis_ad v (Fib >2) | Dis_mil d (Fib <2) | NA | -1.93972 | -1.93972 | NA | NA | NA |
| AB_ 84_2 | Dis_ad v (Fib >2) | Dis_mil d (Fib <2) | SUM.BILE | | 0.81661 | -0.60521 | 0.4855 8 | μmol / L |

| Panel Number | Positive Group | Negative Group | Metabolite / Feature | Bias | Coefficients | Scaling Factors | | Unit |
|---|---|---|---|---|---|---|---|---|
| | | | | $w_0$ | $w_i$ | $m_i$ | $s_i$ | |
| AB_ 84_2 | Dis_ad v (Fib >2) | Dis_mil d (Fib <2) | Etiocholanolone sulfate | | -0.76157 | -0.19521 | 0.3424 4 | P.A. R. |

**Claims**

1. A method for assessing non-alcoholic fatty liver disease (NAFLD) comprising

   (a) determining in a sample from a subject suffering from NAFLD at least the amounts of two metabolite biomarkers, wherein said two metabolite biomarkers are:

   • at least one eicosatrienoic acid metabolite biomarker selected from the group consisting of 14,15-Dihydroxyeicosatrienoic acid (C20:cis[5,8,11]3), 11,12-Dihydroxyeicosatrienoic acid (C20:cis[5,8,14]3), and 8,9-Dihydroxyeicosatrienoic acid (C20:cis[5,11,14]3), or the sum of the amounts of 14,15-Dihydroxyeicosatrienoic acid (C20:cis[5,8,11]3), 11,12-Dihydroxyeicosatrienoic acid (C20:cis[5,8,14]3), and 8,9-Dihydroxyeicosatrienoic acid (C20:cis[5,11,14]3), and
   • at least one androgen metabolite biomarker and/or at least one bile acid metabolite biomarker:

   wherein the at least one androgen metabolite biomarker is Etiocholanolone sulfate, Androsterone sulfate or Dihydrotestosterone sulfate,
   and/or
   wherein the bile acid metabolite biomarker is selected from Glycocholic acid, Taurochenodeoxycholic acid, Taurodeoxycholic acid, and Taurocholic acid, or wherein the bile acid metabolite biomarker is the sum of the amounts of Taurochenodeoxycholic acid, Taurodeoxycholic acid, Taurocholic acid, and Glycocholic acid,

   and

   (b) comparing

   (i) the amounts as determined in step (a) to reference amounts for the metabolite biomarkers, or
   (ii) a score calculated based on the amounts as determined in step (a) to a reference score,

   and
   (c) assessing NAFLD based on the results of step (b).

2. The method of claim 1, wherein the assessment of NAFLD is the differentiation between a mild form and severe form of NAFLD.

3. The method of claims 1 and 2, wherein a mild form of NAFLD is NAFLD with a fibrosis score of lower than 2, and/or wherein a severe form of NAFLD is NAFLD with a fibrosis score of equal to or larger than 2.

4. The method of any one of claims 1 to 3, wherein NAFLD is NASH (non-alcoholic steato-hepatitis), or NAFL (non-alcoholic fatty liver).

5. The method of any one of claims 1 to 4, wherein the sample is blood, serum or plasma sample.

6. The method of any one of claims 1 to 5, wherein the subject is a human subject.

7. The method of any one of claims 1 to 6, wherein at least the amounts of Etiocholanolone sulfate, Glycocholic acid, and 14,15-Dihydroxyeicosatrienoic acid (C20:cis[5,8,11]3) are determined in step (a) of claim 1.

8. The method of any one of claims 1 to 7, wherein the amounts of the metabolite biomarkers are determined by mass spectrometry.

9. The method of any one of claims 1 to 8, wherein in step (a) of claim 1 the amount of at least one further biomarker selected from the group consisting of Tyrosine, Sphingomyelin (d18:2,C18:0), Lysine, Cystine, Creatinine, Cholesterylester C20:4, Glucose, Sphingomyelin (d18:2,C20:0), Creatine, Phenylalanine, Asparagine, Arginine, Glutamine, Tryptophan, Urea, Histidine, Ethanolamine plasmalogen (C39:5), Choline plasmalogen (C36:5), Phosphatidylcholine (C18:0,C20:4), total cholesterol, Ethanolamine plasmalogen (C39:4), Choline plasmalogen (C36:4), TOTAL CHOLESTEROL Sphingomyelin (d18:1,C18:0), Cholesterylester C15:0, Choline plasmalogen (C18-vinyl,C20:4), Testosterone, Androstenedione, Uridine, C-reactive protein, alkaline phosphatase, leukocytes, Dehydroepiandrosterone sulfate, and Testosterone-17-sulfate is determined.

10. The method of any one of claims 1 to 9, wherein the amounts of the biomarkers of any one of the panels shown in Table 2a_1, Table 2a_2), Table 2a_3), Table 2b_1), Table 2b_2), Table 2b_3), Table 2c_1), Table 2c_2), Table 2c_3), Table 2d), Table 2e), or Table 2f), or in particular of in Table 3 are determined in step (a) of the method of claim 1.

11. In vitro use of the at least two metabolite biomarkers as set forth in any of the preceding claims for assessing non-alcoholic fatty liver disease (NAFLD).

Europäisches
Patentamt

European
Patent Office

Office européen
des brevets

# EUROPEAN SEARCH REPORT

Application Number

EP 17 20 9996

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/021192 A2 (LIPOMICS TECHNOLOGIES INC [US]; WATKINS STEVEN M [US]; WIEST MICHELLE) 21 February 2008 (2008-02-21) | 1,2,4-6, 8,11 | INV. G01N33/68 |
| Y | * tables 2,9 * <br> * paragraphs [0038], [0070], [0073] - [0076], [0083], [0091] * <br> * claims 16,18 * | 3,7,9,10 | |
| Y | WO 2017/210147 A1 (JIA WEI [US]) 7 December 2017 (2017-12-07) <br> * tables 1,3A * | 7,9,10 | |
| Y | US 2017/138967 A1 (ABDELMALEK MANAL F [US] ET AL) 18 May 2017 (2017-05-18) <br> * tables 2,7-9 * | 7,9,10 | |
| Y | WO 2015/089102 A1 (UNIV CALIFORNIA [US]) 18 June 2015 (2015-06-18) <br> * paragraphs [0066], [0069] * | 3,7,9,10 | |
| Y | ROHIT LOOMBA ET AL: "Polyunsaturated fatty acid metabolites as novel lipidomic biomarkers for noninvasive diagnosis of nonalcoholic steatohepatitis", JOURNAL OF LIPID RESEARCH, vol. 56, no. 1, 1 January 2015 (2015-01-01), pages 185-192, XP055467622, US ISSN: 0022-2275, DOI: 10.1194/jlr.P055640 <br> * abstract * | 7,9,10 | **TECHNICAL FIELDS SEARCHED (IPC)** <br><br> G01N |
| Y | WO 2016/081534 A1 (METABOLON INC [US]) 26 May 2016 (2016-05-26) <br> * tables 7,8 * | 7,9,10 | |

-/--

The present search report has been drawn up for all claims

| Place of search | Date of completion of the search | Examiner |
|---|---|---|
| Munich | 17 April 2018 | Jacques, Patrice |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 1 of 2

Europäisches
Patentamt

European
Patent Office

Office européen
des brevets

# EUROPEAN SEARCH REPORT

Application Number

EP 17 20 9996

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2012/049874 A1 (HUMAN METABOLOME TECHNOLOGIES INC [JP]; UNIV TOKYO WOMENS MEDICAL [JP]) 19 April 2012 (2012-04-19) * abstract * ----- | 7,9,10 | |

TECHNICAL FIELDS
SEARCHED      (IPC)

The present search report has been drawn up for all claims

| Place of search | Date of completion of the search | Examiner |
|---|---|---|
| Munich | 17 April 2018 | Jacques, Patrice |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

# EP 3 502 703 A1

## ANNEX TO THE EUROPEAN SEARCH REPORT
## ON EUROPEAN PATENT APPLICATION NO.

EP 17 20 9996

This annex lists the patent family members relating to the patent documents cited in the above-mentioned European search report.
The members are as contained in the European Patent Office EDP file on
The European Patent Office is in no way liable for these particulars which are merely given for the purpose of information.

17-04-2018

| Patent document cited in search report | | | Publication date | Patent family member(s) | | | | Publication date |
|---|---|---|---|---|---|---|---|---|
| WO 2008021192 | A2 | | 21-02-2008 | AU | 2007284733 | A1 | | 21-02-2008 |
| | | | | BR | PI0715402 | A2 | | 26-11-2013 |
| | | | | CA | 2662987 | A1 | | 21-02-2008 |
| | | | | CN | 101523221 | A | | 02-09-2009 |
| | | | | EP | 2057473 | A2 | | 13-05-2009 |
| | | | | EP | 2607902 | A1 | | 26-06-2013 |
| | | | | EP | 3285072 | A1 | | 21-02-2018 |
| | | | | ES | 2527438 | T3 | | 26-01-2015 |
| | | | | HK | 1131209 | A1 | | 27-11-2015 |
| | | | | JP | 2010500566 | A | | 07-01-2010 |
| | | | | US | 2010233724 | A1 | | 16-09-2010 |
| | | | | WO | 2008021192 | A2 | | 21-02-2008 |
| WO 2017210147 | A1 | | 07-12-2017 | NONE | | | | |
| US 2017138967 | A1 | | 18-05-2017 | NONE | | | | |
| WO 2015089102 | A1 | | 18-06-2015 | AU | 2014364327 | A1 | | 09-06-2016 |
| | | | | CA | 2933034 | A1 | | 18-06-2015 |
| | | | | CN | 105980861 | A | | 28-09-2016 |
| | | | | EP | 3080613 | A1 | | 19-10-2016 |
| | | | | JP | 2016540218 | A | | 22-12-2016 |
| | | | | KR | 20160096618 | A | | 16-08-2016 |
| | | | | US | 2016305925 | A1 | | 20-10-2016 |
| | | | | WO | 2015089102 | A1 | | 18-06-2015 |
| WO 2016081534 | A1 | | 26-05-2016 | CN | 107002113 | A | | 01-08-2017 |
| | | | | EP | 3221463 | A1 | | 27-09-2017 |
| | | | | JP | 2018502286 | A | | 25-01-2018 |
| | | | | US | 2017370954 | A1 | | 28-12-2017 |
| | | | | WO | 2016081534 | A1 | | 26-05-2016 |
| WO 2012049874 | A1 | | 19-04-2012 | JP | 5636567 | B2 | | 10-12-2014 |
| | | | | JP | WO2012049874 | A1 | | 24-02-2014 |
| | | | | WO | 2012049874 | A1 | | 19-04-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008021192 A
- WO 2009059150 A
- WO 2010018165 A
- WO 2012000770 A
- WO 2016081534 A
- US 4540884 A
- US 5397894 A
- US 7196323 B

### Non-patent literature cited in the description

- American Gastroenterological Association 2002. *Gastroenterology,* vol. 123, 1705-25
- **BRUNT.** *Am J Gastroenterol,* 1999, vol. 94, 2467-74
- **BRUNT.** *Nat Rev Gastroenterol Hepatol,* 2010, vol. 7, 195-203
- **S.C. KALHAN et al.** *Metabolism Clinical and Experimental,* 2011, vol. 60, 404-413
- **KOGA et al.** *Intern Med,* 2011, vol. 50, 1657-1661
- **TOKUSHIGE et al.** *J Gastroenterol,* 2013, vol. 48, 1392-1400
- **DOWDY ; WEARDEN.** Statistics for Research. John Wiley & Sons, 1983
- **TREEPRASERTSUK S et al.** NAFLD fibrosis score: a prognostic predictor for mortality and liver complications among NAFLD patients. *World J Gastroenterol,* 28 February 2013, vol. 19 (8), 1219-29
- **KLEINER et al.** *HEPATOLOGY,* 2005, vol. 41 (6), 2005
- **MARYAM R. KASHI ; DAWN M. TORRES ; STEPHEN A. HARRISON.** Current and Emerging Therapies in Nonalcoholic Fatty Liver Disease: Therapeutic Modalities. *Semin Liver Dis.,* 2008, vol. 28 (4), 396-406
- **NISSEN.** *Journal of Chromatography A,* 1995, vol. 703, 37-57
- **KHAN et al.** *Postgrad Med J.,* May 2006, vol. 82 (967), 353-354
- **COHORT BOYLE ; MARIE P. ; TINIAKOS, DINA G. ; MCPHERSON, STUART et al.** *HEPATOLOGY,* October 2017, vol. 66 (1), 54A-55A
- **HASEGAWA et al.** Usefulness of Cytokeratin-18M65 in Diagnosing Non-Alcoholic Steatohepatitis in Japanese Population. *Dig Dis. 2015,* 2015, vol. 33
- **MUSSO et al.** Meta-analysis: natural history of non-alcoholic fatty liver disease (NAFLD) and diagnostic accuracy of non-invasive tests for liver disease severity. *Ann Med,* 2011
- **FELDSTEIN et al.** Cytokeratin-18 fragment levels as noninvasive biomarkers for nonalcoholic steatohepatitis: a multicenter validation study. *Hepatology,* 2009
- **LEMOINE M et al.** The gamma-glutamyl transpeptidase to platelet ratio (GPR) predicts significant liver fibrosis and cirrhosis in patients with chronic HBV infection. *West Africa Gut.,* August 2016, vol. 65 (8), 1369-76
- **CHIN.** Non-invasive Markers of Liver Fibrosis: Adjuncts or Alternatives to Liver Biopsy?. *Front. Pharmacol.,* 2016, vol. 7, 159
- **RAVENZWAAY, B. et al.** The use of metabolomics for the discovery of new biomarkers of effect. *Toxicol Lett,* 2007, vol. 172, 21-8
- **VAN RAVENZWAAY, B. et al.** The use of metabolomics for the discovery of new biomarkers of effect. *Toxicol Lett,* 2007, vol. 172, 21-8
- **ROESSNER, U. ; WAGNER, C. ; KOPKA, J. ; TRETHEWEY, R.N. ; WILLMITZER, L.** Technical advance: simultaneous analysis of metabolites in potato tuber by gas chromatography-mass spectrometry. *Plant J,* 2000, vol. 23, 131-42
- **MUTCH, D.M. et al.** Metabolite profiling identifies candidate markers reflecting the clinical adaptations associated with Roux-en-Y gastric bypass surgery. *PLoS One,* 2009, vol. 4, e7905
- **CHRISTIE, W.W.** Rapid separation and quantification of lipid classes by high performance liquid chromatography and mass (light-scattering) detection. *J Lipid Res,* 1985, vol. 26, 507-12
- **SCHMIDT, H. ; SCHMIDT, R. ; GEISSLINGER, G.** LC-MS/MS-analysis of sphingosine-1-phosphate and related compounds in plasma samples. *Prostaglandins Other Lipid Mediat,* 2006, vol. 81, 162-70
- **LIAW ; WIENER.** Classification and Regression by random Forest. *R News,* 2002, vol. 2 (3), 18-22
- **ZOU ; HASTIE.** Regularization and variable selection via the elastic net. *Journal of the Royal Statistical Society, Series B,* 2005

- **TIBSHIRANI ; HASTI ; FRIEDMAN.** Elements of Statistical Learning. 2008